(19) Europäisches Patentamt / European Patent Office / Office européen des brevets

(11) **EP 4 328 300 A1**

(12) **EUROPEAN PATENT APPLICATION**
published in accordance with Art. 153(4) EPC

(43) Date of publication:
**28.02.2024 Bulletin 2024/09**

(21) Application number: **22791119.5**

(22) Date of filing: **22.04.2022**

(51) International Patent Classification (IPC):
**C12N 5/0783** (2010.01)     **A61K 35/17** (2015.01)
**A61K 38/20** (2006.01)

(52) Cooperative Patent Classification (CPC):
**A61K 35/17; A61K 38/20; C12N 5/06**

(86) International application number:
**PCT/CN2022/088338**

(87) International publication number:
**WO 2022/223013 (27.10.2022 Gazette 2022/43)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **23.04.2021 PCT/CN2021/089336**

(71) Applicants:
• **Suzhou Grit Biotechnology Co., Ltd.**
  **Shanghai 201315 (CN)**
• **Zhuhai Tuoyu Biotechnology Co., Ltd.**
  **Shanghai 201315 (CN)**
• **Shanghai Grit Biotechnology Co., Ltd.**
  **Shanghai 201315 (CN)**

• **Zhuhai Grit Biotechnology Co., Ltd.**
  **Shanghai 201315 (CN)**

(72) Inventors:
• **LIU, Yarong**
  **Shanghai 201315 (CN)**
• **SUN, Jingwei**
  **Shanghai 201315 (CN)**
• **JIN, Jiahui**
  **Shanghai 201315 (CN)**

(74) Representative: **Jones Day**
**Rechtsanwälte, Attorneys-at-Law, Patentanwälte**
**Prinzregentenstrasse 11**
**80538 München (DE)**

Remarks:
The complete document including Reference Table(s) and the Sequence Listing(s) can be downloaded from the EPO website

(54) **MODIFIED TUMOR-INFILTRATING LYMPHOCYTE AND USE THEREOF**

(57)     Provided are modified tumor-infiltrating lymphocyte and the use thereof, in particular provided is a method for culturing the tumor-infiltrating lymphocyte (TIL), which comprises increasing the expression and/or enhancing the activity of at least one cytokine of the TIL. Also provided is a method for preventing and/or treating tumors by using the tumor-infiltrating lymphocyte.

EP 4 328 300 A1

**Description**

**TECHNICAL FIELD**

The present application relates to the field of biomedicine, and specifically to a modified tumor-infiltrating lymphocyte and a use thereof.

**BACKGROUND OF THE INVENTION**

Treating tumors by using adoptive autologous transferred tumor-infiltrating lymphocytes is an effective approach to treat patients with poor prognosis. However, treating tumors by adoptive autologous transferred tumor-infiltrating lymphocytes requires a large number of tumor-infiltrating lymphocytes.

Therefore, how to provide a robust and reliable method for culturing tumor-infiltrating lymphocytes is an urgent issue to be solved.

**SUMMARY OF THE INVENTION**

The present application provides a modified tumor-infiltrating lymphocyte and a use thereof, and specifically provides a robust and reliable method for culturing tumor-infiltrating lymphocytes, which may have one or more effects selected from the group consisting of: improving the number of TIL cells, enhancing the secretion capacity of TIL cells, enhancing the killing ability of TIL cells, increasing the proportion of NK cells, changing the proportion of TIL cells, increasing the proportion of CD4$^+$ cells, decreasing the proportion of CD8$^+$ cells, increasing the proportion of central memory T cells, decreasing the proportion of regulatory T cells, increasing the proportion of activated T cells, increasing the proportion of tumor-specific T cells, and increasing the proportion of stem cell-like T cells.

In one aspect, the present application provides a method for culturing tumor-infiltrating lymphocytes (TILs), the method comprises improving the expression and/or enhancing the activity of at least one cytokine of the TILs, and co-culturing the TILs with feeder cells after contacting the TILs with T cell activators and/or T cell growth factors for a period of time.

In one embodiment, the method comprises improving the expression and/or enhancing the activity of at least one cytokine of the TILs after co-culturing the TILs with the feeder cells.

In one embodiment, the method comprises improving the expression and/or enhancing the activity of at least one cytokine of the TILs before co-culturing the TILs with the feeder cells.

In one embodiment, the method comprises improving the expression and/or enhancing the activity of at least one cytokine of the TILs after contacting the TILs with the T cell activators and/or the T cell growth factors and before co-culturing the TILs with the feeder cells.

In one embodiment, the method comprises improving the expression and/or enhancing the activity of at least one cytokine of the TILs substantially simultaneously with contacting the TILs with the T cell activators and/or the T cell growth factors.

In one embodiment, the method comprises improving the expression and/or enhancing the activity of at least one cytokine of the TILs substantially simultaneously with co-culturing the TILs with the feeder cells.

A method for culturing tumor-infiltrating lymphocytes (TILs), the method comprises improving the expression and/or enhancing the activity of at least one cytokine of the TILs, where the TILs comprise TILs obtained by co-culturing with feeder cells after contacting the TILs with T cell activators and/or T cell growth factors for a period of time.

A method for culturing tumor-infiltrating lymphocytes (TILs), the method comprises co-culturing the TILs with feeder cells after contacting the TILs with T cell activators and/or T cell growth factors for a period of time, where the TILs comprise TILs obtained by improving the expression and/or enhancing the activity of at least one cytokine of the TILs.

In one embodiment, compared to TILs with unchanged expression and/or activity of the cytokine, TILs obtained by improving the expression and/or enhancing the activity of at least one cytokine of the TILs show improved TIL properties.

In one embodiment, the improved TIL properties comprise one or more properties selected from the group consisting of: increased number of TIL cells, increased proportion of viable cells, enhanced persistence, improved proportion of T cell subpopulations, enhanced cytokine secretion capacity, enhanced tumor cell killing ability, enhanced T cell receptor (TCR) clonal diversity, and increased number of TIL cells in tissues.

In one embodiment, the improved proportion of T cell subpopulations comprises one or more subpopulations selected from the group consisting of: increased proportion of central memory T cells, decreased proportion of regulatory T cells, increased proportion of activated T cells, increased proportion of tumor-specific T cells, and increased proportion of stem cell-like T cells.

In one embodiment, the step of improving the expression and/or enhancing the activity of at least one cytokine of the TILs comprises introducing a nucleic acid encoding the cytokine into the TILs.

In one embodiment, the step of improving the expression and/or enhancing the activity of at least one cytokine of the TILs comprises introducing a vector comprising the nucleic acid into the TILs.

In one embodiment, the nucleic acid encoding the cytokine is integrated into the genome of the TILs.

In one embodiment, the vector comprises a viral vector.

In one embodiment, the viral vector comprises a retroviral vector.

In one embodiment, the retroviral vector comprises a lentiviral vector.

In one embodiment, the cytokine comprises interleukin (IL).

In one embodiment, the cytokine comprises interleukin-15 (IL-15) and/or a functionally active fragment thereof.

In one embodiment, the IL-15 comprises membrane-anchored IL-15 and/or secreted IL-15.

In one embodiment, the IL-15 comprises an IL-15 domain.

In one embodiment, the IL-15 domain comprises an amino acid sequence as shown in SEQ ID NO: 3.

In one embodiment, the IL-15 comprises an IL-15Ra extracellular domain.

In one embodiment, the IL-15Ra extracellular domain comprises an amino acid sequence as shown in SEQ ID NO: 16.

In one embodiment, the IL-15 domain is directly or indirectly linked to the IL-15Ra extracellular domain.

In one embodiment, the indirect linkage comprises linking through a linker.

In one embodiment, the linker comprises an amino acid sequence selected from the group consisting of: SEQ ID NOs: 4-10, (SEQ ID NO: 11)$_l$, (SEQ ID NO: 12)$_m$, (SEQ ID NO: 13)$_n$, (SEQ ID NO: 14)$_p$, and (SEQ ID NO: 15)$_q$, and any combination of the above, where l, m, n, p, and q are each independently at least 1.

In one embodiment, the II,-15 comprises a transmembrane domain.

In one embodiment, the transmembrane domain comprises an amino acid sequence as shown in any one of SEQ ID NOs: 17-22.

In one embodiment, the transmembrane domain is directly or indirectly linked to the IL-15Ra extracellular domain.

In one embodiment, the IL-15 comprises an intracellular domain.

In one embodiment, the intracellular domain comprises an amino acid sequence as shown in any one of SEQ ID NOs: 23-26.

In one embodiment, the intracellular domain is directly or indirectly linked to the transmembrane domain.

In one embodiment, the IL-15 comprises a signal peptide domain.

In one embodiment, the signal peptide domain comprises an amino acid sequence as shown in SEQ ID NO: 2.

In one embodiment, the signal peptide domain is directly or indirectly linked to the IL-15 domain.

In one embodiment, the functionally active fragment of the IL-15 comprises an amino acid sequence as shown in SEQ ID NO: 3.

In one embodiment, the IL-15 comprises an amino acid sequence as shown in SEQ ID NO: 1.

In one embodiment, the enhanced expression of the cytokine comprises enhanced synthesis and/or secretion of the cytokine.

In one embodiment, compared to TILs with unchanged expression and/or activity of the cytokine, the proportion of cells expressing the cytokine in the TILs obtained by improving the expression and/or enhancing the activity of at least one cytokine of the TILs is increased.

In one embodiment, compared to TILs with unchanged expression and/or activity of the cytokine, the proportion of cells expressing the cytokine in the TILs obtained by improving the expression and/or enhancing the activity of at least one cytokine of the TILs is increased by at least about 5% or above.

In one embodiment, in the TILs obtained by improving the expression and/or enhancing the activity of at least one cytokine of the TILs, the proportion of cells expressing the cytokine is at least about 5% or above.

In one embodiment, the method further comprises: subjecting TILs derived from tumor tissues and not expanded *in vitro* to at least one stage of *in vitro* expansion, where the TILs are co-cultured with the feeder cells during the at least one stage *of in vitro* expansion.

In one embodiment, the method comprises co-culturing the TILs with the feeder cells during a single stage *of in vitro* expansion.

In one embodiment, the expression of at least one cytokine of the TILs is increased and/or the activity thereof is enhanced, and the TILs are co-cultured with the feeder cells during a single stage *of in vitro* expansion.

In one embodiment, the TILs derived from tumor tissues and not expanded *in vitro* are subjected to a first stage *of in vitro* expansion and a second stage *of in vitro* expansion, and during the second stage *of in vitro* expansion, co-culturing the TILs with the feeder cells

In one embodiment, the first stage of *in vitro* expansion is carried out for at least about 7 days.

In one embodiment, the first stage of *in vitro* expansion is carried out for about 7 days to about 14 days.

In one embodiment, the second stage *of in vitro* expansion is carried out for at least about 7 days.

In one embodiment, the second stage *of in vitro* expansion is carried out for about 7 days to about 14 days.

In one embodiment, co-culturing the TILs with the feeder cells after contacting the TILs with the T cell activators and/or the T cell growth factors for at least about 2 hours.

In one embodiment, co-culturing the TILs with the feeder cells after contacting the TILs with the T cell activators and/or the T cell growth factors for about 6 hours to about 72 hours.

In one embodiment, co-culturing the TILs with the feeder cells after contacting the TILs with the T cell activators and/or the T cell growth factors for about 12 hours to about 48 hours.

In one embodiment, co-culturing the TILs with the feeder cells after contacting the TILs with the T cell activators and/or the T cell growth factors for about 6 hours, about 12 hours, about 24 hours, about 48 hours, or about 72 hours.

In one embodiment, the feeder cells comprise antigen-presenting cells.

In one embodiment, the feeder cells comprise one or more cells selected from the group consisting of: peripheral mononuclear cells, dendritic cells, and artificial antigen-presenting cells.

In one embodiment, the feeder cells are peripheral mononuclear cells.

In one embodiment, the feeder cells are irradiated feeder cells.

In one embodiment, the step of co-culturing the TILs with the feeder cells comprises contacting the surface of the feeder cells with the surface of the TILs.

In one embodiment, the step of co-culturing the TILs with the feeder cells comprises adding the feeder cells into the cell culture medium of the TILs.

In one embodiment, the feeder cells are added into the cell culture medium of the TILs at a proportion of the feeder cells to the TILs from about 40:1 to about 400:1.

In one embodiment, the method further comprises: subjecting TILs derived from tumor tissues and not expanded *in vitro* to at least one stage of *in vitro* expansion, where contacting the TILs with the T cell activators during the at least one stage *of in vitro* expansion.

In one embodiment, the method comprises contacting the TILs with the T cell activators during a single stage *of in vitro* expansion.

In one embodiment, the method comprises increasing the expression of at least one cytokine of the TILs and/or enhancing the activity thereof, and contacting the TILs with the T cell activators during the single stage of *in vitro* expansion.

In one embodiment, the TILs derived from tumor tissues and not expanded *in vitro* are subjected to the first stage *of in vitro* expansion and the second stage *of in vitro* expansion, and during the second stage *of in vitro* expansion, contacting the TILs with the T cell activators.

In one embodiment, the T cell activators comprise one or more activators selected from the group consisting of: cluster of differentiation 80 (CD80), CD86, CD276, 4-1BB ligand (4-1BBL), CD27, CD30, CD134, CD275, CD40, CD258, and functionally active fragments thereof.

In one embodiment, the T cell activators comprise agonists of one or more targets selected from the group consisting of: CD3, CD28, herpes virus entry mediator (HVEM), CD40L, OX40, and 4-1BB.

In one embodiment, the T cell activators comprise a CD3 agonist and/or a CD28 agonist.

In one embodiment, the T cell activators comprise a CD3 agonist.

In one embodiment, the T cell activators comprise an anti-CD3 antibody and/or an antigen-binding fragment thereof.

In one embodiment, the T cell activators comprise a CD28 agonist.

In one embodiment, the T cell activators comprise an anti-CD28 antibody and/or an antigen-binding fragment thereof, CD80 and/or a functionally active fragment thereof and/or CD86 and/or a functionally active fragment thereof.

In one embodiment, the step of contacting the TILs with the T cell activators comprises one or more ways selected from the group consisting of: (1) adding the T cell activators into the cell culture medium of the TILs; (2) adding engineered cells expressing the T cell activators into the cell culture medium of the TILs; and (3) adding a solid medium comprising the T cell activators into the cell culture medium of the TILs.

In one embodiment, the initial concentration of each of the T cell activators in the cell culture medium of the TILs is each independently at least about 30 ng/mL.

In one embodiment, the initial concentration of each of the T cell activators in the cell culture medium of the TILs is each independently about 30 ng/mL to about 300 ng/mL.

In one embodiment, the diameter of the solid medium is about 500 nm to about 10 μm.

In one embodiment, the diameter of the solid medium is about 1 nm to about 500 nm.

In one embodiment, the diameter of the solid medium is measured by transmission electron microscopy.

In one embodiment, the solid medium comprises a polymer.

In one embodiment, the amount of each of the T cell activators comprised in each mg of the solid medium is independently at least about 25 μg.

In one embodiment, the solid medium comprising the T cell activators is added into the cell culture medium of the TILs at a proportion of the solid medium to the TILs from about 2:1 to about 1:2.

In one embodiment, the solid medium comprising the T cell activators is added into the cell culture medium of the TILs at a proportion of the solid medium to the TILs from about 1:100 to about 1:2000.

In one embodiment, the method further comprises: subjecting TILs derived from tumor tissues and not expanded *in vitro* to at least one stage of *in vitro* expansion, where contacting the TILs with the T cell growth factors during the at least one stage of *in vitro* expansion.

In one embodiment, contacting the TILs with the T cell growth factors during the single stage of *in vitro* expansion.

In one embodiment, contacting the TILs with the T cell activators and the T cell growth factors during the single stage of *in vitro* expansion.

In one embodiment, the TILs derived from tumor tissues and not expanded *in vitro* are subjected to the first stage *of in vitro* expansion and the second stage *of in vitro* expansion, and during the second stage of *in vitro* expansion, contacting the TILs with the T cell growth factors.

In one embodiment, the method comprises contacting the TILs with the T cell activators and the T cell growth factors substantially simultaneously.

In one embodiment, the T cell growth factors are one or more factors selected from the group consisting of: IL-2, IL-7, IL-12, IL-15, IL-21, interferon γ, and the functionally active fragments thereof.

In one embodiment, the T cell growth factors comprise IL-2 and/or a functionally active fragment thereof.

In one embodiment, the step of contacting the TILs with the T cell growth factors comprises adding the T cell growth factors into the cell culture medium of the TILs.

In one embodiment, the initial concentration of each of the T cell growth factors in the cell culture medium of the TILs is each independently at least about 300 IU/mL.

In one embodiment, the TILs are TILs derived from tumor tissue debris and/or TILs resuscitated after cryopreservation.

In one embodiment, the debris has a volume of about 1 mm$^3$ to about 27 mm$^3$.

In another aspect, the present application further provides a method for culturing tumor-infiltrating lymphocytes (TILs), which comprises:

contacting a first TIL population derived from tumor tissues and not expanded *in vitro* with T cell growth factors, where a second TIL population is obtained via the step (A);

improving the expression and/or enhancing the activity of at least one cytokine of the second TIL population, and co-culturing the second TIL population with feeder cells after contacting the second TIL population with T cell activators and/or T cell growth factors for a period of time, where a third TIL population is obtained via the step (B).

In another aspect, the present application further provides a method for culturing tumor-infiltrating lymphocytes (TILs), which comprises:

resuscitating and/or continuing culturing an *in vitro* TIL population to obtain a second TIL population, where the *in vitro* TIL population comprises a TIL population obtained by *in vitro* expansion of the first TIL population derived from tumor tissues and not expanded *in vitro*;

improving the expression and/or enhancing the activity of at least one cytokine of the second TIL population, and co-culturing the second TIL population with feeder cells after contacting the second TIL population with T cell activators and/or T cell growth factors for a period of time, where a third TIL population is obtained via the step (B).

In one embodiment, the *in vitro* TIL population comprises a TIL population obtained by contacting the first TIL population with T cell growth factors.

In one embodiment, the *in vitro* TIL population comprises a TIL population obtained by cryopreserving the first TIL population.

In one embodiment, the step (A) is carried out for about 7 days to about 14 days.

In one embodiment, the step (B) is carried out for about 7 days to about 14 days.

In another aspect, the present application further provides a method for culturing tumor-infiltrating lymphocytes (TILs), which comprises:

contacting a first TIL population derived from tumor tissues and not expanded *in vitro* with T cell growth factors, where a second TIL population is obtained via the step (A);

improving the expression and/or enhancing the activity of at least one cytokine of the second TIL population, and contacting the second TIL population with T cell activators and/or T cell growth factors, where a third TIL population is obtained via the step (B);

co-culturing the third TIL population with feeder cells, where a fourth TIL population is obtained via the step (C).

In another aspect, the present application further provides a method for culturing tumor-infiltrating lymphocytes (TILs), which comprises:

resuscitating and/or continuing culturing an *in vitro* TIL population to obtain a second TIL population, where the *in vitro* TIL population comprises a TIL population obtained by *in vitro* expansion of a first TIL population derived from tumor tissues and not expanded *in vitro;*
improving the expression and/or enhancing the activity of at least one cytokine of the second TIL population, and contacting the second TIL population with T cell activators and/or T cell growth factors, where a third TIL population is obtained via the step (B);
co-culturing the third TIL population with feeder cells, where a fourth TIL population is obtained via the step (C).

In one embodiment, the *in vitro* TIL population comprises a TIL population obtained by contacting the first TIL population with T cell growth factors.
In one embodiment, the *in vitro* TIL population comprises a TIL population obtained by cryopreserving the first TIL population.
In one embodiment, the step (A) is carried out for about 7 days to about 14 days.
In one embodiment, the step (B) is carried out for about 0 days to about 8 days.
In one embodiment, the step (C) is carried out for about 5 days to about 14 days.
In another aspect, the present application further provides a method for culturing tumor-infiltrating lymphocytes (TILs), which comprises:

contacting a first TIL population derived from tumor tissues and not expanded *in vitro* with T cell growth factors, where a second TIL population is obtained via the step (A);
contacting the second TIL population with T cell activators and/or T cell growth factors, where a third TIL population is obtained via the step (B);
improving the expression and/or enhancing the activity of at least one cytokine of the third TIL population, where a fourth TIL population is obtained via the step (C);
co-culturing the fourth TIL population with feeder cells, where a fifth TIL population is obtained via the step (D).

In another aspect, the present application further provides a method for culturing tumor-infiltrating lymphocytes (TILs), which comprises:

resuscitating and/or continuing culturing an *in vitro* TIL population to obtain a second TIL population, where the *in vitro* TIL population comprises a TIL population obtained by *in vitro* expansion of a first TIL population derived from tumor tissues and not expanded *in vitro;*
contacting the second TIL population with T cell activators and/or T cell growth factors, where a third TIL population is obtained via the step (B);
improving the expression and/or enhancing the activity of at least one cytokine of the third TIL population, where a fourth TIL population is obtained via the step (C);
co-culturing the fourth TIL population with feeder cells, where a fifth TIL population is obtained via the step (D).

In one embodiment, the *in vitro* TIL population comprises a TIL population obtained by contacting the first TIL population with T cell growth factors.
In one embodiment, the *in vitro* TIL population comprises a TIL population obtained by cryopreserving the first TIL population.
In one embodiment, the step (A) is carried out for about 7 days to about 14 days.
In one embodiment, the step (B) is carried out for about 0 days to about 4 days.
In one embodiment, the step (C) is carried out for about 0 days to about 4 days.
In one embodiment, the step (D) is carried out for about 5 days to about 14 days.
In one embodiment, compared to TILs with unchanged expression and/or activity of the cytokine, TILs obtained by improving the expression and/or enhancing the activity of at least one cytokine of the TILs show improved TIL properties.
In one embodiment, the improved TIL properties comprise one or more properties selected from the group consisting of: increased number of TIL cells, increased proportion of viable cells, enhanced persistence, improved proportion of T cell subpopulations, enhanced cytokine secretion capacity, enhanced tumor cell killing ability, enhanced T cell receptor (TCR) clonal diversity, and increased number of TIL cells in tissues.
In one embodiment, the improved proportion of T cell subpopulations comprises one or more subpopulations selected from the group consisting of: increased proportion of central memory T cells, decreased proportion of regulatory T cells, increased proportion of activated T cells, increased proportion of tumor-specific T cells, and increased proportion of stem cell-like T cells.

In one embodiment, the step of improving the expression and/or enhancing the activity of at least one cytokine of the TILs comprises introducing a nucleic acid encoding the cytokine into the TILs.

In one embodiment, the step of improving the expression and/or enhancing the activity of at least one cytokine of the TILs comprises introducing a vector comprising the nucleic acid into the TILs.

In one embodiment, the nucleic acid encoding the cytokine is integrated into the genome of the TILs.

In one embodiment, the vector comprises a viral vector.

In one embodiment, the viral vector comprises a retroviral vector.

In one embodiment, the retroviral vector comprises a lentiviral vector.

In one embodiment, the cytokine comprises interleukin (IL).

In one embodiment, the cytokine comprises interleukin-15 (IL-15) and/or a functionally active fragment thereof.

In one embodiment, the IL-15 comprises membrane-anchored IL-15 and/or secreted IL-15.

In one embodiment, the IL-15 comprises an IL-15 domain.

In one embodiment, the IL-15 domain comprises an amino acid sequence as shown in SEQ ID NO: 3.

In one embodiment, the IL-15 comprises an IL-15Ra extracellular domain.

In one embodiment, the IL-15Ra extracellular domain comprises an amino acid sequence as shown in SEQ ID NO: 16.

In one embodiment, the IL-15 domain is directly or indirectly linked to the IL-15Ra extracellular domain.

In one embodiment, the indirect linkage comprises linking through a linker.

In one embodiment, the linker comprises an amino acid sequence selected from the group consisting of: SEQ ID NOs: 4-10, (SEQ ID NO: 11)$_l$, (SEQ ID NO: 12)$_m$, (SEQ ID NO: 13)$_n$, (SEQ ID NO: 14)$_p$, and (SEQ ID NO: 15)$_q$, and any combination of the above, where l, m, n, p, and q are each independently at least 1.

In one embodiment, the IL-15 comprises a transmembrane domain.

In one embodiment, the transmembrane domain comprises an amino acid sequence as shown in any one of SEQ ID NOs: 17-22.

In one embodiment, the transmembrane domain is directly or indirectly linked to the IL-15Ra extracellular domain.

In one embodiment, the IL-15 comprises an intracellular domain.

In one embodiment, the intracellular domain comprises an amino acid sequence as shown in any one of SEQ ID NOs: 23-26.

In one embodiment, the intracellular domain is directly or indirectly linked to the transmembrane domain.

In one embodiment, the IL-15 comprises a signal peptide domain.

In one embodiment, the signal peptide domain comprises an amino acid sequence as shown in SEQ ID NO: 2.

In one embodiment, the signal peptide domain is directly or indirectly linked to the IL-15 domain.

In one embodiment, the functionally active fragment of the IL-15 comprises an amino acid sequence as shown in SEQ ID NO: 3.

In one embodiment, the IL-15 comprises an amino acid sequence as shown in SEQ ID NO: 1.

In one embodiment, the enhanced expression of the cytokine comprises enhanced synthesis and/or secretion of the cytokine.

In one embodiment, compared to TILs with unchanged expression and/or activity of the cytokine, the proportion of cells expressing the cytokine in the TILs obtained by improving the expression and/or enhancing the activity of at least one cytokine of the TILs is increased.

In one embodiment, compared to TILs with unchanged expression and/or activity of the cytokine, the proportion of cells expressing the cytokine in the TILs obtained by improving the expression and/or enhancing the activity of at least one cytokine of the TILs is increased by at least about 5% or above.

In one embodiment, in the TILs obtained by improving the expression and/or enhancing the activity of at least one cytokine of the TILs, the proportion of cells expressing the cytokine is at least about 5% or above.

In one embodiment, co-culturing the TILs with the feeder cells after contacting the TILs with the T cell activators and/or the T cell growth factors for at least about 2 hours.

In one embodiment, co-culturing the TILs with the feeder cells after contacting the TILs with the T cell activators and/or the T cell growth factors for about 6 hours to about 72 hours.

In one embodiment, co-culturing the TILs with the feeder cells after contacting the TILs with the T cell activators and/or the T cell growth factors for about 12 hours to about 48 hours.

In one embodiment, co-culturing the TILs with the feeder cells after contacting the TILs with the T cell activators and/or the T cell growth factors for about 6 hours, about 12 hours, about 24 hours, about 48 hours, or about 72 hours.

In one embodiment, the feeder cells comprise antigen-presenting cells.

In one embodiment, the feeder cells comprise one or more cells selected from the group consisting of: peripheral mononuclear cells, dendritic cells, and artificial antigen-presenting cells.

In one embodiment, the feeder cells are peripheral mononuclear cells.

In one embodiment, the feeder cells are irradiated feeder cells.

In one embodiment, the step of co-culturing the TILs with the feeder cells comprises contacting the surface of the feeder cells with the surface of the TILs.

In one embodiment, the step of co-culturing the TILs with the feeder cells comprises adding the feeder cells into the cell culture medium of the TILs.

In one embodiment, the feeder cells are added into the cell culture medium of the TILs at a proportion of the feeder cells to the TILs from about 40:1 to about 400:1.

In one embodiment, the T cell activators comprise one or more activators selected from the group consisting of: cluster of differentiation 80 (CD80), CD86, CD276, 4-1BB ligand (4-1BBL), CD27, CD30, CD134, CD275, CD40, CD258, and functionally active fragments thereof.

In one embodiment, the T cell activators comprise agonists of one or more targets selected from the group consisting of: CD3, CD28, herpes virus entry mediator (HVEM), CD40L, OX40, and 4-1BB.

In one embodiment, the T cell activators comprise a CD3 agonist and/or a CD28 agonist.

In one embodiment, the T cell activators comprise a CD3 agonist.

In one embodiment, the T cell activators comprise an anti-CD3 antibody and/or an antigen-binding fragment thereof.

In one embodiment, the T cell activators comprise a CD28 agonist.

In one embodiment, the T cell activators comprise an anti-CD28 antibody and/or an antigen-binding fragment thereof, CD80 and/or a functionally active fragment thereof and/or CD86 and/or a functionally active fragment thereof.

In one embodiment, the step of contacting the TILs with the T cell activators comprises one or more ways selected from the group consisting of: (1) adding the T cell activators into the cell culture medium of the TILs; (2) adding engineered cells expressing the T cell activators into the cell culture medium of the TILs; and (3) adding a solid medium comprising the T cell activators into the cell culture medium of the TILs.

In one embodiment, the initial concentration of each of the T cell activators in the cell culture medium of the TILs is each independently at least about 30 ng/mL.

In one embodiment, the initial concentration of each of the T cell activators in the cell culture medium of the TILs is each independently about 30 ng/mL to about 300 ng/mL.

In one embodiment, the diameter of the solid medium is about 500 nm to about 10 $\mu$m.

In one embodiment, the diameter of the solid medium is about 1 nm to about 500 nm.

In one embodiment, the diameter of the solid medium is measured by transmission electron microscopy.

In one embodiment, the solid medium comprises a polymer.

In one embodiment, the amount of each of the T cell activators comprised in each mg of the solid medium is independently at least about 25 $\mu$g.

In one embodiment, the solid medium comprising the T cell activators is added into the cell culture medium of the TILs at a proportion of the solid medium to the TILs from about 2:1 to about 1:2.

In one embodiment, the solid medium comprising the T cell activators is added into the cell culture medium of the TILs at a proportion of the solid medium to the TILs from about 1:100 to about 1:2000.

In one embodiment, the method comprises contacting the TILs with the T cell activators and the T cell growth factors substantially simultaneously.

In one embodiment, the T cell growth factors are one or more factors selected from the group consisting of: IL-2, IL-7, IL-12, IL-15, IL-21, interferon $\gamma$, and functionally active fragments thereof.

In one embodiment, the T cell growth factors comprise IL-2 and/or a functionally active fragment thereof.

In one embodiment, the step of contacting the TILs with the T cell growth factors comprises adding the T cell growth factors into the cell culture medium of the TILs.

In one embodiment, the initial concentration of each of the T cell growth factors in the cell culture medium of the TILs is each independently at least about 300 IU/mL.

In one embodiment, the TILs are TILs derived from tumor tissue debris and/or TILs resuscitated after cryopreservation.

In one embodiment, the debris has a volume of about 1 mm$^3$ to about 27 mm$^3$.

In another aspect, the present application further provides a method for culturing tumor-infiltrating lymphocytes (TILs), the method including improving the expression and/or enhancing the activity of at least one cytokine of the TILs and contacting the TILs with a CD28 agonist.

In one embodiment, the method comprises improving the expression and/or enhancing the activity of at least one cytokine of the TILs after contacting the TILs with the CD28 agonist.

In one embodiment, the method comprises improving the expression and/or enhancing the activity of at least one cytokine of the TILs before contacting the TILs with the CD28 agonist.

A method for culturing tumor-infiltrating lymphocytes (TILs), the method including improving the expression and/or enhancing the activity of at least one cytokine of the TILs, where the TILs comprise TILs obtained by contacting the TILs with a CD28 agonist.

A method for culturing tumor-infiltrating lymphocytes (TILs), the method including contacting the TILs with a CD28 agonist, where the TILs comprise TILs obtained by improving the expression and/or enhancing the activity of at least one cytokine of the TILs.

In one embodiment, compared to TILs with unchanged expression and/or activity of the cytokine, TILs obtained by improving the expression and/or enhancing the activity of at least one cytokine of the TILs show improved TIL properties.

In one embodiment, the improved TIL properties comprise one or more properties selected from the group consisting of: increased number of TIL cells, increased proportion of viable cells, enhanced persistence, improved proportion of T cell subpopulations, enhanced cytokine secretion capacity, enhanced tumor cell killing ability, enhanced T cell receptor (TCR) clonal diversity, and increased number of TIL cells in tissues.

In one embodiment, the improved proportion of T cell subpopulations comprises one or more subpopulations selected from the group consisting of: increased proportion of central memory T cells, decreased proportion of regulatory T cells, increased proportion of activated T cells, increased proportion of tumor-specific T cells, and increased proportion of stem cell-like T cells.

In one embodiment, compared to corresponding TILs that have not been contacted with the CD28 agonist during the stage *of in vitro* expansion, the TILs that have been contacted with the CD28 agonist during at least one stage *of in vitro* expansion show an improved gene editing effect.

In one embodiment, the improved gene editing effect comprises an enhanced gene knockout efficiency.

In one embodiment, the step of improving the expression and/or enhancing the activity of at least one cytokine of the TILs comprises introducing a nucleic acid encoding the cytokine into the TILs.

In one embodiment, the step of improving the expression and/or enhancing the activity of at least one cytokine of the TILs comprises introducing a vector comprising the nucleic acid into the TILs.

In one embodiment, the nucleic acid encoding the cytokine is integrated into the genome of the TILs.

In one embodiment, the vector comprises a viral vector.

In one embodiment, the viral vector comprises a retroviral vector.

In one embodiment, the retroviral vector comprises a lentiviral vector.

In one embodiment, the cytokine comprises interleukin (IL).

In one embodiment, the cytokine comprises interleukin-15 (IL-15) and/or a functionally active fragment thereof.

In one embodiment, the IL-15 comprises membrane-anchored IL-15 and/or secreted IL-15.

In one embodiment, the IL-15 comprises an IL-15 domain.

In one embodiment, the IL-15 domain comprises an amino acid sequence as shown in SEQ ID NO: 3.

In one embodiment, the IL-15 comprises an IL-15Ra extracellular domain.

In one embodiment, the IL-15Ra extracellular domain comprises an amino acid sequence as shown in SEQ ID NO: 16.

In one embodiment, the IL-15 domain is directly or indirectly linked to the IL-15Ra extracellular domain.

In one embodiment, the indirect linkage comprises linking through a linker.

In one embodiment, the linker comprises an amino acid sequence selected from the group consisting of: SEQ ID NOs: 4-10, (SEQ ID NO: 11)$_l$, (SEQ ID NO: 12)$_m$, (SEQ ID NO: 13)$_n$, (SEQ ID NO: 14)$_p$, and (SEQ ID NO: 15)$_q$, and any combination of the above, where l, m, n, p, and q are each independently at least 1.

In one embodiment, the IL-15 comprises a transmembrane domain.

In one embodiment, the transmembrane domain comprises an amino acid sequence as shown in any one of SEQ ID NOs: 17-22.

In one embodiment, the transmembrane domain is directly or indirectly linked to the IL-15Ra extracellular domain.

In one embodiment, the IL-15 comprises an intracellular domain.

In one embodiment, the intracellular domain comprises an amino acid sequence as shown in any one of SEQ ID NOs: 23-26.

In one embodiment, the intracellular domain is directly or indirectly linked to the transmembrane domain.

In one embodiment, the IL-15 comprises a signal peptide domain.

In one embodiment, the signal peptide domain comprises an amino acid sequence as shown in SEQ ID NO: 2.

In one embodiment, the signal peptide domain is directly or indirectly linked to the IL-15 domain.

In one embodiment, the functionally active fragment of the IL-15 comprises an amino acid sequence as shown in SEQ ID NO: 3.

In one embodiment, the IL-15 comprises an amino acid sequence as shown in SEQ ID NO: 1.

In one embodiment, the enhanced expression of the cytokine comprises enhanced synthesis and/or secretion of the cytokine.

In one embodiment, compared to TILs with unchanged expression and/or activity of the cytokine, the proportion of cells expressing the cytokine in the TILs obtained by improving the expression and/or enhancing the activity of at least one cytokine of the TILs is increased.

In one embodiment, compared to TILs with unchanged expression and/or activity of the cytokine, the proportion of cells expressing the cytokine in the TILs obtained by improving the expression and/or enhancing the activity of at least one cytokine of the TILs is increased by at least about 5% or above.

In one embodiment, in the TILs obtained by improving the expression and/or enhancing the activity of at least one cytokine of the TILs, the proportion of cells expressing the cytokine is at least about 5% or above.

In one embodiment, the TILs derived from tumor tissues and not expanded *in vitro* are subjected to at least one stage *of in vitro* expansion, and during the at least one stage of *in vitro* expansion, contacting the TILs with the CD28 agonist.

In one embodiment, the TILs derived from tumor tissues and not expanded *in vitro* are subjected to the first stage *of in vitro* expansion and the second stage *of in vitro* expansion, and during the second stage of *in vitro* expansion, the TILs from the first stage of *in vitro* expansion are contacted with the CD28 agonist.

In one embodiment, the first stage of *in vitro* expansion is carried out for at least about 7 days.

In one embodiment, the first stage of *in vitro* expansion is carried out for about 7 days to about 14 days.

In one embodiment, the second stage *of in vitro* expansion is carried out for at least about 7 days.

In one embodiment, the second stage *of in vitro* expansion is carried out for about 7 days to about 14 days.

In one embodiment, the CD28 agonist comprises an anti-CD28 antibody and/or an antigen-binding fragment thereof, CD80 and/or a functionally active fragment thereof, and/or CD86 and/or a functionally active fragment thereof.

In one embodiment, the method further comprises: subjecting TILs derived from tumor tissues and not expanded *in vitro* to at least one stage of *in vitro* expansion, where contacting the TILs with other T cell activators other than the CD28 agonist during the at least one stage *of in vitro* expansion.

In one embodiment, during a single stage *of in vitro* expansion, contacting the TILs with the other T cell activators.

In one embodiment, during a single stage *of in vitro* expansion, the expression of at least one cytokine of the TILs is increased and/or the activity thereof is enhanced and the TILs are contacted with the other T cell activators.

In one embodiment, the TILs derived from tumor tissues and not expanded *in vitro* are subjected to the first stage *of in vitro* expansion and the second stage *of in vitro* expansion, and during the second stage of *in vitro* expansion, contacting the TILs with the other T cell activators.

In one embodiment, the method comprises contacting the TILs with the CD28 agonist and the other T cell activators substantially simultaneously.

In one embodiment, the other T cell activators comprise agonists of one or more targets selected from the group consisting of: CD3, HVEM, CD40L, OX40, and 4-1BB.

In one embodiment, the other T cell activators comprise a CD3 agonist.

In one embodiment, the other T cell activators comprise an anti-CD3 antibody and/or an antigen-binding fragment thereof.

In one embodiment, the step of contacting the TILs with the CD28 agonist and the other T cell activators comprises one or more ways selected from the group consisting of: (1) adding the CD28 agonist and the other T cell activators into the cell culture medium of the TILs; (2) adding engineered cells expressing the CD28 agonist and the other T cell activators into the cell culture medium of the TILs; (3) adding a solid medium comprising the CD28 agonist and the other T cell activators into the cell culture medium of the TILs.

In one embodiment, the initial concentration of the other T cell activators in the cell culture medium of the TILs is at least about 30 ng/mL.

In one embodiment, the initial concentration of the other T cell activators in the cell culture medium of the TILs is about 30 ng/mL to about 300 ng/mL.

In one embodiment, the diameter of the solid medium is about 500 nm to about 10 $\mu$m.

In one embodiment, the diameter of the solid medium is about 1 nm to about 500 nm.

In one embodiment, the diameter of the solid medium is measured by transmission electron microscopy.

In one embodiment, the solid medium comprises a polymer.

In one embodiment, each mg of the solid medium contains at least about 25 $\mu$g of the CD28 agonist and the other T cell activators.

In one embodiment, the solid medium comprising the CD28 agonist and the other T cell activators is added into the cell culture medium of the TILs at a proportion of the solid medium to the TILs from about 2:1 to about 1:2.

In one embodiment, the solid medium comprising the CD28 agonist and the other T cell activators is added into the cell culture medium of the TILs at a proportion of the solid medium to the TILs from about 1:100 to about 1:2000.

In one embodiment, the method further comprises: subjecting TILs derived from tumor tissues and not expanded *in vitro* to at least one stage of *in vitro* expansion, where the TILs are co-cultured with feeder cells after contacting the TILs with the CD28 agonist for a period of time during the at least one stage *of in vitro* expansion.

In one embodiment, co-culturing the TILs with the feeder cells during the single stage *of in vitro* expansion.

In one embodiment, contacting the TILs with the CD28 agonist and co-cultured with the feeder cells during the single stage *of in vitro* expansion.

In one embodiment, the TILs derived from tumor tissues and not expanded *in vitro* are subjected to the first stage *of in vitro* expansion and the second stage *of in vitro* expansion, and during the second stage *of in vitro* expansion, co-culturing the TILs with the feeder cells

In one embodiment, co-culturing the TILs with the feeder cells after contacting the TILs with the CD28 agonist for at least about 2 hours.

In one embodiment, co-culturing the TILs with the feeder cells after contacting the TILs with the CD28 agonist for about 6 hours to about 72 hours.

In one embodiment, co-culturing the TILs with the feeder cells after contacting the TILs with the CD28 agonist for about 12 hours to about 48 hours.

In one embodiment, co-culturing the TILs with the feeder cells after contacting the TILs with the CD28 agonist for about 6 hours, about 12 hours, about 24 hours, about 48 hours, or about 72 hours.

In one embodiment, the feeder cells comprise antigen-presenting cells.

In one embodiment, the feeder cells comprise one or more cells selected from the group consisting of: peripheral mononuclear cells, dendritic cells, and artificial antigen-presenting cells.

In one embodiment, the feeder cells are peripheral mononuclear cells.

In one embodiment, the feeder cells are irradiated feeder cells.

In one embodiment, the step of co-culturing the TILs with the feeder cells comprises contacting the surface of the feeder cells with the surface of the TILs.

In one embodiment, the step of co-culturing the TILs with the feeder cells comprises adding the feeder cells into the cell culture medium of the TILs.

In one embodiment, the feeder cells are added into the cell culture medium of the TILs at a proportion of the feeder cells to the TILs from about 40:1 to about 400:1.

In one embodiment, the method further comprises: subjecting TILs derived from tumor tissues and not expanded *in vitro* to at least one stage of *in vitro* expansion, where contacting the TILs with T cell growth factors during the at least one stage *of in vitro* expansion.

In one embodiment, contacting the TILs with the T cell growth factors during the single stage of *in vitro* expansion.

In one embodiment, contacting the TILs with the CD28 agonist and the T cell growth factors during a single stage *of in vitro* expansion.

In one embodiment, the TILs derived from tumor tissues and not expanded *in vitro* are subjected to the first stage *of in vitro* expansion and the second stage *of in vitro* expansion, and during the second stage of *in vitro* expansion, contacting the TILs with the T cell growth factors.

In one embodiment, the method comprises contacting the TILs with the CD28 agonist and the T cell growth factors substantially simultaneously.

In one embodiment, the T cell growth factors are one or more factors selected from the group consisting of: IL-2, IL-7, IL-12, IL-15, IL-21, interferon $\gamma$, and functionally active fragments thereof.

In one embodiment, the T cell growth factors comprise IL-2 and/or a functionally active fragment thereof.

In one embodiment, the step of contacting the TILs with the T cell growth factors comprises adding the T cell growth factors into the cell culture medium of the TILs.

In one embodiment, the initial concentration of each of the T cell growth factors in the cell culture medium of the TILs is each independently at least about 300 IU/mL.

In one embodiment, the TILs are TILs derived from tumor tissue debris and/or TILs resuscitated after cryopreservation.

In one embodiment, the debris has a volume of about 1 $mm^3$ to about 27 $mm^3$.

In another aspect, the present application further provides a method for culturing tumor-infiltrating lymphocytes (TILs), which comprises:

contacting a first TIL population derived from tumor tissues and not expanded *in vitro* with T cell growth factors, where a second TIL population is obtained via the step (A);
improving the expression and/or enhancing the activity of at least one cytokine of the second TIL population, and contacting the TILs with a CD28 agonist, where a third TIL population is obtained via the step (B).

In another aspect, the present application further provides a method for culturing tumor-infiltrating lymphocytes (TILs), which comprises:

resuscitating and/or continuing culturing an *in vitro* TIL population to obtain a second TIL population, where the *in vitro* TIL population comprises a TIL population obtained by *in vitro* expansion of a first TIL population derived from tumor tissues and not expanded *in vitro;*

improving the expression and/or enhancing the activity of at least one cytokine of the second TIL population, and contacting the TILs with a CD28 agonist, where a third TIL population is obtained via the step (B).

In one embodiment, the *in vitro* TIL population comprises a TIL population obtained by contacting the first TIL population with T cell growth factors.

In one embodiment, the *in vitro* TIL population comprises a TIL population obtained by cryopreserving the first TIL population.

In one embodiment, the step (A) is carried out for about 7 days to about 14 days.

In one embodiment, the step (B) is carried out for about 7 days to about 14 days.

In one embodiment, compared to TILs with unchanged expression and/or activity of the cytokine, TILs obtained by improving the expression and/or enhancing the activity of at least one cytokine of the TILs show improved TIL properties.

In one embodiment, the improved TIL properties comprise one or more properties selected from the group consisting of: increased number of TIL cells, increased proportion of viable cells, enhanced persistence, improved proportion of T cell subpopulations, enhanced cytokine secretion capacity, enhanced tumor cell killing ability, enhanced T cell receptor (TCR) clonal diversity, and increased number of TIL cells in tissues.

In one embodiment, the improved proportion of T cell subpopulations comprises one or more subpopulations selected from the group consisting of: increased proportion of central memory T cells, decreased proportion of regulatory T cells, increased proportion of activated T cells, increased proportion of tumor-specific T cells, and increased proportion of stem cell-like T cells.

In one embodiment, compared to corresponding TILs that have not been contacted with the CD28 agonist during the stage *of in vitro* expansion, the TILs that have been contacted with the CD28 agonist during at least one stage *of in vitro* expansion show an improved gene editing effect.

In one embodiment, the improved gene editing effect comprises enhanced gene knockout efficiency.

In one embodiment, the step of improving the expression and/or enhancing the activity of at least one cytokine of the TILs comprises introducing a nucleic acid encoding the cytokine into the TILs.

In one embodiment, the step of improving the expression and/or enhancing the activity of at least one cytokine of the TILs comprises introducing a vector comprising the nucleic acid into the TILs.

In one embodiment, the nucleic acid encoding the cytokine is integrated into the genome of the TILs.

In one embodiment, the vector comprises a viral vector.

In one embodiment, the viral vector comprises a retroviral vector.

In one embodiment, the retroviral vector comprises a lentiviral vector.

In one embodiment, the cytokine comprises interleukin (IL).

In one embodiment, the cytokine comprises interleukin-15 (IL-15) and/or a functionally active fragment thereof.

In one embodiment, the IL-15 comprises membrane-anchored IL-15 and/or secreted IL-15.

In one embodiment, the IL-15 comprises an IL-15 domain.

In one embodiment, the IL-15 domain comprises an amino acid sequence as shown in SEQ ID NO: 3.

In one embodiment, the IL-15 comprises an IL-15Ra extracellular domain.

In one embodiment, the IL-15Ra extracellular domain comprises an amino acid sequence as shown in SEQ ID NO: 16.

In one embodiment, the IL-15 domain is directly or indirectly linked to the IL-15Ra extracellular domain.

In one embodiment, the indirect linkage comprises linking through a linker.

In one embodiment, the linker comprises an amino acid sequence selected from the group consisting of: SEQ ID NOs: 4-10, (SEQ ID NO: 11)$_l$, (SEQ ID NO: 12)$_m$, (SEQ ID NO: 13)$_n$, (SEQ ID NO: 14)$_p$, and (SEQ ID NO: 15)$_q$, and any combination of the above, where l, m, n, p, and q are each independently at least 1.

In one embodiment, the IL-15 comprises a transmembrane domain.

In one embodiment, the transmembrane domain comprises an amino acid sequence as shown in any one of SEQ ID NOs: 17-22.

In one embodiment, the transmembrane domain is directly or indirectly linked to the IL-15Ra extracellular domain.

In one embodiment, the IL-15 comprises an intracellular domain.

In one embodiment, the intracellular domain comprises an amino acid sequence as shown in any one of SEQ ID NOs: 23-26.

In one embodiment, the intracellular domain is directly or indirectly linked to the transmembrane domain.

In one embodiment, the IL-15 comprises a signal peptide domain.

In one embodiment, the signal peptide domain comprises an amino acid sequence as shown in SEQ ID NO: 2.

In one embodiment, the signal peptide domain is directly or indirectly linked to the IL-15 domain.

In one embodiment, the functionally active fragment of the IL-15 comprises an amino acid sequence as shown in SEQ ID NO: 3.

In one embodiment, the IL-15 comprises an amino acid sequence as shown in SEQ ID NO: 1.

In one embodiment, the enhanced expression of the cytokine comprises enhanced synthesis and/or secretion of the cytokine.

In one embodiment, compared to TILs with unchanged expression and/or activity of the cytokine, the proportion of cells expressing the cytokine in the TILs obtained by improving the expression and/or enhancing the activity of at least one cytokine of the TILs is increased.

In one embodiment, compared to TILs with unchanged expression and/or activity of the cytokine, the proportion of cells expressing the cytokine in the TILs obtained by improving the expression and/or enhancing the activity of at least one cytokine of the TILs is increased by at least about 5% or above.

In one embodiment, in the TILs obtained by improving the expression and/or enhancing the activity of at least one cytokine of the TILs, the proportion of cells expressing the cytokine is at least about 5% or above.

In one embodiment, the CD28 agonist comprises an anti-CD28 antibody and/or an antigen-binding fragment thereof, CD80 and/or a functionally active fragment thereof, and/or CD86 and/or a functionally active fragment thereof.

In one embodiment, the method comprises contacting the TILs with the CD28 agonist and the other T cell activators substantially simultaneously.

In one embodiment, the other T cell activators comprise agonists of one or more targets selected from the group consisting of: CD3, HVEM, CD40L, OX40, and 4-1BB.

In one embodiment, the other T cell activators comprise a CD3 agonist.

In one embodiment, the other T cell activators comprise an anti-CD3 antibody and/or an antigen-binding fragment thereof.

In one embodiment, the step of contacting the TILs with the CD28 agonist and the other T cell activators comprises one or more ways selected from the group consisting of: (1) adding the CD28 agonist and the other T cell activators into the cell culture medium of the TILs; (2) adding engineered cells expressing the CD28 agonist and the other T cell activators into the cell culture medium of the TILs; (3) adding a solid medium comprising the CD28 agonist and the other T cell activators into the cell culture medium of the TILs.

In one embodiment, the initial concentration of the other T cell activators in the cell culture medium of the TILs is at least about 30 ng/mL.

In one embodiment, the initial concentration of the other T cell activators in the cell culture medium of the TILs is about 30 ng/mL to about 300 ng/mL.

In one embodiment, the diameter of the solid medium is about 500 nm to about 10 $\mu$m.

In one embodiment, the diameter of the solid medium is about 1 nm to about 500 nm.

In one embodiment, the diameter of the solid medium is measured by transmission electron microscopy.

In one embodiment, the solid medium comprises a polymer.

In one embodiment, each mg of the solid medium contains at least about 25 $\mu$g of the CD28 agonist and the other T cell activators.

In one embodiment, the solid medium comprising the CD28 agonist and the other T cell activators is added into the cell culture medium of the TILs at a proportion of the solid medium to the TILs from about 2:1 to about 1:2.

In one embodiment, the solid medium comprising the CD28 agonist and the other T cell activators is added into the cell culture medium of the TILs at a proportion of the solid medium to the TILs from about 1:100 to about 1:2000.

In one embodiment, co-culturing the TILs with the feeder cells after contacting the TILs with the CD28 agonist for at least about 2 hours.

In one embodiment, co-culturing the TILs with the feeder cells after contacting the TILs with the CD28 agonist for about 6 hours to about 72 hours.

In one embodiment, co-culturing the TILs with the feeder cells after contacting the TILs with the CD28 agonist for about 12 hours to about 48 hours.

In one embodiment, co-culturing the TILs with the feeder cells after contacting the TILs with the CD28 agonist for about 6 hours, about 12 hours, about 24 hours, about 48 hours, or about 72 hours.

In one embodiment, the feeder cells comprise antigen-presenting cells.

In one embodiment, the feeder cells comprise one or more cells selected from the group consisting of: peripheral mononuclear cells, dendritic cells, and artificial antigen-presenting cells.

In one embodiment, the feeder cells are peripheral mononuclear cells.

In one embodiment, the feeder cells are irradiated feeder cells.

In one embodiment, the step of co-culturing the TILs with the feeder cells comprises contacting the surface of the feeder cells with the surface of the TILs.

In one embodiment, the step of co-culturing the TILs with the feeder cells comprises adding the feeder cells into the cell culture medium of the TILs.

In one embodiment, the feeder cells are added into the cell culture medium of the TILs at a proportion of the feeder cells to the TILs from about 40:1 to about 400:1.

In one embodiment, the method comprises contacting the TILs with the CD28 agonist and the T cell growth factors substantially simultaneously.

In one embodiment, the T cell growth factors are one or more factors selected from the group consisting of: IL-2, IL-7, IL-12, IL-15, IL-21, interferon $\gamma$, and functionally active fragments thereof.

In one embodiment, the T cell growth factors comprise IL-2 and/or a functionally active fragment thereof.

In one embodiment, the step of contacting the TILs with the T cell growth factors comprises adding the T cell growth factors into the cell culture medium of the TILs.

In one embodiment, the initial concentration of each of the T cell growth factors in the cell culture medium of the TILs is each independently at least about 300 IU/mL.

In one embodiment, the TILs are TILs derived from tumor tissue debris and/or TILs resuscitated after cryopreservation.

In one embodiment, the debris has a volume of about 1 mm$^3$ to about 27 mm$^3$.

In another aspect, the present application further provides a tumor-infiltrating lymphocyte (TIL), which is obtainable by the method of the present application.

In another aspect, the present application further provides a composition, which comprises the TIL of the present application.

In another aspect, the present application further provides a pharmaceutical composition, which comprises the TIL of the present application and/or the composition of the present application, and optionally a pharmaceutically acceptable carrier.

In another aspect, the present application further provides a method for affecting tumor cell growth, which comprises administering to a subject the TIL of the present application, the composition of the present application, and/or the pharmaceutical composition of the present application.

In another aspect, the present application further provides a use of the TIL of the present application, the composition of the present application and/or the pharmaceutical composition of the present application for the manufacture of drugs for preventing and/or treating a tumor.

The use of the present application, where the tumor is a solid tumor.

The use of the present application, where the tumor is one or more tumors selected from the group consisting of: melanoma, ovarian cancer, cervical cancer, lung cancer, bladder cancer, breast cancer, head and neck cancer, pancreatic cancer, liver cancer, gastric cancer, colorectal cancer, and kidney cancer.

Other aspects and advantages of the present application can be readily perceived by those skilled in the art from the following detailed description. In the following detailed description, only exemplary embodiments of the present application are shown and described. As will be recognized by those skilled in the art, the content of the present application enables those skilled in the art to make changes to the disclosed specific embodiments without departing from the spirit and scope of the invention involved in the present application. Correspondingly, the drawings and descriptions in the specification of the present application are merely exemplary, rather than restrictive.

## BRIEF DESCRIPTION OF THE DRAWING

The specific features of the invention involved in the present application are as shown in the appended claims. The characteristics and advantages of the invention involved in the present application can be better understood by referring to the exemplary embodiments described in detail below and the accompanying drawings. A brief description of the drawings is as below:

FIG. 1 shows the IL-15 transduction efficiency of CD4$^+$ cells in each group, for TIL cells derived from donor A.

FIG. 2 shows the IL-15 transduction efficiency of CD8$^+$ cells in each group, for TIL cells derived from donor A.

FIG. 3 shows the normalized expansion rate of TIL cells in each group, for TIL cells derived from donor B.

FIG. 4 shows the viability of TIL cells in each group, for TIL cells derived from donor B.

FIG. 5 shows the proliferation ability of TIL cells in each group, for TIL cells derived from donor C.

FIGs. 6, 7, and 8 show the secretion of cytokines IL-2, IFN-$\gamma$, and IL-10 in each group of TIL cells after being stimulated with the CD3 antibody, respectively, for TIL cells derived from donor D.

FIGs. 9 and 10 show the secretion of cytokines TNF-$\alpha$ and IL-10 in each group of TIL cells after being stimulated with transACT, respectively, for TIL cells derived from donor E.

FIGs. 11 and 12 show the secretion of cytokines IFN-$\gamma$ and IL-2 in each group of TIL cells, respectively, for TIL cells derived from donor F.

FIGs. 13 and 14 show the secretion of cytokines IFN-γ and IL-6 in each group of TIL cells, respectively, for TIL cells derived from donor G cultured without IL-2.

FIG. 15 shows the test results of the killing ability of TIL cells derived from donor H which are co-cultured with tumor cells at an effector-target ratio of 0.3:1.

FIG. 16 shows the test results of the killing ability of TIL cells derived from donor H which are co-cultured with tumor cells at an effector-target ratio of 1:1.

FIG. 17 shows the secretion results of cytokine IL-2 in TIL cells derived from donor H which are co-cultured with tumor cells at an effector-target ratio of 0.3:1 or 1:1.

FIG. 18 shows the secretion results of cytokine IFN-γ in TIL cells derived from donor H which are co-cultured with tumor cells at an effector-target ratio of 0.3:1 or 1:1.

FIG. 19 shows the diversity of $CD8^+$ T cell TCR Vβ clones on day 8 after transduction of TIL cells derived from donor I.

FIG. 20 shows the diversity of $CD8^+$ T cell TCR Vβ clones on day 26 after transduction of TIL cells derived from donor I.

FIG. 21 shows the diversity of $CD4^+$ T cell TCR Vβ clones on day 8 after transduction of TIL cells derived from donor I.

FIG. 22 shows the diversity of $CD4^+$ T cell TCR Vβ clones on day 26 after transduction of TIL cells derived from donor I.

FIGs. 23, 24, 25, 26, and 27 show the number of T cells in the various tissues on days 6 and 21 after injection of TIL cells, respectively.

FIG. 28 shows the analysis results of the proliferation ability of TILs cultured with feeder cells added at different times.

FIGs. 29 and 30 show the proportion of $CD45RA^-CCR7^+$ central memory T cells (Terns) in the TIL cells obtained by culturing with feeder cells added at 0, 24 or 48 hours after the addition of OKT3 and IL-2.

FIG. 31 shows the proportion of $CD4^+CD25^+Foxp3^+$ regulatory T cells (Treg) in the TIL cells obtained by culturing with feeder cells added at 0, 24 or 48 hours after the addition of OKT3 and IL-2.

FIGs. 32 and 33 show the proportion of activated T cells in the TIL cells obtained by culturing with feeder cells added at 0, 24 or 48 hours after the addition of OKT3 and IL-2.

FIG. 34 shows the proportion of $CD103^+CD39^+$ tumor-specific T cells in the TIL cells obtained by culturing with feeder cells added at 0, 24 or 48 hours after the addition of OKT3 and IL-2.

FIG. 35 shows the proportion of $TCF1^+$ stem cell-like T cells in the TIL cells obtained by culturing with feeder cells added at 0, 24 or 48 hours after the addition of OKT3 and IL-2.

FIG. 36 shows the analysis results of the proliferation ability of the test groups added with different forms of CD28 agonists and the control group.

FIGs. 37 and 38 show the proportion of T cell subpopulations in the TIL cells obtained from culture in the mixed antibody group and the control group, respectively, for TILs from different donor sources.

FIGs. 39 and 40 show the proportion of T cell subpopulations in the TIL cells obtained from culture in the magnetic bead group and the control group, respectively, for TILs from different donor sources.

FIG. 41 shows the proportion of T cell subpopulations in the TIL cells obtained from culture in the nanomatrix group and the control group.

FIG. 42 shows the cell killing ability of the TIL cells obtained from culture in the nanomatrix group and the control group.

FIG. 43 shows the results of intracellular factor expression assay of TIL cells obtained from culture in the mixed antibody group and the control group.

FIGs. 44, 45, 46, and 47 show the results of intracellular factor expression assay of TIL cells obtained from culture in the magnetic bead group and the control group, respectively, for TILs from different donor sources.

FIG. 48 shows the results of intracellular factor expression assay of TIL cells obtained from culture in the nanomatrix group and the control group.

FIG. 49 shows the results of cytokine secretion assay of TIL cells obtained from culture in the nanomatrix group and the control group.

FIG. 50 shows the results of cytokine secretion assay of TIL cells obtained from culture in the nanomatrix group and the control group after co-incubating with tumor cells.

FIGs. 51 and 52 show the results of gene knockout efficiency of TIL cells obtained from culture in the nanomatrix group and the control group, respectively, for TILs from different donor sources.

FIGs. 53, 54, and 55 show the analysis results of the proliferation ability of the test groups upon *in vitro* expansion in different ways in the terminal stimulation stage, respectively, for TILs from different donor sources.

FIGs. 56, 57, and 58 show the serial killing results of TILs after being transduced with the IL-15 of the present application, respectively, for TILs from different donor sources.

FIG. 59 shows that, TIL cells transduced with IL-12 may show stronger tumor volume suppressive ability.

FIG. 60 shows that, TIL cells transduced with IL-15 may show higher and more durable ability of IFN-γ secretion.

FIG. 61A shows the cell proliferation ability of TIL cells obtained by culturing with feeder cells added at 0, 24 or 48 hours after the addition of OKT3 and IL-2.

FIG. 61B shows the proportion of $CD45RA^-CCR7^+$ central memory T cells (Terns) in the TIL cells obtained by culturing with feeder cells added at 0, 24 or 48 hours after the addition of OKT3 and IL-2.

FIG. 61C shows the proportion of TCF1+ stem cell-like T cells in the TIL cells obtained by culturing with feeder cells added at 0, 24 or 48 hours after the addition of OKT3 and IL-2.

FIG. 61D shows the proportion of CD4+CD25+Foxp3+ regulatory T cells (Treg) in the TIL cells obtained by culturing with feeder cells added at 0, 24 or 48 hours after the addition of OKT3 and IL-2.

FIG. 61E shows the proportion of activated T cells (PD-1+) in the TIL cells obtained by culturing with feeder cells added at 0, 24 or 48 hours after the addition of OKT3 and IL-2.

FIG. 61F shows the proportion of CD103+CD39+ tumor-specific T cells in the TIL cells obtained by culturing with feeder cells added at 0, 24 or 48 hours after the addition of OKT3 and IL-2.

FIG. 61G shows the proportion of activated T cells (CD28+) in the TIL cells obtained by culturing with feeder cells added at 0, 24 or 48 hours after the addition of OKT3 and IL-2.

FIG. 61H shows the proportion of activated T cells (41BB+) in the TIL cells obtained by culturing with feeder cells added at 0, 24 or 48 hours after the addition of OKT3 and IL-2.

FIG. 61I shows the proportion of activated T cells (CD25+) in the TIL cells obtained by culturing with feeder cells added at 0, 24 or 48 hours after the addition of OKT3 and IL-2.

FIG. 61J shows the results of intracellular factor expression assay of TIL cells obtained by culturing with feeder cells added at 0, 24 or 48 hours after the addition of OKT3 and IL-2.

FIG. 61K shows the results of cytokine secretion assay of TIL cells obtained by culturing with feeder cells added at 0, 24 or 48 hours after the addition of OKT3 and IL-2.

FIG. 61L shows the proliferation ability of TIL cells obtained by culturing with feeder cells added at 0, 6, 12, 24, 48, 72 hours, or 5 days after the addition of OKT3 and IL-2.

FIG. 61M shows the proportion of CD8+ T cells in the TIL cells obtained by culturing with feeder cells added at 0, 6, 12, 24, 48, 72 hours, or 5 days after the addition of OKT3 and IL-2.

FIG. 61N shows the proportion of CD45RO+CD62L+ T cells in the TIL cells obtained by culturing with feeder cells added at 0, 6, 12, 24, 48, 72 hours, or 5 days after the addition of OKT3 and IL-2.

FIG. 61O shows the proportion of NK T cells in the TIL cells obtained by culturing with feeder cells added at 0, 6, 12, 24, 48, 72 hours, or 5 days after the addition of OKT3 and IL-2.

FIG. 61P shows the proportion of CD4+CD25+Foxp3+ regulatory T cells (Treg) in the TIL cells obtained by culturing with feeder cells added at 0, 6, 12, 24, 48, 72 hours, or 5 days after the addition of OKT3 and IL-2.

FIG. 61Q shows the cell killing ability of TIL cells obtained by culturing with feeder cells added at 48 hours after the addition of OKT3 and IL-2.

## DETAILED DESCRIPTION

The implementation of the present application will be illustrated below by specific examples, and other advantages and effects of the present application will be easily known by those familiar with the art from the contents disclosed in the specification.

## Definition of terms

In the present application, the term "interleukin" generally refers to a cytokine. For example, the interleukin can activate and regulate immune cells, mediate the activation, proliferation or differentiation of T cells and B cells, and may play an important role in the inflammatory response. In the present application, the interleukin may encompass unprocessed interleukin, any forms of processed interleukin, variants of interleukin or substances comprising functionally active fragments of the interleukin.

In the present application, the term "interleukin-15" or "IL-15" generally refers to one of cytokines. For example, IL-15 can be found in GenBank Accession No. P40933. The IL-15 protein of the present application may also encompass functionally active fragments thereof, not limited to substances comprising functionally active fragments of IL-15 produced from processing and/or modification occurring in cells. For example, the IL-15 of the present application may comprise functionally active fragments of IL-15 and any other domains.

In the present application, the term "IL-15 domain" generally refers to a domain comprising the functionally active fragments of IL-15. For example, the IL-15 domain of the present application may encompass functionally active fragments of IL-15 and variants thereof. For example, the substances comprising the IL-15 domain of the present application may have the function of IL-15 to regulate immune cells.

In the present application, the term "IL-15Ra extracellular domain" generally refers to the extracellular portion of a receptor of IL-15. For example, the receptor of IL-15 may be IL-15Ra. For example, IL-1 5Ra can be found in GenBank Accession No. Q13261. For example, IL-15Ra extracellular domain may comprise the functionally active fragments of IL-15Ra, not limited to substances comprising functionally active fragments of IL-15Ra produced from processing and/or modification occurring in cells. For example, the IL-15Ra extracellular domain of the present application may have the function of binding to IL-15 or fragments thereof.

In the present application, the term "transmembrane domain" generally refers to the portion of the transmembrane domain that spans the cell membrane. For example, the portion in the cell membrane may be, e.g., transmembrane regions of various polypeptides. The fragments of the transmembrane domain may comprise functionally active fragments, truncated bodies and/or mutant variants of a wild-type transmembrane domain. For example, the transmembrane domain of the present application may have a function of enabling substances comprising the transmembrane domain bind to the cell membrane.

In the present application, the term "intracellular domain" generally refers to the intracellular portion located inside the cell membrane. For example, the intracellular domain of the present application may comprise functionally active fragments, truncated bodies and/or mutant variants of a wild-type intracellular domain. For example, the intracellular domain of the present application may not or may have any intracellular signaling function. For example, the intracellular domain of the present application may have the effect of enabling, maintaining and/or promoting the overall structural stability.

In the present application, the term "signal peptide domain" generally refers to a short peptide chain that directs the transfer of newly synthesized proteins to the secretory pathway. For example, the signal peptide domain of the present application may comprise functionally active fragments, truncated bodies and/or mutant variants of a wild-type signal peptide domain. For example, the signal peptide domain of the present application may have a function of directing the transmembrane transfer of substances comprising the signal peptide domain. For example, the signal peptide domain of the present application may bind to signal peptide binding substances, allowing all or part of the substance comprising the signal peptide domain to be transferred to the extracellular region outside the cell membrane.

In the present application, the term "CD80" generally refers to a cell stimulating molecule. For example, CD80 may be the ligand of CD28. For example, CD80 can be found in GenBank Accession No. P33681. The CD80 protein of the present application may also encompass functionally active fragments thereof, not limited to substances comprising the functionally active fragments of CD80 produced from processing and/or modification occurring in the cells. For example, the CD80 of the present application may comprise functionally active fragments of CD80 and any other domains.

In the present application, the term "CD86" generally refers to a cell stimulating molecule. For example, CD86 may be the ligand of CD28. For example, CD86 can be found in GenBank Accession No. P42081. The CD86 protein of the present application may also encompass functionally active fragments thereof, not limited to substances comprising the functionally active fragments of CD86 produced from processing and/or modification occurring in the cells. For example, the CD86 of the present application may comprise functionally active fragments of CD86 and any other domains.

In the present application, the term "membrane-anchored" generally means that one substance may exist in a form that is localized to the cell membrane. For example, a membrane-anchored substance may mean that the substance may be distributed on the cell membrane. For example, a membrane-anchored substance may be partially or fully embedded in the cell membrane or on both the inner and outer sides. For example, the membrane-anchored substances may penetrate the cell membrane, the membrane-anchored substances may bind to the components of the cell membrane through ionic bonding, hydrogen bonding or other interactions, and the membrane-anchored substances may also interact with other substances and bind to the membrane indirectly. For example, whether a substance is a membrane-anchored substance can be tested by flow cytometry or other methods for detecting cells. For example, a membrane-anchored substance may be present in a membrane-anchored form for a period of time and/or in a certain proportion.

In the present application, the term "secretion" generally means that a substance may be localized to the extracellular part of the cell. For example, the secreted substance may be synthesized inside the cell and then transported to the extracellular space of the cell. For example, whether a substance is a secreted substance may be tested by enzyme-linked immunosorbent assay or other detection methods.

In the present application, the term "viral vector" generally refers to a virus that can deliver the nucleic acid. For example, a viral vector can be used to infect a target cell and deliver the target nucleic acid into the target cell, enabling the target cell to express the gene encoded by the target nucleic acid. For example, the viral vector may be a lentiviral vector.

In the present application, the term "retroviral vector" generally refers to a vector derived from at least a portion of the retroviral genome. For example, a retroviral vector can convert RNA to cDNA under the action of reverse transcriptase and, upon infection of a target cell, can express one or more transgenes contained within a heterologous nucleic acid sequence in the target cell. For example, a retroviral vector can be used clinically, also including, but not limited to, a lentiviral vector. In the present application, the term "lentiviral vector" generally refers to a vector derived from at least a portion of the lentiviral genome. For example, the lentiviral envelope protein may comprise a gene encoding an env protein or a part thereof. For example, a host cell can be transfected with a transfer vector and one or more packaging vectors to produce a virus that can be used to infect a target cell to express one or more transgenes contained within a heterologous nucleic acid sequence in the target cell.

In the present application, the term "introduction" generally refers to the incorporation of a nucleic acid into a eukaryotic or prokaryotic cell. For example, introduction may refer to the incorporation of a heterologous or isolated nucleic acid into a eukaryotic or prokaryotic cell, where the nucleic acid can be incorporated into the genome (e.g., chromosomal, plasmid, plastid, or mitochondrial DNA) of a cell, and converted into an autonomous replicon or expressed transiently (e.g., transfected mRNA). For example, introduction can be achieved by "infection", "transfection", "conversion" and "transduction". For example, a nucleic acid can be introduced into a prokaryotic cell by a variety of methods, including electroporation, calcium phosphate precipitation, lipid mediated, and viral vectortransduced transfection.

In the present application, the term "enhanced expression" generally refers to the enhanced expression of the product. For example, the amount of the product synthesized from the cells is enhanced. For example, when the substance is a secreted substance, the amount of the substance secreted from the cells is enhanced. For example, when the substance is a membrane-anchored substance, the proportion of cells comprising the substance in the cell membrane is enhanced, or alternatively, the amount of the substance comprised in the cell membrane is enhanced.

In the present application, the term "activity" generally refers to a biological function of a substance. For example, the activity of cytokine may refer to its ability to affect the activation, proliferation or differentiation of cells.

In the present application, the term "T cell receptor" or "TCR" generally refers to a complex of membrane proteins involved in the activation of T cells in response to antigen presentation. TCRs may be responsible for recognizing antigens bound to major histocompatibility complex molecules. TCRs may be composed of heterodimers of alpha ($\alpha$) and beta ($\beta$) chains, or of gamma and delta ($\gamma/\delta$) chains. TCRs may exist in $\alpha/\beta$ and $\gamma/\delta$ forms, which are structurally similar but have distinct anatomical locations and functions. For example, TCRs may be TCRs modified on any cells expressing TCRs. For example, the type of TCR can be analyzed with TCR subtype analysis reagents.

In the present application, the term "clonal diversity" generally means that a certain substance has multiple clonotypes. For example, the clonal diversity of a TCR may mean that the TCR may have different sequence structures and/or antigen recognition abilities. For example, the diversity of TCRs is often distinguished by $\beta$-chain subtypes, which can comprise V$\beta$ 23, V$\beta$ 7.2, V$\beta$ 5.2, V$\beta$ 11, V$\beta$ 16, V$\beta$ 3, etc. When one T-cell population has more $\beta$-chain subtypes, the T-cell population can be considered to have a higher clonal diversity.

In the present application, "CD4$^+$ cells" generally refers to CD4 positive cells, e.g., T cells. The terms "CD4$^+$ cells" and "CD4 positive cells" may be used synonymously. These cells can be identified by methods known in the art, for example, by staining the cells with a fluorescently labeled antibody against CD4 and using fluorescence activated cell sorting. For example, it has demonstrated from existing data that an increase in the proportion of CD4$^+$ cells can lead to an increase in the ability of the cell population to secrete IFN-$\gamma$ and/or TNF and can improve the effect of the T cell population on promoting the tumor suppression. For example, see Tay, R. E., Richardson, E. K. et, al. (2020). Cancer Gene Therapy, 1-13. However, there is a lack of a method to increase the proportion of CD4$^+$ cells in the field, and the present application may provide a method to affect the proportion of CD4$^+$ cells.

In the present application, "CD8$^+$ cells" generally refers to CD8 positive cells, e.g., T cells. The terms "CD8$^+$ cells" and "CD8 positive cells" may be used synonymously. These cells can be identified by methods known in the art, for example, by staining the cells with a fluorescently labeled antibody against CD8 and using fluorescence activated cell sorting.

In the present application, the term "IC$_{50}$ value" or "IC50 value" generally refers to the concentration of a target required to obtain 50% inhibition of a biological process. The IC50 value can be converted to an absolute inhibition constant (Ki) using the Cheng-Prusoff equation (Biochem. Pharmacol. (1973) 22:3099).

In the present application, the term "K$_D$ value" or "KD value" generally refers to the dissociation constant, which can be determined by surface plasmon resonance. Generally, surface plasmon resonance analysis uses the BIAcore system (Pharmacia Biosensor, Piscataway, NJ) to measure real-time binding interactions between a ligand (a substance immobilized on a biosensor substrate) and an analyte (a substance in a solution) via surface plasmon resonance (SPR). Surface plasmon analysis can also be performed by immobilizing the analyte (substances on the biosensor substrate) and presenting the ligand.

In the present application, the term "encoding" generally refers to the ability to infer, directly or indirectly, information about the structure or composition of one type of molecule from information about the structure or composition of another type of molecule that is related to it, based on essentially defined rules. For example, the nucleotide sequence of an amino acid can be inferred from its sequence, for example, based on the properties of deoxyribonucleic acid-transcribed complementary nucleic acids, including those that can be translated into polypeptides. For example, deoxyribonucleic acids can encode RNAs transcribed from the deoxyribonucleic acids. Deoxyribonucleic acids can similarly encode polypeptides translated by RNAs that are transcribed from the deoxyribonucleic acids.

In the present application, the term "small molecule compounds" generally refers to peptides, peptidomimetics, amino acids, amino acid analogues, polynucleotides, polynucleotide analogues, nucleotides, nucleotide analogues, organic or inorganic substances of molecular weight less than about 10,000 g/mol (i.e., including heterologous organic substances and organometallic compounds), organic or inorganic substances of molecular weight less than about 5,000 g/mol, organic or inorganic substances of molecular weight less than about 1,000 g/mol, organic or inorganic substances of molecular weight less than about 500 g/mol, as well as salts, esters and other pharmaceutically acceptable forms of such drugs.

In the present application, the term "NK cell", also referred to as "natural killer cell", generally refers to a cell with large granules in the cytoplasm. NK cells are developed from bone marrow lymphoid stem cells and can differentiate and develop depending on the bone marrow or thymus microenvironment. In the present application, the proportion of NK cells in TIL cells can be altered by the methods of the present application.

In the present application, the term "antibody" generally refers to an immunoglobulin or fragments or derivatives thereof, including any polypeptides comprising antigen binding sites, no matter produced *in vitro* or *in vivo*. The term comprises, but not limited to, polyclonal, monoclonal, mono-specific, multi-specific, non-specific, humanized, single chain, chimeric, synthetic, recombinant, hybrid, mutant and transplanted antibodies. Unless otherwise modified by the term "intact", such as in the "intact antibody", for the purpose of the present application, the term "antibody" also comprises antibody fragments, such as Fab, F(ab')2, Fv, scFv, Fd, dAb and other antibody fragments that maintain the antigen binding function (e.g., specifically binding to CD3). Generally, such fragments should comprise antigen binding domains. A basic 4-chain antibody unit is heterotetrameric glycoprotein consisting of two identical light (L) chains and two identical heavy (H) chains. An IgM antibody consists of 5 basic heterotetrameric unit and an additional polypeptide known as J-chain and contains 10 antigen binding sites; while an IgA antibody consists of 2-5 basic 4-chain units that can combine and polymerize with J-chains to form multivalent combinations. With regard to IgG, a 4-chain unit is generally about 150,000 Dalton. Each L chain is linked to an H chain through a covalent disulfide bond, while two H chains are linked to each other through one or more disulfide bonds depending on the isotype of the H chain. Each of H and L chains also has regularly spaced intra-chain disulfide bridges. Each H chain has a variable domain (VH) at the N-terminus, which is followed by three constant domains (CHs) for each of $\alpha$ and $\gamma$ chains or followed by four CH domains for $\mu$ and $\epsilon$ isotypes. Each L chain has a variable domain (VL) at the N-terminus and has a constant domain at the other terminus. VL corresponds to VH, and CL corresponds to the first constant domain (CH1) of the heavy chain. Specific amino acid residues are considered to form an interface between the light chain and heavy chain variable domains. VH is paired with VL to form a single antigen-binding site. L chains from any vertebrate species can be classified into one of two distinct types based on the amino acid sequences of their constant domains, called kappa and lambda. Depending on the amino acid sequences of its heavy chain (CH) constant domain, immunoglobulin can be classified into different types or isotypes. There are currently five types of immunoglobulin: IgA, IgD, IgE, IgG and IgM, which have heavy chains named $\alpha$, $\delta$, $\epsilon$, $\gamma$ and $\mu$, respectively.

In the present application, the term "antigen binding fragments" generally refers to one or more polypeptide fragments having the ability of specifically binding to an antigen (e.g., CD3). In the present application, the antigen binding fragments may comprise Fab, Fab', F(ab)$_2$, Fv fragments, F(ab')$_2$, scFv, di-scFv, and/or dAb.

In the present application, the term "solid medium" or "medium" generally refers to a solid material having the binding function. For example, the solid medium of the present application may refer to a material which binds one or more substances within the medium and/or on the surface of the medium by covalent binding and/or non-covalent binding. For example, the solid medium of the present application may refer to a material which binds CD28 antibody or antigen-binding fragments thereof and CD3 antibody or antigen-binding fragments thereof within the medium and/or on the surface of the medium by covalent binding and/or non-covalent binding. For example, the solid medium of the present application may be a polymeric material.

In the present application, the term "expression" generally refers to the process of transcription and/or translation of genes encoding a target polypeptide that occurs within a cell. The transcription level of the genes encoding the target polypeptide in the host cell can be determined by measuring the amount of corresponding mRNA present in the cell. For example, mRNA transcribed from the genes encoding the target polypeptide can be quantitatively measured by PCR or by RNA hybridization. The translation level of the genes encoding the target polypeptide can be measured by a variety of methods, such as by ELISA, by polypeptide bioactivity assays, or by Western blotting or radioimmunoassay. In the present application, the term "expression" may also generally refer to the transcriptional and/or translational processes that occur in the product. For example, the expression of cytokine may be the processes of transcribing and/or translating the cytokine by cells. For example, the expression of cytokine can be determined by detecting the amount of corresponding mRNA present in the cells or by detecting the amount of the cytokine produced by the cells, or both.

In the present application, the "stage" in the terms "one stage *of in vitro* expansion", "a single stage *of in vitro* expansion", or "the first stage *of in vitro* expansion", etc. generally refers to a process of expansion that TILs are subjected to *in vitro*. In one embodiment, each stage can be divided depending on the change in the number of TIL cells. In one embodiment, when the number of the TIL cells is increased by at least about 1-fold, it can be considered that the TIL cells enter the next stage *of in vitro* expansion. In some embodiments, when the number of the TIL cells is increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold, it can be considered that the TIL cells enter the next stage *of in vitro* expansion. In one embodiment, each stage can also be divided by the culture conditions of the TIL cells. In one embodiment, after T cell activators and/or T cell growth factors are added or supplemented into the cell culture medium, it can be considered that the TIL cells enter the next stage of *in vitro* expansion. In one embodiment, after TIL cells have been centrifuged and/or washed, it can be considered that the TIL cells enter the next stage *of in vitro* expansion. In one embodiment, each stage can also be divided by the culture days of the TIL cells. In one embodiment, after the TIL cells have been cultured *in vitro* for about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 30 days, about 40 days, about 50 days, or about 100 days, it can be considered that the TIL cells enter the next stage *of in vitro* expansion.

In the present application, the term "the first stage *of in vitro* expansion" generally refers to a stage of expansion using T cell growth factors after primary TILs are obtained from tissues. In one embodiment, the tissues in the present application may be one or more tissues selected from the group consisting of tumor tissues and pleural effusion, and the pleural effusion in the present application may be pleural effusion from patients with metastatic carcinomas. In one embodiment, the expansion in the present application may be autologous or allogeneic in vivo expansion, or it may be *in vitro* expansion. The first stage of *in vitro* expansion in the present application can also be referred to as a preREP (pre-rapid expansion protocol) stage. For example, TILs derived from tumor tissues and not expanded *in vitro* can be referred to as the first TIL population. For example, the TILs obtained after the first stage of *in vitro* expansion in the two-step culture method of the present application can be referred to as the second TIL population.

In the present application, the term "the second stage *of in vitro* expansion" generally refers to a stage of expansion again, after a tissue has been removed from a subject and expanded. In one embodiment, compared to the TILs subjected to the first stage *of in vitro* expansion, the number of the TIL cells subjected to the second stage *of in vitro* expansion is increased, e.g., it may be increased by at least about 10-fold (or at least about 20, 30, 40, 50, 60, 70, 80 or 90-fold), or in one embodiment, the cell number may be increased by at least about 100-fold. In one embodiment, the second and the first stages *of in vitro* expansion may be different in culture conditions, e.g., the culture substances added may be different. For example, the second stage of *in vitro* expansion in the two-step culture method of the present application can also be referred to as a REP (rapid expansion protocol) stage. For example, the TILs obtained after the second stage *of in vitro* expansion in the two-step culture method of the present application can be referred to as the third TIL population.

In the present application, the term *"in vivo"* generally refers to an event that occurs in the body of a subject.

In the present application, the term *"in vitro"* generally refers to an event that occurs outside the body of a subject.

In the present application, the term *"ex vivo"* generally refers to an event that involves a treatment or surgery on cells, tissues and/or organs which have been removed from a subject. In one embodiment, the cells, tissues and/or organs can be returned into the subject's body by a surgery or treatment method.

In the present application, the term "secretion" generally refers to the transfer of expressed polypeptide or protein by cells to the extracellular environment.

In the present application, the term "secretion capacity" generally refers to the ability of a cell to express a polypeptide or protein and transfer the polypeptide or protein of the present application to the extracellular environment.

In the present application, the term "irradiation" generally refers to the treatment of a substance by means of radiation. For example, in one embodiment, irradiation may refer to irradiating a substance with X-rays, alpha rays, beta rays, or gamma rays.

In the present application, the term "engineered cells" generally refers to cells which have been genetically modified by adding additional genetic material in the form of DNA or RNA to the total genetic material of the cells. In one embodiment, the engineered cells can be TILs genetically modified to express the T cell activators and/or T cell growth factors of the present application.

In the present application, the term "co-culturing" generally refers to culturing two or more different populations of cells with some degree of contact between them. The "contact" between the two or more different populations of cells of the present application, in one embodiment, can be through direct contact, i.e., the cells of one population are directly physically contacted with the cells of another population. Alternatively, in one embodiment, the contact can be through indirect contact mediated by sharing a culture medium. The shared culture medium in the present application may contain metabolites produced and released by at least one population of the co-cultured cells and is used to culture the cells of another population.

In the present application, the term "contacting" generally means that two or more substances of different types are brought into contact together in any order, in any manner, and for any length of time. In one embodiment, the contact can be through direct contact, for example, one or more feeder cells, T cell activators and/or T cell growth factors can be added into the culture medium of the TIL cells; for example, a culture medium comprising one or more feeder cells, T cell activators and/or T cell growth factors can be added into and/or used to replace the culture medium of the TIL cells; for example, a culture medium comprising one or more feeder cells, T cell activators and/or T cell growth factors can be used for the culture of the TIL cells. In one embodiment, the contact can be through indirect contact, for example, metabolites produced and released by feeder cells can be used to culture the TIL cells.

In the present application, the term "mixture" generally refers to a combination of two or more different substances. For example, the CD28 antibody or antigen-binding fragments thereof and the CD3 antibody or antigen-binding fragments thereof in the present application may be added into the cell culture medium as a mixture after mixing.

In the present application, the terms "simultaneous contact", "concurrent contact", "while contacting with", "simultaneously" and "concurrently" generally refer to the administration of two or more substances to a subject or cells such that the substances are present in the subject and/or the environment in which the cells are cultured at the same time. Simultaneous contact can comprise administration of different compositions at the same time, administration of different compositions at different times, or administration of a composition in which two or more active pharmaceutical ingredients are present. For example, "simultaneous contact" in the present application generally may refer to contacting substantially simultaneously.

In the present application, the term "expansion" generally refers to a several-fold increase in the number of cells over a period of time. In one embodiment, the cell number can be increased by at least about 3-fold (or 4, 5, 6, 7, 8 or 9-fold). In one embodiment, the cell number can be increased by at least about 10-fold (or 20, 30, 40, 50, 60, 70, 80 or 90-fold). Alternatively, in one embodiment, the cell number can be increased by at least about 100-fold. In the present application, the term "expanded" generally means that the cells of the present application have been subjected to one or more of the expansions described above.

In the present application, the term "polymer" generally refers to a molecule composed of individual chemical moieties linked together, which moieties can be the same or different in the present application. In one embodiment, the term "polymer" may refer to individual chemical moieties linked tail to tail to form a linear molecule, as well as individual chemical moieties linked together in the form of branched (e.g., "multi-arm" or "star") structures. In one embodiment, a polymer may comprise, for example, polysaccharide, glucan, hydrogel, polyethylene glycol, or poloxamer. Poloxamer is a nonionic triblock copolymer with a central hydrophobic chain of polyoxypropylene (polypropylene oxide)) and two pendant hydrophilic chains of polyoxyethylene (polyethylene oxide)). The substances encompassed in the present application may be formulated with, or administered with, any polymers described herein or known in the art.

In the present application, the term "chimeric antibody" generally refers to an antibody that is formed from the fusion of the variable region of a mouse-derived antibody with the constant region of a human antibody, which can reduce the immune response induced by the mouse-derived antibody. Chimeric antibodies are established so that a hybridoma that secretes murine-derived specific monoclonal antibodies can be established and variable region genes are then cloned from the murine hybridoma cells, constant region genes of the human antibody can be cloned as needed, murine variable region genes are ligated with human constant region genes to form chimeric genes which are then inserted into expression vectors, and the chimeric antibody molecules can be expressed in eukaryotic or prokaryotic systems.

In the present application, the term "humanized antibody", also referred to as CDR-grafted antibody, generally refers to an antibody produced by grafting the murine CDR sequences into the human antibody variable region frameworks, that is, an antibody produced in different types of human germline antibody framework sequences. Humanized antibodies can overcome heterologous responses induced by chimeric antibodies which carry a large number of murine protein components. Such framework sequences can be obtained from public DNA database covering germline antibody gene sequences or published references. For example, germline DNA sequences of human heavy and light chain variable region genes can be found in "VBase" human germline sequence database.

In the present application, the term "fully humanized antibody", "fully human antibody" or "wholly humanized antibody", also referred to as "fully humanized monoclonal antibody" means that both the variable and constant regions of the antibody can be of human origin, with immunogenicity and toxicities removed. The development of monoclonal antibodies has gone through four stages, namely: murine-derived monoclonal antibodies, chimeric monoclonal antibodies, humanized monoclonal antibodies and fully humanized monoclonal antibodies. The antibodies or ligands of the present application may be fully humanized monoclonal antibodies. The relevant technologies for preparing fully human antibodies may be human hybridoma technology, EBV transformed B lymphocyte technology, phage display technology, transgenic mouse antibody preparation technology, single B cell antibody preparation technology, and the like.

In the present application, the term "CDR" generally refers to one of the six hypervariable regions within the variable domain of an antibody that are primarily responsible for promoting the antigen binding. One of the most commonly used definitions of the six CDRs may be provided by Kabat E.A. et, al., Chothia et, al., and MacCallum et, al. As used in the present application, the Kabat definition of CDRs can be applied to CDR1, CDR2 and CDR3 of the light chain variable domain (CDR L1, CDR L2, CDR L3 or L1, L2, L3) and CDR1, CDR2 and CDR3 of the heavy chain variable domain (CDR H1, CDR H2, CDR H3 or H1, H2, H3).

In the present application, the term "anti-CD3 antibody" generally refers to an antibody targeting CD3 or variants thereof, for example, monoclonal antibodies, including human, humanized, chimeric or murine antibodies, which are directed against CD3 receptors in the T cell antigen receptors of mature T cells. Anti-CD3 antibodies may comprise OKT-3. Anti-CD3 antibodies may comprise SP34. Anti-CD3 antibodies may also comprise other anti-CD3 antibodies, including, e.g., in one embodiment, otelixizumab, teplizumab, and visilizumab.

In the present application, the term "IL-2" or "IL2" generally refers to T cell growth factors known as interleukin 2 and comprises all forms of IL-2, which may comprise, in one embodiment, human and mammalian forms, conservative amino acid substitutions, glycoform modifications or variants, or active fragments thereof. The GeneID encoding IL-2 genes may be 3558.

In the present application, the term "antigen-presenting cells" or "APCs" generally refers to immune system cells, such as helper cells (e.g., B cells, dendritic cells, etc.), that display exogenous antigens complexed with major histocompatibility complexes (MHCs) on their surface. T cells can recognize these complexes using their T cell receptors (TCRs). APCs can process antigens and present them to T cells. In one embodiment, the antigen-presenting cells may comprise one or more cells selected from the group consisting of peripheral mononuclear cells, dendritic cells, and artificial antigen-presenting cells.

In the present application, the term "TIL properties" generally refers to the properties of TIL cells obtained by the culture method of the present application. The changes in TIL properties may comprise increased number of TIL cells, increased proportion of viable cells, enhanced persistence, improved proportion of T cell subpopulations, enhanced cytokine secretion capacity, enhanced tumor cell killing ability, enhanced T cell receptor (TCR) clonal diversity, and increased number of TIL cells in tissues, or any combinations thereof. The changes in the present application may be improvement or decreasement.

In the present application, the term "persistence" generally refers to the existence of cells in a subject. For example, the enhanced persistence of TIL cells may refer to an increase in the time that TIL cells are present in the body. For example, the enhanced persistence may refer to an increase in the time that the cells are present in the tissues of the subject, such as tumors, spleen, bone marrow, lung tissues, and blood.

In the present application, the term "nanoparticles" generally refers to microscopic particles with at least one dimension less than 100 nm. Generally, nanoparticles have diameters ranging from 50 nm to 500 nm (i.e., from 0.05 $\mu$m to 0.5 $\mu$m); are structurally stable in the physiological environment; and can accommodate smaller molecules (e.g., drugs or other biologically active agents) that can then be delivered to the desired sites. For example, the nanoparticles in the present application may comprise CD28 antibodies or antigen-binding fragments thereof. For example, the nanoparticles in the present application may comprise CD28 antibodies or antigen-binding fragments thereof and CD3 antibodies or antigen-binding fragments thereof. For example, the anti-CD3 antibodies may comprise OKT3. For example, the anti-CD28 antibodies may comprise 15E8.

In the present application, the term "artificial antigen-presenting cells" generally refers to immune cells artificially constructed for the presentation of exogenous antigens, e.g., the presentation of exogenous antigens may be by means of comprising a complex of the exogenous antigen with a major histocompatibility complex (MHC) on the surface of an artificial antigen-presenting cell. In one embodiment, isolated artificial antigen-presenting cells (aAPCa) can be comprised, which may comprise cells expressing HLA-A/B/C (the GeneID of the gene encoding it can be 3105, 3106 or 3107), CD64 (the GeneID of the gene encoding it can be 2209), CD80 (the GeneID of the gene encoding it can be 941), ICOS-L (the GeneID of the gene encoding it can be 23308) and CD58 (the GeneID of the gene encoding it can be 965), and can be modified to express more than one T cell activator, with the "more than" in the present application being inclusive.

In the present application, the term "fusion protein" generally refers to a polypeptide or protein comprising the amino acid sequence of a first polypeptide or protein or fragments thereof, analogues or derivatives and the amino acid sequence of a heterologous polypeptide or protein (i.e., a second polypeptide or protein or fragments, analogues or derivatives thereof different from the first polypeptide or protein or fragments, analogues or derivatives thereof, or usually not a part of the first polypeptide or protein or fragments, analogues or derivatives thereof). In some cases, fusion protein may comprise prophylactic or therapeutic drugs fused with heterologous protein, polypeptide or peptide. Where, the heterologous protein, polypeptide or peptide of the present application may be or may not be different types of prophylactic or therapeutic drugs. For example, two different proteins, polypeptides or peptides with immunomodulatory activity can be fused together to form a fusion protein. In some cases, the fusion protein may maintain or have increased activity compared to the activity of the initial polypeptide or protein prior to the fusion of heterologous protein, polypeptide or protein. For example, the fusion protein of the present application may be a fusion protein incorporating a CD28 antibody or antigen-binding fragment thereof and a CD3 antibody or antigen-binding fragment thereof.

In the present application, the term "killing ability" generally refers to the ability to kill target cells by contacting the cells of the application with an effective amount of substances. In one embodiment, the substances of the present application may be TIL cells. The killing in the present application may comprise killing cells by itself or by promoting CDC, apoptosis, ADCC, and/or phagocytosis of other cells or substances, or by a combination of two or more of these mechanisms.

In the present application, the term "administering" generally refers to the delivery of a substance to a subject in need thereof by any route known in the art. Pharmaceutically acceptable carriers and formulations or compositions are also well known in the art. Routes of administration may comprise intravenous, intramuscular, intradermal, subcutaneous, transdermal, mucosal, intratumoral, and/or mucosal.

In the present application, the term "kit" generally refers to two or more components packaged together in a container, a receptacle, or other containers, one of which corresponds to the substance of the present application. For example, the TIL cells of the present application are comprised.

In the present application, the term "subject" generally refers to cells or animals, which may be mammals, such as human, non-human primates (apes, gibbons, gorillas, chimpanzees, orangutans, macaques), domestic animals (dogs and cats), farm animals (poultry such as chickens and ducks, horses, cows, goats, sheep, pigs), and laboratory animals (mice, rats, rabbits, guinea pigs). Human subjects comprise fetal, neonatal, infant, adolescent, and adult subjects. Subjects comprise animal disease models, such as tumor animal models, and other animal models known to those of skill in the art.

In the present application, the term "feeder cells" generally refers to culture cells that can be used to support the growth of another type of cells of interest. For example, at least one factor can be secreted to the culture medium by *in vitro* growth. In one embodiment, feeder cells may comprise antigen-presenting cells.

In the present application, the term "specifically binding" generally refers to a binding substance that recognizes a specific target substance but does not substantially recognize or bind other molecules in the sample. For example, if a binding substance can specifically bind the specific target substance of the present application from one species, the binding substance of the present application can also specifically bind the target substance of the present application or homologous target substances thereof from one or more other species. This interspecific reactivity by itself may not change the classification of the binding substance as specific. In some cases, a binding substance that specifically binds to a target substance may also bind to a different allelic form of the target substance.

In the present application, the term "the complete culture process" generally refers to the complete process of isolating cells from isolated tumor tissues in the patient, and then subjecting to one or more expansions to finally obtain cells that can be administered to the subj ect.

In the present application, the term "cell culture medium" generally refers to a nutrient solution in which cells, such as mammalian cells, are grown. The formulation of cell culture media is well known in the art. Typically, the cell culture media comprise buffer, salts, carbohydrates, amino acids, vitamins, and essential trace elements. The cell culture media may or may not contain sera, peptone, and/or protein. The cell culture media may be supplemented with additional components or increased concentrations of components, such as amino acids, salts, sugars, vitamins, hormones, growth factors, buffers, antibiotics, lipids, trace elements, etc., depending on the requirements of the cells to be cultured and/or the desired cell culture parameters.

In the present application, the term "pharmaceutical composition" or "pharmaceutical formulation" generally refers to a preparation that allows the biological activity of the active ingredient to be effective and may not contain additional components that are unacceptably toxic to the subject to whom the formulation is to be administered. Such formulations are sterile. "Pharmaceutically acceptable" excipients (carriers, additives) are those which can reasonably be administered to a test mammal to provide an effective dose of the active ingredient employed.

In the present application, the term "tumor-infiltrating lymphocytes" or "TILs" generally refers to a population of cells initially acquired as leukocytes, which in the present application have left the bloodstream of the subject and migrated into the tumor. TILs may comprise, but not limited to, CD8$^+$ cytotoxic T cells (lymphocytes), Th1 and Th17 CD4$^+$ T cells, natural killer cells, dendritic cells and M1 macrophages. TILs may comprise primary TIL and secondary TILs. The "primary TILs" may be those TIL cells obtained from the tissue samples of the subject, while the "secondary TILs" may be any TIL populations that have been expanded or expanded in the present application. In some embodiments, the tumor-infiltrating lymphocytes of the present application may have not been isolated and purified or may be infiltrated with tumor cells. In one embodiment, the TILs of the present application may refer to TIL populations.

In the present application, the term "central memory T cells" generally refers to T cells that have long-term memory and can be restimulated by antigens. Central memory T cells may have a phenotype of CD45RA$^-$CCR7$^+$, for example, central memory T cells can be identified by CD45RA$^-$ and CCR7$^+$. Central memory T cells may have a stronger ability to resist tumor growth compared with regular T cells.

In the present application, the term "regulatory T cells" generally refers to a subpopulation of T cells that control autoimmune reactivity in the body. Regulatory T cells may have a phenotype of CD4$^+$CD25$^+$Foxp3$^+$, for example, regulatory T cells can be identified by CD4$^+$, CD25$^+$, and Foxp3$^+$. Regulatory T cells may have an ability to inhibit the anti-tumor growth of T cells.

In the present application, the term "activated T cells" generally refers to T cells that have been activated to have the ability to resist the tumor growth. Activated T cells may have a phenotype of PD1$^+$, LAG3$^+$ or CD28$^+$, for example, activated T cells can be identified by PD1$^+$, LAG3$^+$ or CD28$^+$. Activated T cells may have an ability to resist the tumor growth.

In the present application, the term "tumor-specific T cells" generally refers to T cells that can specifically resist the tumor growth. Tumor-specific T cells may have a phenotype of CD103$^+$CD39$^+$, for example, tumor-specific T cells can be identified by CD103$^+$ and CD39$^+$. Tumor-specific T cells may have a more specific ability to resist the tumor growth compared to regular T cells.

In the present application, the term " stem cell-like T cells" generally refers to a class of T cells that may have the potential for self-proliferation and/or differentiation. Stem cell-like T cells may have a phenotype of TCF1$^+$, for example, stem cell-like T cells can be identified by TCF1$^+$. Tumor-specific T cells may have a stronger and/or longer-term ability to resist the tumor growth compared to regular T cells.

In the present application, the term "tumor debris" generally refers to tumor debris that can be formed by mechanical disruption, enzymatic digestion and/or other disruption methods after the tumor tissue is removed from the subject.

In the present application, the term "composition" or "pharmaceutical composition" generally refers to a mixture of at least one cell as well as at least one and optionally more than one other pharmaceutically acceptable chemical components such as carriers, stabilizers, diluents, dispersing agents, suspending aids, thickening agents and/or excipients.

In the present application, the term "pharmaceutically acceptable carrier" generally refers to one or more nontoxic materials that do not interfere with the active ingredients. For example, the pharmaceutically acceptable carriers may not interfere with the biological activity of the active ingredients; for example, the pharmaceutically acceptable carriers may not interfere with the effectiveness of the biological activity of the active ingredients. Such formulations may conventionally contain salts, buffers, preservatives, compatible carriers, and optionally other therapeutic agents. Such pharmaceutically acceptable formulations may also contain compatible solid or liquid fillers, diluents or encapsulating substances suitable for administration to human. Other contemplated carriers, excipients, and/or additives that can be used in the formulations described herein can comprise, for example, flavoring agents, antimicrobial agents, sweeteners, antioxidants, antistatic agents, lipids, protein excipients (such as serum albumin, gelatin, and casein), salt-forming counterions (such as sodium), and the like. These and other known pharmaceutical carriers, excipients and/or additives suitable for use in the formulations described herein are known in the art. In the present application, the "pharmaceutically acceptable carrier" may be understood as not including vectors in the form of nucleic acid used in genetic engineering.

In the present application, the term "functionally active fragment" generally refers to a fragment having a partial region of a full-length protein or nucleic acid but maintaining or partially maintaining the biological activity or function of the full-length protein or nucleic acid. For example, a functionally active fragment may maintain or partially maintain the ability of the full-length protein to bind another molecule. For example, the functionally active fragment of the growth factor IL-2 may maintain or partially maintain the biologically active function of the full-length IL-2 to cause cell proliferation.

In the present application, the term "T cell activator" generally refers to a substance that binds the corresponding binding receptor on T cells and mediates the costimulatory response of the T cells. T cell activators may be substances other than antigen receptors that are required for T cells to produce effective immune responses. T cell activators may refer to T cell costimulatory molecules. For example, the T cell activators in the present application may comprise variants, homologues thereof or any substances comprising functionally active fragments thereof. T cell activators may comprise, but not limited to, MHC type I molecules, TNF receptor protein, immunoglobulin-like protein, cytokine receptors, integrins, signaling lymphocytic activation molecules (SLAM protein), NK cell activation receptors, BTLA (the GeneID of the gene encoding it can be 151888), Toll ligand receptors, OX40 (the GeneID of the gene encoding it can be 7293), CD2 (the GeneID of the gene encoding it can be 914), CD7 (the GeneID of the gene encoding it can be 924), CD27 (the GeneID of the gene encoding it can be 939), CD28 (the GeneID of the gene encoding it can be 940), CD30 (the GeneID of the gene encoding it can be 943), CD40 (the GeneID of the gene encoding it can be 958), CDS, ICAM-1 (the GeneID of the gene encoding it can be 3383), LFA-1 (CD11a/CD18) (the GeneID of the gene encoding it can be 3689), 4-1BB (CD137) (the GeneID of the gene encoding it can be 3604), B7-H3 (the GeneID of the gene encoding it can be 80381), ICOS (CD278) (the GeneID of the gene encoding it can be 29851), GITR (the GeneID of the gene encoding it can be 8784), BAFFR (the GeneID of the gene encoding it can be 115650), LIGHT (the GeneID of the gene encoding it can be 8740), HVEM (LIGHTR) (the GeneID of the gene encoding it can be 8764), KIRDS2, SLAMF7 (the GeneID of the gene encoding it can be 57823), NKp80 (KLRF1) (the GeneID of the gene encoding it can be 51348), NKp44 (the GeneID of the gene encoding it can be 9436), NKp30 (the GeneID of the gene encoding it can be 259197), NKp46 (the GeneID of the gene encoding it can be 9437), CD19 (the GeneID of the gene encoding it can be 930), CD4 (the GeneID of the gene encoding it can be 920), CD8α (the GeneID of the gene encoding it can be 925), CD8β (the GeneID of the gene encoding it can be 926), IL-2Rβ, IL-2Ry, IL7Rα (the GeneID of the gene encoding it can be ), ITGA4 (the GeneID of the gene encoding it can be 3676), VLA1 (the GeneID of the gene encoding it can be 3672), CD49a (the GeneID of the gene encoding it can be 3672), IA4 (the GeneID of the gene encoding it can be 3732), CD49D (the GeneID of the gene encoding it can be 3676), ITGA6 (the GeneID of the gene encoding it can be 3655), VLA-6 (the GeneID of the gene encoding it can be 3655), CD49f (the GeneID of the gene encoding it can be 3655), ITGAD (the GeneID of the gene encoding it can be 3681), CD11d (the GeneID of the gene encoding it can be 3681), ITGAE (the GeneID of the gene encoding it can be 3682), CD103 (the GeneID of the gene encoding it can be 3682), ITGAL (the GeneID of the gene encoding it can be 3683), CD11a (the GeneID of the gene encoding it can be 3683), LFA-1 (the GeneID of the gene encoding it can be 3683), ITGAM (the GeneID of the gene encoding it can be 3684), CD11b (the GeneID of the gene encoding it can be 3684), ITGAX (the GeneID of the gene encoding it can be 3687), CD11c (the GeneID of the gene encoding it can be 3687), ITGB1 (the GeneID of the gene encoding it can be 3688), CD29 (the GeneID of the gene encoding it can be 3688), ITGB2 (the GeneID of the gene encoding it can be 3689), CD18 (the GeneID of the gene encoding it can be 3689), LFA-1 (the GeneID of the gene encoding it can be 3689), ITGB7 (the GeneID of the gene encoding it can be 3695), NKG2D (the GeneID of the gene encoding it can be 22914), NKG2C (the GeneID of the gene encoding it can be 3822), TNFR2 (the GeneID of the gene encoding it can be 7133), TRANCE/RANKL (the GeneID of the gene encoding it can be 8600), DNAM1 (CD226) (the GeneID of the gene encoding it can be 10666), SLAMF4 (CD244, 2B4) (the GeneID of the gene encoding it can be 51744), CD84 (the GeneID of the gene encoding it can be 8832), CD96(Tactile) (the GeneID of the gene encoding it can be 10225), CEACAM1 (the GeneID of the gene encoding it can be 634), CRTAM (the GeneID of the gene encoding it can be 56253), Ly9 (CD229) (the GeneID of the gene encoding it can be 4063), CD160 (BY55) (the GeneID of the gene encoding it can be 11126), PSGL1 (the GeneID of the gene encoding it can be 6404), CD100 (SEMA4D) (the GeneID of the gene encoding it can be 10507), CD69 (the GeneID of the gene encoding it can be 969), SLAMF6 (NTB-A, Ly108) (the GeneID of the gene encoding it can be 114836), SLAM (SLAMF1, CD150, IPO-3) (the GeneID of the gene encoding it can be 6504), BLAME (SLAMF8) (the GeneID of the gene encoding it can be 56833), SELPLG (CD162) (the GeneID of the gene encoding it can be 6404), LTBR (the GeneID of the gene encoding it can be 4055), LAT (the GeneID of the gene encoding it can be 27040), GADS (the GeneID of the gene encoding it can be 9402), SLP-76 (the GeneID of the gene encoding it can be 3937), PAG/Cbp (the GeneID of the gene encoding it can be 55824), CD19a, and ligands specifically binding to CD3, ligands specifically binding to CD28, ligands specifically binding to HVEM, ligands specifically binding to CD40L, ligands specifically binding to OX40, and ligands specifically binding to 4-1BB. A costimulatory intracellular signaling domain may refer to the intracellular portion of a T cell activator. An intracellular signaling domain may comprise the complete intracellular portion of a molecule from which it is derived or the complete natural intracellular signaling domain or a functional fragment thereof.

In the present application, the term "T cell growth factor" generally refers to a biological active polypeptide or a small molecular compound that causes cell proliferation. For example, the T cell growth factors in the present application may comprise variants, homologues thereof or any substances comprising functionally active fragments thereof. In one embodiment, the T cell growth factors may be one or more factors selected from the group consisting of: IL-2 (the GeneID of the gene encoding it can be 3558), IL-4 (the GeneID of the gene encoding it can be 3565), IL-6 (the GeneID of the gene encoding it can be 3569), IL-7 (the GeneID of the gene encoding it can be 3574), IL-10 (the GeneID of the gene encoding it can be 3586), IL-12 (the GeneID of the gene encoding it can be 3592 or 3593), IL-15 (the GeneID of the gene encoding it can be 3600), IL-21 (the GeneID of the gene encoding it can be 59067), TNF-$\alpha$ (the GeneID of the gene encoding it can be 100137091), interferon $\gamma$ (the GeneID of the gene encoding it can be 3458), etc.

In the present application, the term "substantially simultaneously" generally means that TILs can be in contact with two or more substances simultaneously within a period of time during the contact process but may not be limited to the fact that the TILs are always in contact with the two or more substances simultaneously during the entire contact process. In one embodiment, substantially simultaneously may mean that the TILs can be in contact with at least 10%, 20%, 30%, 40%, 50%, 60%, 70%, 75%, 80%, 85%, 90%, and 95% of each of the two or more substances simultaneously within a period of time.

In the present application, the term "solid medium" or "medium" generally refers to a solid phase material with a binding function. For example, the solid medium of the present application may refer to a material that binds one or more substances in the medium and/or on the surface of the medium through covalent binding and/or non-covalent binding actions. For example, the solid medium of the present application may bind one or more T cell activators. For example, the solid medium of the present application may refer to a material that binds CD28 antibody or an antigen-binding fragment thereof and CD3 antibody or an antigen-binding fragment thereof in the medium and/or on the surface of the medium through covalent binding and/or non-covalent binding actions. For example, the solid medium of the present application may be microspheres including OKT3 antibodies and 15E8 antibodies and having a diameter from about 500 nm to about 10 $\mu$m. For example, the solid medium of the present application may be polymeric materials. For example, the solid medium of the present application may be microspheres having a diameter of at least about 500 nm. For example, the solid medium of the present application may be nanomatrix. For example, the solid medium of the present application may be nanomatrix including OKT3 antibodies and 15E8 antibodies and having a diameter from about 1 nm to about 500 nm.

In the present application, the term "nanomatrix" generally refers to a material having a diameter from about 1 nm to about 500 nm. In the present application, the nanomatrix may have a binding function, for example, the nanomatrix in the present application may bind one or more T cell activators. In the present application, the nanomatrix may comprise polymers, for example, the nanomatrix in the present application may comprise degradable polymers. In the present application, the nanomatrix may comprise polysaccharide and/or glucan.

In the present application, the term "dendritic cell" generally refers to antigen-presenting cells that are present *in vivo*, *in vitro*, *ex vivo* or in the host or subject, or can be derived from hematopoietic stem cells or monocytes. Dendritic cells and precursors thereof may be isolated from various lymphoid organs such as spleen, lymphatic gland and bone marrow, and peripheral blood. The dendritic cells in the present application may have characteristic morphology, such as thin layers (lamellipodia) extending in multiple directions of the dendritic cell body. Generally, dendritic cells can express high levels of MHCs and costimulatory (e.g., B7-1 and B7-2) molecules. Dendritic cells can induce antigen-specific differentiation of T cells *in vitro* and can elicit primary T cell responses *in vitro* and *in vivo.*

In the present application, the term "*in vitro* expansion" generally refers to changes in the number of cells resulting from culture, but the expanded cells may also subj ect to changes in the number and/or proportion of cells, changes in secretion capacity, changes in killing ability or changes in expression ability, or any combination thereof. The changes in the present application may be improvement or decreasement. In the present application, *in vitro* expansion may be for the purpose of expansion, or for detecting the function of TIL cells, e.g., for detecting the ability of TIL cells to release cytokines. However, the operation steps performed on TIL cells (e.g., adding one or more substances to the culture medium of TIL cells to detect the ability of TIL cells to release cytokines) may not belong to the *in vitro* expansion of the present application.

In the present application, the term "peripheral mononuclear cells" or "peripheral blood mononuclear cells" generally refers to cells with single nuclei in peripheral blood. For example, in the present application, the peripheral blood mononuclear cells of the present application may comprise lymphocytes, monocytes and/or dendritic cells.

In the present application, the term "cytokine" generally refers to a protein released by one cell population that acts as an intercellular regulator for another cell. The cytokine in the present application may be lymphokines, monokines and polypeptide hormones. The cytokine in the present application may comprise interleukins (ILs), such as IL-1, IL-1$\alpha$, IL-2, IL-3, IL-4, IL-5, IL-6, IL-7, IL-8, IL-9, IL-10, IL-11, IL-15, IL-21, and/or IL-12. In the present application, the term cytokine may comprise proteins from natural sources or from recombinant cell cultures, biologically active equivalents of natural sequence cytokines, and functionally active fragments thereof.

In the present application, the term "diameter" generally refers to the diameter of the cross-section of the substance of the present application. For example, when the substance of the present application is not spherical, the term "diameter" generally refers to the maximum diameter and/or average diameter of the largest cross section of the substance of the present application. The method for determining the diameter of the substance may be a method commonly used in the art, such as transmission electron microscopy.

In the present application, the term "tumor" generally refers to any new pathological tissue proliferation. The tumors of the present application may be benign or malignant. The tumors of the present application may be solid or hematologic. The term "tumor" may be one or more of the tumors selected from the group consisting of: melanoma, ovarian cancer, cervical cancer, lung cancer, bladder cancer, breast cancer, head and neck cancer, pancreatic cancer, liver cancer, gastric cancer, colorectal cancer, and kidney cancer.

In the present application, the term "tumor tissue" generally refers to samples of any tissues from tumors in a subject, including any solid tumors and/or non-solid tumors in the subj ect.

In the present application, the term "CD28 agonist" generally refers to compounds that bind CD28 protein on the cell surface and elicit responses in the cells. For example, the CD28 agonist of the present application may be a small molecule formulation that binds CD28. For example, the CD28 agonist of the present application may be an antibody that binds CD28 or antigen-binding fragments thereof.

In the present application, the term "proportion of T cell subpopulations" generally refers to the proportions of different T cell subpopulations in TIL cells or TIL populations. For example, the different T cell subpopulations in the present application have different immune activities and/or differentiation capacities. For example, the T cell subpopulations in the present application may be distinguished based on T cell surface markers. For example, central memory T cells may have a phenotype of CD45RA$^-$CCR7$^+$. For example, regulatory T cells may have a phenotype of CD4$^+$CD25$^+$Foxp3$^+$. For example, activated T cells may have a phenotype of CD25$^+$, CD28$^+$, TIM3$^+$, PD1$^+$ or 41BB$^+$. For example, tumor-specific T cells may have a phenotype of CD103$^+$CD39$^+$. For example, stem cell-like T cells may have a phenotype of TCF1$^+$.

In the present application, the term "number of TIL cells" generally refers to the cell number in the TIL cells of the present application. In the present application, the number of TIL cells may refer to the cell number in the TIL population obtained in any one stage of the present application. For example, the number of TIL cells may refer to the cell number in the first TIL population derived from tumor tissues and not expanded *in vitro.* For example, the number of TIL cells may refer to the cell number of the second TIL population through the first stage *of in vitro* expansion. For example, the number of TIL cells may refer to the cell number of the third TIL population through the second stage of *in vitro* expansion. For example, the number of TIL cells may refer to the TIL cells finally obtained by any one culture method of the present application. In the present application, the number of TIL cells may be determined by methods commonly used in the art, which may comprise, for example, but not limited to, manual cell counting on a cell counting plate and/or counting with an automatic cell counter.

In the present application, the terms "about" and "approximately" generally refer to being within a statistically significant numerical range. Such a range can be within an order of magnitude of a given value or range, e.g., it can be within 50%, within 20%, within 10%, or within 5%. The permissible variation encompassed by the term "about" or "approximately" depends on the particular system under study, and can be readily understood by one of ordinary skill in the art. The terms "above", "below", "at most" and "at least" can comprise the number itself.

**Detailed description of the invention**

In one aspect, the present application provides a method for culturing tumor-infiltrating lymphocytes (TILs), the method may comprise improving the expression and/or enhancing the activity of at least one cytokine of the TILs, and co-culturing with feeder cells after contacting the TILs with T cell activators and/or T cell growth factors for a period of time.

For example, the method may comprise improving the expression and/or enhancing the activity of at least one cytokine of the TILs after co-culturing the TILs with the feeder cells.

For example, the method may comprise co-culturing the TILs with the feeder cells after improving the expression and/or enhancing the activity of at least one cytokine of the TILs.

For example, the method may comprise improving the expression and/or enhancing the activity of at least one cytokine of the TILs after contacting the TILs with the T cell activators and/or the T cell growth factors and before co-culturing the TILs with the feeder cells.

For example, the method may comprise improving the expression and/or enhancing the activity of at least one cytokine of the TILs substantially simultaneously with contacting the TILs with the T cell activators and/or the T cell growth factors.

For example, the method may comprise improving the expression and/or enhancing the activity of at least one cytokine of the TILs substantially simultaneously with co-culturing the TILs with the feeder cells.

In another aspect, the present application further provides a method for culturing tumor-infiltrating lymphocytes (TILs), the method may comprise improving the expression and/or enhancing the activity of at least one cytokine of the TILs, where the TILs comprise TILs obtained by co-culturing with feeder cells after contacting with T cell activators and/or T cell growth factors for a period of time.

In another aspect, the present application further provides a method for culturing tumor-infiltrating lymphocytes (TILs), the method may comprise co-culturing the TILs with feeder cells after contacting the TILs with T cell activators and/or T cell growth factors for a period of time, where the TILs comprise TILs obtained by improving the expression and/or enhancing the activity of at least one cytokine of the TILs.

In another aspect, the present application further provides a method for culturing tumor-infiltrating lymphocytes (TILs), the method may comprise improving the expression and/or enhancing the activity of at least one cytokine of the TILs and contacting the TILs with a CD28 agonist.

In another aspect, the present application further provides a method for culturing tumor-infiltrating lymphocytes (TILs), the method may comprise improving the expression and/or enhancing the activity of at least one cytokine of the TILs, where the TILs comprise TILs obtained by contacting the TILs with a CD28 agonist.

In another aspect, the present application further provides a method for culturing tumor-infiltrating lymphocytes (TILs), the method may comprise contacting the TILs with a CD28 agonist, where the TILs comprise TILs obtained by improving the expression and/or enhancing the activity of at least one cytokine of the TILs.

For example, the CD28 agonist comprises an anti-CD28 antibody and/or an antigen-binding fragment thereof, CD80 and/or a functionally active fragment thereof and/or CD86 and/or a functionally active fragment thereof, and recombinant protein of the above substances.

For example, compared to TILs with unchanged expression and/or activity of the cytokine, TILs obtained by improving the expression and/or enhancing the activity of at least one cytokine of the TILs may show improved TIL properties. In one embodiment, TILs with unchanged expression and/or activity of the cytokine may refer to TIL cells which are derived from the same donor and in which the expression of at least one cytokine of the TILs has not been improved and/or the activity thereof has not been enhanced. In one embodiment, TILs with unchanged expression and/or activity of the cytokine may refer to TIL cells which are derived from the same donor and in which the expression of the substances (e.g., green fluorescent protein) other than the cytokine of the TILs has not been improved and/or the activity thereof has not been enhanced.

In one embodiment, corresponding TILs in which the expression of at least one cytokine of the TILs has not been improved and/or the activity thereof has not been enhanced may refer to TIL cells which are derived from the same donor and isolated in the same way and in which the expression of at least one cytokine of the TILs has not been improved and/or the activity thereof has not been enhanced. In one embodiment, corresponding TILs in which the expression of at least one cytokine of the TILs has not been improved and/or the activity thereof has not been enhanced may refer to TIL cells which are derived from the same tumor source of the same donor and in which the expression of at least one cytokine of the TILs has not been improved and/or the activity thereof has not been enhanced. In one embodiment, corresponding TILs in which the expression of at least one cytokine of the TILs has not been improved and/or the activity thereof has not been enhanced may refer to TIL cells which are derived from the same tumor source of the same donor and isolated in the same way and in which the expression of at least one cytokine of the TILs has not been improved and/or the activity thereof has not been enhanced. In one embodiment, corresponding TILs in which the expression of at least one cytokine of the TILs has not been improved and/or the activity thereof has not been enhanced may mean that, the TIL cells derived from the same donor are divided into two groups, where one group of TIL cells in which the expression of at least one cytokine of the TILs has not been improved and/or the activity thereof has not been enhanced may be corresponding TILs in which the expression of at least one cytokine of the TILs has not been improved and/or the activity thereof has not been enhanced. In one embodiment, corresponding TILs in which the expression of at least one cytokine of the TILs has not been improved and/or the activity thereof has not been enhanced may mean that, the TIL cells derived from the same donor and isolated in the same way are divided into two groups, where one group of TIL cells in which the expression of at least one cytokine of the TILs has not been improved and/or the activity thereof has not been enhanced may be corresponding TILs in which the expression of at least one cytokine of the TILs has not been improved and/or the activity thereof has not been enhanced. In one embodiment, corresponding TILs in which the expression of at least one cytokine of the TILs has not been improved and/or the activity thereof has not been enhanced may mean that, TIL cells derived from the same tumor source of the same donor are divided into two groups, where one group of TIL cells in which the expression of at least one cytokine of the TILs has not been improved and/or the activity thereof has not been enhanced may be corresponding TILs in which the expression of at least one cytokine of the TILs has not been improved and/or the activity thereof has not been enhanced. In one embodiment, corresponding TILs in which the expression of at least one cytokine of the TILs has not been improved and/or the activity thereof has not been enhanced may mean that, the TIL cells derived from the same tumor source of the same donor and isolated in the same way are divided into two groups, where one group of TIL cells in which the expression of at least one cytokine of the TILs has not been improved and/or the activity thereof has not been enhanced may be corresponding TILs in which the expression of at least one cytokine of the TILs has not been improved and/or the activity thereof has not been enhanced. For example, improving the expression and/or enhancing the activity of at least one cytokine may mean that, natural cells may not express the cytokine, and can express various forms of the cytokine through the processing of the present application, that is, improved expression of the cytokine may mean to make the natural cells change from not expressing the cytokine to expressing a certain amount of the cytokine.

In one embodiment, the improved TIL properties in the present application comprise one or more properties selected from the group consisting of: increased number of TIL cells, increased proportion of viable cells, enhanced persistence, improved proportion of T cell subpopulations, enhanced cytokine secretion capacity, enhanced tumor cell killing ability, enhanced T cell receptor (TCR) clonal diversity, and increased number of TIL cells in tissues.

In one embodiment, the enhanced TIL cell number in tissues in the present application may mean that, compared to corresponding TILs in which the expression of at least one cytokine of the TILs has not been improved and/or the activity thereof has not been enhanced during the stage *of in vitro* expansion, the T cell number of the TIL cells of the present application in which the expression of at least one cytokine of the TILs is increased and/or the activity thereof is enhanced during at least one stage of *in vitro* expansion in tumor tissues, blood tissues, bone marrow tissues, lung tissues and/or spleen tissues may increase by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold.

In one embodiment, the improved TIL cell number in the present application may mean that, compared to corresponding TILs in which the expression of at least one cytokine of the TILs has not been improved and/or the activity thereof has not been enhanced during the stage *of in vitro* expansion, the cell number of the TILs in the present application in which the expression of at least one cytokine of the TILs is increased and/or the activity thereof is enhanced during at least one stage *of in vitro* expansion may increase by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. For example, the improved TIL cell number may display as the increase in the TIL cell viability. In one embodiment, the increased TIL cell number in the present application may mean that, compared to corresponding TILs in which the expression of at least one cytokine of the TILs has not been improved and/or the activity thereof has not been enhanced during the stage of *in vitro* expansion, the cell number of the TILs in the present application in which the expression of at least one cytokine of the TILs is increased and/or the activity thereof is enhanced during at least one stage *of in vitro* expansion may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%.

In one embodiment, the enhanced cytokine secretion capacity in the present application may refer to the enhanced secretion capacity of cytokine of TIL cells that is one or more cytokines selected from the group consisting of CD107a, GZMB, IL-2, TNF-$\alpha$ and IFN-$\gamma$. In one embodiment, the enhanced cytokine secretion capacity in the present application may mean that, compared to corresponding TILs in which the expression of at least one cytokine of the TILs has not been improved and/or the activity thereof has not been enhanced during the stage *of in vitro* expansion, the cytokine secretion capacity of the TILs in the present application in which the expression of at least one cytokine of the TILs is increased and/or the activity thereof is enhanced during at least one stage of *in vitro* expansion may increase by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. In one embodiment, the enhanced cytokine secretion capacity in the present application may mean that, compared to corresponding TILs in which the expression of at least one cytokine of the TILs has not been improved and/or the activity thereof has not been enhanced during the stage of *in vitro* expansion, the cytokine secretion capacity of the TILs in the present application in which the expression of at least one cytokine of the TILs is increased and/or the activity thereof is enhanced during at least one stage of *in vitro* expansion may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%. In one embodiment, the cytokine secretion capacity of the TILs in the present application may be determined by measuring the cytokine expression capacity of the TIL cells. In one embodiment, the cytokine secretion capacity of the TILs in the present application may be determined by measuring the cytokine release capacity of the TILs in the present application. In one embodiment, the cytokine secretion capacity of the TILs in the present application is determined by CBA (Cytometric Bead Array).

In one embodiment, the increased NK cell proportion in the present application may be the increase in the proportion of NK cells in the TIL cells. For example, the proportion of NK cells in the TIL cells may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%.

In one embodiment, the enhanced tumor cell killing ability in the present application may mean that, compared to corresponding TILs in which the expression of at least one cytokine of the TILs has not been improved and/or the activity thereof has not been enhanced during the stage of *in vitro* expansion, the tumor cell killing rate of the TILs in the present application in which the expression of at least one cytokine of the TILs is increased and/or the activity thereof is enhanced during at least one stage *of in vitro* expansion may increase by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. In one embodiment, the enhanced tumor cell killing ability in the present application may mean that, compared to corresponding TILs in which the expression of at least one cytokine of the TILs has not been improved and/or the activity thereof has not been enhanced during the stage *of in vitro* expansion, the tumor cell killing rate of the TILs in the present application in which the expression of at least one cytokine of the TILs is increased and/or the activity thereof is enhanced during at least one stage *of in vitro* expansion may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%. In one embodiment, the tumor cell killing rate of the TILs in the present application may be measured by CFSE and DAPI staining method. In one embodiment, the tumor cell killing rate of the TILs in the present application may be determined by measuring the activity of Caspase-3/7 using an IncuCyte system. In one embodiment, the tumor cell killing of the TILs in the present application may refer to the ability of TILs to kill solid tumor cells. In one embodiment, the tumor cell killing of the TILs in the present application may refer to the ability of TILs to kill cervical cancer cells.

In one embodiment, the enhanced T cell receptor (TCR) clonal diversity in the present application may comprise that, during the long-term culture, compared to corresponding TILs in which the expression of at least one cytokine of the TILs has not been improved and/or the activity thereof has not been enhanced during the stage of *in vitro* expansion, there are more kinds of TCRs being expressed in the TIL cell populations of the present application in which the expression of at least one cytokine of the TILs is increased and/or the activity thereof is enhanced during at least one stage *of in*

*vitro* expansion, e.g., it may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%.

In one embodiment, the improved proportion of T cell subpopulations in the present application may comprise one or more subpopulations selected from the group consisting of: increased proportion of CD4$^+$ cells, decreased proportion of CD8$^+$ cells, increased proportion of central memory T cells, decreased proportion of regulatory T cells, increased proportion of activated T cells, increased proportion of tumor-specific T cells, and increased proportion of stem cell-like T cells.

In one embodiment, the increased proportion of CD4$^+$ cells in the present application may be the increase in the proportion of CD4 positive cells in TIL cells. For example, the proportion of CD4$^+$ cells in TIL cells may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%.

In one embodiment, the decreased proportion of CD8$^+$ cells in the present application may be the decrease in the proportion of CD8 positive cells in TIL cells. For example, the proportion of CD8$^+$ cells in TIL cells may decrease by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%.

In one embodiment, the increased proportion of central memory T cells in the present application may be the increase in the proportion of CD45RA$^-$CCR7$^+$ cells in TIL cells. For example, the proportion of central memory T cells in TIL cells may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%.

In one embodiment, the decreased proportion of regulatory T cells in the present application may be the decrease in the proportion of CD4$^+$CD25$^+$Foxp3$^+$ cells in TIL cells. For example, the proportion of regulatory T cells in TIL cells may decrease by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%.

In one embodiment, the increased proportion of activated T cells in the present application may be the increase in the proportion of CD25$^+$, CD28$^+$, TIM3$^+$, PD1$^+$ or 41BB$^+$ cells in TIL cells. For example, the proportion of activated T cells in TIL cells may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%, or may increase by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. For example, the proportion of CD25$^+$ cells in TIL cells may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%, or may increase by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. For example, the proportion of CD28$^+$ cells in TIL cells may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%, or may increase by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. For example, the proportion of TIM3$^+$ cells in TIL cells may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%, or may increase by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. For example, the proportion of PD1$^+$ cells in TIL cells may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%, or may increase by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. For example, the proportion of 41BB$^+$ cells in TIL cells may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%, or may increase by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold.

For example, the step of improving the expression and/or enhancing the activity of at least one cytokine of the TILs in the method of the present application may comprise introducing a nucleic acid encoding the cytokine into the TILs.

For example, the step of improving the expression and/or enhancing the activity of at least one cytokine of the TILs in the method of the present application may comprise introducing a vector comprising the nucleic acid into the TILs, where the nucleic acid encodes one or more cytokines. For example, the nucleic acid encoding the cytokine in the method of the present application can be integrated into the genome of the TILs. For example, when the nucleic acid encoding the cytokine can be integrated into the genome of the TILs, the cytokine can be expressed in the cell for a long time or continuously.

For example, the vector in the method of the present application may comprise a viral vector. For example, the viral vector in the method of the present application may comprise a retroviral vector. For example, the retroviral vector in the method of the present application may comprise a lentiviral vector.

For example, the cytokine in the method of the present application may comprise interleukin (IL). For example, the cytokine in the method of the present application may comprise interleukin-15 (IL-15) and/or a functionally active fragment thereof.

For example, the IL-15 in the method of the present application may comprise membrane-anchored IL-15 and/or secreted IL-15. For example, the L-15 may comprise a variety of domains. For example, the IL-15 of the present application may be not limited to only including an IL-15 domain.

For example, the IL-15 in the method of the present application may comprise, but not limited to, an IL-15 domain. For example, the IL-15 domain in the method of the present application may comprise an amino acid sequence as shown in SEQ ID NO: 3.

For example, the IL-15 in the method of the present application may comprise, but not limited to, an IL-15Ra extracellular domain. For example, the IL-15Ra extracellular domain of the present application functions to bind the IL-15 domain of the present application. For example, the IL-15Ra extracellular domain in the method of the present application may comprise an amino acid sequence as shown in SEQ ID NO: 16.

For example, the IL-15 domain in the method of the present application may be directly or indirectly linked to the IL-15Ra extracellular domain. For example, the indirect linkage in the method of the present application may comprise, but not limited to, linking through a linker. For example, the linker in the method of the present application may comprise an amino acid sequence selected from the group consisting of: SEQ ID NOs: 4-10, (SEQ ID NO: 11)$_l$, (SEQ ID NO: 12)$_m$, (SEQ ID NO: 13)$_n$, (SEQ ID NO: 14)$_p$, and (SEQ ID NO: 15)$_q$, and any combination of the above, where l, m, n, p, and q may are each independently at least 1. For example, l, m, n, p, and q are the number of repeating units in each respective sequence, and when the sequence can be a repeat, the number of repeating units indicates the number of the repeats. For example, l may be 1, 2, 3, 4, 5, or 10, m may be 1, 2, 3, 4, 5, or 10, n may be 1, 2, 3, 4, 5, or 10, p may be 1, 2, 3, 4, 5, or 10, and q may be 1, 2, 3, 4, 5, or 10.

For example, the IL-15 in the method of the present application may comprise, but not limited to, a transmembrane domain. For example, the transmembrane domain of the present application may enable the IL-15 of the present application to be bound on the cell membrane. For example, the transmembrane domain in the method of the present application may comprise an amino acid sequence as shown in any one of SEQ ID NOs: 17-22. For example, the transmembrane domain in the method of the present application may be directly or indirectly linked to the IL-15Ra extracellular domain.

For example, the IL-15 in the method of the present application may comprise, but not limited to, an intracellular domain. For example, the intracellular domain in the method of the present application may comprise an amino acid sequence as shown in any one of SEQ ID NOs: 23-26. For example, the intracellular domain in the method of the present application may be directly or indirectly linked to the transmembrane domain.

For example, the IL-15 in the method of the present application may comprise, but not limited to, a signal peptide domain. For example, the signal peptide domain in the method of the present application may comprise an amino acid sequence as shown in SEQ ID NO: 2. For example, the signal peptide domain in the method of the present application may be directly or indirectly linked to the IL-15 domain.

For example, the functionally active fragment of the IL-15 in the method of the present application may comprise an amino acid sequence as shown in SEQ ID NO: 3.

For example, the IL-15 in the method of the present application may comprise an amino acid sequence as shown in SEQ ID NO: 1.

For example, the enhanced expression of the cytokine in the method of the present application may comprise enhanced synthesis and/or secretion of the cytokine.

For example, in the method of the present application, compared to TILs with unchanged expression and/or activity of the cytokine, the proportion of cells expressing the cytokine in the TILs obtained by improving the expression and/or enhancing the activity of at least one cytokine of the TILs can be improved.

For example, in the method of the present application, compared to TILs with unchanged expression and/or activity of the cytokine, the proportion of cells expressing the cytokine in the TILs obtained by improving the expression and/or enhancing the activity of at least one cytokine of the TILs may be improved by at least about 5% or above. For example, the proportion of cells expressing the IL-15 can be improved by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, or at least about 5%. For example, the proportion of cells expressing the IL-15 may be from 0% to an observable cell proportion. For example, the proportion of cells expressing the IL-15 can be improved to at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, or at least about 1%. For example, the proportion of cells expressing the IL-15 can be detected by flow cytometry.

For example, in the method of the present application, the proportion of cells expressing the cytokine in the TILs obtained by improving the expression and/or enhancing the activity of at least one cytokine of the TILs may be at least about 5% or above. For example, the proportion of cells expressing the IL-15 may be at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, or at least about 5%. For example, the proportion of cells expressing the IL-15 can be detected by flow cytometry.

In one embodiment, the method of the present application may also comprise: subjecting TILs derived from tumor tissues and not expanded *in vitro* to at least one stage of *in vitro* expansion, where the TILs of the present application can be co-cultured with feeder cells during the at least one stage *of in vitro* expansion in the present application.

In one embodiment, during a single stage of *in vitro* expansion in the present application, the expression of at least one cytokine of the TILs may be improved and/or the activity thereof may be enhanced, and the TILs may be co-cultured with the feeder cells of the present application. In one embodiment, the single stage of *in vitro* expansion in the present application may refer to the same one stage *of in vitro* expansion in the present application. For example, it may be all in the first stage of *in vitro* expansion in the present application, all in the second stage *of in vitro* expansion in the present application, or all in the third stage *in vitro* expansion in the present application, etc.

In one embodiment, during the first stage of *in vitro* expansion in the present application, the expression of at least one cytokine of the TILs may be improved and/or the activity thereof may be enhanced and the TILs may be co-cultured with the feeder cells of the present application. In one embodiment, during the second stage of *in vitro* expansion in the present application, the expression of at least one cytokine of the TILs may be improved and/or the activity thereof may be enhanced and the TILs may be co-cultured with the feeder cells of the present application. In one embodiment, during the third stage *of in vitro* expansion in the present application, the expression of at least one cytokine of the TILs may be improved and/or the activity thereof may be enhanced and the TILs may be co-cultured with the feeder cells of the present application.

In one embodiment, each stage of *in vitro* expansion may be divided depending on the change in the number of TIL cells. In one embodiment, when the number of TIL cells increases by at least about 1-fold, it can be considered that the TIL cells enter the next stage of *in vitro* expansion. In some embodiments, when the number of TIL cells increases by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, at least about 50-fold, at least about 100-fold, at least about 200-fold, at least about 500-fold, or at least about 1000-fold, it can be considered that the TIL cells enter the next stage *of in vitro* expansion. In one embodiment, each stage *of in vitro* expansion may also be divided depending on the change in the culture conditions of the TIL cells. In one embodiment, after T cell activators and/or T cell growth factors are added or supplemented into the cell culture medium, it can be considered that the TIL cells enter the next stage *of in vitro* expansion. For example, when IL-2 is added or supplemented into the cell culture medium, it can be considered that the TIL cells enter the next stage *of in vitro* expansion. For example, when the expression of at least one cytokine of the TILs is increased and/or the activity thereof is enhanced, it can be considered that the TIL cells enter the next stage *of in vitro* expansion. For example, when feeder cells are added or supplemented into the cell culture medium, it can be considered that the TIL cells enter the next stage of *in vitro* expansion. In one embodiment, when TIL cells have subjected to centrifugation and/or washing, it can be considered that the TIL cells enter the next stage of *in vitro* expansion. In one embodiment, each stage may also be divided depending on the culture days of the TIL cells. In one embodiment, after the TIL cells are cultured *in vitro* for about 1 day, about 2 days, about 3 days, about 4 days, about 5 days, about 6 days, about 7 days, about 8 days, about 9 days, about 10 days, about11 days, about 12 days, about 13 days, about 14 days, about 15 days, about 16 days, about 17 days, about 18 days, about 19 days, about 20 days, about 30 days, about 40 days, about 50 days or about 100 days, it can be considered that the TIL cells enter the next stage *of in vitro* expansion.

For example, the second stage of *in vitro* expansion can be carried out for at least about 7 days. For example, the second stage *of in vitro* expansion can be carried out for at least about 9 days. For example, the second stage *of in vitro* expansion can be carried out for at most about 14 days. For example, the second stage *of in vitro* expansion can be carried out for at most about 13 days. For example, the second stage *of in vitro* expansion can be carried out for about 7 days to about 14 days, about 9 days to about 14 days, about 7 days to about 13 days or about 9 days to about 13 days. For example, the second stage *of in vitro* expansion in the present application can be carried out for at least about 9 days, at least about 10 days, at least about11 days, at least about 12 days, at least about 13 days, or at least about 14 days. For example, the second stage *of in vitro* expansion in the present application can be carried out for about 9 days to about 14 days. For example, the second stage *of in vitro* expansion in the present application can be carried out for about 9 days to about 14 days, about 10 days to about 14 days, about11 days to about 14 days, about 12 days to about 14 days, about 13 days to about 14 days, about 9 days to about 13 days, about 10 days to about 13 days, about11 days to about 13 days, about 12 days to about 13 days, about 9 days to about 12 days, about 10 days to about 12 days, about11 days to about 12 days, or about 10 days to about 11 days. For example, the second stage of *in vitro* expansion in the present application can be considered as a REP (rapid expansion protocol) stage.

For example, the first stage of *in vitro* expansion can be carried out for at least about 7 days. For example, the first stage of *in vitro* expansion can be carried out for about 7 days to about 14 days. For example, the first stage *of in vitro* expansion in the present application can be carried out for at least about 7 days, at least about 8 days, at least about 9 days, at least about 10 days, at least about11 days, at least about 12 days, at least about 13 days, or at least about 14 days. For example, the first stage of *in vitro* expansion in the present application can be carried out for about 7 days to about 14 days, about 8 days to about 14 days, about 9 days to about 14 days, about 10 days to about 14 days, about11 days to about 14 days, about 12 days to about 14 days, about 13 days to about 14 days, about 9 days to about 13 days, about 10 days to about 13 days, about11 days to about 13 days, about 12 days to about 13 days, about 9 days to about 12 days, about 10 days to about 12 days, about11 days to about 12 days, or about 10 days to about11 days. For example, the first stage of *in vitro* expansion in the present application can be considered as a preREP stage.

In one embodiment, the days for the second stage *of in vitro* expansion in the present application may be calculated from the time when the second stage *of in vitro* expansion starts. For example, just when the second stage *of in vitro* expansion starts, it can be considered that the second stage *of in vitro* expansion has been carried out for about 0 days. For example, after the second stage of *in vitro* expansion has been carried out for about 24 hours, it can be considered that the second stage *of in vitro* expansion has been carried out for about 1 day. For example, on the day when the second stage of *in vitro* expansion starts, it can be considered that the second stage of *in vitro* expansion has been carried out for about 0 days. In one embodiment, the days for the second stage *of in vitro* expansion in the present application may be calculated by the days the second stage *of in vitro* expansion is carried out. For example, on the second day after the second stage *of in vitro* expansion starts, it can be considered that the second stage *of in vitro* expansion has been carried out for about 1 day.

In one embodiment, the culture method of the present application may be divided into two steps. For example, (A) a first TIL population derived from tumor tissues and not expanded *in vitro* can be contacted with T cell growth factors, where a second TIL population is obtained via the step (A); (B) the expression of at least one cytokine of the second TIL population can be improved and/or the activity thereof can be enhanced, and the second TIL population is co-cultured with feeder cells after contacting the second TIL population with T cell activators and/or T cell growth factors for a period of time, where a third TIL population is obtained via the step (B). For example, the step (A) can be carried out for about 7 days to about 14 days. For example, the step (B) can be carried out for about 7 days to about 14 days.

In one embodiment, the culture method of the present application may be divided into three steps. For example, (A) a first TIL population derived from tumor tissues and not expanded *in vitro* can be contacted with T cell growth factors, where a second TIL population is obtained via the step (A); (B) the expression of at least one cytokine of the second TIL population can be improved and/or the activity thereof can be enhanced, and the second TIL population can be contacted with T cell activators and/or T cell growth factors, where a third TIL population is obtained via the step (B); (C) the third TIL population can be co-cultured with feeder cells, where a fourth TIL population is obtained via the step (C). For example, the step (A) can be carried out for about 7 days to about 14 days. For example, the step (B) can be carried out for about 0 days to about 8 days. For example, the step (C) can be carried out for about 5 days to about 14 days.

In one embodiment, the culture method of the present application may be divided into four steps. For example, (A) a first TIL population derived from tumor tissues and not expanded *in vitro* can be contacted with T cell growth factors, where a second TIL population is obtained via the step (A); (B) the second TIL population can be contacted with T cell activators and/or T cell growth factors, where a third TIL population is obtained via the step (B); (C) the expression of at least one cytokine of the third TIL population can be improved and/or the activity thereof can be enhanced, where a fourth TIL population is obtained via the step (C); (D) the fourth TIL population can be co-cultured with feeder cells, where a fifth TIL population is obtained via the step (D). For example, the step (A) can be carried out for about 7 days to about 14 days. For example, the step (B) can be carried out for about 0 days to about 4 days. For example, the step (C) can be carried out for about 0 days to about 4 days. For example, the step (D) can be carried out for about 5 days to about 14 days.

In one embodiment, the step (A) in the culture method of the present application is to resuscitate and/or continue culturing an *in vitro* TIL population to obtain a second TIL population. For example, the *in vitro* TIL population may comprise a TIL population obtained by *in vitro* expansion of a first TIL population derived from tumor tissues and not expanded *in vitro.* For example, the *in vitro* TIL population may comprise a TIL population obtained by contacting the first TIL population with T cell growth factors. For example, the *in vitro* TIL population may comprise a TIL population obtained by cryopreserving the first TIL population. For example, the *in vitro* TIL population may comprise a TIL population obtained by contacting the first TIL population with T cell growth factors and cryopreservation. For example, when the step (A) in the present application is to resuscitate and/or continue culturing an *in vitro* TIL population to obtain the second TIL population, the step (A) can be carried out for about 2 hours to about 4 days.

In one embodiment, during a single stage of *in vitro* expansion in the present application, the TILs of the present application can be contacted with one or more T cell activators and/or one or more T cell growth factors of the present application for a period of time and co-cultured with feeder cells of the present application. In one embodiment, the period of time in the present application may be at least about 2 hours. In one embodiment, the period of time in the present application may be at least about 1 hour, at least about 2 hours, at least about 3 hours, at least about 4 hours, at least about 5 hours, at least about 6 hours, at least about 7 hours, at least about 8 hours, at least about 9 hours, at least about 10 hours, at least about 11 hours, at least about 12 hours, at least about 13 hours, at least about 14 hours, at least about 15 hours, at least about 16 hours, at least about 17 hours, at least about 18 hours, at least about 19 hours, at least about 20 hours, at least about 21 hours, at least about 22 hours, at least about 23 hours, at least about 24 hours, at least about 36 hours, at least about 48 hours, at least about 60 hours or at least about 72 hours. In one embodiment, the period of time in the present application can be about 6 hours to about 72 hours. In one embodiment, the period of time in the present application can be about 6 hours to about 7 hours, about 6 hours to about 8 hours, about 6 hours to about 9 hours, about 6 hours to about 10 hours, about 6 hours to about 11 hours, about 6 hours to about 12 hours, about 6 hours to about 13 hours, about 6 hours to about 14 hours, about 6 hours to about 15 hours, about 6 hours to about 16 hours, about 6 hours to about 17 hours, about 6 hours to about 18 hours, about 6 hours to about 19 hours, about 6 hours to about 20 hours, about 6 hours to about 21 hours, about 6 hours to about 22 hours, about 6 hours to about 23 hours, about 6 hours to about 24 hours, about 6 hours to about 36 hours, about 6 hours to about 48 hours, about 6 hours to about 60 hours or about 6 hours to about 72 hours. In one embodiment, the period of time in the present application can be about 12 hours to about 13 hours, about 12 hours to about 14 hours, about 12 hours to about 15 hours, about 12 hours to about 16 hours, about 12 hours to about 17 hours, about 12 hours to about 18 hours, about 12 hours to about 19 hours, about 12 hours to about 20 hours, about 12 hours to about 21 hours, about 12 hours to about 22 hours, about 12 hours to about 23 hours, about 12 hours to about 24 hours, about 12 hours to about 36 hours, about 12 hours to about 48 hours, about 12 hours to about 60 hours or about 12 hours to about 72 hours. In one embodiment, the period of time in the present application can be about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about 24 hours, about 36 hours, about 48 hours, about 60 hours, or about 72 hours.

In one embodiment, the feeder cells of the present application may comprise antigen-presenting cells. In one embodiment, the feeder cells of the present application may comprise one or more cells selected from the group consisting of: peripheral mononuclear cells, dendritic cells, and artificial antigen-presenting cells. In one embodiment, the feeder cells of the present application may be peripheral mononuclear cells. In one embodiment, the feeder cells of the present application may be irradiated feeder cells. For example, the feeder cells of the present application may be isolated artificial antigen-presenting cells (aAPCs), and the artificial antigen-presenting cells of the present application may comprise cells expressing HLA-A/B/C, CD64, CD80, ICOS-L, and/or CD58, can be modified to express more than one T cell activator of the present application. In one embodiment, the feeder cells of the present application may be irradiated, e.g., irradiated with gamma rays or irradiated with X-rays.

In one embodiment, the step of co-culturing the TILs of the present application with the feeder cells of the present application may comprise contacting the surface of the feeder cells of the present application with the surface of the TILs of the present application. In one embodiment, the step of co-culturing the TILs of the present application with the feeder cells of the present application comprises adding the feeder cells of the present application into the cell culture medium of the TILs of of the present application.

In one embodiment, the feeder cells of the present application may be added into the cell culture medium of the TILs of the present application at a proportion of the feeder cells of the present application to the TILs of the present application from about 40:1 to about 400:1 in the present application. In one embodiment, the feeder cells of the present application may be added into the cell culture medium of the TILs of the present application at a proportion of the feeder cells of the present application to the TILs of the present application from about 40:1 to about 400:1, from about 40:1 to about 300:1, from about 40:1 to about 200:1, from about 40:1 to about 100:1, from about 40:1 to about 90:1, from about 40:1 to about 80:1, from about 40:1-about 70:1, from about 40:1 to about 60:1, from about 40:1 to about 50:1, from about 50:1 to about 400:1, from about 60:1 to about 400:1, from about 70:1 to about 400:1, from about 80:1 to about 400:1, from about 90:1 to about 400:1, from about 100:1 to about 400:1, from about 200:1 to about 400:1, or from about 300:1 to about 400:1 in the present application.

In one embodiment, the method of the present application may also comprise: subjecting TILs derived from tumor tissues and not expanded *in vitro* to at least one stage of *in vitro* expansion, where the TILs of the present application are contacted with one or more T cell activators during the at least one stage *of in vitro* expansion in the present application.

In one embodiment, during a single stage of *in vitro* expansion in the present application, contacting the TILs with the one or more T cell activators. For example, the T cell activators may comprise agonists of one or more targets selected from the group consisting of: CD3, CD28, HVEM, CD40L, OX40, and 4-1BB. In one embodiment, during the single stage *of in vitro* expansion, the expression of at least one cytokine of the TILs is increased and/or the activity thereof is enhanced, and contacting the TILs with one or more T cell activators of the present application. In one embodiment, during the first stage of *in vitro* expansion in the present application, the expression of at least one cytokine of the TILs may be improved and/or the activity thereof may be enhanced and the TILs may be contacted with one or more T cell activators of the present application. In one embodiment, during the second stage of *in vitro* expansion in the present application, the expression of at least one cytokine of the TILs may be improved and/or the activity thereof may be enhanced and the TILs may be contacted with one or more T cell activators of the present application. In one embodiment, during the third stage *of in vitro* expansion in the present application, the expression of at least one cytokine of the TILs may be improved and/or the activity thereof may be enhanced and the TILs may be contacted with one or more T cell activators of the present application.

In one embodiment, during a single stage of *in vitro* expansion in the present application, the expression of at least one cytokine of the TILs may be improved and/or the activity thereof may be enhanced substantially simultaneously with contacting the TILs with one or more T cell activators of the present application. In one embodiment, during the single stage *of in vitro* expansion in the present application, the expression of at least one cytokine of the TILs may be improved and/or the activity thereof may be enhanced first, e.g., 2 hours, 4 hours 8 hours, 12 hours, 24 hours, or 48 hours etc., before contacting the TILs with one or more T cell activators of the present application. In one embodiment, during the single stage of *in vitro* expansion in the present application, the TILs of the present application may be contacted with one or more T cell activators of the present application, e.g., 2 hours, 4 hours, 8 hours, 12 hours, 24 hours, or 48 hours etc., before improving the expression and/or enhancing the activity of at least one cytokine of the TILs.

In one embodiment, during the first stage of *in vitro* expansion in the present application, the expression of at least one cytokine of the TILs may be improved and/or the activity thereof may be enhanced substantially simultaneously with contacting the TILs with one or more T cell activators of the present application. In one embodiment, during the second stage *of in vitro* expansion in the present application, the expression of at least one cytokine of the TILs may be improved and/or the activity thereof may be enhanced substantially simultaneously with contacting the TILs with one or more T cell activators of the present application. In one embodiment, during the third stage of *in vitro* expansion in the present application, the expression of at least one cytokine of the TILs may be improved and/or the activity thereof may be enhanced substantially simultaneously with contacting the TILs with one or more T cell activators of the present application.

In one embodiment, the T cell activators of the present application may comprise one or more activators selected from the group consisting of: CD80, CD86, B7-H3, 4-1BBL, CD27, CD30, CD134, B7h, CD40, LIGHT, and functionally active fragments thereof. In one embodiment, the T cell activators of the present application may comprise agonists of one or more targets selected from the group consisting of: CD3, CD28, HVEM, CD40L, OX40, and 4-1BB. In one embodiment, the T cell activators of the present application may comprise one or more antibodies selected from the group consisting of CD3, CD28, HVEM, CD40L, OX40, and 4-1BB, and antigen binding fragments thereof. In one embodiment, the T cell activators of the present application may comprise a CD3 agonist. In one embodiment, the T cell activators of the present application may comprise an anti-CD3 antibody and/or an antigen-binding fragment thereof, e.g., it may be OKT3 from Miltenyi Biotech, or SP34 from BD. In one embodiment, the T cell activators of the present application may comprise a CD28 agonist. In one embodiment, the T cell activators of the present application may comprise an anti-CD28 antibody and/or an antigen-binding fragment thereof, e.g., it may be 15E8 from Merck. The T cell activators of the present application may comprise CD80 and/or a functionally active fragment thereof and/or CD86 and/or a functionally active fragment thereof, and recombinant protein of the above substances.

In one embodiment, the T cell activators of the present application may comprise an anti-CD3 antibody and/or an antigen-binding fragment thereof, e.g., they may comprise light chain VL and heavy chain VH of OKT3 from Miltenyi Biotech, or light chain VL and heavy chain VH of SP34 from BD. In one embodiment, the T cell activators of the present application may comprise a CD28 agonist. In one embodiment, the T cell activators of the present application may comprise an anti-CD28 antibody and/or an antigen-binding fragment thereof, e.g., they may comprise light chain VL and heavy chain VH of 15E8 from Merck. In one embodiment, the T cell activators of the present application may comprise an anti-CD3 antibody and/or an antigen-binding fragment thereof, e.g., they may comprise light chain LCDRs 1-3 and heavy chain HCDRs 1-3 of OKT3 from Miltenyi Biotech, or light chain LCDRs 1-3 and heavy chain HCDRs 1-3 of SP34 from BD, and the an anti-CD3 antibody and/or an antigen-binding fragment thereof in the present application may have the ability of binding CD3. In one embodiment, the T cell activators of the present application may comprise a CD28 agonist. In one embodiment, the T cell activators of the present application may comprise an anti-CD28 antibody and/or an antigen-binding fragment thereof, e.g., they may comprise light chain LCDRs 1-3 and heavy chain HCDRs 1-3 of 15E8 from Merck, and the an anti-CD28 antibody and/or an antigen-binding fragment thereof in the present application may have the ability of binding CD28. In the present application, the antibodies or the antigen binding proteins thereof in the present application comprise at least one CDR in the heavy chain variable region VH of the antibodies. The CDRs in the present application may be defined according to the nomenclature of IMGT, the CDRs in the present application may be defined according to Chothia, or the CDRs in the present application may be defined according to Kabat.

In one embodiment, the CD3 agonist of the present application may be a CD3 antibody or an antigen binding protein thereof.

In the present application, the antibodies or antigen binding proteins thereof in the present application comprise at least one CDR in the heavy chain variable region VH of the antibodies. The CDRs in the present application may be defined according to the nomenclature of IMGT, the CDRs in the present application may be defined according to Chothia, or the CDRs in the present application may be defined according to Kabat.

For example, the antibodies or antigen binding proteins thereof in the present application may comprise an HCDR1, and the HCDR1 in the present application may comprise an amino acid sequence as shown in any one of SEQ ID NOs: 28 and 38; the CDRs in the present application may be defined according to the nomenclature of IMGT; the CDRs in the present application may be defined according to Kabat; for example, the antigen binding proteins in the present application may have the ability of binding CD3.

For example, the antibodies or antigen binding proteins thereof in the present application may comprise an HCDR2, and the HCDR2 in the present application may comprise an amino acid sequence as shown in any one of SEQ ID NOs: 29 and 39; the CDRs in the present application may be defined according to the nomenclature of IMGT; the CDRs in the present application may be defined according to Kabat; for example, the antigen binding proteins in the present application may have the ability of binding CD3.

For example, the antibodies or antigen binding proteins thereof in the present application may comprise an HCDR3, and the HCDR3 in the present application may comprise an amino acid sequence as shown in any one of SEQ ID NOs: 30 and 40; the CDRs in the present application may be defined according to the nomenclature of IMGT; the CDRs in the present application may be defined according to Kabat; for example, the antigen binding proteins in the present application may have the ability of binding CD3.

For example, the antibodies or antigen binding proteins thereof in the present application may comprise HCDRs 1-3, where the HCDR1 in the present application may comprise an amino acid sequence as shown in any one of SEQ ID NOs: 28 and 38, the HCDR2 in the present application may comprise an amino acid sequence as shown in any one of SEQ ID NOs: 29 and 39, and the HCDR3 in the present application may comprise an amino acid sequence as shown in any one of SEQ ID NOs: 30 and 40; the CDRs in the present application may be defined according to the nomenclature of IMGT; the CDRs in the present application may be defined according to Kabat; for example, the antigen binding proteins in the present application may have the ability of binding CD3.

For example, the antibodies or antigen binding proteins thereof in the present application may comprise the same HCDRs 1-3 as those of OKT3, where the HCDR1 in the present application may comprise an amino acid sequence as shown in SEQ ID NO: 28, the HCDR2 in the present application may comprise an amino acid sequence as shown in SEQ ID NO: 29, and the HCDR3 in the present application may comprise an amino acid sequence as shown in SEQ ID NO: 30; the CDRs in the present application may be defined according to the nomenclature of IMGT; for example, the antigen binding proteins in the present application may have the ability of binding CD3.

For example, the antibodies or antigen binding proteins thereof in the present application may comprise the same HCDRs 1-3 as those of SP34, where the HCDR1 in the present application may comprise an amino acid sequence as shown in SEQ ID NO: 38, the HCDR2 in the present application may comprise an amino acid sequence as shown in SEQ ID NO: 39, and the HCDR3 in the present application may comprise an amino acid sequence as shown in SEQ ID NO: 40; the CDRs in the present application may be defined according to Kabat; for example, the antigen binding proteins in the present application may have the ability of binding CD3.

In the present application, the antibodies or antigen binding proteins thereof in the present application comprise at least one CDR in the light chain variable region VL of the antibodies. The CDRs in the present application may be defined according to the nomenclature of IMGT, or the CDRs in the present application may be defined according to Kabat.

For example, the antibodies or antigen binding proteins thereof in the present application may comprise an LCDR1, and the LCDR1 in the present application may comprise an amino acid sequence as shown in any one of SEQ ID NOs: 31 and 41; the CDRs in the present application may be defined according to the nomenclature of IMGT; the CDRs in the present application may be defined according to Kabat; for example, the antigen binding proteins in the present application may have the ability of binding CD3.

For example, the antibodies or antigen binding proteins thereof in the present application may comprise an LCDR2, and the LCDR2 in the present application may comprise an amino acid sequence as shown in any one of SEQ ID NOs: 32 and 42; the CDRs in the present application may be defined according to the nomenclature of IMGT; the CDRs in the present application may be defined according to Kabat; for example, the antigen binding proteins in the present application may have the ability of binding CD3.

For example, the antibodies or antigen binding proteins thereof in the present application may comprise an LCDR3, and the LCDR3 in the present application may comprise an amino acid sequence as shown in any one of SEQ ID NOs: 33 and 43; the CDRs in the present application may be defined according to the nomenclature of IMGT; the CDRs in the present application may be defined according to Kabat; for example, the antigen binding proteins in the present application may have the ability of binding CD3.

For example, the antibodies or antigen binding proteins thereof in the present application may comprise LCDRs 1-3, where the LCDR1 in the present application may comprise an amino acid sequence as shown in any one of SEQ ID NOs: 31 and 41, the LCDR2 in the present application may comprise an amino acid sequence as shown in any one of SEQ ID NOs: 32 and 42, and the LCDR3 in the present application may comprise an amino acid sequence as shown in any one of SEQ ID NOs: 33 and 43; the CDRs in the present application may be defined according to the nomenclature of IMGT; the CDRs in the present application may be defined according to Kabat; for example, the antigen binding proteins in the present application may have the ability of binding CD3.

For example, the antibodies or antigen binding proteins thereof in the present application may comprise the same LCDRs 1-3 as those of OKT3, where the LCDR1 in the present application may comprise an amino acid sequence as shown in SEQ ID NO: 31, the LCDR2 in the present application may comprise an amino acid sequence as shown in SEQ ID NO: 32, and the LCDR3 in the present application may comprise an amino acid sequence as shown in SEQ ID NO: 33; the CDRs in the present application may be defined according to the nomenclature of IMGT; for example, the antigen binding proteins in the present application may have the ability of binding CD3.

For example, the antibodies or antigen binding proteins thereof in the present application may comprise the same LCDRs 1-3 the same as those of SP34, where the LCDR1 in the present application may comprise an amino acid sequence as shown in SEQ ID NO: 41, the LCDR2 in the present application may comprise an amino acid sequence as shown in SEQ ID NO: 42, and the LCDR3 in the present application may comprise an amino acid sequence as shown in SEQ ID NO: 43; the CDRs in the present application may be defined according to Kabat; for example, the antigen binding proteins in the present application may have the ability of binding CD3.

For example, the antibodies or antigen binding proteins thereof in the present application may comprise HCDRs1-3 and LCDRs 1-3, where the HCDR1 in the present application may comprise an amino acid sequence as shown in any one of SEQ ID NOs: 28 and 38, the HCDR2 in the present application may comprise an amino acid sequence as shown in any one of SEQ ID NOs: 29 and 39, the HCDR3 in the present application may comprise an amino acid sequence as shown in any one of SEQ ID NOs: 30 and 40, the LCDR1 in the present application may comprise an amino acid sequence as shown in any one of SEQ ID NOs: 31 and 41, the LCDR2 in the present application may comprise an amino acid sequence as shown in any one of SEQ ID NOs: 32 and 42, and the LCDR3 in the present application may comprise an amino acid sequence as shown in any one of SEQ ID NOs: 33 and 43; the CDRs in the present application may be defined according to the nomenclature of IMGT; the CDRs in the present application may be defined according to Kabat; for example, the antigen binding proteins in the present application may have the ability of binding CD3.

For example, the antibodies or antigen binding proteins thereof in the present application may comprise the same HCDRs 1-3 and LCDRs 1-3 as those of OKT3, where the HCDR1 in the present application may comprise an amino acid sequence as shown in SEQ ID NO: 28, the HCDR2 in the present application may comprise an amino acid sequence as shown in SEQ ID NO: 29, the HCDR3 in the present application may comprise an amino acid sequence as shown in SEQ ID NO: 30, the LCDR1 in the present application may comprise an amino acid sequence as shown in SEQ ID NO: 31, the LCDR2 in the present application may comprise an amino acid sequence as shown in SEQ ID NO: 32, and the LCDR3 in the present application may comprise an amino acid sequence as shown in SEQ ID NO: 33; the CDRs in the present application may be defined according to the nomenclature of IMGT; for example, the antigen binding proteins in the present application may have the ability of binding CD3.

For example, the antibodies or antigen binding proteins thereof in the present application may comprise the same HCDRs 1-3 as those of SP34, where the HCDR1 in the present application may comprise an amino acid sequence as shown in SEQ ID NO: 38, the HCDR2 in the present application may comprise an amino acid sequence as shown in SEQ ID NO: 39, the HCDR3 in the present application may comprise an amino acid sequence as shown in SEQ ID NO: 40, the LCDR1 in the present application may comprise an amino acid sequence as shown in SEQ ID NO: 41, the LCDR2 in the present application may comprise an amino acid sequence as shown in SEQ ID NO: 42, and the LCDR3 in the present application may comprise an amino acid sequence as shown in SEQ ID NO: 43; the CDRs in the present application may be defined according to Kabat; for example, the antigen binding proteins in the present application may have the ability of binding CD3.

In one embodiment, the antibodies or antigen binding proteins thereof in the present application may comprise a heavy chain variable region VH, and the VH in the present application may comprise an amino acid sequence as shown in any one of SEQ ID NOs: 34 and 44; for example, the antigen binding proteins in the present application may have the ability of binding CD3.

For example, the antibodies or antigen binding proteins thereof in the present application may comprise the same VH as that of OKT3, and the VH in the present application may comprise an amino acid sequence as shown in SEQ ID NO: 34; for example, the antigen binding proteins in the present application may have the ability of binding CD3.

For example, the antibodies or antigen binding proteins thereof in the present application may comprise the same VH as that of SP34, and the VH in the present application may comprise an amino acid sequence as shown in SEQ ID NO: 44; for example, the antigen binding proteins in the present application may have the ability of binding CD3.

In one embodiment, the antibodies or antigen binding proteins thereof in the present application may comprise a light chain variable region VL, and the VL in the present application may comprise an amino acid sequence as shown in any one of SEQ ID NOs: 35 and 45; for example, the antigen binding proteins in the present application may have the ability of binding CD3.

For example, the antibodies or antigen binding proteins thereof in the present application may comprise the same VL as that of OKT3, and the VL in the present application may comprise an amino acid sequence as shown in SEQ ID NO: 35; for example, the antigen binding proteins in the present application may have the ability of binding CD3.

For example, the antibodies or antigen binding proteins thereof in the present application may comprise the same VL as that of SP34, and the VL in the present application may comprise an amino acid sequence as shown in SEQ ID NO: 45; for example, the antigen binding proteins in the present application may have the ability of binding CD3.

In one embodiment, the antibodies or antigen binding proteins thereof in the present application may comprise a heavy chain variable region VH and a light chain variable region VL, and the VH in the present application may comprise an amino acid sequence as shown in any one of SEQ ID NOs: 34 and 44, and the VL in the present application may comprise an amino acid sequence as shown in any one of SEQ ID NOs: 35 and 45; for example, the antigen binding proteins in the present application may have the ability of binding CD3.

For example, the antibodies or antigen binding proteins thereof in the present application may comprise the same VH and VL as those of OKT3, and the VH in the present application may comprise an amino acid sequence as shown in SEQ ID NO: 34, the VL in the present application may comprise an amino acid sequence as shown in SEQ ID NO: 35; for example, the antigen binding proteins in the present application may have the ability of binding CD3 .

For example, the antibodies or antigen binding proteins thereof in the present application may comprise the same VH and VL as those of SP34, and the VH in the present application may comprise an amino acid sequence as shown in SEQ ID NO: 44, the VL in the present application may comprise an amino acid sequence as shown in SEQ ID NO: 45; for example, the antigen binding proteins in the present application may have the ability of binding CD3 .

In one embodiment, the antibodies or antigen binding proteins thereof in the present application may comprise a heavy chain, and the heavy chain in the present application may comprise an amino acid sequence as shown in any one of SEQ ID NOs: 36 and 46; for example, the antigen binding proteins in the present application may have the ability of binding CD3.

For example, the antibodies or antigen binding proteins thereof in the present application may comprise the same heavy chain as that of OKT3, and the heavy chain in the present application may comprise an amino acid sequence as shown in SEQ ID NO: 36; for example, the antigen binding proteins in the present application may have the ability of binding CD3 .

For example, the antibodies or antigen binding proteins thereof in the present application may comprise the same heavy chain as that of SP34, and the heavy chain in the present application may comprise an amino acid sequence as shown in SEQ ID NO: 46; for example, the antigen binding proteins in the present application may have the ability of binding CD3 .

In one embodiment, the antibodies or antigen binding proteins thereof in the present application may comprise a light chain, and the light chain in the present application may comprise an amino acid sequence as shown in any one of SEQ ID NOs: 37 and 47; for example, the antigen binding proteins in the present application may have the ability of binding CD3.

For example, the antibodies or antigen binding proteins thereof in the present application may comprise the same light chain as that of OKT3, and the light chain in the present application may comprise an amino acid sequence as shown in SEQ ID NO: 37; for example, the antigen binding proteins in the present application may have the ability of binding CD3 .

For example, the antibodies or antigen binding proteins thereof in the present application may comprise the same light chain as that of SP34, and the light chain in the present application may comprise an amino acid sequence as shown in SEQ ID NO: 47; for example, the antigen binding proteins in the present application may have the ability of binding CD3.

In one embodiment, the antibodies or antigen binding proteins thereof in the present application may comprise a heavy chain and a light chain, and the heavy chain in the present application may comprise an amino acid sequence as shown in any one of SEQ ID NOs: 36 and 46, the light chain in the present application may comprise an amino acid sequence as shown in any one of SEQ ID NOs: 37 and 47; for example, the antigen binding proteins in the present application may have the ability of binding CD3.

For example, the antibodies or antigen binding proteins thereof in the present application may comprise the same heavy chain and light chain as those of OKT3, and the heavy chain in the present application may comprise an amino acid sequence as shown in SEQ ID NO: 36, the light chain in the present application may comprise an amino acid sequence as shown in SEQ ID NO: 37; for example, the antigen binding proteins in the present application may have the ability of binding CD3.

For example, the antibodies or antigen binding proteins thereof in the present application may comprise the same heavy chain and light chain as those of SP34, and the heavy chain in the present application may comprise an amino acid sequence as shown in SEQ ID NO: 46, the light chain in the present application may comprise an amino acid sequence as shown in SEQ ID NO: 47; for example, the antigen binding proteins in the present application may have the ability of binding CD3.

In one embodiment, the CD28 agonist of the present application may be a CD28 antibody or an antigen binding protein thereof.

In the present application, the antibodies or antigen binding proteins thereof in the present application comprise at least one CDR in the heavy chain variable region VH of the antibodies. The CDRs in the present application may be defined according to the nomenclature of IMGT, or the CDRs in the present application may be defined according to Kabat.

For example, the antibodies or antigen binding proteins thereof in the present application may comprise an HCDR1, and the HCDR1 in the present application may comprise an amino acid sequence as shown in SEQ ID NO: 48; the CDRs in the present application may be defined according to the nomenclature of IMGT; the CDRs in the present application may be defined according to Kabat; for example, the antigen binding proteins in the present application may have the ability of binding CD28.

For example, the antibodies or antigen binding proteins thereof in the present application may comprise an HCDR2, and the HCDR2 in the present application may comprise an amino acid sequence as shown in SEQ ID NO: 49; the CDRs in the present application may be defined according to the nomenclature of IMGT; the CDRs in the present application may be defined according to Kabat; for example, the antigen binding proteins in the present application may have the ability of binding CD28.

For example, the antibodies or antigen binding proteins thereof in the present application may comprise an HCDR3, and the HCDR3 in the present application may comprise an amino acid sequence as shown in SEQ ID NO: 50; the CDRs in the present application may be defined according to the nomenclature of IMGT; the CDRs in the present application may be defined according to Kabat; for example, the antigen binding proteins in the present application may have the ability of binding CD28.

For example, the antibodies or antigen binding proteins thereof in the present application may comprise HCDRs 1-3, where the HCDR1 in the present application may comprise an amino acid sequence as shown in SEQ ID NO: 48, the HCDR2 in the present application may comprise an amino acid sequence as shown in SEQ ID NO: 49, and the HCDR3 in the present application may comprise an amino acid sequence as shown in SEQ ID NO: 50; the CDRs in the present application may be defined according to the nomenclature of IMGT; the CDRs in the present application may be defined according to Kabat; for example, the antigen binding proteins in the present application may have the ability of binding CD28.

For example, the antibodies or antigen binding proteins thereof in the present application may comprise the same HCDRs 1-3 as those of 15E8, where the HCDR1 in the present application may comprise an amino acid sequence as shown in SEQ ID NO: 48, the HCDR2 in the present application may comprise an amino acid sequence as shown in SEQ ID NO: 49, and the HCDR3 in the present application may comprise an amino acid sequence as shown in SEQ ID NO: 50; the CDRs in the present application may be defined according to the nomenclature of IMGT; for example, the antigen binding proteins in the present application may have the ability of binding CD28.

In the present application, the antibodies or antigen binding proteins thereof in the present application comprises at least one CDR in the light chain variable region VL of the antibodies. The CDRs in the present application may be defined according to the nomenclature of IMGT, or the CDRs in the present application may be defined according to Kabat.

For example, the antibodies or antigen binding proteins thereof in the present application may comprise an LCDR1, and the LCDR1 in the present application may comprise an amino acid sequence as shown in SEQ ID NO: 51; the CDRs in the present application may be defined according to the nomenclature of IMGT; the CDRs in the present application may be defined according to Kabat; for example, the antigen binding proteins in the present application may have the ability of binding CD28.

For example, the antibodies or antigen binding proteins thereof in the present application may comprise an LCDR2, and the LCDR2 in the present application may comprise an amino acid sequence as shown in SEQ ID NO: 52; the CDRs in the present application may be defined according to the nomenclature of IMGT; the CDRs in the present application may be defined according to Kabat; for example, the antigen binding proteins in the present application may have the ability of binding CD28.

For example, the antibodies or antigen binding proteins thereof in the present application may comprise an LCDR3, and the LCDR3 in the present application may comprise an amino acid sequence as shown in SEQ ID NO: 53; the CDRs in the present application may be defined according to the nomenclature of IMGT; the CDRs in the present application may be defined according to Kabat; for example, the antigen binding proteins in the present application may have the ability of binding CD28.

For example, the antibodies or antigen binding proteins thereof in the present application may comprise LCDRs 1-3, where the LCDR1 in the present application may comprise an amino acid sequence as shown in SEQ ID NO: 51, the LCDR2 in the present application may comprise an amino acid sequence as shown in SEQ ID NO: 52, and the LCDR3 in the present application may comprise an amino acid sequence as shown in SEQ ID NO: 53; the CDRs in the present application may be defined according to the nomenclature of IMGT; the CDRs in the present application may be defined according to Kabat; for example, the antigen binding proteins in the present application may have the ability of binding CD28.

For example, the antibodies or antigen binding proteins thereof in the present application may comprise the same LCDRs 1-3 as those of 15E8, where the LCDR1 in the present application may comprise an amino acid sequence as shown in SEQ ID NO: 51, the LCDR2 in the present application may comprise an amino acid sequence as shown in SEQ ID NO: 52, and the LCDR3 in the present application may comprise an amino acid sequence as shown in SEQ ID NO: 53; the CDRs in the present application may be defined according to the nomenclature of IMGT; for example, the antigen binding proteins in the present application may have the ability of binding CD28.

For example, the antibodies or antigen binding proteins thereof in the present application may comprise HCDRs 1-3 and LCDRs 1-3, where the HCDR1 in the present application may comprise an amino acid sequence as shown in SEQ ID NO: 48, the HCDR2 in the present application may comprise an amino acid sequence as shown in SEQ ID NO: 49, the HCDR3 in the present application may comprise an amino acid sequence as shown in SEQ ID NO: 50, the LCDR1 in the present application may comprise an amino acid sequence as shown in SEQ ID NO: 51, the LCDR2 in the present application may comprise an amino acid sequence as shown in SEQ ID NO: 52, and the LCDR3 in the present application may comprise an amino acid sequence as shown in SEQ ID NO: 53; the CDRs in the present application may be defined according to the nomenclature of IMGT; the CDRs in the present application may be defined according to Kabat; for example, the antigen binding proteins in the present application may have the ability of binding CD28.

For example, the antibodies or antigen binding proteins thereof in the present application may comprise the same HCDRs 1-3 and LCDRs 1-3 as those of 15E8, where the HCDR1 in the present application may comprise an amino acid sequence as shown in SEQ ID NO: 48, the HCDR2 in the present application may comprise an amino acid sequence as shown in SEQ ID NO: 49, the HCDR3 in the present application may comprise an amino acid sequence as shown in SEQ ID NO: 50, the LCDR1 in the present application may comprise an amino acid sequence as shown in SEQ ID NO: 51, the LCDR2 in the present application may comprise an amino acid sequence as shown in SEQ ID NO: 52, and the LCDR3 in the present application may comprise an amino acid sequence as shown in SEQ ID NO: 53; the CDRs in the present application may be defined according to the nomenclature of IMGT; for example, the antigen binding proteins in the present application may have the ability of binding CD28.

In one embodiment, the antibodies or antigen binding proteins thereof in the present application may comprise a heavy chain variable region VH, and the VH in the present application may comprise an amino acid sequence as shown in any one of SEQ ID NOs: 54 and 55; for example, the antigen binding proteins in the present application may have the ability of binding CD28.

For example, the antibodies or antigen binding proteins thereof in the present application may comprise the same VH as that of one 15E8, and the VH in the present application may comprise an amino acid sequence as shown in SEQ ID NO: 54; for example, the antigen binding proteins in the present application may have the ability of binding CD28.

For example, the antibodies or antigen binding proteins thereof in the present application may comprise the same VH as that of another 15E8, and the VH in the present application may comprise an amino acid sequence as shown in SEQ ID NO: 55; for example, the antigen binding proteins in the present application may have the ability of binding CD28.

In one embodiment, the antibodies or antigen binding proteins thereof in the present application may comprise a light chain variable region VL, and the VL in the present application may comprise an amino acid sequence as shown in SEQ ID NO: 56; for example, the antigen binding proteins in the present application may have the ability of binding CD28.

For example, the antibodies or antigen binding proteins thereof in the present application may comprise the same VL as that of 15E8, and the VL in the present application may comprise an amino acid sequence as shown in SEQ ID NO: 56; for example, the antigen binding proteins in the present application may have the ability of binding CD28.

In one embodiment, the antibodies or antigen binding proteins thereof in the present application may comprise a heavy chain variable region VH and a light chain variable region VL, and the VH in the present application may comprise an amino acid sequence as shown in any one of SEQ ID NOs: 54 and 55, the VL in the present application may comprise an amino acid sequence as shown in SEQ ID NO: 56; for example, the antigen binding proteins in the present application may have the ability of binding CD28.

For example, the antibodies or antigen binding proteins thereof in the present application may comprise the same VH and VL as those of one 15E8, and the VH in the present application may comprise an amino acid sequence as shown in SEQ ID NO: 54, the VL in the present application may comprise an amino acid sequence as shown in SEQ ID NO: 56; for example, the antigen binding proteins in the present application may have the ability of binding CD28.

For example, the antibodies or antigen binding proteins thereof in the present application may comprise the same VH and VL as those of another 15E8, and the VH in the present application may comprise an amino acid sequence as shown in SEQ ID NO: 55, the VL in the present application may comprise an amino acid sequence as shown in SEQ ID NO: 56; for example, the antigen binding proteins in the present application may have the ability of binding CD28.

In one embodiment, the antibodies or antigen binding proteins thereof in the present application may comprise a heavy chain, and the heavy chain in the present application may comprise an amino acid sequence as shown in any one of SEQ ID NOs: 57 and 58; for example, the antigen binding proteins in the present application may have the ability of binding CD28.

For example, the antibodies or antigen binding proteins thereof in the present application may comprise the same heavy chain as that of one 15E8, and the heavy chain in the present application may comprise an amino acid sequence as shown in SEQ ID NO: 57; for example, the antigen binding proteins in the present application may have the ability of binding CD28.

For example, the antibodies or antigen binding proteins thereof in the present application may comprise the same heavy chain as that of another 15E8, and the heavy chain in the present application may comprise an amino acid sequence as shown in SEQ ID NO: 58; for example, the antigen binding proteins in the present application may have the ability of binding CD28.

In one embodiment, the antibodies or antigen binding proteins thereof in the present application may comprise a light chain, and the light chain in the present application may comprise an amino acid sequence as shown in SEQ ID NO: 59; for example, the antigen binding proteins in the present application may have the ability of binding CD28.

For example, the antibodies or antigen binding proteins thereof in the present application may comprise the same light chain as that of 15E8, and the light chain in the present application may comprise an amino acid sequence as shown in SEQ ID NO: 59; for example, the antigen binding proteins in the present application may have the ability of binding CD28.

In one embodiment, the antibodies or antigen binding proteins thereof in the present application may comprise a heavy chain and a light chain, and the heavy chain in the present application may comprise an amino acid sequence as shown in any one of SEQ ID NOs: 57 and 58, the light chain in the present application may comprise an amino acid sequence as shown in SEQ ID NO: 59; for example, the antigen binding proteins in the present application may have the ability of binding CD28.

For example, the antibodies or antigen binding proteins thereof in the present application may comprise the same heavy chain and light chain as those of one 15E8, and the heavy chain in the present application may comprise an amino acid sequence as shown in SEQ ID NO: 57, the light chain in the present application may comprise an amino acid sequence as shown in SEQ ID NO: 59; for example, the antigen binding proteins in the present application may have the ability of binding CD28.

For example, the antibodies or antigen binding proteins thereof in the present application may comprise the same heavy chain and light chain as those of another 15E8, and the heavy chain in the present application may comprise an amino acid sequence as shown in SEQ ID NO: 58, the light chain in the present application may comprise an amino acid sequence as shown in SEQ ID NO: 59; for example, the antigen binding proteins in the present application may have the ability of binding CD28.

In one embodiment, the step of contacting the TILs of the present application with one or more T cell activators of the present application may comprise one or more ways selected from the group consisting of: (1) adding the T cell activators of the present application into the cell culture medium of the TILs of of the present application; (2) adding engineered cells expressing the T cell activators of the present application into the cell culture medium of the TILs of of the present application; (3) adding a solid medium comprising the T cell activators of the present application into the cell culture medium of the TILs of of the present application. In one embodiment, the step of contacting the TILs of the present application with one or more T cell activators of the present application may comprise adding a solid medium comprising the T cell activators of the present application into the cell culture medium of the TILs of of the present application. In one embodiment, the step of contacting the TILs of the present application with one or more T cell activators of the present application may comprise adding a solid medium comprising the CD28 antibodies and CD3 antibodies of the present application into the cell culture medium of the TILs of of the present application.

In one embodiment, the initial concentration of the T cell activators in the cell culture medium of the TILs of the present application may be at least about 30 ng/mL. For example, the initial concentration of the CD28 antibodies of the present application in the cell culture medium of the TILs of the present application may be at least about 30 ng/mL; for example, the initial concentration of the CD3 antibodies of the present application in the cell culture medium of the TILs of the present application may be at least about 30 ng/mL. For example, the choice of the initial concentration of the CD28 antibodies of the present application may be independent of the choice of the initial concentration of the CD3 antibodies of the present application; for example, the initial concentrations of the CD28 antibodies of the present application and the CD3 antibodies of the present application in the cell culture medium of the TILs of the present application may be in any combination. For example, the initial concentration of the CD28 antibodies of the present application in the cell culture medium of the TILs of the present application may be optionally selected from about 30 ng/mL to about 300 ng/mL. For example, the initial concentration of the CD3 antibodies of the present application in the cell culture medium of the TILs of the present application may be optionally selected from about 30 ng/mL to about 300 ng/mL. For example, the initial concentration of the CD28 antibodies of the present application in the cell culture medium of the TILs of the present application may be optionally selected from about 30 ng/mL to about 300 ng/mL, and the initial concentration of the CD3 antibodies of the present application in the cell culture medium of the TILs of the present application may be optionally selected from about 30 ng/mL to about 300 ng/mL, the choice of the initial concentration of the CD28 antibodies of the present application may be independent of the choice of the initial concentration of the CD3 antibodies of the present application. In one embodiment, the diameter of the solid medium of the present application can be about 500 nm to about 10 $\mu$m. In one embodiment, the diameter of the solid medium of the present application can be measured by transmission electron microscopy. In one embodiment, the diameter of the solid medium of the present application can be about 1 nm to about 500 nm. In one embodiment, the diameter of the solid medium of the present application can be about 100 nm to about 500 nm. In one embodiment, the diameter of the solid medium of the present application can be about 200 nm to about 500 nm. In one embodiment, the diameter of the solid medium of the present application can be measured by transmission electron microscopy.

In one embodiment, the solid medium of the present application may comprise a polymer. In one embodiment, the solid medium of the present application may comprise glucan.

In one embodiment, each mg of the solid medium of the present application comprises at least about 25 $\mu$g of the T cell activators of the present application.

In one embodiment, the solid medium comprising the T cell activators of the present application is added into the cell culture medium of the TILs of the present application at a ratio of the solid medium of the present application to the TILs of the present application from about 1:100 to about 1:2000. In one embodiment, the solid medium comprising the T cell activators of the present application is added into the cell culture medium of the TILs of the present application at a ratio of the solid medium of the present application to the TILs of the present application from about 2:1 to about 1:2.

For example, when the diameter of the solid medium of the present application is about 100 nm to about 500 nm, the solid medium comprising the T cell activators of the present application may be added into the cell culture medium of the TILs of the present application at a ratio of the solid medium of the present application to the TILs of the present application from about 2:1 to about 1:2. For example, when the diameter of the solid medium of the present application is about 100 nm to about 500 nm, the solid medium comprising the T cell activators of the present application, e.g., the CD3 agonist and/or the CD28 agonist, may be added into the cell culture medium of the TILs of the present application at a ratio of the solid medium of the present application to the TILs of the present application from about 2:1 to about 1:2, from about 2:1 to about 1:1, or from about 1:1 to about 1:2.

For example, when the diameter of the solid medium of the present application is about 100 nm to about 500 nm, the solid medium comprising the T cell activators of the present application may be added into the cell culture medium of the TILs of the present application at a ratio of the solid medium of the present application to the TILs of the present application from about 1:100 to about 1:2000. For example, when the diameter of the solid medium of the present application is about 100 nm to about 500 nm, the solid medium comprising the CD28 agonist and CD3 agonist of the present application may be added into the cell culture medium of the TILs of the present application at a ratio of the solid medium of the present application to the TILs of the present application from about 1:100 to about 1:2000, from about 1:200 to about 1:2000, from about 1:300 to about 1:2000, from about 1:400 to about 1:2000, from about 1:500 to about 1:2000, from about 1:600 to about 1:2000, from about 1:700 to about 1:2000, from about 1:800 to about 1:2000, from about 1:900 to about 1:2000, from about 1:1000 to about 1:2000, from about 1:1200 to about 1:2000from about 1:1400 to about 1:2000, from about 1:1600 to about 1:2000, or from about 1:1800 to about 1:2000.

In one embodiment, the method of the present application may also comprise: subjecting TILs derived from tumor tissues and not expanded *in vitro* to at least one stage of *in vitro* expansion, where the TILs of the present application are contacted with one or more T cell growth factors during the at least one stage *of in vitro* expansion in the present application.

In one embodiment, during a single stage of *in vitro* expansion in the present application, the TILs of the present application may be contacted with the T cell activators of the present application and contacted with one or more T cell growth factors of the present application. For example, during the first stage *of in vitro* expansion in the present application, the TILs of the present application may be contacted with the T cell activators of the present application and contacted with one or more T cell growth factors of the present application. For example, during the second stage of *in vitro* expansion in the present application, the TILs of the present application may be contacted with the T cell activators of the present application and contacted with one or more T cell growth factors of the present application. For example, during the third stage of *in vitro* expansion in the present application, the TILs of the present application may be contacted with the T cell activators of the present application and contacted with one or more T cell growth factors of the present application.

In one embodiment, during a single stage of *in vitro* expansion in the present application, the expression of at least one cytokine of the TILs may be improved and/or the activity thereof may be enhanced substantially simultaneously with contacting the TILs with the T cell growth factors. In one embodiment, during a single stage *of in vitro* expansion in the present application, the expression of at least one cytokine of the TILs may be improved and/or the activity thereof may be enhanced substantially simultaneously with contacting the TILs with the T cell growth factors. In one embodiment, during a single stage of *in vitro* expansion in the present application, the expression of at least one cytokine of the TILs may be improved and/or the activity thereof may be enhanced first, for example, 2 hours, 4 hours, 8 hours, 12 hours, 24 hours, or 48 hours before contacting the TILs of the present application with one or more T cell growth factors of the present application. In one embodiment, during a single stage of *in vitro* expansion in the present application, the TILs of the present application may be contacted with one or more T cell growth factors of the present application first, for example, 2 hours, 4 hours, 8 hours, 12 hours, 24 hours, or 8 hours before improving the expression and/or enhancing the activity of at least one cytokine of the TILs.

For example, during the first stage of *in vitro* expansion in the present application, the expression of at least one cytokine of the TILs may be improved and/or the activity thereof may be enhanced substantially simultaneously with contacting the TILs with the T cell growth factors. For example, during the second stage of *in vitro* expansion in the present application, the expression of at least one cytokine of the TILs may be improved and/or the activity thereof may be enhanced substantially simultaneously with contacting the TILs with the T cell growth factors. For example, during the third stage *of in vitro* expansion in the present application, the expression of at least one cytokine of the TILs may be improved and/or the activity thereof may be enhanced substantially simultaneously with contacting the TILs with the T cell growth factors.

In one embodiment, the T cell growth factors of the present application may be one or more of the factors selected from the group consisting of: IL-2, IL-7, IL-12, IL-15, IL-21, interferon γ, and functionally active fragments thereof. In one embodiment, the T cell growth factors of the present application may comprise IL-2 and/or a functionally active fragment thereof. For example, the functionally active fragments of IL-2 may comprise fragments of IL-2 capable of binding to IL-2 receptors of T cells that are known in the art.

In one embodiment, the step of contacting the TILs of the present application with one or more T cell growth factors of the present application may comprise adding the T cell growth factors of the present application into the cell culture medium of the TILs of the present application. In one embodiment, the initial concentration of the T cell growth factors of the present application in the cell culture medium of the TILs of the present application may be at least about 300 IU/mL. In one embodiment, the initial concentration of the IL-2 of the present application in the cell culture medium of the TILs of the present application may be at least about 350 IU/mL, at least about 400 IU/mL, at least about 500 IU/mL, at least about 600 IU/mL, at least about 700 IU/mL, at least about 800 IU/mL, at least about 900 IU/mL, at least about 1000 IU/mL, at least about 1100 IU/mL, at least about 1200 IU/mL, at least about 1300 IU/mL, at least about 1400 IU/mL, at least about 1500 IU/mL, at least about 2000 IU/mL, at least about 2500 IU/mL, at least about 2600 IU/mL, at least about 2700 IU/mL, at least about 2800 IU/mL, at least about 2900 IU/mL, at least about 3000 IU/mL, at least about 3100 IU/mL, at least about 3200 IU/mL, at least about 3300 IU/mL, at least about 3400 IU/mL, at least about 3500 IU/mL, at least about 4000 IU/mL, at least about 4500 IU/mL, at least about 5000 IU/mL, at least about 5500 IU/mL, at least about 6000 IU/mL, at least about 6500 IU/mL, at least about 7000 IU/mL, at least about 7500 IU/mL, at least about 8000 IU/mL, at least about 8500 IU/mL, or at least about 9000 IU/mL.

In one embodiment, the TILs of the present application may are TILs derived from the debris of the tumor tissues of the present application. In one embodiment, the TILs of the present application may be obtained by processing the tumor tissues into tumor debris. In one embodiment, the tumor debris of the present application has a volume of about 1-27 mm$^3$. In one embodiment, the tumor debris of the present application has a volume of about 1 mm$^3$, about 2 mm$^3$, about 3 mm$^3$, about 4 mm$^3$, about 5 mm$^3$, about 6 mm$^3$, about 7 mm$^3$, about 8 mm$^3$, about 9 mm$^3$, about 10 mm$^3$, about 11 mm$^3$, about 12 mm$^3$, about 13 mm$^3$, about 15 mm$^3$, about 17 mm$^3$, about 19 mm$^3$, about 20 mm$^3$, about 21 mm$^3$, about 23 mm$^3$, about 24 mm$^3$, about 25 mm$^3$, about 26 mm$^3$, or about 27 mm$^3$.

In another aspect, the present application provides a method for culturing tumor-infiltrating lymphocytes (TILs), which may comprise: (A) contacting a first TIL population derived from tumor tissues and not expanded *in vitro* with T cell growth factors, where a second TIL population is obtained via the step (A); (B) improving the expression and/or enhancing the activity of at least one cytokine of the TILs, and co-culturing the second TIL population with feeder cells after contacting the second TIL population with T cell activators and/or T cell growth factors for a period of time, where a third TIL population is obtained via the step (B).

In another aspect, the present application provides a method for culturing tumor-infiltrating lymphocytes (TILs), which may comprise: (A) resuscitating and/or continuing culturing an *in vitro* TIL population to obtain a second TIL population, where the *in vitro* TIL population comprises a TIL population obtained by the *in vitro* expansion of the first TIL population, the first TIL population is a TIL population derived from tumor tissues and not expanded *in vitro*; (B) improving the expression and/or enhancing the activity of at least one cytokine of the TILs, and co-culturing the second TIL population with feeder cells after contacting the second TIL population with T cell activators and/or T cell growth factors for a period of time, where a third TIL population is obtained via the step (B).

For example, a TIL population derived from tumor tissues at a certain time and/or at a certain location and not expanded *in vitro* may be contacted with T cell growth factors first to obtain an *in vitro* TIL population. The *in vitro* TIL population may be, in one aspect, cultured continually to subject to the step (B), or, in another aspect, may be cryopreserved first and resuscitated when needed, and then subjected to the step (B).

In another aspect, the present application provides a method for culturing tumor-infiltrating lymphocytes (TILs), which may comprise: (A) contacting a first TIL population derived from tumor tissues and not expanded *in vitro* with T cell growth factors, where a second TIL population is obtained via the step (A); (B) improving the expression and/or enhancing the activity of IL-15 of the TILs, and co-culturing the second TIL population with feeder cells after contacting the second TIL population with T cell activators and/or T cell growth factors for a period of time, where a third TIL population is obtained via the step (B).

In another aspect, the present application provides a method for culturing tumor-infiltrating lymphocytes (TILs), which may comprise: (A) contacting a first TIL population derived from tumor tissues and not expanded *in vitro* with T cell growth factors, where a second TIL population is obtained via the step (A); (B) improving the proportion of TIL cells expressing IL-15 in the TILs by at least 5%, and co-culturing the second TIL population with feeder cells after contacting the second TIL population with T cell activators and/or T cell growth factors for a period of time, where a third TIL population is obtained via the step (B).

In the terms used in one embodiment, the first stage of *in vitro* expansion in the present application and the step (A) in the methods of the above aspects can be optionally used interchangeably. In the terms used in one embodiment, the second stage of *in vitro* expansion in the present application and the step (B) in the methods of the above aspects can be optionally used interchangeably. In the terms used in one embodiment, the TILs upon the first stage of *in vitro* expansion in the present application and the second TIL population obtained from the step (A) in the methods of the above aspects can be optionally used interchangeably. In the terms used in one embodiment, the TILs upon the second stage of *in vitro* expansion in the present application and the third TIL population obtained from the step (B) in the methods of the above aspects can be optionally used interchangeably. In the terms used in one embodiment, if needed, the third stage *in vitro* expansion of the present application and the optionally added step (C) in the methods of the above aspects can be optionally used interchangeably. In the terms used in one embodiment, if needed, the TILs upon the third stage *of in vitro* expansion in the present application and the fourth TIL population obtained from the optionally added step (C) in the methods of the above aspects can be optionally used interchangeably.

In another aspect, the present application provides a method for culturing tumor-infiltrating lymphocytes (TILs), which may comprise: (A) contacting the first TIL population derived from tumor tissues and not expanded *in vitro* with a variety of T cell growth factors; where a second TIL population is obtained via the step (A); (B) contacting the second TIL population and a variety of T cell growth factors with a variety of T cell activators, improving the expression and/or enhancing the activity of at least one cytokine of the TILs, and co-culturing the TILs with feeder cells; where a third TIL population is obtained via the step (B).

In another aspect, the present application provides a method for culturing tumor-infiltrating lymphocytes (TILs), which may comprise: (A) contacting the first TIL population derived from tumor tissues and not expanded *in vitro* with a variety of T cell growth factors; where a second TIL population is obtained via the step (A); (B) contacting the second TIL population with a variety of T cell growth factor and a variety of T cell activators, improving the expression of IL-15 of the TILs and/or enhancing the activity thereof, and co-culturing the TILs with feeder cells; where a third TIL population is obtained via the step (B).

In another aspect, the present application provides a method for culturing tumor-infiltrating lymphocytes (TILs), which may comprise: (A) contacting a first TIL population derived from tumor tissues and not expanded *in vitro* with a variety of T cell growth factors; where a second TIL population is obtained via the step (A); (B) contacting the second TIL population with a variety of T cell growth factors and a variety of T cell activators, improving the proportion of TIL cells expressing IL-15 in the TILs by at least 5%, and co-culturing the TILs with feeder cells; where a third TIL population is obtained via the step (B).

In another aspect, the present application provides a method for culturing tumor-infiltrating lymphocytes (TILs), which may comprise: (A) contacting a first TIL population derived from tumor tissues and not expanded *in vitro* with a variety of T cell growth factors; where a second TIL population is obtained via the step (A); (B) contacting the second TIL population with a variety of T cell growth factors and a variety of T cell activators, improving the proportion of TIL cells expressing IL-15 in the TILs by at least 5%, and co-culturing the TILs with feeder cells at least 2 hours later; where a third TIL population is obtained via the step (B).

In another aspect, the present application provides a method for culturing tumor-infiltrating lymphocytes (TILs), which may comprise: (A) contacting a first TIL population derived from tumor tissues and not expanded *in vitro* with a variety of T cell growth factors; where a second TIL population is obtained via the step (A); (B) contacting the second TIL population with a variety of T cell growth factors and a variety of T cell activators, improving the proportion of TIL cells expressing IL-15 in the TILs by at least 5%, and co-culturing the TILs with feeder cells at least 2 hours later, the feeder cells may comprise peripheral mononuclear cells, the feeder cells are added into the cell culture medium of the TILs; where a third TIL population is obtained via the step (B).

In another aspect, the present application provides a method for culturing tumor-infiltrating lymphocytes (TILs), which may comprise: (A) contacting a first TIL population derived from tumor tissues and not expanded *in vitro* with a variety of T cell growth factors; where a second TIL population is obtained via the step (A); (B) contacting the second TIL population with a variety of T cell growth factors and a variety of T cell activators, improving the proportion of TIL cells expressing IL-15 in the TILs by at least 5%, and co-culturing the TILs with feeder cells at least 2 hours later, the feeder cells may comprise peripheral mononuclear cells, and the feeder cells may be added into the cell culture medium of the TILs at a proportion of the feeder cells to the TILs from about 40:1 to about 400:1; where a third TIL population is obtained via the step (B).

In another aspect, the present application provides a method for culturing tumor-infiltrating lymphocytes (TILs), which may comprise: (A) contacting a first TIL population derived from tumor tissues and not expanded *in vitro* with IL-2; where a second TIL population is obtained via the step (A); (B) contacting the second TIL population with IL-2 and a variety of T cell activators, improving the proportion of TIL cells expressing IL-15 in the TILs by at least 5%, and co-culturing the TILs with feeder cells at least 2 hours later, the feeder cells may comprise peripheral mononuclear cells and may be added into the cell culture medium of the TILs at a proportion of the feeder cells to the TILs from about 40:1 to about 400:1; where a third TIL population is obtained via the step (B).

In another aspect, the present application provides a method for culturing tumor-infiltrating lymphocytes (TILs), which may comprise: (A) contacting a first TIL population derived from tumor tissues and not expanded *in vitro* with IL-2 of which the initial concentration in the cell culture medium of the TILs may be at least about 300 IU/mL; where a second TIL population is obtained via the step (A); (B) contacting the second TIL population with IL-2 of which the initial concentration in the cell culture medium of the TILs may be at least about 300 IU/mL, and CD3 antibodies of which the initial concentration in the cell culture medium of the TILs is at least about 30 ng/mL, improving the proportion of TIL cells expressing IL-15 in the TILs by at least 5%, and co-culturing the TILs with feeder cells at least 2 hours later, the feeder cells may comprise peripheral mononuclear cells and may be added into the cell culture medium of the TILs at a proportion of the feeder cells to the TILs from about 40:1 to about 400:1; where a third TIL population is obtained via the step (B).

In another aspect, the present application provides a method for culturing tumor-infiltrating lymphocytes (TILs), which may comprise: (A) contacting a first TIL population derived from tumor tissues and not expanded *in vitro* with IL-2; where a second TIL population is obtained via the step (A); (B) contacting the second TIL population with IL-2 and a nanomatrix comprising CD3 antibodies and CD28 antibodies, improving the proportion of TIL cells expressing IL-15 in the TILs by at least 5%, and co-culturing the TILs with feeder cells; where a third TIL population is obtained via the step (B).

In another aspect, the present application provides a method for culturing tumor-infiltrating lymphocytes (TILs), which may comprise: (A) contacting a first TIL population derived from tumor tissues and not expanded *in vitro* with IL-2; where a second TIL population is obtained via the step (A); (B) contacting the second TIL population with IL-2 and a nanomatrix comprising CD3 antibodies and CD28 antibodies, the diameter of the nanomatrix can be about 1 nm to about 500 nm, and each mg of the nanomatrix may comprise each about 25 μg of CD3 antibodies and CD28 antibodies, respectively; improving the proportion of TIL cells expressing IL-15 in the TILs by at least 5%, and co-culturing the TILs with feeder cells; where a third TIL population is obtained via the step (B).

In another aspect, the present application provides a method for culturing tumor-infiltrating lymphocytes (TILs), which may comprise: (A) contacting a first TIL population derived from tumor tissues and not expanded *in vitro* with IL-2; where a second TIL population is obtained via the step (A); (B) contacting the second TIL population with IL-2 and a nanomatrix comprising CD3 antibodies and CD28 antibodies, the diameter of the nanomatrix can be about 1 nm to about 500 nm, each mg of the nanomatrix may comprise each about 25 μg of CD3 antibodies and CD28 antibodies, respectively, and the nanomatrix may be added into the cell culture medium of the TILs at a proportion of the nanomatrix to the TILs from about 1:100 to about 1:2000; improving the proportion of TIL cells expressing IL-15 in the TILs by at least 5%, and co-culturing the TILs with feeder cells; where a third TIL population is obtained via the step (B).

In another aspect, the present application provides a method for culturing tumor-infiltrating lymphocytes (TILs), which may comprise: (A) contacting a first TIL population derived from tumor tissues and not expanded *in vitro* with IL-2 of which the initial concentration in the cell culture medium of the TILs may be at least about 300 IU/mL; where a second TIL population is obtained via the step (A); (B) contacting the second TIL population with IL-2 of which the initial concentration in the cell culture medium of the TILs may be at least about 300 IU/mL, and a nanomatrix comprising CD3 antibodies and CD28 antibodies of which the diameter can be about 1 nm to about 500 nm, each mg of the nanomatrix may comprise each about 25 μg of CD3 antibodies and CD28 antibodies, respectively, and the nanomatrix may be added into the cell culture medium of the TILs at a proportion of the nanomatrix to the TILs from about 1:100 to about 1:2000; improving the proportion of TIL cells expressing IL-15 in the TILs by at least 5%, and co-culturing the TILs with feeder cells, the feeder cells may comprise peripheral mononuclear cells and may be added into the cell culture medium of the TILs at a proportion of the feeder cells to the TILs from about 40:1 to about 400:1; where a third TIL population is obtained via the step (B).

In another aspect, the present application provides a method for culturing tumor-infiltrating lymphocytes (TILs). The TIL cells obtained from the tissue samples of a subject can be orthotopic tumor samples or metastatic tumor samples surgically obtained from the patient, and the weight can be at least about 1 g, or multiple pieces of tissues can be combined. The tumor tissues are transported at about 2-8 degrees in the sample transport solution, for example, a commonly used commercial tumor tissue transport solution, tumor tissue preservation solution or tumor tissue transfer solution, and processed within 48 hours. Tissue pieces can be broken mechanically to a size of about 1-27 mm$^3$ each, transferred into a gas-permeable culture bag or Grex, added with T cell serum-free culture medium and IL-2 at a concentration of 300-9000 IU/mL (e.g., the concentration may be 1000-9000 IU/mL, e.g., 6000 IU/mL) and cultured for about 3-14 days. The harvested TIL cells can be cryopreserved and then resuscitated, or can be directly collected in the cell culture medium, and transferred into gas-permeable culture bag, or Grex, or Xuri unit. The T cell serum-free culture medium can be added with CD28 antibodies, CD3 antibodies and CD28 antibodies, magnetic beads (e.g., Dynabeads) comprising CD3 antibodies and CD28 antibodies, and/or nanomatrix (e.g., transACT) comprising CD3 antibodies and CD28 antibodies of the present application, IL-2 at a concentration of 300-9000 IU/mL (e.g., the concentration may be 1000-9000 IU/mL, e.g., 6000 IU/mL), and retroviruses carrying IL-15 can be used for transduction to improve the proportion of TIL cells expressing IL-15 in the TILs by at least 5%. After the TILs of the present application are activated for a period of time, irradiated PBMCs are added (at a ratio of TILs to PBMCs of about 1:40 to about 1:400 and cultured by expansion for about 3-14 days. The cells in the culture medium are collected using a cell processing system, washed and cryopreserved, and detected. In the final products, the proportion of CD3 can be greater than 80%, the viability of the cells can be greater than 50%, and more than 80% of the T cells may be memory effector T cells and effector T cells. Upon stimulation, IFN-γ can be secreted, and/or the final products may have a characteristic of up-regulated proportion of activated T cells.

In one aspect, the present application provides tumor-infiltrating lymphocytes (TILs) which are obtained by the culture method of the present application. In one embodiment, the TILs provided in the present application may comprise one kind or one batch of TILs obtained by the culture method of the present application. In one embodiment, the TILs provided in the present application may comprise multiple kinds or multiple batches of TILs obtained by the culture method of the present application and combined in any proportion.

In some embodiments, the TILs expanded by the method of the present application can be administered to patients as a pharmaceutical composition. In some embodiments, pharmaceutical composition may be a suspension of TILs in sterile buffer. The TILs expanded with the PBMCs of the present application can be administered in any suitable routes known in the art. In some embodiments, T cells can be administered in a single fusion intraarterially or intravenously, and the infusion may last about 30 to 60 minutes. Other suitable administration routes may comprise intraperitoneal, intrathecal, and intralymphatic administration.

In some embodiments, any suitable dose of TILs can be administered. In some embodiments, for example, when the tumor is melanoma, about $2.3 \times 10^9$ to about $13.7 \times 10^{10}$ TILs can be administered. In some embodiments, about $1 \times 10^9$ to about $12 \times 10^{10}$ TILs can be administered. In some embodiments, about $1.2 \times 10^{10}$ to about $4.3 \times 10^{10}$ TILs can be administered. In some embodiments, about $3 \times 10^{10}$ to about $12 \times 10^{10}$ TILs can be administered. In some embodiments, about $4 \times 10^{10}$ to about $10 \times 10^{10}$ TILs can be administere. In some embodiments, about $5 \times 10^{10}$ to about $8 \times 10^{10}$ TILs can be administered. In some embodiments, about $6 \times 10^{10}$ to about $8 \times 10^{10}$ TILs can be administered. In some embodiments, about $7 \times 10^{10}$ to about $8 \times 10^{10}$ TILs can be administered. In some embodiments, the therapeutically effective dose can be about $2.3 \times 10^9$ to about $13.7 \times 10^{10}$. In some embodiments, the therapeutically effective dose can be about $1 \times 10^9$ to about $12 \times 10^{10}$ TILs. In some embodiments, the therapeutically effective dose can be about $1.2 \times 10^{10}$ to about $4.3 \times 10^{10}$ TILs. In some embodiments, the therapeutically effective dose can be about $3 \times 10^{10}$ to about $12 \times 10^{10}$ TILs. In some embodiments, the therapeutically effective dose can be about $4 \times 10^{10}$ to about $10 \times 10^{10}$ TILs. In some embodiments, the therapeutically effective dose can be about $5 \times 10^{10}$ to about $8 \times 10^{10}$ TILs. In some embodiments, the therapeutically effective dose can be about $6 \times 10^{10}$ to about $8 \times 10^{10}$ TILs. In some embodiments, the therapeutically effective dose can be about $7 \times 10^{10}$ to about $8 \times 10^{10}$ TILs.

In some embodiments, the number of TILs provided in the composition of the present application can be about $1 \times 10^6$, about $2 \times 10^6$, about $3 \times 10^6$, about $4 \times 10^6$, about $5 \times 10^6$, about $6 \times 10^6$, about $7 \times 10^6$, about $8 \times 10^6$, about $9 \times 10^6$, about $1 \times 10^7$, about $2 \times 10^7$, about $3 \times 10^7$, about $4 \times 10^7$, about $5 \times 10^7$, about $6 \times 10^7$, about $7 \times 10^7$ about $8 \times 10^7$, about $9 \times 10^7$, about $1 \times 10^8$, about $2 \times 10^8$, about $3 \times 10^8$, about $4 \times 10^8$, about $5 \times 10^8$, about $6 \times 10^8$, about $7 \times 10^8$, about $8 \times 10^8$, about $9 \times 10^8$, about $1 \times 10^9$, about $2 \times 10^9$, about $3 \times 10^9$, about $4 \times 10^9$, about $5 \times 10^9$, about $6 \times 10^9$, about $7 \times 10^9$, about $8 \times 10^9$, about $9 \times 10^9$, about $1 \times 10^{10}$, about $2 \times 10^{10}$, about $3 \times 10^{10}$, about $4 \times 10^{10}$, about $5 \times 10^{10}$, about $6 \times 10^{10}$, about $7 \times 10^{10}$, about $8 \times 10^{10}$, about $9 \times 10^{10}$, about $1 \times 10^{11}$, about $2 \times 100^{11}$, about $3 \times 10^{11}$, about $4 \times 10^{11}$, about $5 \times 10^{11}$, about $6 \times 10^{11}$, about $7 \times 10^{11}$, about $8 \times 10^{11}$, about $9 \times 10^{11}$, about $1 \times 10^{12}$, about $2 \times 10^{12}$, about $3 \times 10^{12}$, about $4 \times 10^{12}$, about $5 \times 10^{12}$, about $6 \times 10^{12}$, about $7 \times 10^{12}$, about $8 \times 10^{12}$, about $9 \times 10^{12}$, about $1 \times 10^{13}$, about $2 \times 10^{13}$, about $3 \times 10^{13}$, about $4 \times 10^{13}$, about $5 \times 10^{13}$, about $6 \times 10^{13}$, about $7 \times 10^{13}$, about $8 \times 10^{13}$, or about $9 \times 10^{13}$. In some embodiments, the number of the TILs provided in the composition of the present application can range from about $1 \times 10^6$ to $5 \times 10^6$, about $5 \times 10^6$ to $1 \times 10^7$, about $1 \times 10^7$ to $5 \times 10^7$, about $5 \times 10^7$ to $1 \times 10^8$, about $1 \times 10^8$ to $5 \times 10^8$, about $5 \times 10^8$ to $1 \times 10^9$, about $1 \times 10^9$ to $5 \times 10^9$, about $5 \times 10^9$ to $1 \times 10^{10}$, about $1 \times 10^{10}$ to $5 \times 10^{10}$, about $5 \times 10^{10}$ to $1 \times 10^{11}$, about $5 \times 10^{11}$ to $1 \times 10^{12}$, about $1 \times 10^{12}$ to $5 \times 10^{12}$, or about $5 \times 10^{12}$ to $1 \times 10^{13}$.

In some embodiments, the concentration of the TILs provided in the composition of the present application can be less than, e.g., about 100%, about 90%, about 80%, about 70%, about 60%, about 50%, about 40%, about 30%, about 20%, about 19%, about 18%, about 17%, about 16%, about 15%, about 14%, about 13%, about 12%, about 11%, about 10%, about 9%, about 8%, about 7%, about 6%, about 5%, about 4%, about 3%, about 2%, about 1%, about 0.5%, about 0.4%, about 0.3%, about 0.2%, about 0.1%, about 0.09%, about 0.08%, about 0.07%, about 0.06%, about 0.05%, about 0.04%, about 0.03%, about 0.02%, about 0.01%, about 0.009%, about 0.008%, about 0.007%, about 0.006%, about 0.005%, about 0.004%, about 0.003%, about 0.002%, about 0.001%, about 0.0009%, about 0.0008%, about 0.0007%, about 0.0006%, about 0.0005%, about 0.0004%, about 0.0003%, about 0.0002%, or about 0.0001% w/w, w/v or v/v of the composition.

In some embodiments, the concentration of the TILs provided in the composition of the present application can be greater than about 90%, about 80%, about 70%, about 60%, about 50%, about 40%, about 30%, about 20%, about 19.75%, about 19.50%, about 19.25%, about 19%, about 18.75%, about 18.50%, about 18.25%, about 18%, about 17.75%, about 17.50%, about 17.25%, about 17%, about 16.75%, about 16.50%, about 16.25%, about 16%, about 15.75%, about 15.50%, about 15.25%, about 15%, about 14.75%, about 14.50%, about 14.25%, about 14%, about 13.75%, about 13.50%, about 13.25%, about 13%, about 12.75%, about 12.50%, about 12.25%, about 12%, about 11.75%, about 11.50%, about 11.25%, about 11%, about 10.75%, about 10.50%, about 10.25%, about 10%, about 9.75%, about 9.50%, about 9.25%, about 9%, about 8.75%, about 8.50%, about 8.25%, about 8%, about 7.75%, about 7.50%, about 7.25%, about 7%, about 6.75%, about 6.50%, about 6.25%, about 6%, about 5.75%, about 5.50%, about 5.25%, about 5%, about 4.75%, about 4.50%, about 4.25%, about 4%, about 3.75%, about 3.50%, about 3.25%, about 3%, about 2.75%, about 2.50%, about 2.25%, about 2%, about 1.75%, about 1.50%, about 125%, about 1%, about 0.5%, about 0.4%, about 0.3%, about 0.2%, about 0.1%, about 0.09%, about 0.08%, about 0.07%, about 0.06%, about 0.05%, about 0.04%, about 0.03%, about 0.02%, about 0.01%, about 0.009%, about 0.008%, about 0.007%, about 0.006%, about 0.005%, about 0.004%, about 0.003%, about 0.002%, about 0.001%, about 0.0009%, about 0.0008%, about 0.0007%, about 0.0006%, about 0.0005%, about 0.0004%, about 0.0003%, about or 0.0002%, or about 0.0001% w/w, w/v or v/v of the composition.

In some embodiments, the concentration of the TILs provided in the composition of the present application can range from about 0.0001% to about 50%, about 0.001% to about 40%, about 0.01% to about 30%, about 0.02% to about 29%, about 0.03% to about 28%, about 0.04% to about 27%, about 0.05% to about 26%, about 0.06% to about 25%, about 0.07% to about 24%, about 0.08% to about 23%, about 0.09% to about 22%, about 0.1% to about 21%, about 0.2% to about 20%, about 0.3% to about 19%, about 0.4% to about 18%, about 0.5% to about 17%, about 0.6% to about 16%, about 0.7% to about 15%, about 0.8% to about 14%, about 0.9% to about 12%, or about 1% to about 10% w/w, w/v or v/v of the composition.

In some embodiments, the concentration of the TILs provided in the composition of the present application can range from about 0.001% to about 10%, about 0.01% to about 5%, about 0.02% to about 4.5%, about 0.03% to about 4%, about 0.04% to about 3.5%, about 0.05% to about 3%, about 0.06% to about 2.5%, about 0.07% to about 2%, about 0.08% to about 1.5%, about 0.09% to about 1%, or about 0.1% to about 0.9% w/w, w/v or v/v of the composition.

In some embodiments, the amount of the TILs provided in the composition of the present application can be equal to or less than about 10 g, about 9.5 g, about 9.0 g, about 8.5 g, about 8.0 g, about 7.5 g, about 7.0 g, about 6.5 g, about 6.0 g, about 5.5 g, about 5.0 g, about 4.5 g, about 4.0 g, about 3.5 g, about 3.0 g, about 2.5 g, about 2.0 g, about 1.5 g, about 1.0 g, about 0.95 g, about 0.9 g, about 0.85 g, about 0.8 g, about 0.75 g, about 0.7 g, about 0.65 g, about 0.6 g, about 0.55 g, about 0.5 g, about 0.45 g, about 0.4 g, about 0.35 g, about 0.3 g, about 0.25 g, about 0.2 g, about 0.15 g, about 0.1 g, about 0.09 g, about 0.08 g, about 0.07 g, about 0.06 g, about 0.05 g, about 0.04 g, about 0.03 g, about 0.02 g, about 0.01 g, about 0.009 g, about 0.008 g, about 0.007 g, about 0.006 g, about 0.005 g, about 0.004 g, about 0.003 g, about 0.002 g, about 0.001 g, about 0.0009 g, about 0.0008 g, about 0.0007 g, about 0.0006 g, about 0.0005 g, about 0.0004 g, about 0.0003 g, about 0.0002 g, or about 0.0001 g.

In some embodiments, the amount of the TILs provided in the composition of the present application can be greater than about 0.0001 g, about 0.0002 g, about 0.0003 g, about 0.0004 g, about 0.0005 g, about 0.0006 g, about 0.0007 g, about 0.0008 g, about 0.0009 g, about 0.001 g, about 0.0015 g, about 0.002 g, about 0.0025 g, about 0.003 g, about 0.0035 g, about 0.004 g, about 0.0045 g, about 0.005 g, about 0.0055 g, about 0.006 g, about 0.0065 g, about 0.007 g, about 0.0075 g, about 0.008 g, about 0.0085 g, about 0.009 g, about 0.0095 g, about 0.01 g, about 0.015 g, about 0.02 g, about 0.025 g, about 0.03 g, about 0.035 g, about 0.04 g, about 0.045 g, about 0.05 g, about 0.055 g, about 0.06 g, about 0.065 g, about 0.07 g, about 0.075 g, about 0.08 g, about 0.085 g, about 0.09 g, about 0.095 g, about 0.1 g, about 0.15 g, about 0.2 g, about 0.25 g, about 0.3 g, about 0.35 g, about 0.4 g, about 0.45 g, about 0.5 g, about 0.55 g, about 0.6 g, about 0.65 g, about 0.7 g, about 0.75 g, about 0.8 g, about 0.85 g, about 0.9 g, about 0.95 g, about 1 g, about 1.5 g, about 2 g, about 2.5 g, about 3 g, about 3.5 g, about 4 g, about 4.5 g, about 5 g, about 5.5 g, about 6 g, about 6.5 g, about 7 g, about 7.5 g, about 8 g, about 8.5 g, about 9 g, about 9.5 g, or about 10 g.

In some embodiments, the TILs can be administered in a single dose. Such administration can be by injection, e.g., intravenous injection. In some embodiments, the TILs can be administered in multiple doses. The doses can be once, twice, three times, four times, five times, six times or more than six times per year. The doses can be once a month, once every two weeks, once a week or once every 2 days. In some embodiments, the administration of the TILs can be continuous.

In one aspect, the present application provides a pharmaceutical composition. In some embodiments, the pharmaceutical composition may comprise the TILs of the present application and/or the composition of the present application, as well as a pharmaceutically acceptable carrier.

In one aspect, the present application provides a kit, which may comprise T cell activators, T cell growth factors and/or feeder cells used in the method for culturing tumor-infiltrating lymphocytes (TILs) in the present application, and instructions describing the steps of the method for culturing tumor-infiltrating lymphocytes (TILs) in the present application. In one aspect, the present application provides a kit, which may comprise the TILs of the present application and/or the pharmaceutical composition of the present application.

In one aspect, the present application provides a method for affecting the tumor cell growth, which may comprise administering to a subject the TILs of the present application and/or the pharmaceutical composition of the present application. In some embodiments, affecting the tumor growth may comprise reducing the volume of the tumor to, e.g., about 99%, about 95%, about 90%, about 80%, about 70%, about 60%, about 50%, about 40%, about 30%, about 20%, about 19%, about 18%, about 17%, about 16%, about 15%, about 14%, about 13%, about 12%, about 11%, about 10%, about 9%, about 8%, about 7%, about 6%, about 5%, about 4%, about 3%, about 2%, about 1%, about 0.5%, about 0.4%, about 0.3%, about 0.2% or about 0.1% of its volume before the administration.

In one aspect, the present application provides use of the TILs of the present application and/or the pharmaceutical composition of the present application for the manufacture of drugs which can be used for preventing and/or treating a tumor. In some embodiments, the tumors in the present application are selected from solid tumors. In some embodiments, the tumors in the present application can be one or more of the tumors selected from the group consisting of: melanoma, ovarian cancer, cervical cancer, lung cancer, bladder cancer, breast cancer, head and neck cancer, pancreatic cancer, liver cancer, gastric cancer, colorectal cancer, and kidney cancer.

In one aspect, the present application provides a method for preventing and/or treating a tumor, which may comprise administering to a subject the TILs of the present application and/or the pharmaceutical composition of the present application. In some embodiments, the tumors in the present application are selected from solid tumors. In some embodiments, the tumors in the present application can be one or more of the tumors selected from the group consisting of: melanoma, ovarian cancer, cervical cancer, lung cancer, bladder cancer, breast cancer, head and neck cancer, pancreatic cancer, liver cancer, gastric cancer, colorectal cancer, and kidney cancer.

In one aspect, the present application provides the TILs of the present application and/or the pharmaceutical composition of the present application, which can be used for preventing and/or treating a tumor. In some embodiments, the tumors in the present application are selected from solid tumors. In some embodiments, the tumors in the present application can be one or more of the tumors selected from the group consisting of: melanoma, ovarian cancer, cervical cancer, lung cancer, bladder cancer, breast cancer, head and neck cancer, pancreatic cancer, liver cancer, gastric cancer, colorectal cancer, and kidney cancer.

Without intending to be limited by any theory, the examples below are intended only to illustrate the method and use of the present application and are not intended to limit the inventive scope of the present application.

**Examples**

**Example 1 Method for culturing tumor-infiltrating lymphocytes (TILs)**

1.1 Reception and preparation of feeder cells

1.1.1 Reception of apheresis blood

The information of the apheresis blood, batch numbers and volumes were recorded, and the blood sample was rewarmed to room temperature.

1.1.2 Manual isolation and cryopreservation of PBMCs (peripheral blood mononuclear cells)

A blood bag was sterilized with 75% alcohol and transferred into a biosafety cabinet. After the blood bag cutting with a sterile scissor, the apheresis blood was transferred into a 50 mL centrifuge tubes. The blood bag was washed with 20 mL of PBS or normal saline injected by a 20 mL syringe. The washing solution was also transferred into the 50 mL centrifuge tubes. The liquid volume in each 50 mL centrifuge tube should not exceed 30 mL. The apheresis blood was centrifuged at 3000 g for 10 minutes. During the centrifugation, 6-8 centrifuge tubes of 50 mL were prepared, into each of which was added rewarmed lymphocyte separation solution (Ficoll, Tianjin Haoyang) at 20 mL per tube. At the end of centrifugation, the upper layer of plasma was discarded, and the cell pellets were diluted with PBS or normal saline. The diluted blood cell mixed solution was slowly added dropwise onto the upper layer of the lymphocyte separation solution without destroying the interface at about 25 mL of samples per tube, and the final volume within each tube should be no more than 28 mL.

Centrifugation was carried out at temperature of 18-22°C and 500-600 g for 15-30 minutes using a horizontal rotor. After centrifugation, the resulting buffy coat will be at the interface between normal saline and the lymphocyte isolation solution Ficoll. The upper layer of plasma and normal saline were aspirated off, and the middle buffy coat was transferred to another 50 mL sterile centrifuge tube with a pipette. The collected buffy coat was diluted with PBS or normal saline and centrifuged at room temperature and 600 g for 10 minutes. At the end of centrifugation, the supernatant was discarded. The cells were washed once with PBS or normal saline and centrifuged at room temperature and 500 g for 5 minutes.

If there were lots of remanent red blood cells, the red blood cells could be lysed after the centrifugation. A red blood cell lysis solution was added at a volume ratio of 1:2 to 1:3 of the cell pellets to the red blood cell lysis solution and mixed well. The red blood cells were lysed at room temperature for 10 minutes, during which the centrifuge tubes were gently mixed 2-3 times to ensure the lysis effect. After the lysis was completed, PBS or normal saline were added to wash the cells. After the lysis, the cells were washed twice, centrifuged at 400 g for 6 minutes, and counted before the last centrifugation.

The supernatant was discarded, the cells were resuspended in the basic medium at a cell density adjusted to about $2\text{-}3 \times 10^7$/mL, where the liquid level should be not higher than 1 cm, and the volume in each T225 culture flask should be less than 200 mL. The suspension was irradiated with X-rays at 50 Gy in the tiled state. The supernatant was discarded after centrifugation, and the cells were cryopreserved according to the counting results at about $1\text{-}2 \times 10^8$/mL and 1-2 mL/tube. The cells were placed in a programmed cooling box and transferred to a -80°C freezer for cryopreservation.

1.1.3 Automatic isolation and cryopreservation of PBMCs

The tubing of the blood bag was aseptically connected to the input end of a cpro isolation kit (Cytiva). If the blood volume was more than 120 mL, a pre-concentration step might be performed to concentrate the blood volume to less than 120 mL. A neatcell procedure might be used to isolate and wash PBMCs, where the washing solution was normal saline, and the intermediate volume was 20 mL; the resuspending solution was the basic medium and was added at 80 mL/batch. After isolation, the PBMCs for each donor were one bag of 100 mL. When in the tiled state, the liquid level might be no more than 1 cm, and the suspension was irradiated with X-rays at 50 Gy. Sampling and counting were carried out after irradiation. Cells were collected and washed three times using a culture wash procedure, where the washing solution was normal saline; the intermediate volume and the final volume were set so that the volume per $1\times10^9$ cells was not less than 2 mL/; an equal amount to 2-fold amount of cryopreservation solution was added and mixed well. The cell density was adjusted to about $1\times10^7$ cells/mL to $2\times10^8$ cells/mL with a 1-fold cryopreservation solution. The suspension was subpackaged at 20 mL per bag, cryopreserved in a programmed cooler, and stored in liquid nitrogen.

1.2 Reception and processing of tumor tissues

1.2.1 Reception of tissues

Tumor tissues and blood samples were received from donors. The sample information was checked and recorded, and corresponding sample labels were printed.

1.2.2 Tissue processing and culture

The sample tubes and blood collection tubes were sterilized with 75% alcohol and transferred into a biosafety cabinet. The PBMC cells in the blood samples were isolated and cryopreserved according to the above procedures for manual isolation and cryopreservation of PBMCs. A kind of culture flasks and bags with gas permeable surfaces, e.g., a culture bag (Origen), were taken, into which were added 300 mL of rewarmed complete medium that could be optionally selected from X-vivo 15 culture medium or other commercially available T cell culture media, e.g., T cell culture media of Stem Cell, Lonza, Thermo, Miltenyi etc., as well as essential amino acids and antibiotics, and IL-2 at an added concentration of about 300 to 9000 IU/mL (e.g., the concentration may be 1000 to 9000 IU/mL, e.g., it may be 6000 IU/mL). Several culture dishes of 10 cm were taken, into which was added an appropriate amount of culture medium. The tumor tissues were taken out from the sample tubes into the 10 cm culture dishes using sterile ophthalmic forceps. The amount of the culture medium should be just above the tumor tissues, and the tissue morphology was observed and recorded. The tissues were washed and the culture dishes were replaced. The tissues were cut initially using ophthalmic scissors and ophthalmic forceps to remove adipose tissues and necrotic tissues. Each tissue piece was further cut to a size of about 27 mm$^3$. Non-suspended tumor tissue pieces were taken. A 20 mL syringe, after removing the inner piston, was connected to the culture bag, through which about 1 g of tissue pieces were transferred into the culture bag using a pipette. The culture bag was placed in a carbon dioxide incubator for culture. The scissors and forceps were cleaned and initially disinfected with 75% alcohol, and then sterilized after ultrasonic cleaning to obtain the first TIL population.

1.3 Step (A): *In vitro* expansion and harvest

1.3.1 *In vitro* expansion in step (A)

According to the cell growth status, the culture medium was replenished or half-changed every 3-7 days to ensure cell nutrition. A complete culture medium was used, which may be optionally selected from X-vivo 15 culture medium or other commercially available T cell culture media, e.g. T cell culture media of Stem Cell, Lonza, Thermo, Miltenyi etc., and into which were added essential amino acids and antibiotics as well as IL-2 (SL Pharm) at an added concentration of 300 to 9000 IU/mL (e.g., the concentration may be 1000 to 9000 IU/mL, e.g., it may be 6000 IU/mL). Sampling and counting were carried out on days 3-14, e.g., on days 13 or 14, of the step (A). If the cell number was between $5 \times 10^5$ and $5 \times 10^8$, the cells entered the harvest step of the step (A).

1.3.2 Harvest in step (A)

The cells at the end of *in vitro* expansion of step (A) were collected and centrifuged. The culture medium was discarded. The cells were washed once with PBS or normal saline to obtain the TILs that have been subjected to the *in vitro* expansion of step (A). Sampling and counting were carried out to leave an amount of about $5 \times 10^5$ to $2 \times 10^8$ cells to enter the subsequent step *of in vitro* expansion; an amount of about $5 \times 10^5$ cells could be taken for quality control detection; the rest of the cells were cryopreserved in a cryopreservation solution as the cryopreserved preREP TIL *in vitro* cells.

1.4 Step (B): Activation of TILs

The TILs (the second TIL population) expanded *in vitro* by step (A) were continued to be cultured, or the cryopreserved preREP TIL cells are resuscitated *in vitro* for TIL activation in step (B).
A complete medium was used, which may be optionally selected from X-vivo 15 culture medium or other commercially available T cell culture media, e.g., T cell culture media of Stem Cell, Lonza, Thermo, Miltenyi etc. Moreover, essential amino acids and antibiotics could be added into a suspension 24-well culture plate at 1 mL/well to adjust the cell density to $5 \times 10^5$ to $2 \times 10^6$ cells/mL, and IL-2 was also added at a concentration of 300 to 9000 IU/mL (e.g., the concentration may be 1000 to 9000 IU/mL, e.g., it may be 6000 IU/mL). Into the culture medium of each TIL cell population could also be added T cell activators, e.g., a CD3 agonist and/or a CD28 agonist, e.g., about 30 ng/mL of CD3 antibody (Miltenyi Biotech, OKT3), about 30 ng/mL of CD28 antibody (Merck, 15E8), magnetic beads (diameter of about 1 to 10 $\mu$m, Dynabeads, thermo Fisher) at a ratio of magnetic beads to TILs of about 1:2 to 2:1, and/or transACT at a ratio of transACT (diameter of about 100 to 500 nm, Miltenyi) to TILs of about 1: 100 to 1 :2000. Upon culture for about 0-4 days, a third TIL population was obtained.

1.5 Step (C): Transduction of TIL cells

One day before transduction, a 24-well suspension culture plate was coated with recombinant human fibrin fragments (Retronectin, Takara) at a final concentration of 15 $\mu$g/mL, 250 $\mu$L for per well of the 24-well plate. The plate was protected from light and left at 4°C overnight for later use. The coated 24-well plate was taken out, from which the coating solution was aspirated and discarded, and into which was add 500 $\mu$L of 2% BSA blocking solution to block at room temperature for 30 minutes. The blocking solution was aspirated and discarded, the plate was washed twice with 500 $\mu$L of cleaning liquid comprising 2.5% HEPES for each well, and the cleaning liquid was then aspirated and discarded. The test group was transduced by retroviruses carrying IL-15 (its amino acid sequence may be as shown in SEQ ID NO: 1), and the GFP control group was transduced by retroviruses carrying GFP.
The plasmids of retroviruses carrying IL-15 may be constructed by synthesizing the IL-15 gene fragments of the present application, digesting with EcoRI+NotI, and recycling the IL-15 exogenous fragments. The plasmid MP71 was digested with EcoRI+NotI, and the vector fragments were recycled. The exogenous fragments were ligated to the vector fragments with T4 Ligase to obtain plasmid of retroviruses carrying IL-15. The plasmids of retroviruses carrying GFP were constructed by a similar method.
0.25-2 mL of retrovirus solution was added into each well and centrifuged at 2000 g at 32°C for 2 hours. The negative control group was not subjected to cell transduction. The supernatant of the 24-well plate was discarded, and a volume of 300-500 $\mu$L of the third TIL population was added into each well of the 24-well plate at a cell concentration of about $1\times10^6$/mL and centrifuged at 1000 g at 30-32°C for 10 minutes. At the end of centrifugation, the culture plate was incubated in a 5% $CO_2$ incubator at 37°C to obtain transduced cells. After the transduction, the cells were cultured for about 0-4 days to obtain the fourth TIL population.

1.6 Step (D): Culture of TIL cells after transduction

Feeder cells were added into the fourth TIL cells population for culture. The time of TILs contacting with feeder cells needs to be at a certain time Tn later after the contact of TILs with IL-2 and T cell activators in step (B) ($T_n$ for each test group may be 0 hours to 12 days, e.g., 24 hours or 48 hours). First, feeder cells mixed from 1-5 donors were resuscitated; the activated TIL cells were mixed with the feeder cells at a ratio of TIL cells to feeder cells of about 1:200, and transferred into G-Rex100 culture flasks or gas-permeable bags which was supplemented with a complete medium. Sampling and counting were carried out every 1-3 days, and the medium was replenished or half-changed according to the cell status until the total number of the cells were greater than $1\times10^9$, or the culture time *of in vitro* expansion in step (D) reached about 5 days to 14 days, then the *in vitro* expansion culture in step (D) was terminated.

1.7 Harvest of tumor-infiltrating lymphocytes

The cells expanded in step (D) were centrifuged, and after discarding the supernatant of the culture medium, the cells were washed three times with PBS or normal saline or a compound electrolyte solution to obtain TILs subjected to expansion in step (D) (the fifth TIL population). Sampling and counting were carried out during the third washing. According to the counting results, the supernatant was discarded after the last centrifugation, and $3\times10^6$ cells were taken for quality control detection; all the remaining cells were added into a cryopreservation solution, and the cell density was adjusted to $1-3\times10^8$/mL for cryopreservation.

**Example 2 Detection of the transduction efficiency of TIL cells**

Starting from day 14 after the transduction, the transduction efficiency of the TIL cells of the present application was detected by a flow cytometer (Beckman CouLter) every 3 days.

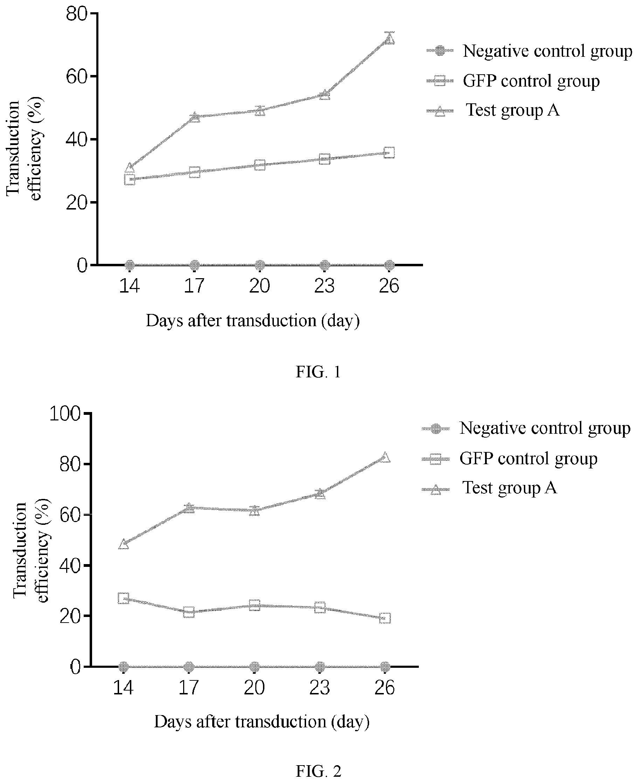

FIG. 1 shows the IL-15 transduction efficiency of CD4$^+$ cells in each group, for TIL cells derived from donor A.
FIG. 2 shows the IL-15 transduction efficiency of CD8$^+$ cells in each group, for TIL cells derived from donor A. The results show that the IL-15 of the present application may achieve high transduction efficiency in TIL cells, and the transduction efficiency of IL-15 continues to increase.

**Example 3 Detection of the expansion efficiency and viability of TIL cells**

IL-2 is an important factor to regulate T cell growth. This Example is intended to detect whether the TIL cells transduced with IL-15 of the present application can still survive and/or expand independent of IL-2.
Starting from day 8 after transduction, each group of TIL cells were re-plated with the same total number of cells and cultured with IL-2-free cell culture medium, i.e., IL-2 was withdrawn for culture. The expansion efficiency and viability of TIL cells were analyzed by a cell counter every 3 days. Where, the expansion efficiency was expressed as normalized expansion rate: the total cell number on the day when IL-2 was withdrawn (day 0) was taken as 100%, the normalized expansion rate on day n was (the total cell number on day n / the total cell number on day 0)$\times$100%; the viability indicated the number of surviving cells as a percentage of the total cell number.

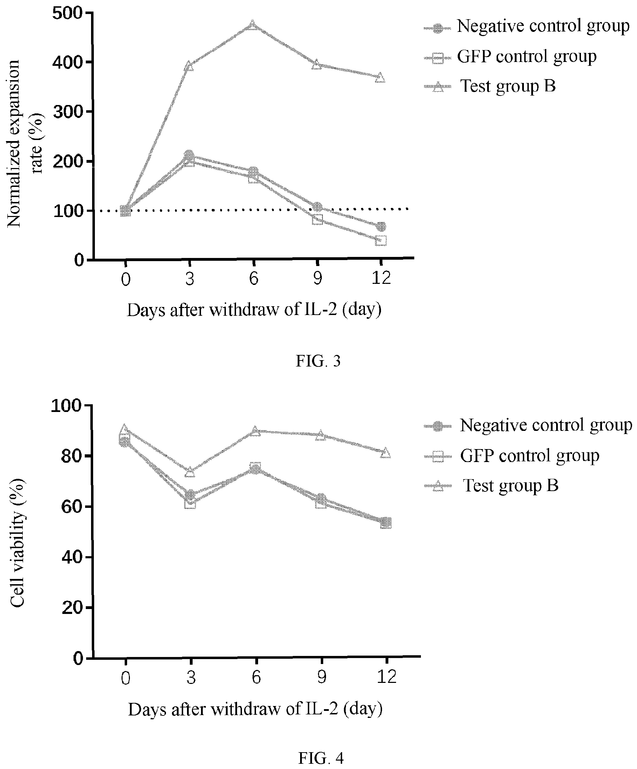

FIG. 3 shows the normalized expansion rate of TIL cells in each group, for TIL cells derived from donor B.
FIG. 4 shows the viability of TIL cells in each group, for TIL cells derived from donor B. The results show that, even with the withdrawal of IL-2, the TIL cells transduced with IL-15 in the present application can still have significant expansion capacity and can maintain a high cell viability.

**Example 4 Expansion assay of TIL cells**

Starting from day 8 after transduction, each group of TIL cells were re-plated with the same total number of cells. 3 days later, the expansion was detected.
According to the instructions of CTGkit (CellTiter-Glo Luminescent Cell Viability Assay, Promega), CTG substrate (CellTiter-Glo Substrate) was mixed with CTG buffer (CellTiter-Glo Buffer) to prepare a CTG reaction solution. The cell suspension to test was added into a 96-well microtiter plate at 50 $\mu$L/well, and the well comprising only the culture medium was set as the background value of fluorescence. An equal volume of CTG reaction solution was added to each well, shaken on a horizontal shaker for 2 minutes and left at room temperature for 10 minutes to stabilize the fluorescence signal, and then the fluorescence value was read.

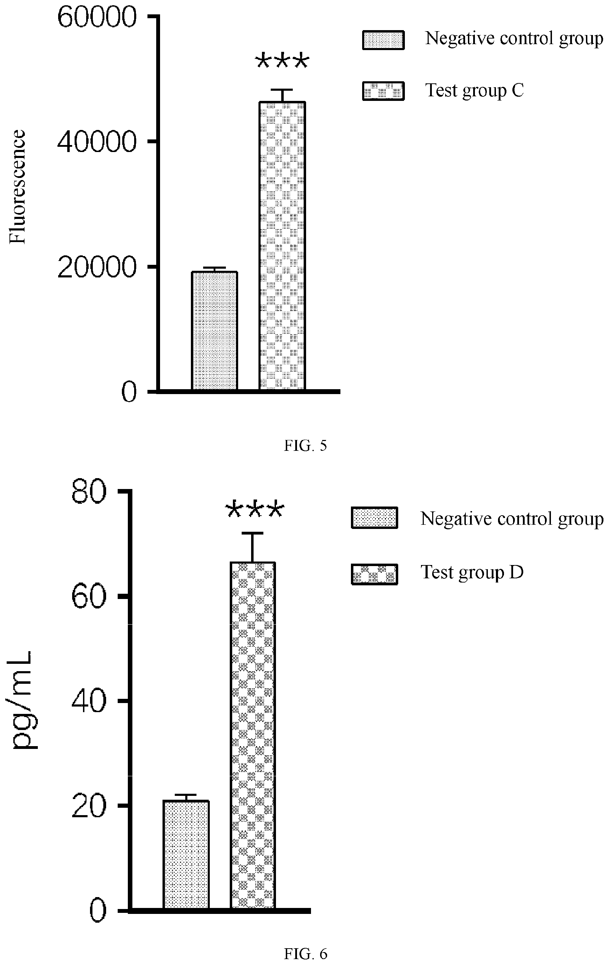

FIG. 5 shows the proliferation ability of TIL cells in each group, for TIL cells derived from donor C. *** indicated $p < 0.001$. The results show that TIL cells transduced with IL-15 may have significant expansion capacity.

**Example 5 Cytokine secretion assay of TIL cells**

Starting from day 8 after transduction, each group of TIL cells were re-plated with the same total number of cells, and CD3 antibodies (T&L Biotechnology) were simultaneously added to stimulate the TIL cells. 24 hours later, the supernatant was taken for cytokine secretion assay.

The cytokine secretion assay method can refer to the instructions of a cytokine detection kit (BD). The human Th1/Th2/Th17 cytokine standard lyophilized powders (BD) were reconstituted in 2 mL of Assay Diluent (BD) (the concentration of each cytokine in the standard stock solution was 5000 pg/mL), and gradiently diluted in the following order: 1:2, 1:4, 1:8, 1:16, 1:32, 1:64, 1:128, 1:256, 1: 512, and 1: 1024, and labeled as "standard tubes". A tube comprising Assay Diluent only was taken as a reference. Each kind of Capture Beads (BD) were added at 2 $\mu$L/Beads/well, then a PE Detection Reagent (BD) was added at 10 $\mu$L/well, and they were mixed to prepare a mix which was added to a V-bottom 96-well plate at 22 $\mu$L/well. Then, each standard and the supernatant of the test groups were added at 10 $\mu$L/well and mixed, and incubated at room temperature in dark for 3 hours.

At the end of the incubation, 200 $\mu$L of Wash Buffer (BD) was added to each well and centrifuged at 500 g for 3 minutes. At the end of the centrifugation, 100 $\mu$L of Wash Buffer (BD) was added to each well to resuspend for flow analysis.

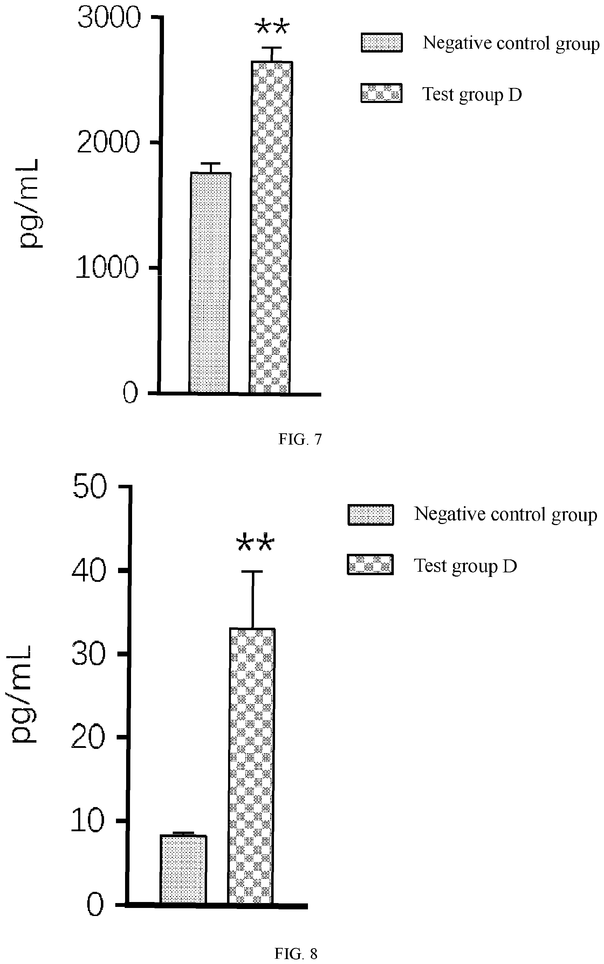

FIGs. 6, 7, and 8 show the secretion of cytokines IL-2, IFN-$\gamma$, and IL-10 in each group of TIL cells after being stimulated with the CD3 antibody, respectively, for TIL cells derived from donor D. *** indicated $p < 0.001$, ** indicated $p < 0.01$. The results show that the TIL cells transduced with IL-15 may have more significant cytokine secretion capacity after being stimulated with the CD3 antibody.

Starting from day 8 after transduction, each group of TIL cells were re-plated with the same total number of cells, and transACT (nanomaterials comprising CD3 antibodies and CD28 antibodies, Miltenyi) was simultaneously added to stimulate the TIL cells. 24 hours later, the supernatant was taken for cytokine secretion assay.

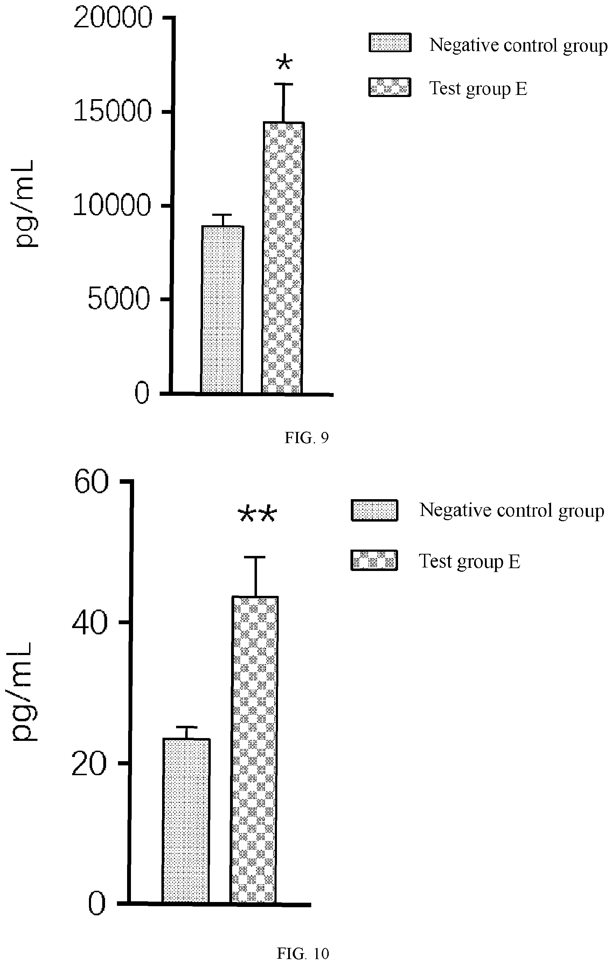

FIGs. 9 and 10 show the secretion of cytokines TNF-$\alpha$ and IL-10 in each group of TIL cells after being stimulated with transACT, respectively, for TIL cells derived from donor E. ** indicated $p < 0.01$, and * indicated $p < 0.05$. The results show that, TIL cells transduced with IL-15 may have more significant cytokine secretion capacity after being stimulated with the CD28 antibody.

Starting from day 8 after transduction, each group of TIL cells were re-plated with the same total number of cells, with no stimulation on the TIL cells. 24 hours later, the supernatant was taken for cytokine secretion assay.

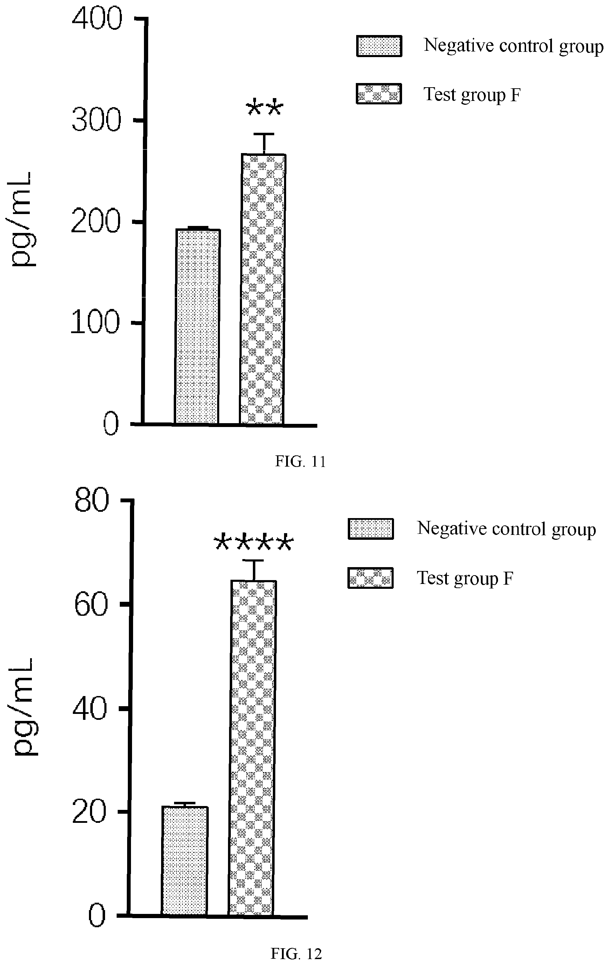

FIGs. 11 and 12 show the secretion of cytokines IFN-$\gamma$ and IL-2 in each group of TIL cells, respectively, for TIL cells derived from donor F. **** indicated $p < 0.0001$, ** indicated $p < 0.01$. The results show that, TIL cells transduced with IL-15 may have more significant cytokine secretion capacity.

Starting from day 8 after transduction, each group of TIL cells were re-plated with the same total number of cells and cultured with IL-2-free cell culture medium, i.e., IL-2 was withdrawn for culture. On the 15th day after IL-2 was withdrawn and the culture was continued, the supernatant was taken for cytokine secretion assay.

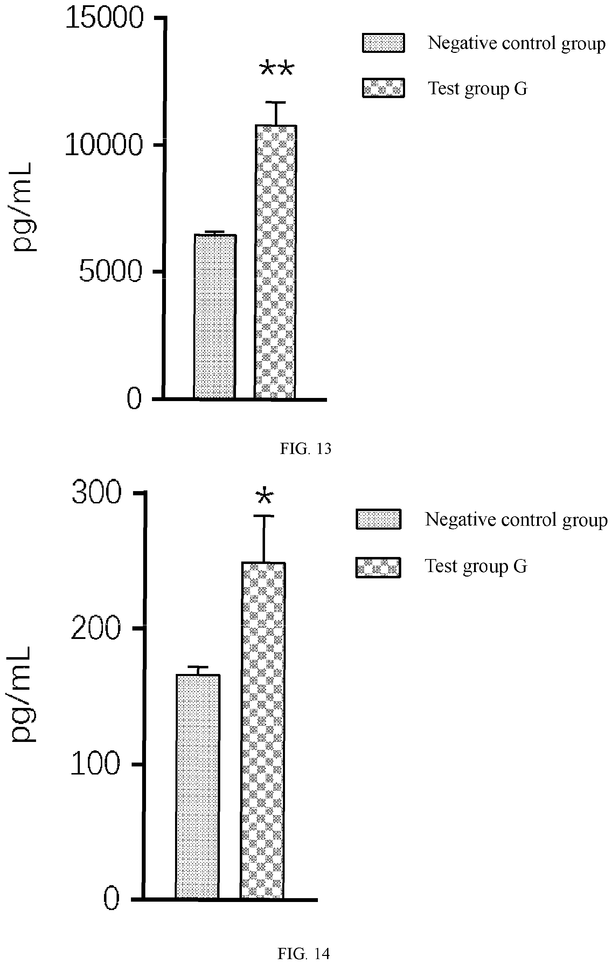

FIGs. 13 and 14 show the secretion of cytokines IFN-$\gamma$ and IL-6 in each group of TIL cells, respectively, for TIL cells derived from donor F which were cultured without IL-2. ** indicated $p < 0.01$, * indicated $p < 0.05$. The results show that, TIL cells transduced with IL-15 may have more significant cytokine secretion capacity in the absence of IL-2.

**Example 6 Detection of the killing ability of TIL cells**

Starting from day 7 after transduction, A375 tumor cell lines were plated in a 96-well plate at $2 \times 10^4$/well. The next day, each group of TIL cells were co-cultured with the above A375 cells at an effector-target ratio of (TIL cells : tumor cells) of 0.3:1 or 1:1.

According to the instructions of apoptosis detection reagents (Incucyte Caspase-3/7 Green Dye for Apoptosis, Sartorius), Incucyte® Caspase-3/7 Green Dye for Apoptosis was added at 0.2 $\mu$L/well, and the culture medium was added at 25 $\mu$L/well to dilute the Caspase 3/7 Green Dye. The activity of Caspase 3/7 was recorded by an Incucyte recorder (Sartorius) to analyze the killing ability of TILs against tumor cells, once every 3 hours and for a total record time of about 5 days.

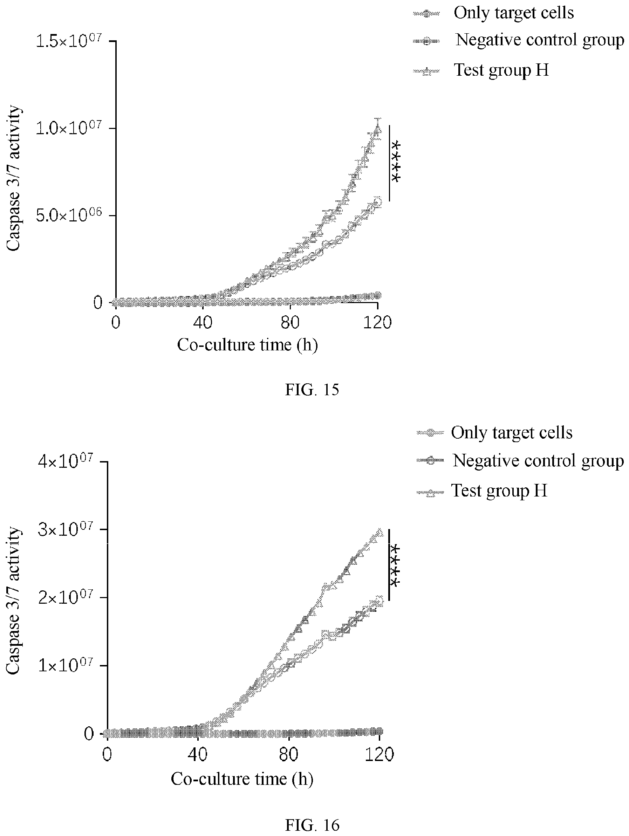

FIG. 15 shows the test results of the killing ability of TIL cells derived from donor H which were co-cultured with tumor cells at an effector-target ratio of 0.3:1. **** indicated $p < 0.0001$.

FIG. 16 shows the test results of the killing ability of TIL cells derived from donor H which are co-cultured with tumor cells at an effector-target ratio of 1:1. **** indicated $p < 0.0001$. The results show that, TIL cells transduced with IL-15 may have more significant cell killing ability.

The supernatant was taken for cytokine secretion assay 24 hours after the co-culture.

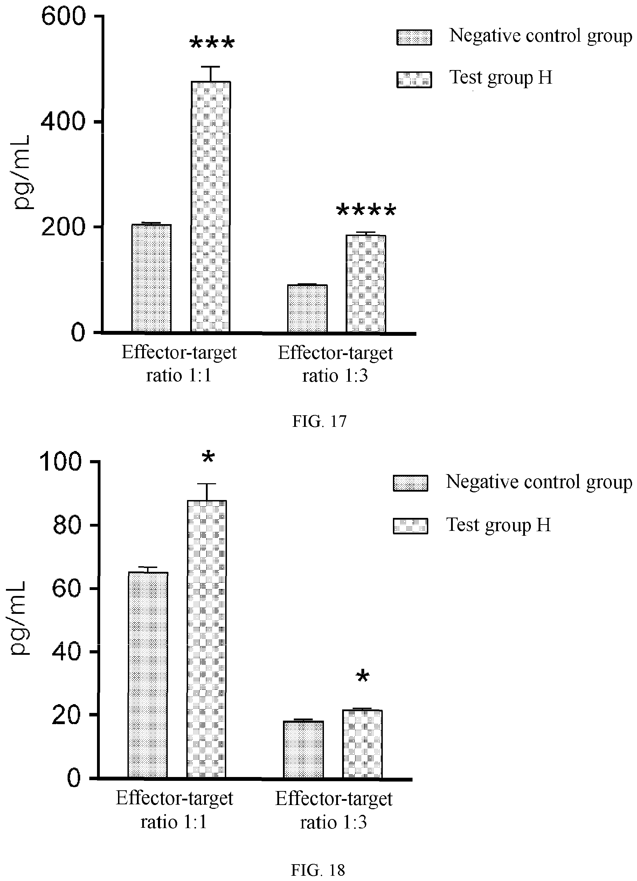

FIG. 17 shows the secretion results of cytokine IL-2 in TIL cells derived from donor H which are co-cultured with tumor cells at an effector-target ratio of 0.3:1 or 1:1. *** indicated $p < 0.001$, **** indicated $p < 0.0001$.

FIG. 18 shows the secretion results of cytokine IFN-$\gamma$ in TIL cells derived from donor H which are co-cultured with tumor cells at an effector-target ratio of 0.3:1 or 1:1. * indicated $p < 0.05$. The results show that, TIL cells transduced with IL-15 may have more significant cytokine secretion capacity when contacting with tumor cells.

**Example 7 Detection of the TCR clonal diversity of TIL cells**

Starting from day 8 after transduction, each group of TIL cells were re-plated with the same total number of cells and cultured with IL-2-free cell culture medium, i.e., IL-2 was withdrawn for culture. The clonal diversity of T cell receptors (TCRs) in TILs was analyzed on days 8 and 26 after transduction.

According to the instructions of TCR repertoire kit (Beta Mark TCR V$\beta$ Repertoire Kit, Beckman Coulter), into each of eight tubes from A to H were respectively added 4 $\mu$L of Beta Mark TCR V$\beta$ Repertoire Kit Tube A-H antibody, as well as 1 $\mu$L of BV510-labeled CD3 antibody (BD), 1 $\mu$L of PerCP-cy5.5-labeled CD8 antibody (BD), 1 $\mu$L of PE-cy7-labeled CD4 antibody (BD), and 0.01 $\mu$L of eFluor 780-labeled live/dead cell dye (eBioscience). The cells to test were divided into 8 portions, which were respectively added into the tubes A to H, mixed well, and incubated at 4°C for 30 minutes. At the end of staining, centrifugation was carried out and the supernatant was discarded. The cells were resuspended and washed once with PBS for assay by a flow cytometer.

The TCR diversity of TIL cells was identified according to the instructions of the TCR Repertoire kit (Beta Mark TCR V$\beta$ Repertoire Kit, Beckman Coulter). Table 1 shows the correspondence between the fluorescence from tubes A to H and the TCR V$\beta$ clones.

Table 1 Correspondence between fluorescence of tubes A to H and TCR V$\beta$ clones

| | TCR V$\beta$ Clones | Fluorescent Markers |
|---|---|---|
| Tube A | V$\beta$ 5.3 (TRBV5-5) | PE |
| | V$\beta$ 7.1 (TRBV4-1, TRBV4-2, TRBV4-3) | PE+FITC |
| | V$\beta$ 3 (TRBV28) | FITC |
| Tube B | V$\beta$ 9 (TRBV3-1) | PE |
| | V$\beta$ 17 (TRBV19) | PE+FITC |
| | V$\beta$ 16 (TRBV14) | FITC |
| Tube C | V$\beta$ 18 (TRBV18) | PE |
| | V$\beta$ 5.1 (TRBV5-1) | PE+FITC |
| | V$\beta$ 20 (TRBV30) | FITC |
| Tube D | V$\beta$ 13.1 (TRBV6-5, TRBV6-6, TRBV6-9) | PE |
| | V$\beta$ 13.6 (TRBV6-6) | PE+FITC |
| | V$\beta$ 8 (TRBV12-3, TRBV12-4) | FITC |
| Tube E | V$\beta$ 5.2 (TRBV5-6) | PE |
| | V$\beta$ 2 (TRBV20-1) | PE+FITC |
| | V$\beta$ 12 (TRBV10-3) | FITC |
| Tube F | V$\beta$ 23 (TRBV13) | PE |
| | V$\beta$ 1 (TRBV9) | PE+FITC |
| | V$\beta$ 21.3 (TRBV11-2) | FITC |
| Tube G | V$\beta$ 11 (TRBV25-1) | PE |
| | V$\beta$ 22 (TRBV2) | PE+FITC |
| | V$\beta$ 14 (TRBV27) | FITC |

(continued)

|  | TCR Vβ Clones | Fluorescent Markers |
|---|---|---|
| Tube H | Vβ 13.2 (TRBV6-2) | PE |
|  | Vβ 4 (TRBV29-1) | PE+FITC |
|  | Vβ 7.2 (TRBV4-3) | FITC |

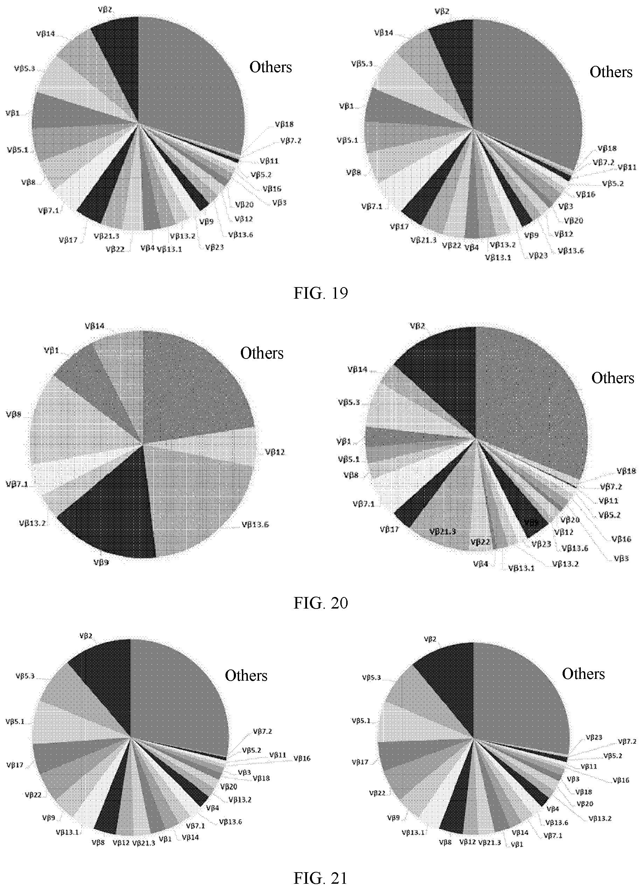

Table 2 and FIG. 19 show the diversity of CD8[+] T cell TCR Vβ clones on day 8 after transduction of TIL cells derived from donor I.

Table 2 Diversity of CD8[+] T cell TCR Vβ clones on day 8 after transduction

| TCR Vβ clones | Diversity of TCR Vβ clones in the negative control group (%) | Diversity of TCR Vβ clones in the test group I transduced with IL-15 (%) |
|---|---|---|
| Others | 29.78 | 31.28 |
| Vβ 23 | 0.3 | 0.41 |
| vβ 7.2 | 0.4 | 0.4 |
| vβ 5.2 | 0.59 | 0.86 |
| Vβ 11 | 0.8 | 1.08 |
| Vβ 16 | 0.85 | 0.98 |
| Vβ 3 | 1.13 | 1.83 |
| Vβ 18 | 1.14 | 0.97 |
| Vβ 20 | 1.67 | 1.5 |
| Vβ 13.2 | 1.74 | 1.48 |
| Vβ 4 | 1.84 | 1.85 |
| Vβ 13.6 | 1.97 | 1.81 |
| Vβ 7.1 | 2.07 | 2.13 |
| Vβ 14 | 2.33 | 2.48 |
| Vβ 1 | 2.7 | 2.27 |
| Vβ 21.3 | 3.12 | 3.16 |
| Vβ 12 | 3.32 | 3.46 |
| Vβ 8 | 4.21 | 3.6 |
| Vβ 13.1 | 4.51 | 5.17 |
| Vβ 9 | 4.64 | 4.72 |
| Vβ 22 | 5.12 | 4.61 |
| vβ 17 | 5.58 | 5.1 |
| Vβ 5.1 | 6.24 | 6.2 |
| Vβ 5.3 | 6.43 | 5.86 |
| Vβ 2 | 7.52 | 6.79 |

Table 3 and FIG. 20 show the diversity of CD8[+] T cell TCR Vβ clones on day 26 after transduction of TIL cells derived from donor I. Where, TCR Vβ clones are others, and may mean that T cells at such a proportion does not contain TCR Vβ that can be identified by the TCR repertoire kit.

Table 3 Diversity of CD8[+] T cell TCR Vβ clones on day 26 after transduction

| TCR Vβ clones | Diversity of TCR Vβ clones in the negative control group (%) | Diversity of TCR Vβ clones in the test group I transduced with IL-15 (%) |
|---|---|---|
| Others | 22.54 | 31.01 |
| Vβ 23 | 0 | 1.09 |
| vβ 7.2 | 0 | 0.017 |
| Vβ 5.2 | 0 | 0.21 |
| Vβ 11 | 0 | 0.95 |
| Vβ 16 | 0 | 1.08 |
| Vβ 3 | 0 | 1.27 |
| Vβ 18 | 0 | 0.73 |
| Vβ 20 | 5.56 | 1.47 |
| Vβ 13.2 | 20 | 0.89 |
| Vβ 4 | 15.8 | 3.74 |
| Vβ 13.6 | 0 | 1.34 |
| Vβ 7.1 | 3.7 | 1.55 |
| Vβ 14 | 0 | 1.65 |
| Vβ 1 | 0 | 0.64 |
| Vβ 21.3 | 0 | 3.34 |
| Vβ 12 | 0 | 9.24 |
| Vβ 8 | 0 | 3.2 |
| Vβ 13.1 | 4.55 | 5.71 |
| Vβ 9 | 13.3 | 2.29 |
| Vβ 22 | 0 | 2.34 |
| Vβ 17 | 7.14 | 2.98 |
| Vβ 5.1 | 0 | 6.61 |
| Vβ 5.3 | 7.41 | 3.25 |
| Vβ 2 | 22.54 | 13.4 |

Table 4 and FIG. 21 show the diversity of CD4[+] T cell TCR Vβ clones on day 8 after transduction of TIL cells derived from donor I.

Table 4 Diversity of CD4[+] T cell TCR Vβ clones on day 8 after transduction

| TCR Vβ clones | Diversity of TCR Vβ clones in the negative control group (%) | Diversity of TCR Vβ clones in the test group I transduced with IL-15 (%) |
|---|---|---|
| Others | 28.12 | 27.73 |
| Vβ 23 | 0 | 0.31 |
| vβ 7.2 | 0.17 | 0.12 |
| vβ 5.2 | 0.55 | 0.84 |
| Vβ 11 | 0.59 | 0.61 |
| Vβ 16 | 0.6 | 0.59 |
| Vβ 3 | 0.97 | 1.12 |

(continued)

| TCR Vβ clones | Diversity of TCR Vβ clones in the negative control group (%) | Diversity of TCR Vβ clones in the test group I transduced with IL-15 (%) |
|---|---|---|
| Vβ 18 | 1.25 | 1.13 |
| Vβ 20 | 1.48 | 1.68 |
| Vβ 13.2 | 1.56 | 1.44 |
| Vβ 4 | 2.12 | 1.91 |
| Vβ 13.6 | 2.19 | 1.97 |
| Vβ 7.1 | 2.2 | 2.21 |
| Vβ 14 | 2.35 | 2.09 |
| Vβ 1 | 2.58 | 2.71 |
| Vβ 21.3 | 2.81 | 2.76 |
| Vβ 12 | 2.9 | 2.58 |
| Vβ 8 | 3.77 | 4.11 |
| Vβ 13.1 | 3.84 | 3.86 |
| Vβ 9 | 3.93 | 4.25 |
| Vβ 22 | 4.83 | 5.41 |
| Vβ 17 | 5.1 | 5 |
| Vβ 5.1 | 7.2 | 6.91 |
| Vβ 5.3 | 7.69 | 7.66 |
| Vβ 2 | 11.2 | 11 |

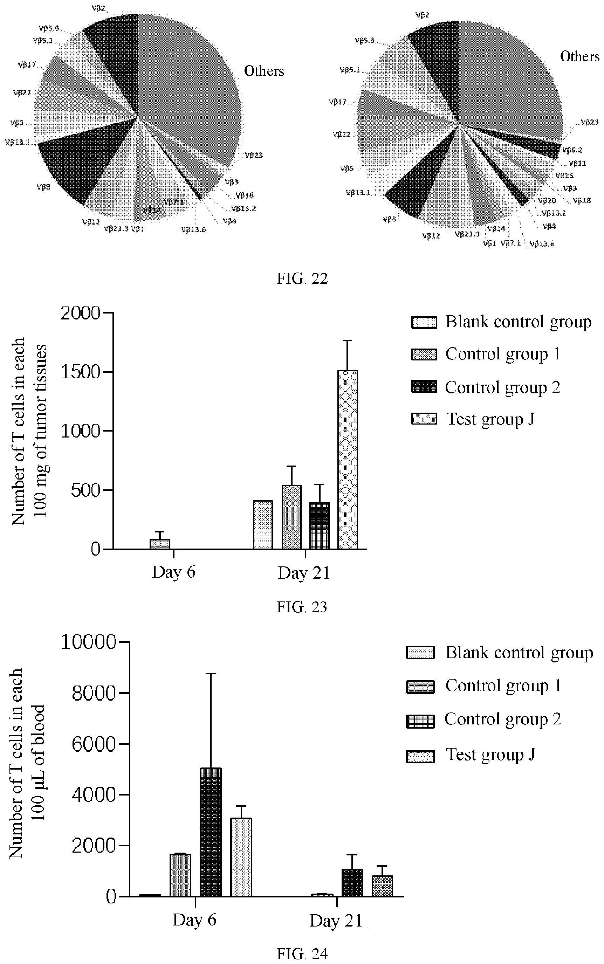

Table 5 and FIG. 22 show the diversity of CD4[+] T cell TCR Vβ clones on day 26 after transduction of TIL cells derived from donor I. Where, TCR Vβ clones are others, and may mean that T cells at such a proportion does not contain TCR Vβ that can be identified by the TCR repertoire kit.

Table 5 Diversity of CD4[+] T cell TCR Vβ clones on day 26 after transduction

| TCR Vβ clones | Diversity of TCR Vβ clones in the negative control group (%) | Diversity of TCR Vβ clones in the test group I transduced with IL-15 (%) |
|---|---|---|
| Others | 33.1 | 27.49 |
| Vβ 23 | 1.13 | 0.57 |
| vβ 7.2 | 0 | 0 |
| vβ 5.2 | 0 | 2.65 |
| Vβ 11 | 0 | 0.68 |
| Vβ 16 | 0 | 1.87 |
| Vβ 3 | 0.65 | 0.82 |
| Vβ 18 | 3.08 | 0.89 |
| Vβ 20 | 0 | 1.45 |
| Vβ 13.2 | 1.24 | 1.76 |
| Vβ 4 | 0.62 | 1.55 |
| Vβ 13.6 | 1.72 | 1.74 |
| Vβ 7.1 | 3.92 | 1.21 |

(continued)

| TCR Vβ clones | Diversity of TCR Vβ clones in the negative control group (%) | Diversity of TCR Vβ clones in the test group I transduced with IL-15 (%) |
|---|---|---|
| Vβ 14 | 4.05 | 1.42 |
| Vβ 1 | 1.13 | 3.51 |
| Vβ 21.3 | 3.39 | 2.21 |
| Vβ 12 | 4.82 | 6.76 |
| Vβ 8 | 12.1 | 6.48 |
| Vβ 13.1 | 1.15 | 3.64 |
| Vβ 9 | 4.22 | 3.96 |
| Vβ 22 | 4.73 | 6.16 |
| Vβ 17 | 4.22 | 3.82 |
| Vβ 5.1 | 3.08 | 4.94 |
| Vβ 5.3 | 2.61 | 5.92 |
| Vβ 2 | 9.04 | 8.5 |

The results show that, Vβ18, Vβ7.2, Vβ11, Vβ5.2, Vβ16, Vβ3, Vβ20, Vβ23, Vβ13.1, Vβ4, Vβ22, Vβ21.3, Vβ17, Vβ5.1, Vβ5.3 and Vβ2 in CD8[+] T cells, and Vβ 7.2, Vβ 5.2, Vβ 11, Vβ 16, and Vβ 20 in CD4[+] T cells disappeared on day 26 for non-transduced TIL cells, indicating the decrease of the TCR clonal diversity; TIL cells transduced with IL-15 may retain more β-chain subtypes and may significantly retain the TCR clonal diversity for a long time, thus helping achieve a stronger ability of antigen recognition and immune response against tumor cells.

**Example 8 Detection of the *in vivo* persistence and distribution of TIL cells**

Immunodeficient mice (NOG mice, Vital River, strain code 408) were subcutaneously inoculated with A375 cells at an inoculation number of about $1\times10^6$ cells per mouse. 7 days after inoculation, when tumor size reached about 50-80 mm$^3$, mice were grouped randomly depending on their tumor size (as shown in Table 6).

Table 6 Grouping

| Groups | Cell number | IL-2 injection volume (IU) | Number of mice in a group | Sample collection point |
|---|---|---|---|---|
| Blank control group | 0 | \ | 3 | Day 6, Day 21 |
| Control group | $5\times10^6$ | \ | 9 | Day 6, Day 21 |
| 1 | | | | |
| Control group 2 | $5\times10^6$ | $1\times10^5\times6$ | 6 | Day 6, Day 21 |
| Test group J | $5\times10^6$ | \ | 9 | Day 6, Day 21 |

Each group was injected in the tail vein with TILs derived from donor J (TILs not transduced with IL-15, TILs (not transduced with IL-15) + IL-2, TILs (transduced with IL-15), with an equal volume of PBS as a blank control) at a dose of $5\times10^6$ cells per mouse. The day on which TIL cells were injected was recorded as day 0. For the control group 2, intraperitoneal injection of IL-2 was performed at the same time, once every 12 hours for 6 consecutive times, and the injection dose of IL-2 was $1\times10^5$ IU per time. On days 6 and 21 after the injection of TIL cells, tumors, spleen, bone marrow, lung tissues and blood (anticoagulated) were removed from the mice, and the number of T cells and the dynamic distribution of tissues were analyzed by flow cytometry.

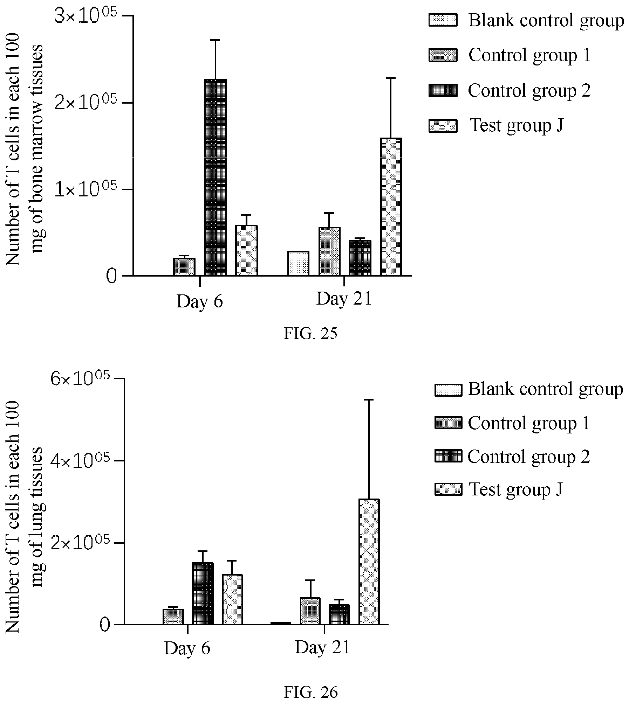

FIGs. 23, 24, 25, 26, and 27 respectively show the number of T cells in each tissue on day 6 and day 21 after injection of TIL cells. The results show that TIL cells transduced with IL-15 can have a longer *in vivo* persistence and that TIL cells transduced with IL-15 can have a stronger infiltration and/or expansion capacity at tumor tissue sites.

**Example 9 Comparison of proliferation ability of TILs cultured with feeder cells added at different time**

After some time $T_n$ ($T_n$ can range from 0 hours to 14 days) after the addition of II,-2 and different forms of T cell activators in Example 1, feeder cells were co-cultured with tumor-infiltrating lymphocytes. In this Example, $T_n$ was taken as 0 hours, 6 hours, 12 hours, 24 hours, 48 hours, 72 hours, 5 days, 7 days, and 9 days to obtain TILs cultured with the feeder cells added at different times, and a comparison experiment on the cell counts was performed.

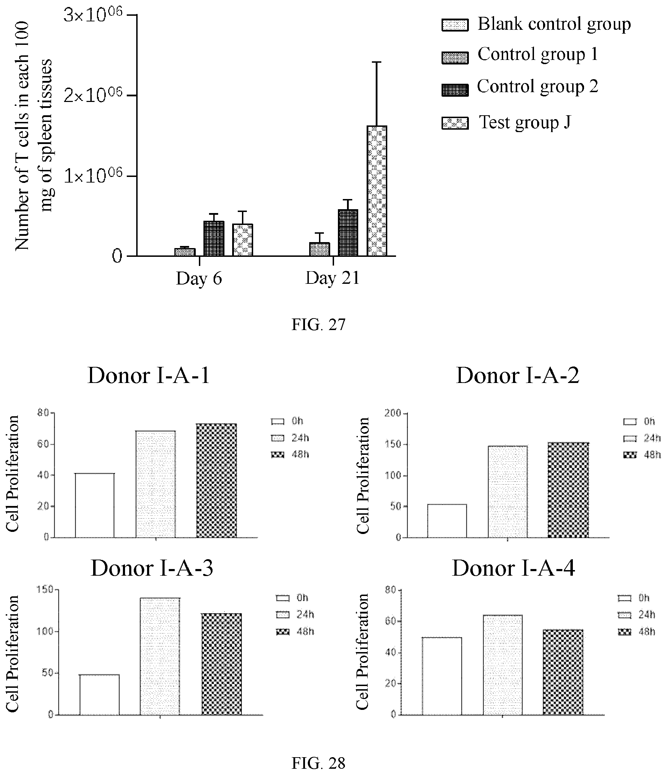

FIG. 28 shows the analysis results of the proliferation ability of TILs cultured with feeder cells added at different times. The values on the vertical coordinates in each group of graphs for TILs which were cultured with feeder cells added at different times indicate the expansion factor of the number of TIL cells after the end *of in vitro* expansion compared with before the start *of in vitro* expansion. The proliferation results of TILs from 4 donors show that the TILs cultured with feeder cells added at 0 hours after the addition of OKT3 and IL-2 (that is, at the same time) have weaker proliferation ability than that of TILs cultured with feeder cells added at 24 or 48 hours after the addition of OKT3 and IL-2.

**Example 10 Comparison of flow cytometry on TILs cultured with feeder cells added at different time**

After some time $T_n$ ($T_n$ can range from 0 hours to 14 days) after the addition of IL-2 and different forms of T cell activators in Example 1, feeder cells were co-cultured with tumor-infiltrating lymphocytes. In this Example, $T_n$ was taken as 0 hours, 6 hours, 12 hours, 24 hours, 48 hours, 72 hours, 5 days, 7 days, and 9 days to obtain TILs cultured with the feeder cells added at different times, and a comparison experiment on the flow cytometry was performed.

Sources of test materials for TIL flow cytometry

Transcription Factor Buffer Set, manufacturer BD, product number 562574; V-bottom 96-well plate, manufacturer Corning, product number 3894; flow tube, manufacturer Corning, product number 352052.

The flow antibodies in this example were purchased from BD or Biolegend. $1 \times 10^5$ to $5 \times 10^5$ cell samples per group were added into flow tubes or V-bottom 96-well plates. Centrifugation was performed at 600 g for 3 minutes and the supernatant was discarded. Washing was carried out once using PBS, with 1 mL/tube for flow tubes, and 250 $\mu$L/well for 96-well plates, and the supernatant was discarded. A prepared antibody working solution was added for cell surface staining. The concentration of the antibodies (BD or Biolegend) was 1:100 to 1:200, and the active detection dye was comprised in 1:10000. Staining was carried out with 100 $\mu$L/tube for flow tubes, and 50 $\mu$L/well for 96-well plates, and incubation was performed at 2-8°C in dark for 30 minutes. The reagents required for transcription factor staining was formulated during the staining process: Transcription Factor Buffer Set (BD) was used to dilute a $4\times$ Fixation/Permeabilization Solution (BD) to produce $1\times$ working solution A; double distilled water was used to dilute a $5\times$ Perm/Wash Buffer (BD) to produce $1\times$ working solution B. The working solutions were pre-cooled at 4 °C for use. After staining, an appropriate amount of PBS was added to wash the cells twice (250 $\mu$L/time for 96-well plates, 1 mL/time for flow tubes). Centrifugation was performed at 600 g for 3 minutes. After the centrifugation, the supernatant was discarded. Cell fixation and permeabilization: the cells were sufficiently resuspended. An appropriate amount (100 $\mu$L/well for 96-well plates, and 1 mL/tube for flow tubes) of $1\times$ working solution A was added to fix and permeabilize. Incubation was performed at 2-8°C in dark for 40-50 minutes. At the end of fixation and permeabilization, $1\times$ working solution B was added to wash the cells (250 $\mu$L/time for 96-well plates, and 2 mL/time for flow tubes). Centrifugation was performed at 2-8°C and at 350 g for 6 minutes, and washing was carried out twice. $1\times$ working solution B was used to prepare intracellular antibodies with 50 $\mu$L/well for 96-well plates, and 100 $\mu$L/tube for flow tubes, and the antibody concentration was 1:100 to 1:200. Staining was performed at 2-8°C in dark for 30 minutes. At the end of staining, $1\times$ working solution B was added to wash the cells (250 $\mu$L/time for 96-well plates, and 2 mL/time for flow tubes). Centrifugation was performed at 2-8°C and at 350 g for 6 minutes, and washing was carried out twice. At the end of surface staining, PBS was used to wash cells once (250 $\mu$L/time for 96-well plates, 1 mL/time for flow tubes). Centrifugation was performed at room temperature and at 600 g for 3 minutes. After the centrifugation, the supernatant was discarded. The cells were resuspended using 100-500 $\mu$L of PBS for flow cytometry assay on machine.

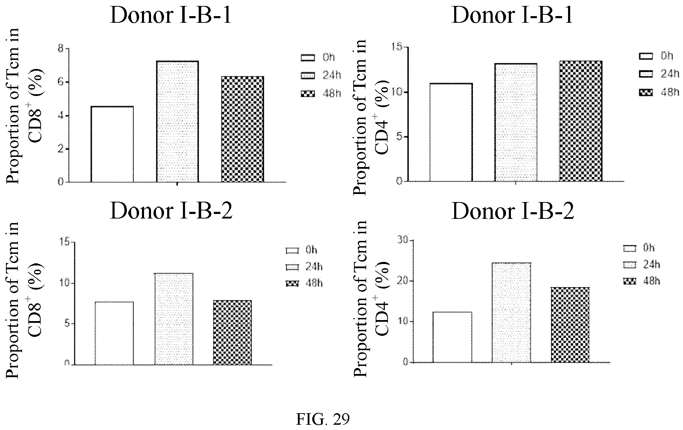

The analyses on flow cytometry results of TILs cultured with feeder cells added at different times are shown in FIG. 29 to FIG. 35.

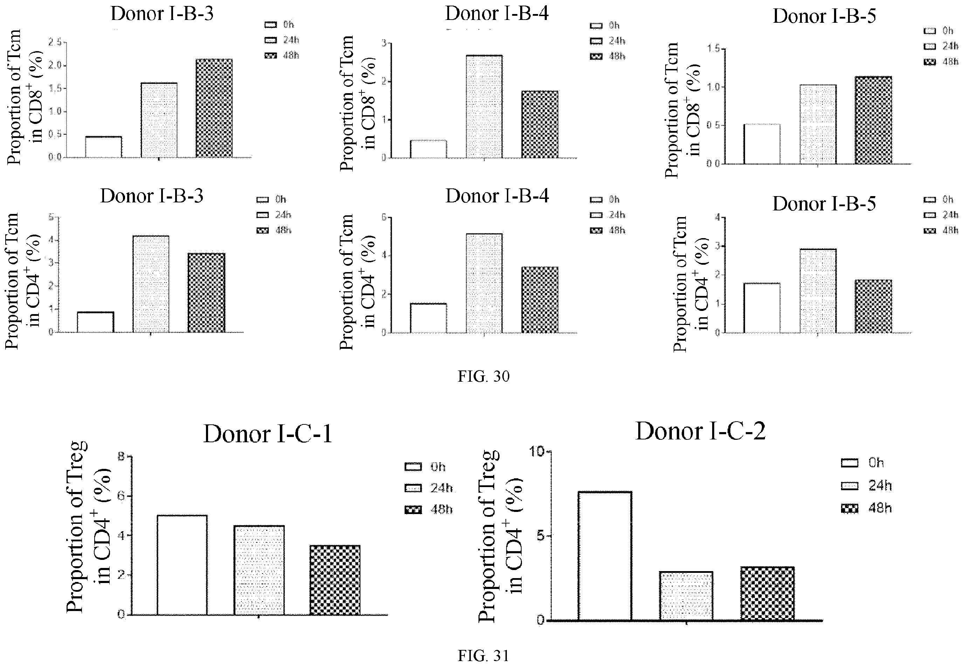

FIGs. 29 and 30 show the proportion of CD45RA$^-$CCR7$^+$ central memory T cells (Terns) in the TIL cells obtained by culturing with feeder cells added at 0, 24 or 48 hours after the addition of OKT3 and IL-2. The results show that the TILs cultured with the feeder cells added after 24 hours or 48 hours had a higher proportion of central memory T cells than that in the TILs cultured with the feeder cells added at the same time.

FIG. 31 shows the proportion of CD4$^+$CD25$^+$Foxp3$^+$ regulatory T cells (Treg) in the TIL cells obtained by culturing with feeder cells added at 0, 24 or 48 hours after the addition of OKT3 and IL-2. The results show that the TILs cultured with the feeder cells added after 24 hours or 48 hours had a lower proportion of regulatory T cells than that in the TILs cultured with the feeder cells added at the same time.

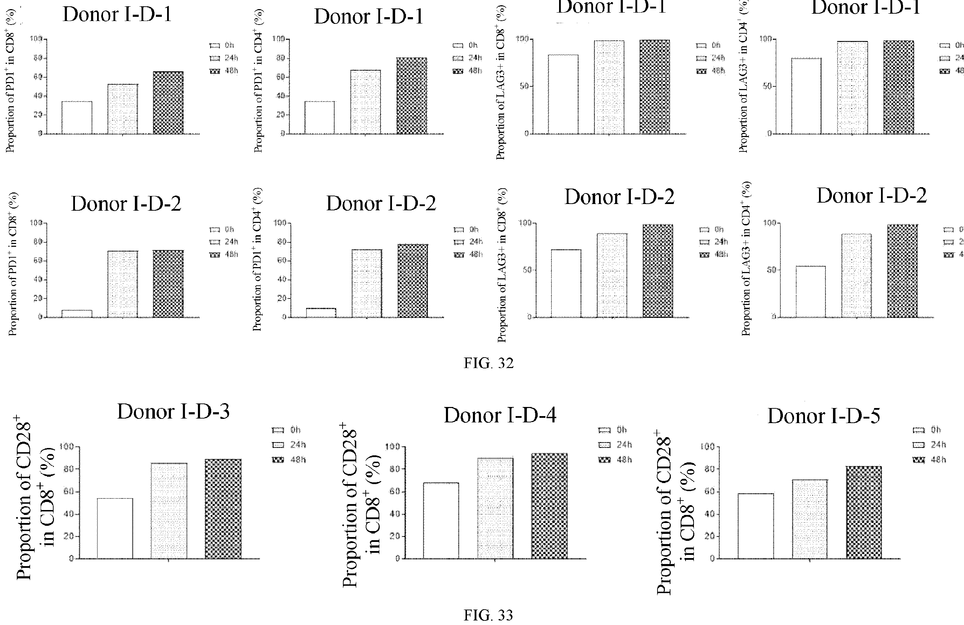

FIGs. 32 and 33 show the proportion of activated T cells in the TIL cells obtained by culturing with feeder cells added at 0, 24 or 48 hours after the addition of OKT3 and IL-2. The results show that the TILs cultured with the feeder cells added after 24 hours or 48 hours had a higher proportion of activated T cells than that in the TILs cultured with the feeder cells added at the same time, for example, a higher proportion of PD1$^+$, LAG3$^+$ and/or CD28$^+$ cells.

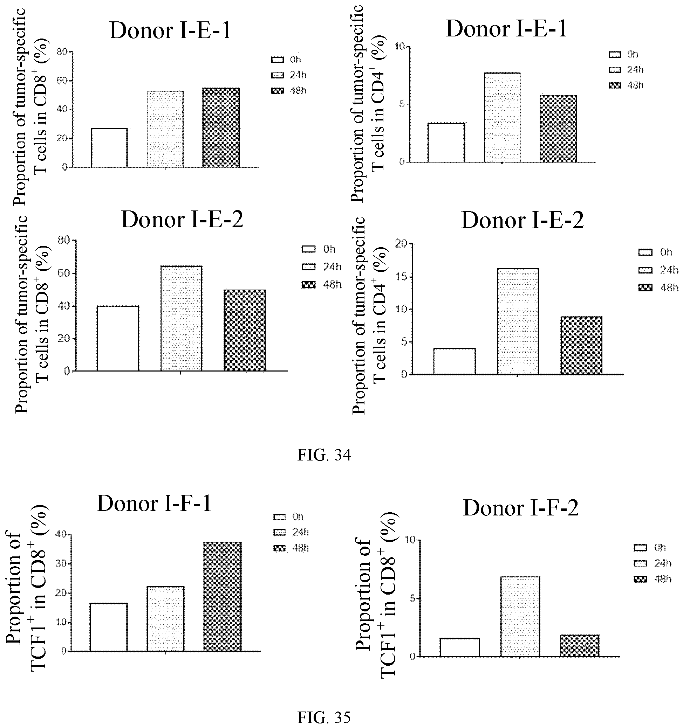

FIG. 34 shows the proportion of CD103$^+$CD39$^+$ tumor-specific T cells in the TIL cells obtained by culturing with feeder cells added at 0, 24 or 48 hours after the addition of OKT3 and IL-2. The results show that the TILs cultured with the feeder cells added after 24 hours or 48 hours had a higher proportion of tumor-specific T cells than that in the TILs cultured with the feeder cells added at the same time.

FIG. 35 shows the proportion of TCF1$^+$ stem cell-like T cells in the TIL cells obtained by culturing with feeder cells added at 0, 24 or 48 hours after the addition of OKT3 and IL-2. The results show that the TILs cultured with the feeder cells added after 24 hours or 48 hours had a higher proportion of stem cell-like T cells than that in the TILs cultured with the feeder cells added at the same time.

**Example 11 Proliferation ability assay of TILs stimulated by adding a CD28 agonist**

Cell counts were performed on TIL populations obtained from culture of each test group in Example 1.

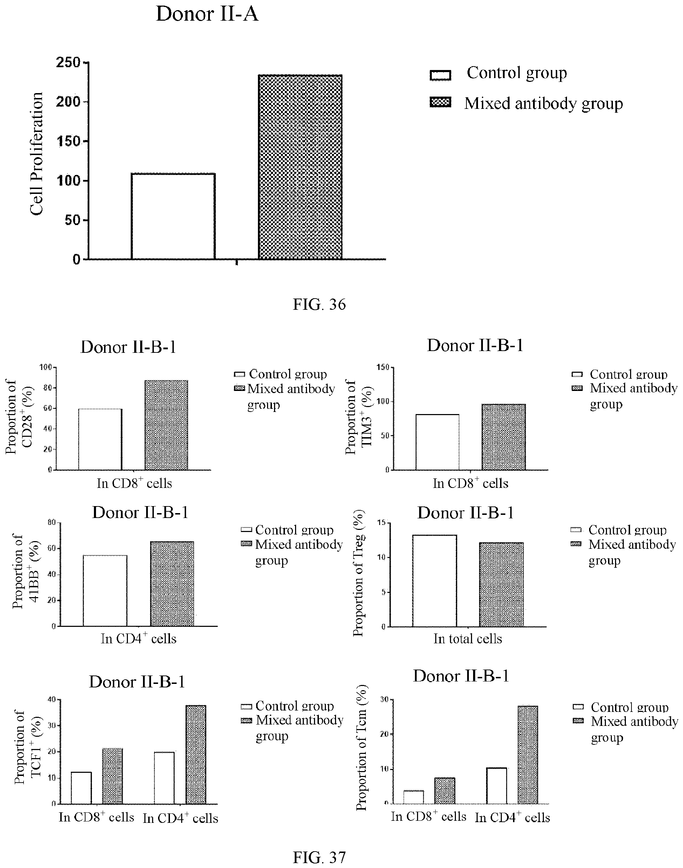

FIG. 36 shows the analysis results of the proliferation ability of the test groups added with different forms of CD28 agonists and the control group. The values on the vertical coordinates in the graphs indicate the expansion factor of the number of TIL cells in the TIL populations obtained in each test group of *in vitro* expansion compared with the TIL populations before the start *of in vitro* expansion. The results show that, the proliferation ability of TILs obtained from the *in vitro* expansion of step (B) in the four-step method with the addition of CD28 antibodies is stronger than that of TILs cultured in the control group (without the addition of CD28 antibodies).

**Example 12 Flow cytometry of TILs stimulated by adding a CD28 agonist**

TIL populations obtained from culture of each test group in Example 1 were detected by flow cytometry.

The analyses on flow cytometry results of TILs cultured by adding different forms of CD28 agonists are shown in FIG. 37 to FIG. 41.

FIG. 37 shows the proportion of T cell subpopulations in the TIL cells obtained from culture in the mixed antibody group and the control group. The results show that the TILs obtained from the *in vitro* expansion of step (B) in the four-step method with the addition of CD28 antibodies have an improved proportion of T cell subpopulations compared with that in the control group (without the addition of CD28 antibodies). For example, a higher proportion of activated T cells (CD28$^+$, TIM3$^+$ or 41BB$^+$), a lower proportion of regulatory T cells (Treg, e.g., CD4$^+$CD25$^+$Foxp3$^+$), a higher proportion of stem cell-like T cells (TCF1$^+$), and/or a higher proportion of central memory T cells (Terns, e.g., CD45RA$^-$CCR7$^+$).

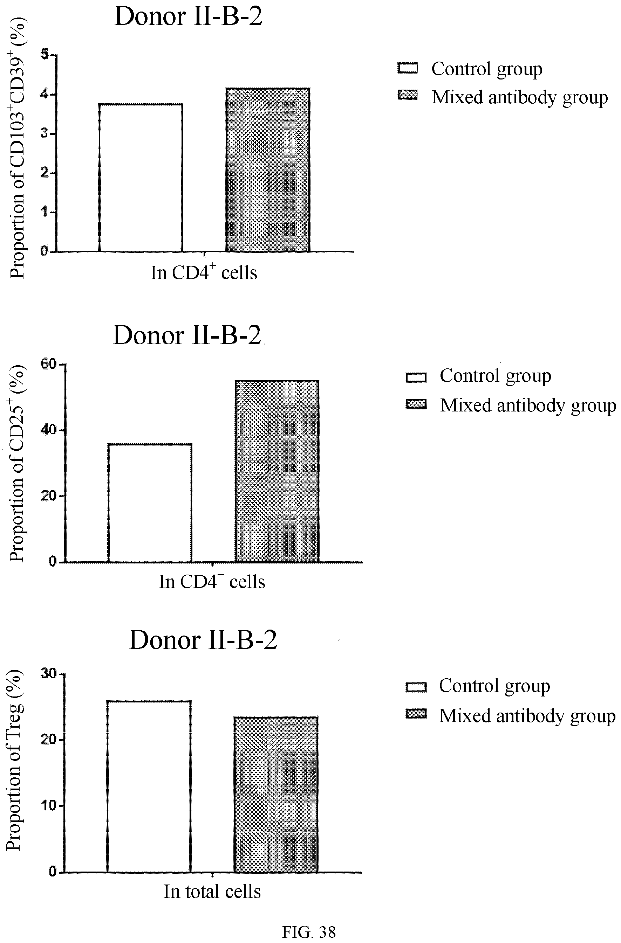

FIG. 38 shows the proportion of T cell subpopulations in the TIL cells obtained from culture in the mixed antibody group and the control group. The results show that, the TILs obtained from the *in vitro* expansion of step (B) in the four-step method with the addition of CD28 antibodies have an improved proportion of T cell subpopulations compared with that in the control group (without the addition of CD28 antibodies). For example, a higher proportion of tumor-specific T cells (CD103$^+$CD39$^+$), a higher proportion of activated T cells (CD25$^+$), and/or a lower proportion of regulatory T cells (Treg, e.g., CD4$^+$CD25$^+$Foxp3$^+$).

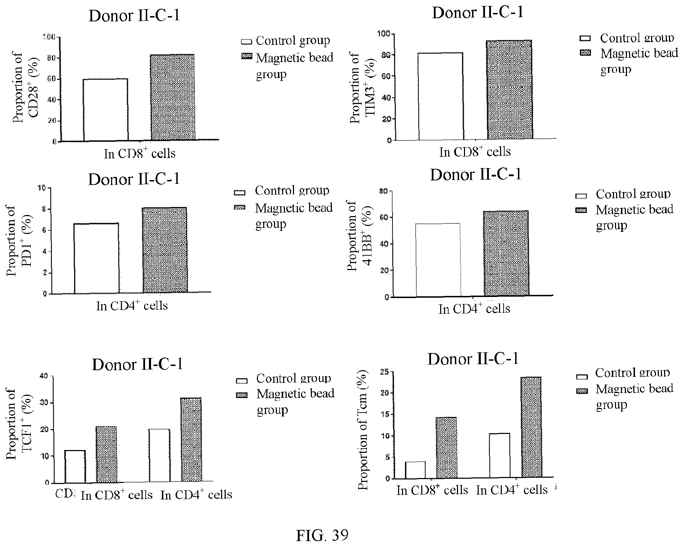

FIG. 39 shows the proportion of T cell subpopulations in TIL cells obtained from culture in the magnetic bead group and the control group. The results show that, the TILs obtained from the *in vitro* expansion of step (B) in the four-step method with the addition of CD28 antibodies (for example, adding magnetic beads comprising CD3 antibodies and CD28 antibodies) have an improved proportion of T cell subpopulations compared with that in the control group (without the addition of CD28 antibodies). For example, a higher proportion of activated T cells (CD28$^+$, TIM3$^+$, PD1$^+$ or 41BB$^+$), a higher proportion of stem cell-like T cells (TCF1$^+$), and/or a higher proportion of central memory T cells (Terns, e.g., CD45RA$^-$CCR7$^+$).

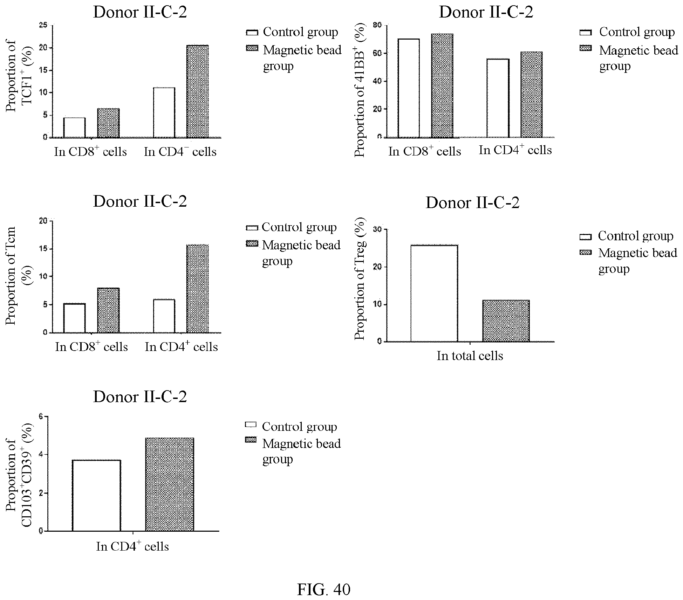

FIG. 40 shows the proportion of T cell subpopulations in TIL cells obtained from culture in the magnetic bead group and the control group. The results show that, the TILs obtained from the *in vitro* expansion of step (B) in the four-step method with the addition of CD28 antibodies (for example, adding magnetic beads comprising CD3 antibodies and CD28 antibodies) have an improved proportion of T cell subpopulations compared with that in the control group (without the addition of CD28 antibodies). For example, a higher proportion of stem cell-like T cells (TCF1$^+$), a higher proportion of activated T cells (41BB$^+$), a higher proportion of central memory T cells (Terns, e.g., CD45RA$^-$CCR7$^+$), lower proportion of regulatory T cells (Treg, e.g., CD4$^+$CD25$^+$Foxp3$^+$), and/or a higher proportion of tumor-specific T cells (CD103$^+$CD39$^+$).

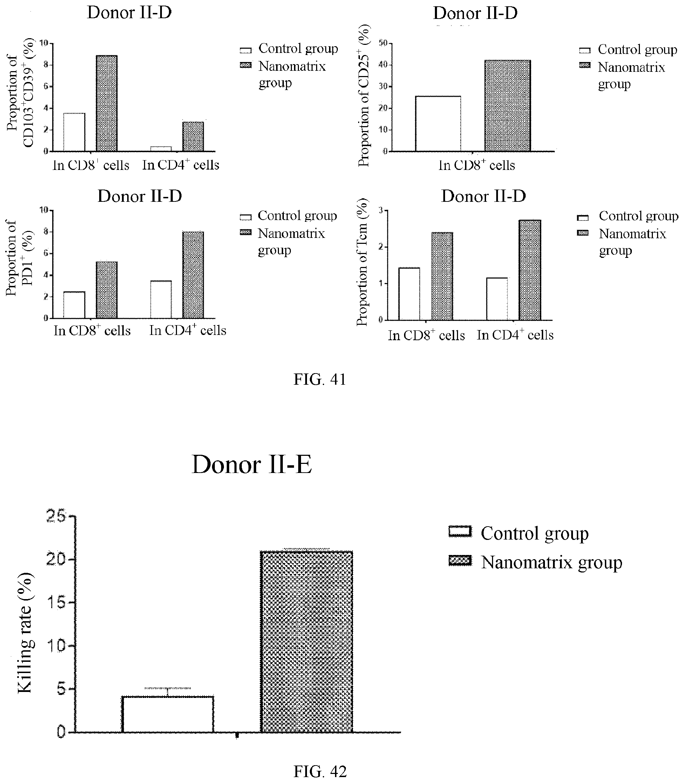

FIG. 41 shows the proportion of T cell subpopulations in TIL cells obtained from culture in the nanomatrix group and the control group. The results show that, the TILs obtained from the *in vitro* expansion of step (B) in the four-step method with the addition of CD28 antibodies (for example, adding transACT comprising CD3 antibodies and CD28 antibodies) have an improved proportion of T cell subpopulations compared with that in the control group (without the addition of CD28 antibodies). For example, a higher proportion of tumor-specific T cells (CD103+CD39+), a higher proportion of activated T cells (CD25+ or PD1+), and/or a higher proportion of central memory T cells (Terns, e.g., CD45RA-CCR7+).

**Example 13 Detection of cell killing ability of TILs stimulated by adding a CD28 agonist**

TIL populations from each test group in Example 1 obtained from the *in vitro* expansion of step (B) in the four-step method were detected for their cell killing ability.

Cell preparation

TILs obtained from each test group for assay and target cells (e.g., AHela tumor cells) for co-culture were prepared.

Assay steps

Labeling the tumor cells with CFSE (5(6)-Carboxyfluorescein diacetate N-succinimidyl ester, Sigma, 21888-25MG-F): The tumor cells were washed with PBS, and resuspended in 500 $\mu$L of PBS; CFSE was added into 500 $\mu$L of PBS, and mixed with 500 $\mu$L of resuspension of the tumor cells in PBS to a final concentration of CFSE of 0.5 $\mu$mol/L. After incubation at 37°C for 6 minutes, a medium comprising 10% FBS was added to wash. Centrifugation was performed at 600 g for 5 minutes. X-vivo 15 medium or other commercially available T cell culture media, e.g. T cell culture media of Stem Cell, Lonza, Thermo, Miltenyi brands etc., were used to resuspend the tumor cells to a concentration of $5\times10^5$ cells/mL. The TIL cells of each test group were centrifuged at 600 g for 5 minutes, and the TIL cells were resuspended according to an effector-target ratio (the ratio of TIL cells to tumor cells) of 3:1 (i.e., the concentration of the resuspended TIL cells was $1.5\times10^6$ cells/mL). Each 100 $\mu$L of tumor cells and TIL cells were added to a U-bottom 96-well plate (Corning), and three replicate wells were set for each group. At the same time, a control group comprising only the tumor cells was set, and different reagents were added for different groups according to the experiment. The well plates were centrifuged at 200 g for 1 minute and incubated at 37°C for 4 hours to overnight.

After the incubation was completed, centrifugation was performed at 600 g for 3 minutes and the supernatant was discarded. Trypsin was added at 20 $\mu$L/well. Incubation was performed in an incubator at 37°C for 3-5 minutes to digest the tumor cells. After the digestion was completed, 180 $\mu$L of culture medium comprising 10% FBS was added to terminate the digestion. Dapi (Beyotime, C0060) was diluted at 1:100, and then the diluted Dapi was added at 20 $\mu$L/well. Flow cytometry was performed on machine.

$$\text{Killing rate\%} = \text{Number of Dapi}^+\text{CFSE}^+\text{ cells/Total CFSE}^+ \times 100\%.$$

FIG. 42 shows the cell killing ability of TIL cells obtained from culture in the nanomatrix group and the control group. The results show that, the TILs obtained from the *in vitro* expansion of step (B) in the four-step method with the addition of CD28 antibodies (for example, adding transACT comprising CD3 antibodies and CD28 antibodies) have higher cell killing ability compared with that in the control group (without the addition of CD28 antibodies).

**Example 14 Intracellular factor expression assay of TILs stimulated by adding a CD28 agonist**

TIL populations from each test group in Example 1 obtained from the *in vitro* expansion of step (B) in the four-step method were detected for intracellular factor expression.

Test preparation

Formulation of the culture medium required for the intracellular factor expression assay: the culture medium of T cells was taken, into which were added CD107a antibodies (BD) at a volume ratio of 1:500.

Assay steps

TILs from each test group were centrifuged, and then resuspended to $1\times10^6$ cells/mL using 600 $\mu$L of the culture medium required for the above intracellular factor expression assay, added to a 96-well plate at 100 $\mu$L/well, and incubated overnight in an incubator at 37°C.

At the end of the incubation, the cells were washed once with PBS at 200 $\mu$L/well, and centrifuged at 600 g for 3 minutes. The supernatant was discarded. An antibody mixed working solution of CD3/CD4/CD8 (BD) was formulated for cell surface staining, with an antibody concentration of 1:100, a viability of 1: 10000, and a staining volume of 50 μL/group. Incubation was performed at 2-8°C in dark for 30 minutes. At the end of the staining, the cells were washed and resuspended in PBS for flow cytometry on machine.

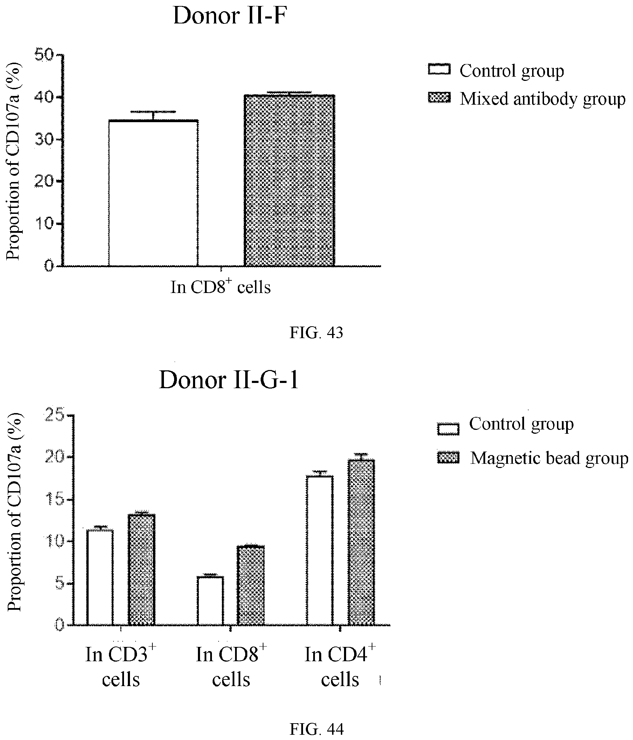

FIG. 43 shows the results of intracellular factor expression assay of TIL cells obtained from culture in the mixed antibody group and the control group. The results show that, the TILs obtained from the *in vitro* expansion of step (B) in the four-step method with the addition of CD28 antibodies have a higher intracellular factor expression capacity compared with that in the control group (without the addition of CD28 antibodies). For example, a higher CD107a expression capacity.

FIG. 44 shows the results of intracellular factor expression assay of TIL cells obtained from culture in the magnetic bead group and the control group. The results show that, the TILs obtained from the *in vitro* expansion of step (B) in the four-step method with the addition of CD28 antibodies (e.g., adding magnetic beads comprising CD3 antibodies and CD28 antibodies) have a higher intracellular factor expression capacity compared with that in the control group (without the addition of CD28 antibodies). For example, a higher CD107a expression capacity.

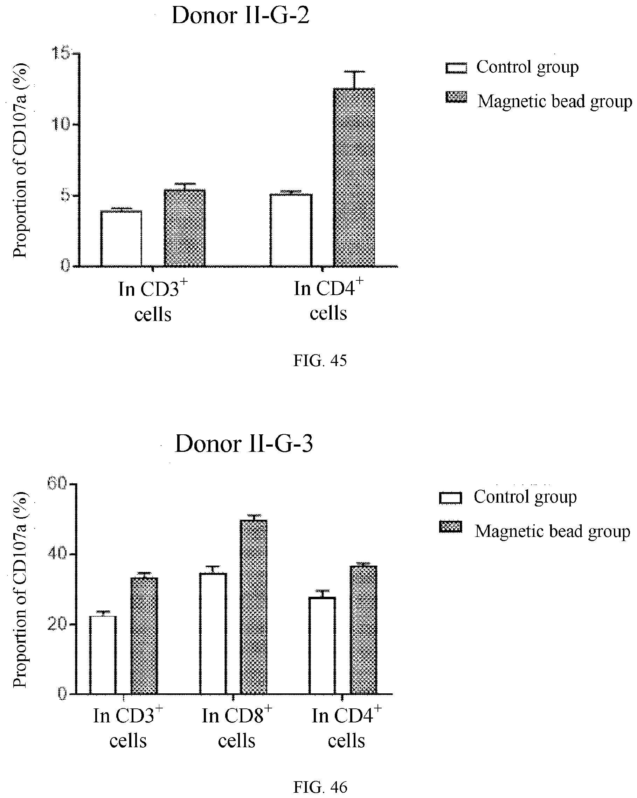

FIG. 45 shows the results of intracellular factor expression assay of TIL cells obtained from culture in the magnetic bead group and the control group. The results show that, the TILs obtained from the *in vitro* expansion of step (B) in the four-step method with the addition of CD28 antibodies (e.g., adding magnetic beads comprising CD3 antibodies and CD28 antibodies) have a higher intracellular factor expression capacity compared with that in the control group (without the addition of CD28 antibodies). For example, a higher CD107a expression capacity.

FIG. 46 shows the results of intracellular factor expression assay of TIL cells obtained from culture in the magnetic bead group and the control group. The results show that, the TILs obtained from the *in vitro* expansion of step (B) in the four-step method with the addition of CD28 antibodies (e.g., adding magnetic beads comprising CD3 antibodies and CD28 antibodies) have a higher intracellular factor expression capacity compared with that in the control group (without the addition of CD28 antibodies). For example, a higher CD107a expression capacity.

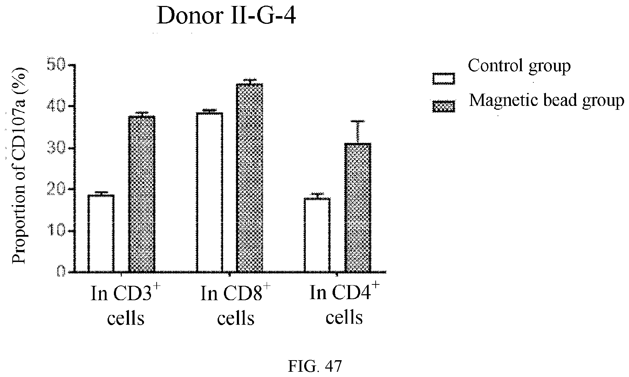

FIG. 47 shows the results of intracellular factor expression assay of TIL cells obtained from culture in the magnetic bead group and the control group. The results show that, the TILs obtained from the *in vitro* expansion of step (B) in the four-step method with the addition of CD28 antibodies (e.g., adding magnetic beads comprising CD3 antibodies and CD28 antibodies) have a higher intracellular factor expression capacity compared with that in the control group (without the addition of CD28 antibodies). For example, a higher CD107a expression capacity.

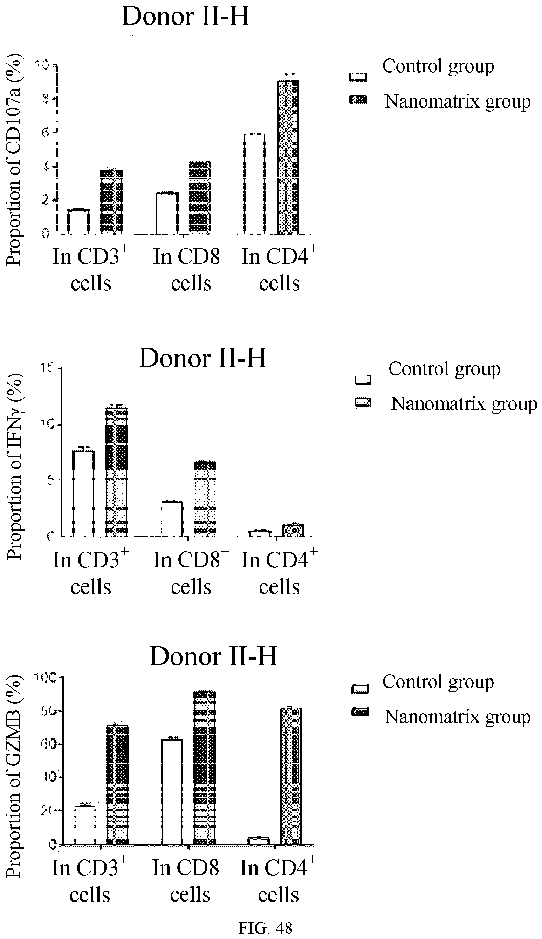

FIG. 48 shows the results of intracellular factor expression assay of TIL cells obtained from culture in the nanomatrix group and the control group. The results show that, the TILs obtained from the *in vitro* expansion of step (B) in the four-step method with the addition of CD28 antibodies (e.g., adding transACT comprising CD3 antibodies and CD28 antibodies) have a higher intracellular factor expression capacity compared with that in the control group (without the addition of CD28 antibodies). For example, a higher CD107a expression capacity, a higher IFN-$\gamma$ expression capacity or a higher GZMB expression capacity.

**Example 15 Cytokine secretion assay of TILs stimulated by adding a CD28 agonist**

TIL populations from each test group in Example 1 obtained from the *in vitro* expansion of step (B) in the four-step method were detected for cytokine secretion.

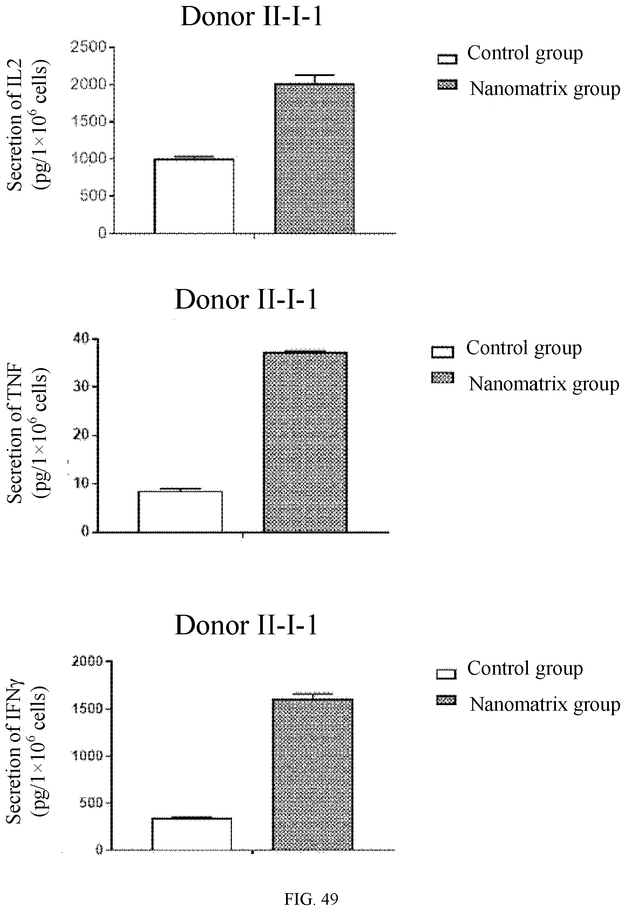

FIG. 49 shows the results of cytokine secretion assay of TIL cells obtained from culture in the nanomatrix group and the control group. The results show that, the TILs obtained from the *in vitro* expansion of step (B) in the four-step method with the addition of CD28 antibodies (e.g., adding transACT comprising CD3 antibodies and CD28 antibodies) have a higher cytokine secretion capacity compared with that in the control group (without the addition of CD28 antibodies). For example, a higher IL-2 secretion capacity, a higher TNF secretion capacity, or a higher IFN-$\gamma$ secretion capacity.

The TILs obtained from each test group were incubated with tumor cells overnight, and the supernatant was taken after the incubation for cytokine secretion assay according to the assay steps of this example.

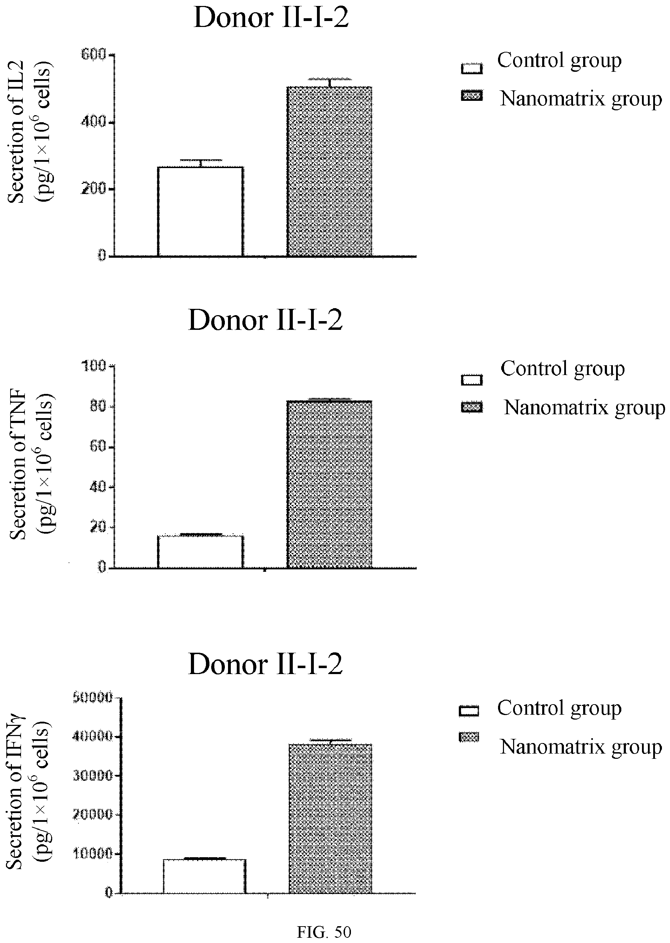

FIG. 50 shows the results of cytokine secretion assay of TIL cells obtained from culture in the nanomatrix group and the control group after being co-cultured with tumor cells. The results show that, the TILs obtained from the *in vitro* expansion of step (B) in the four-step method with the addition of CD28 antibodies (e.g., adding transACT comprising CD3 antibodies and CD28 antibodies) have a higher cytokine secretion capacity compared with that in the control group (without the addition of CD28 antibodies). For example, a higher IL-2 secretion capacity, a higher TNF secretion capacity, or a higher IFN-$\gamma$ secretion capacity.

**Example 16 Detection of gene knockout efficiency of TILs stimulated by adding a CD28 agonist**

TIL cells from each test group in Example 1 obtained after 48 hours of culture by *in vitro* expansion of step (B) in the four-step method were detected for gene knockout efficiency.

Nuclease-free water (commercial source: Shanghai Youfan Biotech Co., Ltd; RT121-02) was used to formulate sgRNA (with its sequence as shown in SEQ ID NO: 27, GGAGAATGACGAGTGGACCC), and the concentration was adjusted to 50 $\mu$mol/L. 2 $\mu$L of gRNA was added to a PCR tube. Nuclease-free water was used as a negative control, and incubated in a PCR instrument at 95°C for 2 minutes and then cooled at room temperature for 10 minutes.

According to a volume ratio of sgRNA:P3 Buffer:Cas9 nuclease = 2:2:1, P3 Buffer (commercial source: Lonza; V4XP-3032) and 61.7 $\mu$mol/L Cas9 nuclease (commercial source: Suzhou Cure Genetics Co., Ltd.; C01-2019-11-001) were successively added into the PCR tube comprising the sgRNA. The PCR tube was placed into the PCR instrument, and incubated at 25°C for 10 minutes to form RNP, which was placed at 4°C for later use.

The T cell culture medium was added at 1 mL/well and placed in a $CO_2$ incubator for preheating. TIL cells from each test group in Example 1 obtained after 48 hours of culture by *in vitro* expansion of step (B) in the four-step method were taken, mixed well and counted. $5\times10^5$ cells were taken from each test group and added into P3 Buffer (20 $\mu$L), and mixed well; the cells were added into a new PCR tube where they were mixed with 5 $\mu$L of RNP; the mixture of the cells and RNP was added into an electroporation strip plate, and electroporated in an electroporator (Lonza) (human T cell stimulated (E0115)). Immediately after the electroporation procedure, 180 $\mu$L of preheated T cell culture medium was added, and the entire volume was transferred to a 24-well suspension plate, and placed in a $CO_2$ incubator for culture. 24 hours later, the cells were counted, and feeder cells (irradiated PBMC cells) were added at a ratio of TILs to feeder cells = 1 :200, and incubated in a $CO_2$ incubator for further 72 hours. The TIL cells from each test group at the end of culture were taken for cell counting. $2\times10^5$ cells were taken from each test group, centrifuged at 500 g for 3 minutes, and the supernatant was aspirated and discarded after the centrifugation.

Formulation of mixed antibodies for flow cytometry assay: Fixable Viability Dye eFluor 780 (commercial source: eBioscience; 65-0865-18) was diluted 10,000 times in PBS; into 100 $\mu$L of the diluted Fixable Viability Dye eFluor 780 in PBS were respectively added 1 $\mu$L of TCR-$\alpha\beta$-APC (commercial source: eBioscience; 17-9986-42), 1 $\mu$L of BB515 Mouse Anti-Hu CD8 (commercial source: BD Pharmingen; 564526), and 1 $\mu$L of PE-Cy7 Mouse Anti-Hu CD4 (Commercial source: BD Pharmingen; 557852), and mixed well.

Into the TIL cell samples from each test group was added 100 $\mu$L of the above mixed antibodies for flow cytometry assay, mixed well and incubated on ice for 30 minutes. After the incubation, centrifugation was performed at 500 g for 3 minutes, and the supernatant was aspirated and discarded after the centrifugation. Then 200 $\mu$L of PBS was added for resuspension. A flow cytometer was used for assay, and the knockdown efficiency of TCR$\beta$ was analyzed by using Flowjo software.

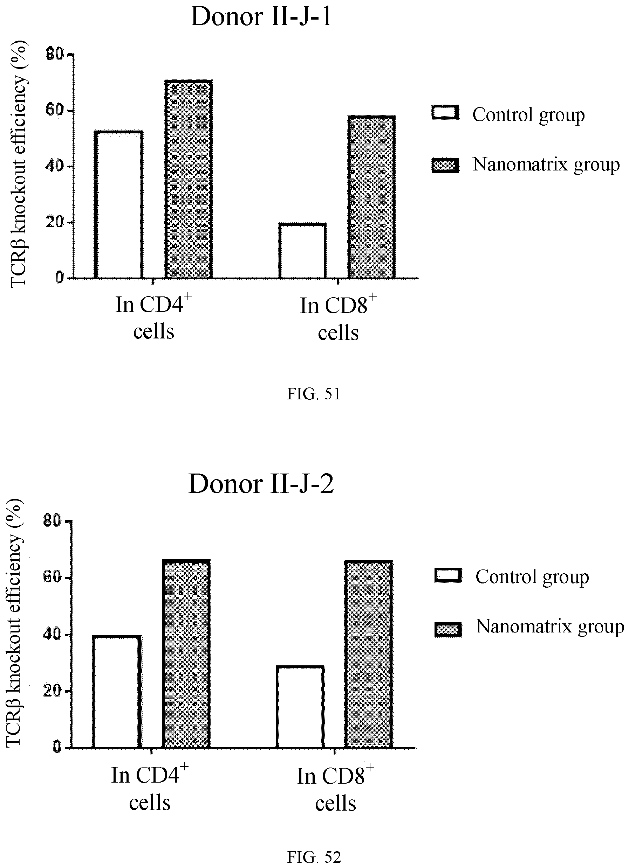

FIG. 51 shows the results of gene knockout efficiency of TIL cells obtained from culture in the nanomatrix group and the control group. The results show that, the TILs obtained from the *in vitro* expansion of step (B) in the four-step method with the addition of CD28 antibodies (for example, adding transACT comprising CD3 antibodies and CD28 antibodies) have an improved gene knockout efficiency compared with that in the control group (without the addition of CD28 antibodies). For example, an enhanced TCR$\beta$ gene knockout efficiency.

FIG. 52 shows the results of gene knockout efficiency of TIL cells obtained from culture in the nanomatrix group and the control group. The results show that, the TILs obtained from the *in vitro* expansion of step (B) in the four-step method with the addition of CD28 antibodies (for example, adding transACT comprising CD3 antibodies and CD28 antibodies) have an improved gene knockout efficiency compared with that in the control group (without the addition of CD28 antibodies). For example, an enhanced TCR$\beta$ gene knockout efficiency.

**Example 17 Proliferation ability assay of TILs with the CD28 agonist added after the end of REP stage**

A first TIL population derived from tumor tissues and not expanded *in vitro* was obtained by reference to Example 1. The first TIL population was subjected to steps (A), (B), (C), and (D) of the four-step method in the same way to obtain the fifth TIL population. The fifth TIL population was randomly divided into 3 groups, and IL-2 was added into the T cell culture medium of each test group, while the blank group was not added any T cell activators, and the group without a CD28 agonist added was added a CD3 antibody at about 30 ng/ mL, and the group with a CD28 agonist added was added a CD3 agonist and a CD28 agonist, for example, transACT was added at a ratio of transACT to TILs of about 1:100-1:2000. The TILs (terminal stimulated cell population) obtained from 3 days of culture were detected for the proliferation ability of TIL cells by cell viability assay using the CellTiter-Glo kit (commercial source: Promega).

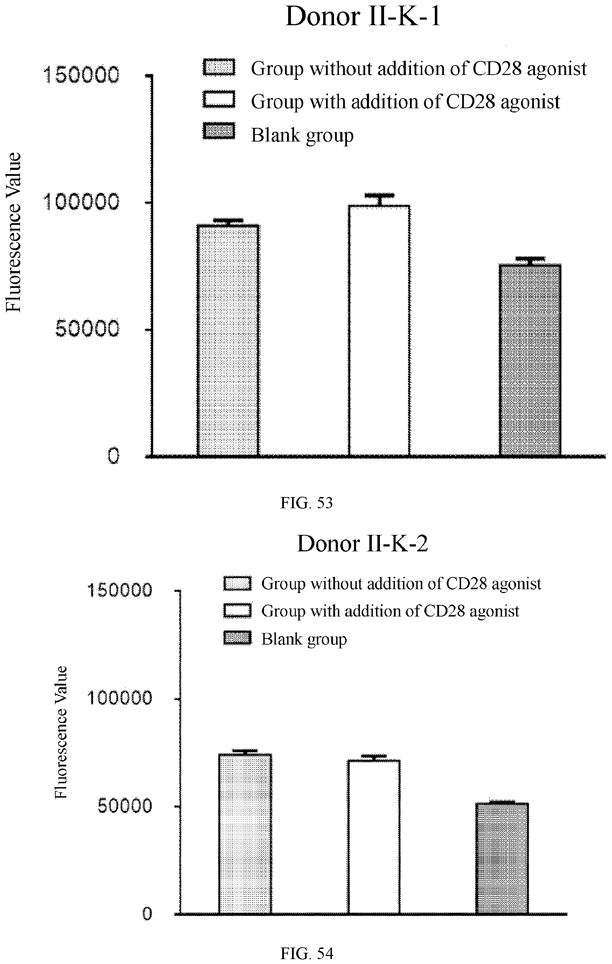

FIGs. 53, 54, and 55 show the analysis results of the proliferation ability of the test groups upon *in vitro* expansion in different ways in the terminal stimulation stage, respectively, for TILs from different donor sources. The fluorescence values on the vertical coordinates of the graphs reflect the proliferation ability of TIL cells subjected to terminal stimulation in different ways in each test group. The results show that the addition of a CD28 agonist during the terminal stimulation resulted in a similar proliferation ability of TILs compared to that resulted from the terminal stimulation without the addition of a CD28 agonist.

**Example 18 Serial killing effect of TILs after transduction**

The A375 tumor cell lines were plated in 96-well plates at $2\times10^4$/well starting from day 7 after transduction. On the following day, each group of TIL cells were co-cultured with the above tumor cells at an effector-target ratio (TIL cells: tumor cells) of 4:1, with at least five wells in each group. Thereafter, all the cells in one well of each group were taken every three days for flow cytometry assay, half of the supernatant in each of the other groups was discarded, and an equal amount of tumor cells were supplemented according to the results of flow cytometry. The steps of flow cytometry assay were as follows: First, all the cells in the original wells were blown off, blown evenly, and an appropriate amount of cells were transferred to a new V-bottom 96-well plate for flow cytometry assay, centrifuged at 600 g for 3 minutes, and the supernatant was discarded; 100 μL of PBS was added to each well for washing once, centrifugation was performed again, and the supernatant was discarded; then into each well were added 0.5 μL of flow antibodies and 50 μL of the formulated live/dead dye and incubated at 4°C for 30 minutes. The flow antibodies comprise BV510 anti-CD3, PC7 anti-CD45, and PE anti-MCSP. Each kind of antibodies was added at 0.5 μL/well. After the incubation was completed, centrifugation was performed at 600 g for 3 minutes, and the supernatant was discarded; after washing once, an appropriate amount of PBS was added to resuspend the cells for flow cytometry assay.

According to the results of flow cytometry, the ratio of tumor cells/TII, cells was calculated. A smaller value indicates a lower relative number of tumor cells and a stronger killing effect of TILs.

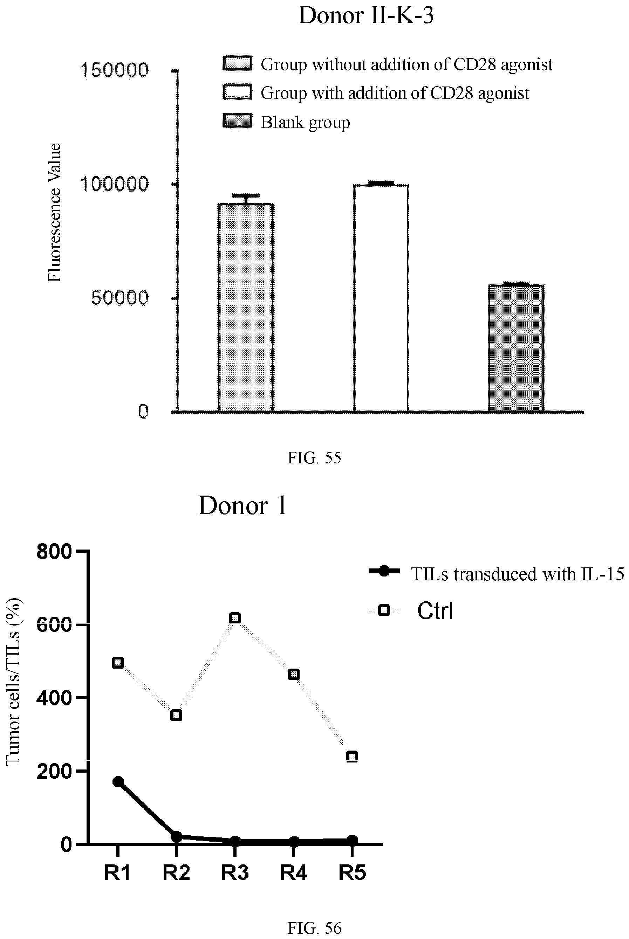

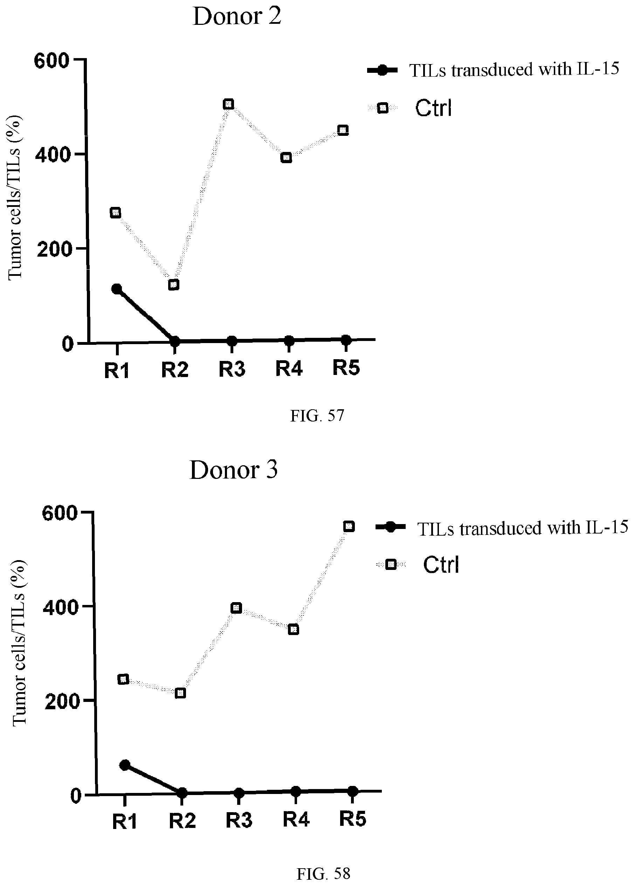

FIGs. 56, 57, and 58 show the serial killing results of TILs after being transduced with the IL-15 of the present application, respectively, for TILs from different donor sources. The results show that, genetically transduced TIL cells may have a stronger serial killing ability.

**Example 19 In vivo validation of TILs after transduction**

Immunodeficient mice (NOG mice, Vital River, strain code 408) were subcutaneously inoculated with A375 cells at an inoculation number of about $1\times10^6$ cells. About 7 days later, when the tumor volume reached about 50-80 mm$^3$, mice were grouped randomly depending on their tumor size (as shown in Table below). Each mouse was injected with control TILs and IL-15-transduced TIL cell injections through the tail vein, and an equal volume of PBS was used as a negative control, which was recorded as D0. Each reinfusion cell group simultaneously received intraperitoneal injection of IL-2, twice a day for 6 consecutive times.

The tumor volume of the mice in each group was measured twice a week, and the group of TILs transduced with IL-15 + low-dose IL-2 showed a more significant tumor suppressive ability, and the tumor suppressive effect was significantly stronger than that of the group of control TILs + low-dose IL-2, the group of TILs transduced with IL-15 also showed a stronger tumor suppressive tendency compared to that of the group of control TILs + low-dose IL-2. Meanwhile, peripheral blood was taken from each group of mice at multiple time points, and the secretion of IFN-γ in serum was analyzed by CBA method. The results showed that the group of TILs transduced with IL-15 + low-dose IL-2 exhibited higher and more durable IFN-γ secretion than the group of control TILs + low-dose IL-2, which was consistent with its better tumor suppressive effect.

| Group | Reinfusion Dose | IL-2 Injection Volume (IU) | Number of mice |
|---|---|---|---|
| PBS | \ | \ | 8 |
| Control TIL + low-dose IL-2 | $5\times10^7$ | $2\times10^4\times3\times$BID | 8 |
| TILs transduced with IL-15 | $5\times10^7$ | \ | 8 |
| TILs transduced with IL-15 + low-dose IL-2 | $5\times10^7$ | $2\times10^4\times3\times$BID | 8 |

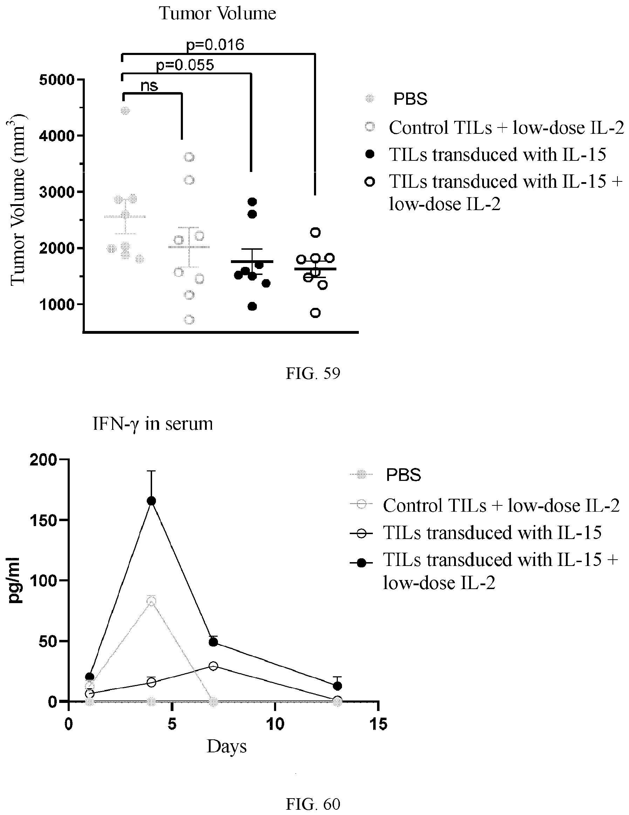

FIG. 59 shows, TIL cells transduced with IL-15 may show stronger tumor volume suppressive ability. FIG. 60 shows, TIL cells transduced with IL-15 may show higher and more durable ability of IFN-γ secretion.

**Example 20 Properties of TILs cultured with feeder cells added at different times**

Statistics of the results of TILs cultured with feeder cells added at different times

During the activation of TILs in the second stage of expansion in 1.4 of Example 1, the amount of cells expanded in the first stage was taken and the cell density was adjusted to $5\times10^5$ to $2\times10^6$/mL. In a suspension 24-well culture plate were added CD3 antibodies at 1 mL/well, e.g., about 30 ng/mL of OKT3, and IL-2 at a concentration of about 1000 to 9000 IU/mL, e.g., 3000 or 6000 IU/mL of IL-2. At 0, 24, and 48 hours after the addition of OKT3 and IL-2 above, feeder cells were added into the culture environment of tumor-infiltrating lymphocytes. Where, TILs and feeder cells may be added at a ratio of 1:40 to 1:400, and all the cells were collected after about 9 to 14 days of culture by the second stage of expansion, and the results of TILs obtained from the culture were detected and counted.

Proliferation ability assay

The TIL cells obtained above by culturing with feeder cells added at different times were counted.

TILs from different donor tumor sources were used as their respective different batches; the data from each batch of the test groups in which feeder cells were added simultaneously with OKT3 and IL-2 (0 h group) was used as the benchmark 1, and the data from the test groups at other time points in the same batch were normalized to count the relative proliferation ability of each test group in the second stage of expansion relative to the 0 h group.

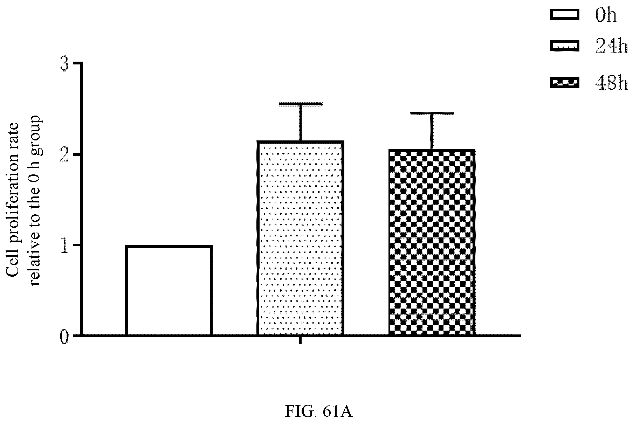

FIG. 61A shows a graph of the cell proliferation ability results of TIL cells obtained by culturing with feeder cells added at 0, 24 or 48 hours after the addition of OKT3 and IL-2. Compared to TILs cultured with feeder cells added at 0 hours after the addition of OKT3 and IL-2 (i.e., simultaneously), the proliferation ability of TILs cultured with feeder cells added at 24 or 48 hours after the addition of OKT3 and IL-2 was significantly enhanced.

Detection of composition of TIL cells by flow cytometry

TIL populations obtained above by culturing with feeder cells added at different addition times were detected by flow cytometry.

TILs from different donor tumor sources were used as their respective different batches; the data from each batch of the test groups in which feeder cells were added simultaneously with OKT3 and IL-2 (0 h group) was used as the benchmark 1, and the data from the test groups at other time points in the same batch were normalized to count the cell composition proportion of each test group in the second stage of expansion relative to the 0 h group.

The test procedure of flow cytometry can refer to the content in Example 3 of the present application.

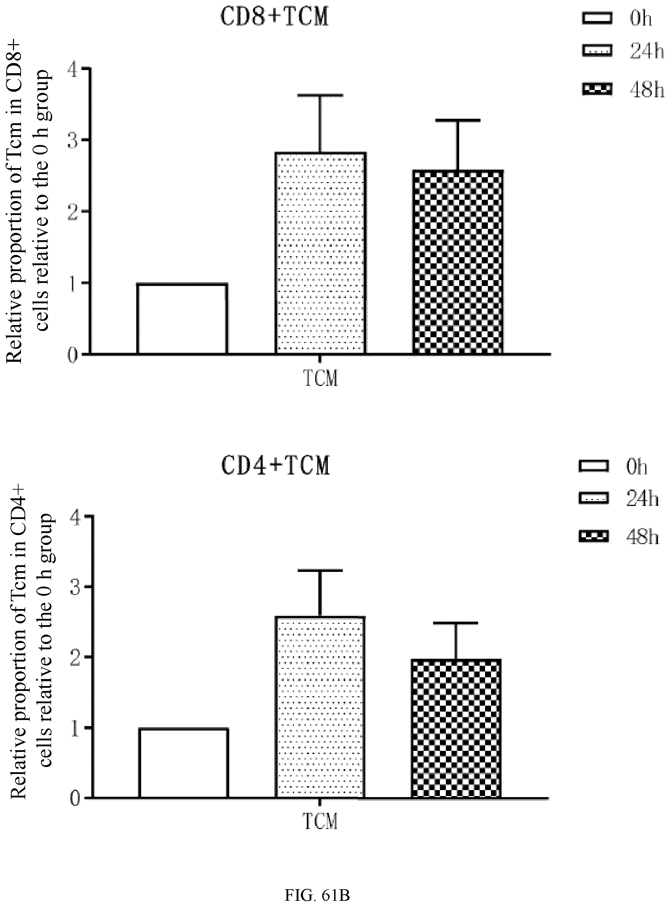

FIG. 61B shows a graph of the proportion results of $CD45RA^-CCR7^+$ central memory T cells (Tcms) in the TIL cells obtained by culturing with feeder cells added at 0, 24 or 48 hours after the addition of OKT3 and IL-2. The results show that, TILs cultured with feeder cells added after 24 hours or 48 hours have higher proportion of central memory T cells in $CD8^+$ and/or $CD4^+$ compared to that in the TILs cultured with feeder cells added simultaneously.

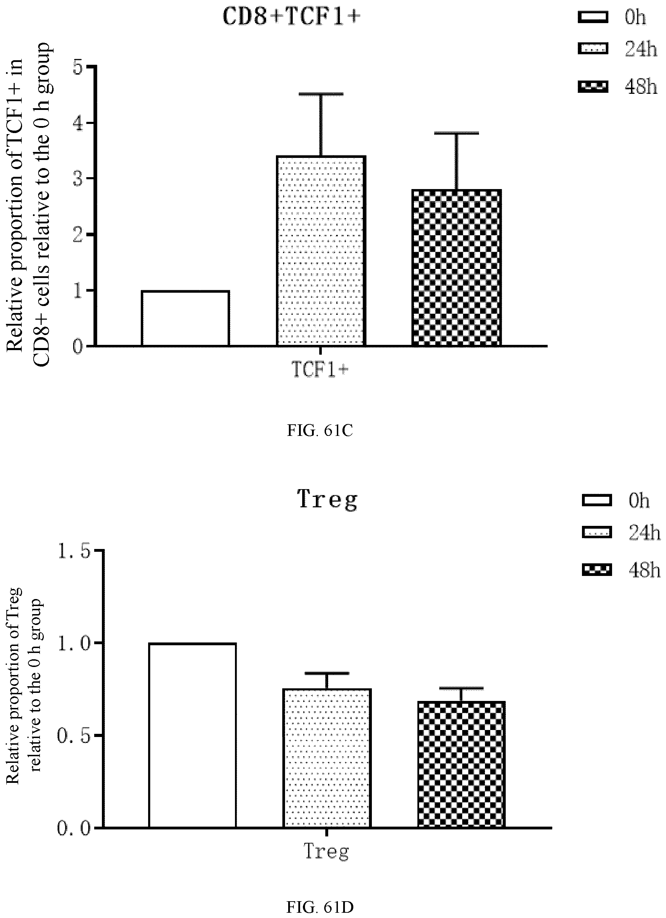

FIG. 61C shows the proportion of $TCF1^+$ stem cell-like T cells in the TIL cells obtained by culturing with feeder cells added at 0, 24 or 48 hours after the addition of OKT3 and IL-2. The results show that, TILs cultured with feeder cells added after 24 hours or 48 hours have higher proportion of stem cell-like T cells in $CD8^+$ compared to that in the TILs cultured with feeder cells added simultaneously.

FIG. 61D shows the proportion of $CD4^+CD25^+Foxp3^+$ regulatory T cells (Tregs) in the TIL cells obtained by culturing with feeder cells added at 0, 24 or 48 hours after the addition of OKT3 and IL-2. The results show that, TILs cultured with feeder cells added after 24 hours or 48 hours have lower proportion of regulatory T cells compared to that in the TILs cultured with feeder cells added simultaneously.

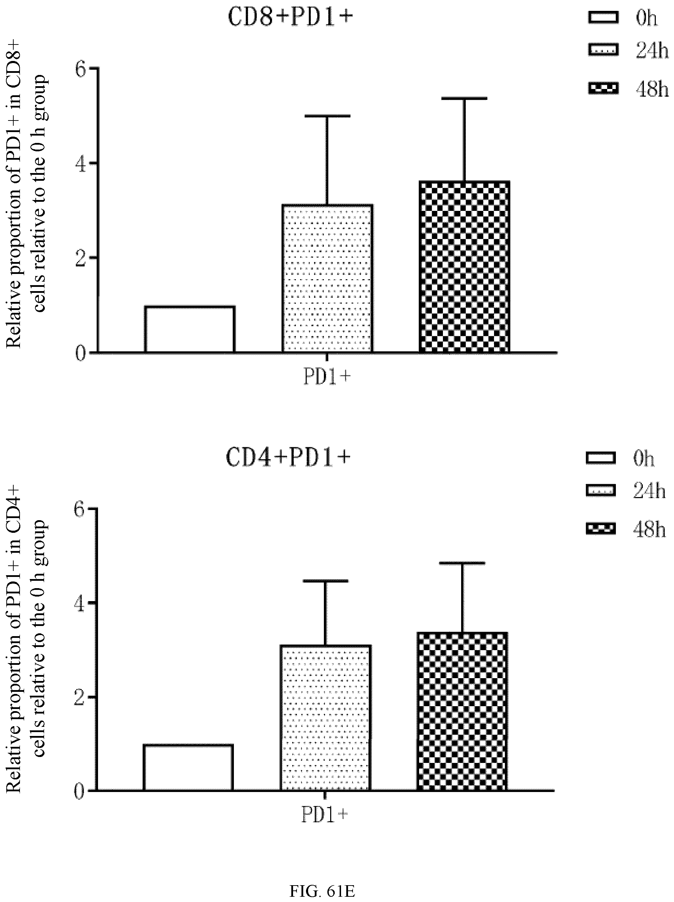

FIG. 61E shows the proportion of activated T cells ($PD-1^+$) in the TIL cells obtained by culturing with feeder cells added at 0, 24 or 48 hours after the addition of OKT3 and IL-2. The results show that, TILs cultured with feeder cells added after 24 hours or 48 hours have higher proportion of activated T cells compared to that in the TILs cultured with feeder cells added simultaneously, e.g., higher proportion of $PD-1^+$ cells in $CD8^+$ and/or $CD4^+$.

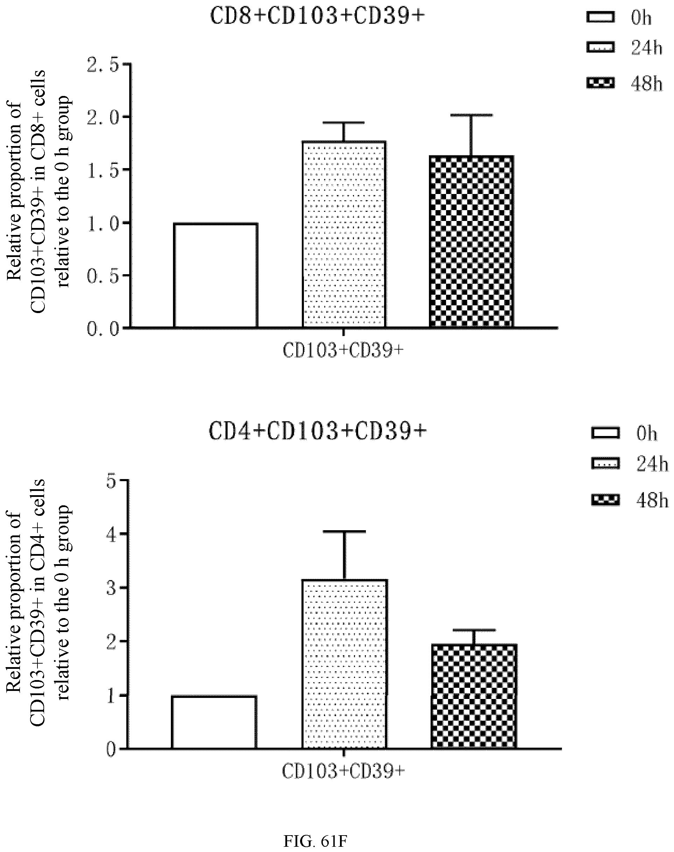

FIG. 61F shows the proportion of $CD103^+CD39^+$ tumor-specific T cells in the TIL cells obtained by culturing with feeder cells added at 0, 24 or 48 hours after the addition of OKT3 and IL-2. The results show that, TILs cultured with feeder cells added after 24 hours or 48 hours have higher proportion of tumor-specific T cells in $CD8^+$ and/or $CD4^+$ compared to that in the TILs cultured with feeder cells added simultaneously.

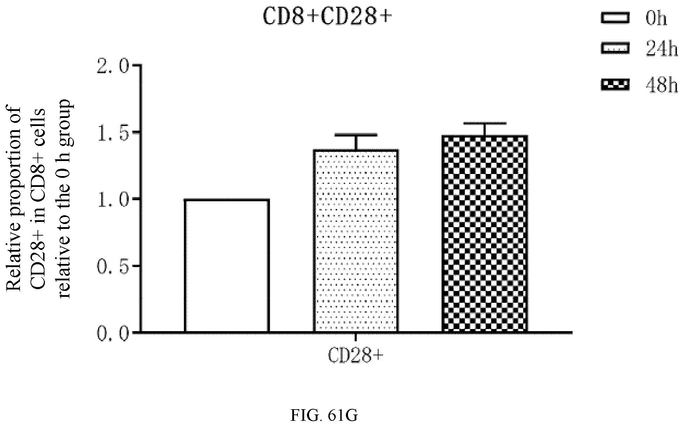

FIG. 61G shows the proportion of activated T cells ($CD28^+$) in the TIL cells obtained by culturing with feeder cells added at 0, 24 or 48 hours after the addition of OKT3 and IL-2. The results show that, TILs cultured with feeder cells added after 24 hours or 48 hours have higher proportion of activated T cells compared to that in the TILs cultured with feeder cells added simultaneously, e.g., higher proportion of $CD8^+CD28^+$ cells.

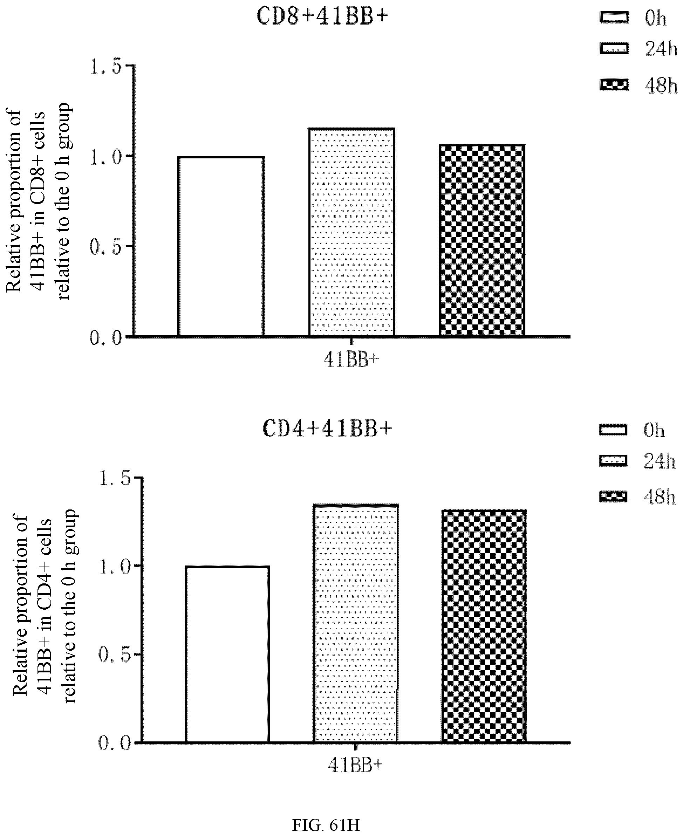

FIG. 61H shows the proportion of activated T cells ($41BB^+$) in the TIL cells obtained by culturing with feeder cells added at 0, 24 or 48 hours after the addition of OKT3 and IL-2. The results show that, TILs cultured with feeder cells added after 24 hours or 48 hours have higher proportion of activated T cells compared to that in the TILs cultured with feeder cells added simultaneously, e.g., higher proportion of $41BB^+$ cells in $CD8^+$ and/or $CD4^+$.

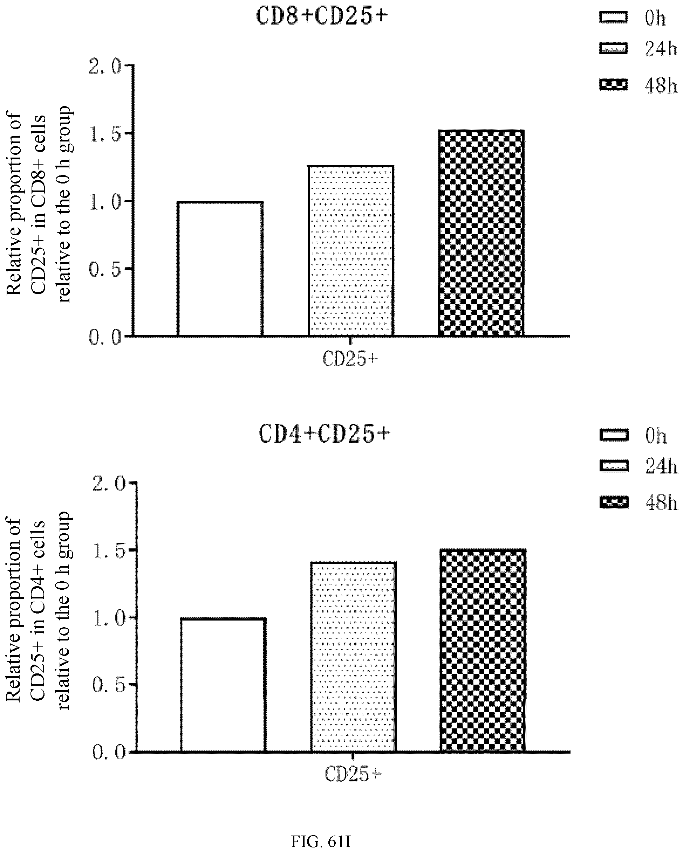

FIG. 61I shows the proportion of activated T cells ($CD25^+$) in the TIL cells obtained by culturing with feeder cells added at 0, 24 or 48 hours after the addition of OKT3 and IL-2. The results show that, TILs cultured with feeder cells added after 24 hours or 48 hours have higher proportion of activated T cells compared to that in the TILs cultured with feeder cells added simultaneously, e.g., higher proportion of $CD25^+$ cells in $CD8^+$ and/or $CD4^+$.

Intracellular factor expression assay

Test preparation

Formulation of the culture medium required for the intracellular factor expression assay: the culture medium of T cells was taken, into which were added CD107a antibodies (BD) at a volume ratio of 1:500.

Assay steps

TILs from each test group were centrifuged, and then resuspended to $1\times10^6$ cells/mL using 600 μL of the culture medium required for the above intracellular factor expression assay, added to a 96-well plate at 100 μL/well, and incubated overnight in an incubator at 37°C.

At the end of the incubation, the cells were washed once with PBS at 200 μL/well, and centrifuged at 600 g for 3 minutes. The supernatant was discarded. An antibody mixed working solution of CD3/CD4/CD8 (BD) was formulated for cell surface staining, with an antibody concentration of 1:100, a viability of 1: 10000, and a staining volume of 50 μL/group. Incubation was performed at 2-8°C in dark for 30 minutes. At the end of the staining, the cells were washed and resuspended in PBS for flow cytometry on machine.

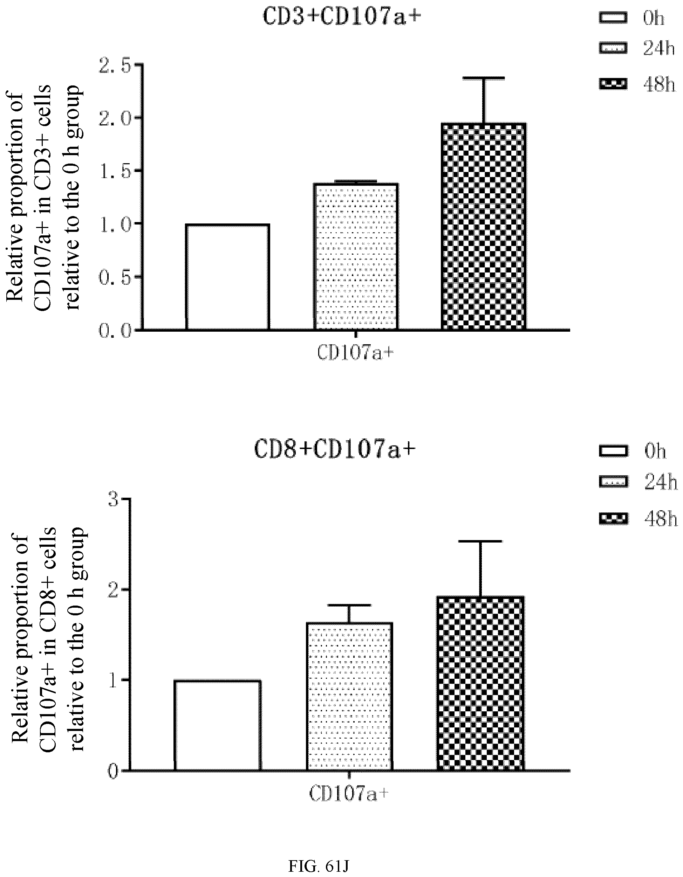

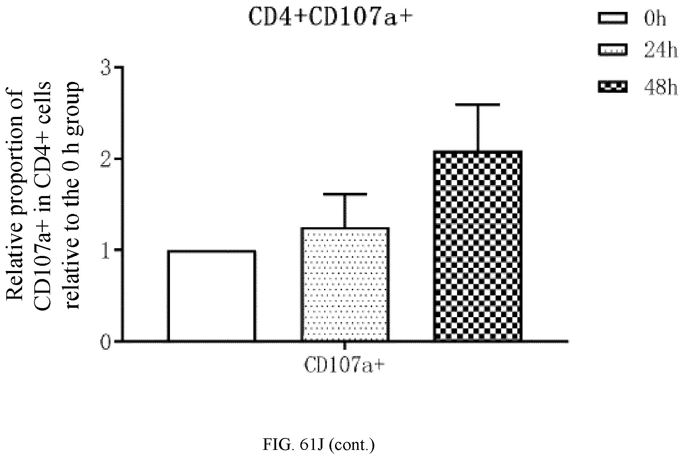

FIG. 61J shows the results of intracellular factor expression assay of TIL cells obtained by culturing with feeder cells added at 0, 24 or 48 hours after the addition of OKT3 and IL-2. The results show that TILs cultured with feeder cells added after 24 hours or 48 hours have a higher intracellular factor expression ability compared with that of TILs cultured with feeder cells added simultaneously. For example, a higher CD107a expression ability in CD3$^+$, CD8$^+$ and/or CD4$^+$.

Cytokine secretion assay

The cytokine secretion assay method can refer to the instructions of the cytokine detection kit (BD). Human Th1/Th2/Th17 cytokine standard freeze-dried powder (BD) was reconstituted with 2 mL of Assay Diluent (BD) (the concentration of each cytokine in the standard stock solution was 5000 pg/mL) and diluted in the following order of gradient dilutions: 1:2, 1:4, 1:8, 1:16, 1:32, 1:64, 1:128, 1:256, 1: 512, 1:1024, labeled as "standard tube". One tube comprising only the Assay Diluent was taken as a reference. Each kind of Capture Beads (BD) were added at 2 μL/Beads/well, then PE Detection Reagent (BD) was added at 10 μL/well and mixed to prepare a mixture (mix), which was added into a V-bottom 96-well plate at 22 μL/well. Into the plate were then added each standard and the supernatant of the test group at 10 μL/well and mixed, then incubated at room temperature in dark for 3 hours.

At the end of the incubation, 200 μL of Wash Buffer (BD) was added to each well and centrifuged at 500 g for 3 min. At the end of the centrifugation, 100 μL of Wash Buffer (BD) was added to each well for resuspension and flow cytometry.

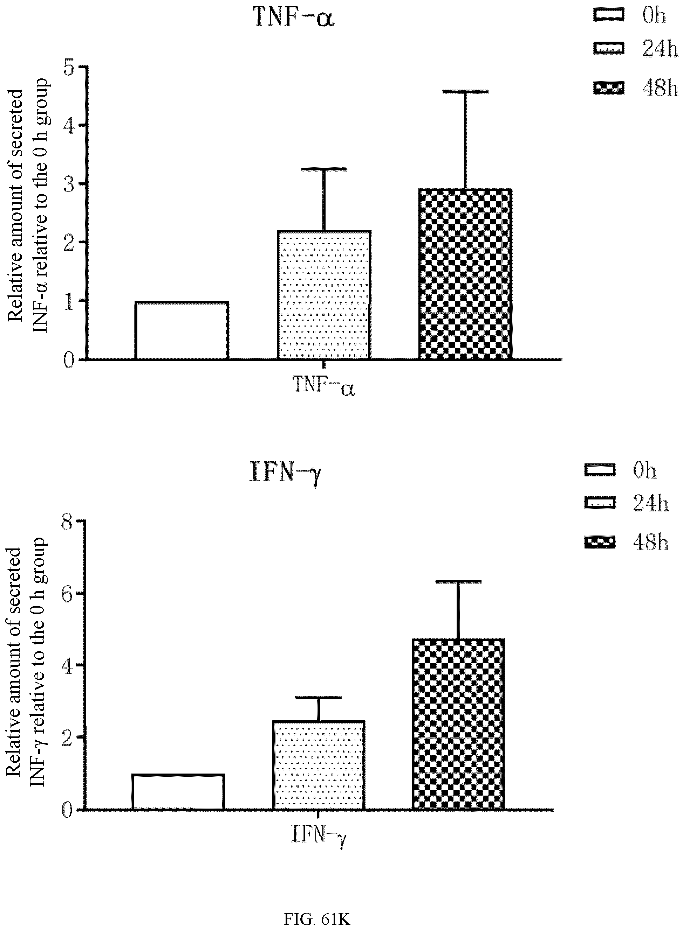

FIG. 61K shows the results of cytokine secretion assay of TIL cells obtained by culturing with feeder cells added at 0, 24 or 48 hours after the addition of OKT3 and IL-2. The results show that, TILs cultured with feeder cells added after 24 hours or 48 hours have a higher cytokine secretion capacity compared with that of the TILs cultured with feeder cells added simultaneously. For example, a higher TNF-α secretion capacity or a higher IFN-γ secretion capacity.

Statistics of the results of TILs cultured with feeder cells added at different times

During the activation of TILs in the second stage of expansion in 1.4 of Example 1, the amount of cells expanded in the first stage was taken and the cell density was adjusted to $5\times10^5$ to $2\times10^6$/mL. In a suspension 24-well culture plate were added CD3 antibodies at 1 mL/well, e.g., about 30 ng/mL of OKT3, and IL-2 at a concentration of about 1000 to 9000 IU/mL, e.g., 3000 or 6000 IU/mL of IL-2. At 0, 6, 12, 24, 48, 72 hours, or 5 days after the addition of OKT3 and IL-2 above, feeder cells were added into the culture environment of tumor-infiltrating lymphocytes. Where, TILs and feeder cells may be added at a ratio of 1:40 to 1:400, e.g., 1:200. All the cells were collected after about 9 to 14 days of culture by the second stage of expansion, and the results of TILs obtained from the culture were detected and counted.

Proliferation ability assay

The TIL cells obtained above by culturing with feeder cells added at different times were counted.

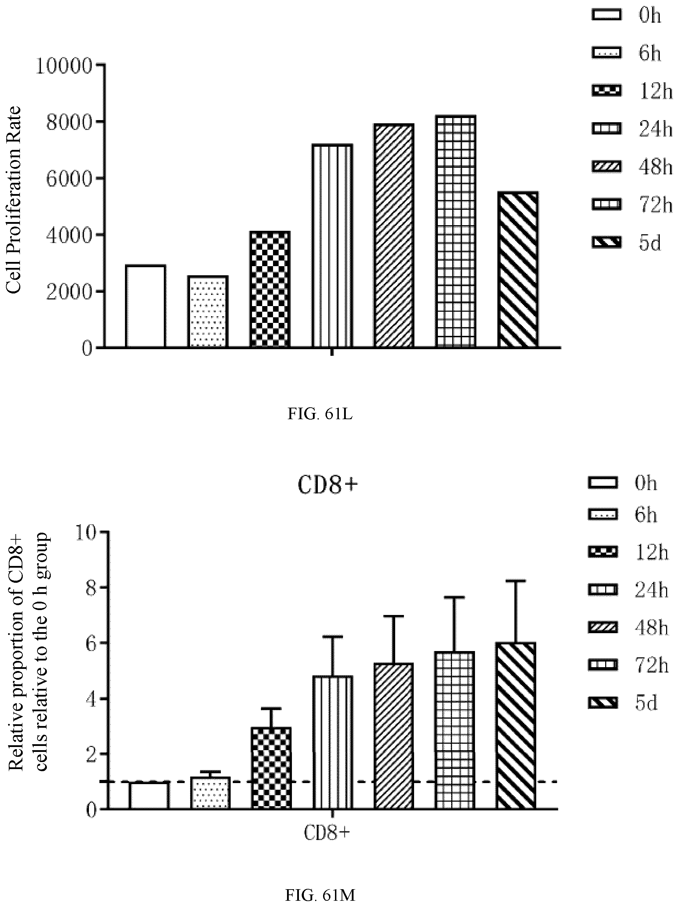

FIG. 61L shows a graph of the cell proliferation ability results of TIL cells obtained by culturing with feeder cells added at 0, 6, 12, 24, 48, 72 hours, or 5 days after the addition of OKT3 and IL-2. Compared to TILs cultured with feeder cells added 0 hours after the addition of OKT3 and IL-2 (i.e., simultaneously), the proliferation ability of TILs cultured with feeder cells added at 12 hours or longer time after the addition of OKT3 and IL-2 was significantly enhanced.

Detection of composition of TIL cells by flow cytometry

TIL populations obtained above by culturing with feeder cells added at different addition times were detected by flow cytometry.

TILs from different donor tumor sources were used as their respective different batches; the data from each batch of the test groups in which feeder cells were added simultaneously with OKT3 and IL-2 (0 h group) was used as the benchmark 1, and the data from the test groups at other time points in the same batch were normalized to count the cell composition proportion of each test group in the second stage of expansion relative to the 0 h group.

The test procedure of flow cytometry can refer to the content in Example 3 of the present application.

FIG. 61M shows the proportion of $CD8^+T$ cells in the TIL cells obtained by culturing with feeder cells added at 0, 6, 12, 24, 48, 72 hours, or 5 days after the addition of OKT3 and IL-2. The results show that, TILs cultured with feeder cells added at 12 hours or longer time after the addition of OKT3 and IL-2 have higher proportion of $CD8^+T$ cells compared to that in the TILs cultured with feeder cells added simultaneously.

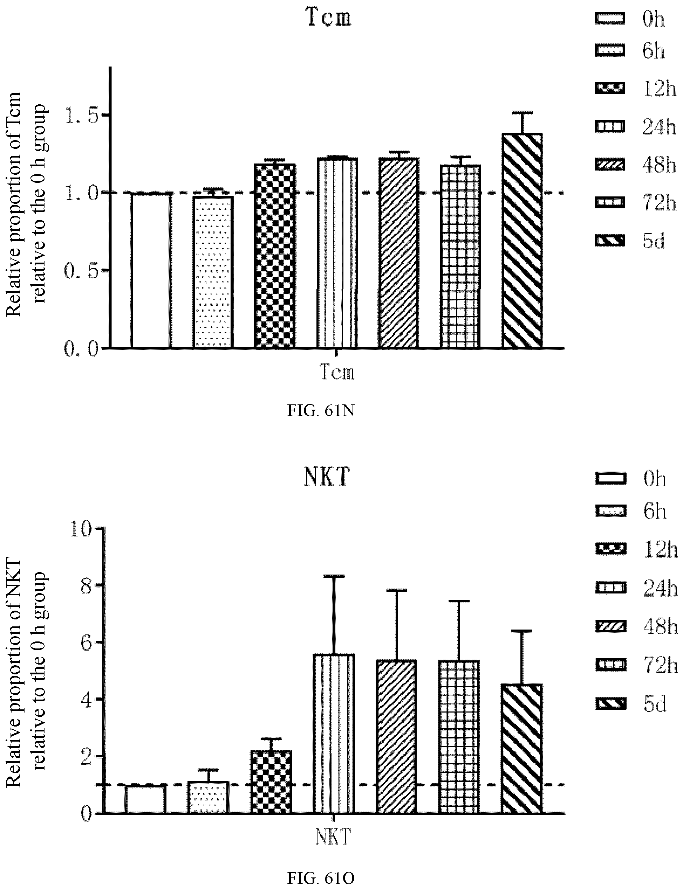

FIG. 61N shows the proportion of $CD45RO^+CD62L^+T$ cells in the TIL cells obtained by culturing with feeder cells added at 0, 6, 12, 24, 48, 72 hours, or 5 days after the addition of OKT3 and IL-2. The results show that, TILs cultured with feeder cells added at 12 hours or longer time after the addition of OKT3 and IL-2 have higher proportion of memory T cells (Terns, $CD45RO^+CD62L^+$) compared to that in the TILs cultured with feeder cells added simultaneously.

FIG. 61O shows the proportion of NK T cells in the TIL cells obtained by culturing with feeder cells added at 0, 6, 12, 24, 48, 72 hours, or 5 days after the addition of OKT3 and IL-2. The results show that, TILs cultured with feeder cells added 1at 2 hours or longer time after the addition of OKT3 and IL-2 have higher proportion of NK T cells compared to that in the TILs cultured with feeder cells added simultaneously.

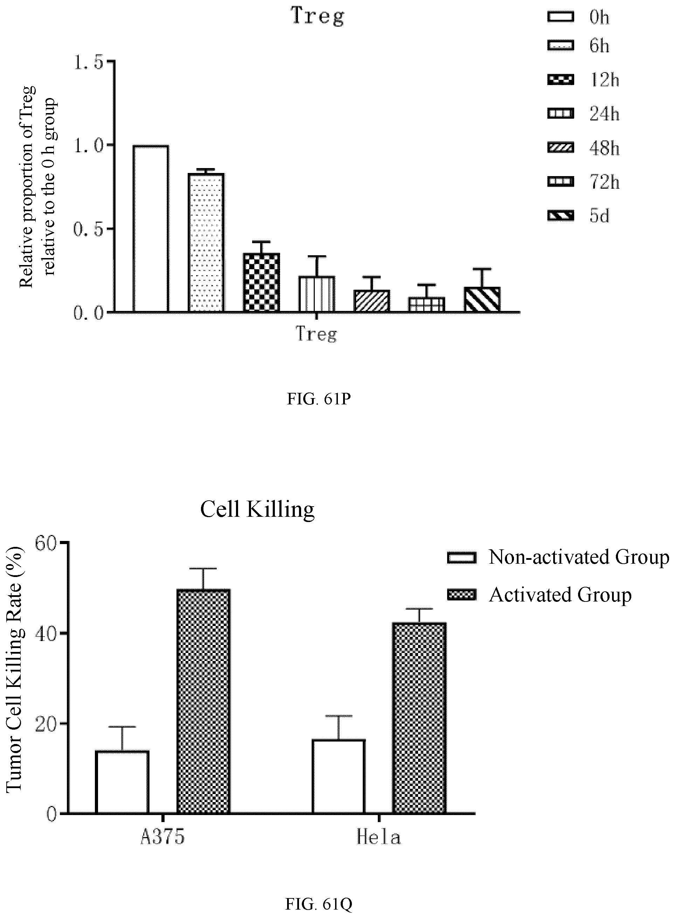

FIG. 61P shows the proportion of $CD4^+CD25^+Foxp3^+$ regulatory T cells (Tregs) in the TIL cells obtained by culturing with feeder cells added at 0, 6, 12, 24, 48, 72 hours, or 5 days after the addition of OKT3 and IL-2. The results show that, TILs cultured with feeder cells added at 12 hours or longer time after the addition of OKT3 and IL-2 have lower proportion of regulatory T cells compared to that in the TILs cultured with feeder cells added simultaneously.

Killing ability assay of TILs cultured in the present application

During the activation of TILs in the second stage of expansion in 1.4 of Example 1, the amount of cells expanded in the first stage was taken and the cell density was adjusted to $5 \times 10^5$ to $2 \times 10^6$/mL. In a suspension 24-well culture plate were added CD3 antibodies at 1 mL/well, e.g., about 30 ng/mL of OKT3, and IL-2 at a concentration of about 1000 to 9000 IU/mL, e.g., 3000 or 6000 IU/mL of IL-2. At 12 hours to 14 days (e.g., 48 hours) after the addition of OKT3 and IL-2 above, feeder cells were added into the culture environment of tumor-infiltrating lymphocytes. Where, TILs and feeder cells were added at a ratio of 1:40 to 1:400, and all the cells were collected after about 9 to 14 days of culture by the second stage of expansion, and the cell killing ability of TILs obtained from the culture was detected and counted.

Cell preparation

TILs obtained from each test group for assay and target cells (e.g., A375 melanoma cells and/or Hela cervical cancer cells) for co-culture were prepared.

Assay steps

Labeling the tumor cells with CFSE (5(6)-Carboxyfluorescein diacetate N-succinimidyl ester, Sigma, 21888-25MG-F): The tumor cells were washed with PBS, and resuspended in 500 μL of PBS; CFSE was added into 500 μL of PBS, and mixed with 500 μL of resuspension of the tumor cells in PBS to a final concentration of CFSE of 0.5 μmol/L. After incubation at 37°C for 6 minutes, a medium comprising 10% FBS was added to wash. Centrifugation was performed at 600 g for 5 minutes. X-vivo 15 medium or other commercially available T cell culture media, e.g. T cell culture media of Stem Cell, Lonza, Thermo, Miltenyi brands etc., were used to resuspend the tumor cells to a concentration of $1 \times 10^6$ cells/mL. The TIL population of each test group was centrifuged at 600 g for 5 minutes, and the TIL cells were resuspended according to an effector-target ratio (the ratio of TIL cells to tumor cells) of 3:1 (i.e., the concentration of the resuspended TIL cells was $3 \times 10^6$ cells/mL). Each 100 μL of tumor cells and TIL cells were added to a U-bottom 96-well plate (Corning), and three replicate wells were set for each group. At the same time, a control group comprising only the tumor cells was set. The well plates were centrifuged at 200 g for 1 minute and incubated at 37°C for 4 hours to overnight. Where, TILs were co-cultured with tumor cells, either without the addition of substances that activate TIL cells as a non-activated group or with the addition of transACT (Miltenyi, a nano-matrix material comprising CD3 antibodies and CD28 antibodies) as an activated group.

After the incubation was completed, centrifugation was performed at 600 g for 3 minutes and the supernatant was discarded. Trypsin was added at 20 μL/well. Incubation was performed in an incubator at 37°C for 3-5 minutes to digest the tumor cells. After the digestion was completed, 180 μL of culture medium comprising 10% FBS was added to terminate the digestion. Dapi (Beyotime, C0060) was diluted at 1:100, and then the diluted Dapi was added at 20 μL/well. Flow cytometry was performed on machine.

Killing rate% = Number of Dapi$^+$CFSE$^+$ cells/Total CFSE$^+$ × 100%, or the killing rate can be expressed by the number of Dapi$^+$ cells/the total number of tumor cells.

FIG. 61Q shows the results of the cell killing ability of TIL cells obtained by culturing with feeder cells added at 48 hours after the addition of OKT3 and IL-2. The results show that, the TILs cultured with feeder cells added at 48 hours after the addition of OKT3 and IL-2 all have significant tumor cell killing ability, e.g., melanoma and/or cervical tumor.

The foregoing detailed description is provided in an illustrative and exemplary manner, and is not intended to limit the scope of the appended claims. Various modifications of embodiments currently listed herein are apparent for those skilled in the art, and are encompassed within the scope of the appended claims and their equivalents.

**Claims**

1. A method for culturing tumor-infiltrating lymphocytes (TILs), wherein the method comprising: improving the expression and/or enhancing the activity of at least one cytokine of the TILs, and co-culturing the TILs with feeder cells after contacting the TILs with T cell activators and/or T cell growth factors for a period of time.

2. The method according to claim 1, wherein the method comprising: improving the expression and/or enhancing the activity of at least one cytokine of the TILs after co-culturing the TILs with the feeder cells.

3. The method according to any one of claims 1-2, wherein the method comprising: improving the expression and/or enhancing the activity of at least one cytokine of the TILs before co-culturing the TILs with the feeder cells.

4. The method according to any one of claims 1-3, wherein the method comprising: improving the expression and/or enhancing the activity of at least one cytokine of the TILs after contacting the TILs with the T cell activators and/or the T cell growth factors and before co-culturing the TILs with the feeder cells.

5. The method according to any one of claims 1-4, wherein the method comprising: improving the expression and/or enhancing the activity of at least one cytokine of the TILs substantially simultaneously with contacting the TILs with the T cell activators and/or the T cell growth factors.

6. The method according to any one of claims 1-5, wherein the method comprising: improving the expression and/or enhancing the activity of at least one cytokine of the TILs substantially simultaneously with co-culturing the TILs with the feeder cells.

7. A method for culturing tumor-infiltrating lymphocytes (TILs), wherein the method comprising: improving the expression and/or enhancing the activity of at least one cytokine of the TILs, wherein the TILs comprise TILs obtained by co-culturing with feeder cells after contacting the TILs with T cell activators and/or T cell growth factors for a period of time.

8. A method for culturing tumor-infiltrating lymphocytes (TILs), wherein the method comprising: co-culturing the TILs with feeder cells after contacting the TILs with T cell activators and/or T cell growth factors for a period of time, wherein the TILs comprise TILs obtained by improving the expression and/or enhancing the activity of at least one cytokine of the TILs.

9. The method according to any one of claims 1-8, wherein TILs obtained by improving the expression and/or enhancing the activity of at least one cytokine of the TILs show improved TIL properties, compared to TILs with unchanged expression and/or activity of the cytokine.

10. The method according to claim 9, wherein the improved TIL properties comprise one or more properties selected from the group consisting of: increased number of TIL cells, increased proportion of viable cells, enhanced persistence, improved proportion of T cell subpopulations, enhanced cytokine secretion capacity, enhanced tumor cell killing ability, enhanced T cell receptor (TCR) clonal diversity, and increased number of TIL cells in tissues.

11. The method according to claim 10, wherein the improved proportion of T cell subpopulations comprises one or more subpopulations selected from the group consisting of: increased proportion of central memory T cells, decreased proportion of regulatory T cells, increased proportion of activated T cells, increased proportion of tumor-specific T cells, and increased proportion of stem cell-like T cells.

12. The method according to any one of claims 1-11, wherein the step of improving the expression and/or enhancing the activity of at least one cytokine of the TILs comprises introducing a nucleic acid encoding the cytokine into the TILs.

13. The method according to claim 12, wherein the step of improving the expression and/or enhancing the activity of at least one cytokine of the TILs comprises introducing a vector comprising the nucleic acid into the TILs.

14. The method according to any one of claims 12-13, wherein the method comprises integrating the nucleic acid encoding the cytokine into a genome of the TILs.

15. The method according to any one of claims 13-14, wherein the vector comprises a viral vector.

16. The method according to claim 15, wherein the viral vector comprises a retroviral vector.

17. The method according to claim 16, wherein the retroviral vector comprises a lentiviral vector.

18. The method according to any one of claims 1-17, wherein the cytokine comprises interleukin (IL).

19. The method according to any one of claims 1-18, wherein the cytokine comprises interleukin-15 (IL-15) and/or a functionally active fragment thereof.

20. The method according to claim 19, wherein the IL-15 comprises membrane-anchored IL-15 and/or secreted IL-15.

21. The method according to any one of claims 19-20, wherein the IL-15 comprises an IL-15 domain.

22. The method according to claim 21, wherein the IL-15 domain comprises an amino acid sequence as shown in SEQ ID NO: 3.

23. The method according to any one of claims 19-22, wherein the IL-15 comprises an IL-15Ra extracellular domain.

24. The method according to claim 23, wherein the IL-15Ra extracellular domain comprises an amino acid sequence as shown in SEQ ID NO: 16.

25. The method according to any one of claims 23-24, wherein the IL-15 domain is directly or indirectly linked to the IL-15Ra extracellular domain.

26. The method according to claim 25, wherein the indirect linkage comprises linking through a linker.

27. The method according to claim 26, wherein the linker comprises an amino acid sequence selected from the group consisting of: SEQ ID NOs: 4-10, $(SEQ\ ID\ NO:\ 11)_l$, $(SEQ\ ID\ NO:\ 12)_m$, $(SEQ\ ID\ NO:\ 13)_n$, $(SEQ\ ID\ NO:\ 14)_p$, and $(SEQ\ ID\ NO:\ 15)_q$, and any combination of the above, wherein l, m, n, p, and q are each independently at least 1.

28. The method according to any one of claims 19-27, wherein the IL-15 comprises a transmembrane domain.

29. The method according to claim 28, wherein the transmembrane domain comprises an amino acid sequence as shown in any one of SEQ ID NOs: 17-22.

30. The method according to any one of claims 28-29, wherein the transmembrane domain is directly or indirectly linked to the IL-15Ra extracellular domain.

31. The method according to any one of claims 19-30, wherein the IL-15 comprises an intracellular domain.

32. The method according to claim 31, wherein the intracellular domain comprises an amino acid sequence as shown in any one of SEQ ID NOs: 23-26.

33. The method according to any one of claims 31-32, wherein the intracellular domain is directly or indirectly linked to the transmembrane domain.

34. The method according to any one of claims 19-33, wherein the IL-15 comprises a signal peptide domain.

35. The method according to claim 34, wherein the signal peptide domain comprises an amino acid sequence as shown in SEQ ID NO: 2.

36. The method according to any one of claims 34-35, wherein the signal peptide domain is directly or indirectly linked to the IL-15 domain.

37. The method according to any one of claims 19-36, wherein the functionally active fragment of the IL-15 comprises an amino acid sequence as shown in SEQ ID NO: 3.

38. The method according to any one of claims 19-37, wherein the IL-15 comprises an amino acid sequence as shown in SEQ ID NO: 1.

39. The method according to any one of claims 1-38, wherein the enhanced expression of the cytokine comprises an enhanced synthesis and/or secretion of the cytokine.

40. The method according to any one of claims 1-39, wherein the proportion of cells expressing the cytokine in the TILs obtained by improving the expression and/or enhancing the activity of at least one cytokine of the TILs is increased, compared to TILs with unchanged expression and/or activity of the cytokine.

41. The method according to any one of claims 1-40, wherein the proportion of cells expressing the cytokine in the TILs obtained by improving the expression and/or enhancing the activity of at least one cytokine of the TILs is increased by at least about 5% or above, compared to TILs with unchanged expression and/or activity of the cytokine.

42. The method according to any one of claims 1-41, wherein in the TILs obtained by improving the expression and/or enhancing the activity of at least one cytokine of the TILs, the proportion of cells expressing the cytokine is at least about 5% or above.

43. The method according to any one of claims 1-42, wherein the method further comprising: subjecting TILs derived from tumor tissues and not expanded *in vitro* to at least one stage of *in vitro* expansion, and co-culturing the TILs with the feeder cells during the at least one stage *of in vitro* expansion.

44. The method according to claim 43, wherein the method comprises co-culturing the TILs with the feeder cells during a single stage *of in vitro* expansion.

45. The method according to any one of claims 43-44, wherein during the single stage of *in vitro* expansion, the method comprises increasing the expression of at least one cytokine of the TILs and/or enhancing the activity thereof, and co-culturing the TILs with the feeder cells.

46. The method according to any one of claims 43-45, wherein the method comprises subjecting the TILs derived from tumor tissues and not expanded *in vitro* to a first stage of *in vitro* expansion and a second stage *of in vitro* expansion, and during the second stage *of in vitro* expansion, co-culturing the TILs with the feeder cells.

47. The method according to claim 46, wherein the first stage of *in vitro* expansion is carried out for at least about 7 days.

48. The method according to any one of claims 46-47, wherein the first stage *of in vitro* expansion is carried out for about 7 days to about 14 days.

49. The method according to any one of claims 46-48, wherein the second stage *of in vitro* expansion is carried out for at least about 7 days.

50. The method according to any one of claims 46-49, wherein the second stage *of in vitro* expansion is carried out for about 7 days to about 14 days.

51. The method according to any one of claims 1-50, comprising co-culturing the TILs with the feeder cells after contacting the TILs with the T cell activators and/or the T cell growth factors for at least about 2 hours.

52. The method according to any one of claims 1-51, comprising the co-culturing TILs with the feeder cells after contacting the TILs with the T cell activators and/or the T cell growth factors for about 6 hours to about 72 hours.

53. The method according to any one of claims 1-52, comprising co-culturing the TILs with the feeder cells after contacting the TILs with the T cell activators and/or the T cell growth factors for about 12 hours to about 48 hours.

54. The method according to any one of claims 1-52, comprising co-culturing the TILs with the feeder cells after contacting the TILs with the T cell activators and/or the T cell growth factors for about 6 hours, about 12 hours, about 24 hours, about 48 hours, or about 72 hours.

55. The method according to any one of claims 1-54, wherein the feeder cells comprise antigen-presenting cells.

56. The method according to any one of claims 1-55, wherein the feeder cells comprise one or more cells selected from the group consisting of: peripheral mononuclear cells, dendritic cells, and artificial antigen-presenting cells.

57. The method according to any one of claims 1-56, wherein the feeder cells are peripheral mononuclear cells.

58. The method according to any one of claims 1-57, wherein the feeder cells are irradiated feeder cells.

59. The method according to any one of claims 1-58, wherein the step of co-culturing the TILs with the feeder cells comprises contacting the surface of the feeder cells with the surface of the TILs.

60. The method according to any one of claims 1-59, wherein the step of co-culturing the TILs with the feeder cells comprises adding the feeder cells into the cell culture medium of the TILs.

61. The method according to claim 60, wherein the method comprising adding the feeder cells into the cell culture medium of the TILs at a proportion of the feeder cells to the TILs from about 40:1 to about 400:1.

62. The method according to any one of claims 1-61, wherein the method further comprising: subjecting TILs derived from tumor tissues and not expanded *in vitro* to at least one stage of *in vitro* expansion, wherein contacting the TILs with the T cell activators during the at least one stage of *in vitro* expansion.

63. The method according to claim 62, wherein contacting the TILs with the T cell activators during the single stage *of in vitro* expansion.

64. The method according to any one of claims 62-63, wherein the method comprises increasing the expression of at least one cytokine of the TILs and/or enhancing the activity thereof, and contacting the TILs with the T cell activators during the single stage of *in vitro* expansion.

65. The method according to any one of claims 62-64, wherein the method comprises subjecting the TILs derived from tumor tissues and not expanded *in vitro* to the first stage of *in vitro* expansion and the second stage of *in vitro* expansion, and during the second stage of *in vitro* expansion, contacting the TILs with the T cell activators.

66. The method according to any one of claims 1-65, wherein the T cell activators comprise one or more activators selected from the group consisting of: cluster of differentiation 80 (CD80), CD86, CD276, 4-1BB ligand (4-1BBL), CD27, CD30, CD134, CD275, CD40, CD258, and the functionally active fragments thereof.

67. The method according to any one of claims 1-66, wherein the T cell activators comprise agonists of one or more targets selected from the group consisting of: CD3, CD28, herpes virus entry mediator (HVEM), CD40L, OX40, and 4-1BB.

68. The method according to any one of claims 1-67, wherein the T cell activators comprise a CD3 agonist and/or a CD28 agonist.

69. The method according to any one of claims 1-68, wherein the T cell activators comprise a CD3 agonist.

**70.** The method according to any one of claims 1-69, wherein the T cell activators comprise an anti-CD3 antibody and/or an antigen-binding fragment thereof.

**71.** The method according to any one of claims 1-70, wherein the T cell activators comprise a CD28 agonist.

**72.** The method according to any one of claims 1-71, wherein the T cell activators comprise an anti-CD28 antibody and/or an antigen-binding fragment thereof, CD80 and/or a functionally active fragment thereof and/or CD86 and/or a functionally active fragment thereof.

**73.** The method according to any one of claims 1-72, wherein the step of contacting the TILs with the T cell activators comprises one or more ways selected from the group consisting of: (1) adding the T cell activators into the cell culture medium of the TILs; (2) adding engineered cells expressing the T cell activators into the cell culture medium of the TILs; and (3) adding a solid medium comprising the T cell activators into the cell culture medium of the TILs.

**74.** The method according to claim 73, wherein the initial concentration of each of the T cell activators in the cell culture medium of the TILs is each independently at least about 30 ng/mL.

**75.** The method according to any one of claims 73-74, wherein the initial concentration of each of the T cell activators in the cell culture medium of the TILs is each independently about 30 ng/mL to about 300 ng/mL.

**76.** The method according to any one of claims 73-75, wherein the diameter of the solid medium is about 500 nm to about 10 $\mu$m.

**77.** The method according to any one of claims 73-76, wherein the diameter of the solid medium is about 1 nm to about 500 nm.

**78.** The method according to any one of claims 76-77, wherein the diameter of the solid medium is measured by transmission electron microscopy.

**79.** The method according to any one of claims 73-78, wherein the solid medium comprises a polymer.

**80.** The method according to any one of claims 73-79, wherein the amount of each of the T cell activators comprised in each mg of the solid medium is each independently at least about 25 $\mu$g.

**81.** The method according to any one of claims 73-80, comprising adding the solid medium comprising the T cell activators into the cell culture medium of the TILs at a proportion of the solid medium to the TILs from about 2:1 to about 1:2.

**82.** The method according to any one of claims 73-81, comprising adding the solid medium comprising the T cell activators into the cell culture medium of the TILs at a proportion of the solid medium to the TILs from about 1: 100 to about 1 :2000.

**83.** The method according to any one of claims 1-82, wherein the method further comprising: subjecting TILs derived from tumor tissues and not expanded *in vitro* to at least one stage of *in vitro* expansion, wherein contacting the TILs with the T cell growth factors during the at least one stage *of in vitro* expansion.

**84.** The method according to claim 83, wherein contacting the TILs with the T cell growth factors during the single stage of *in vitro* expansion.

**85.** The method according to any one of claims 83-84, wherein contacting the TILs with the T cell activators and the T cell growth factors during the single stage *of in vitro* expansion.

**86.** The method according to any one of claims 83-85, wherein subjecting the TILs derived from tumor tissues and not expanded *in vitro* to the first stage *of in vitro* expansion and the second stage of *in vitro* expansion, and during the second stage of *in vitro* expansion, contacting the TILs with the T cell growth factors.

**87.** The method according to any one of claims 1-86, wherein the method comprises contacting the TILs with the T cell activators and the T cell growth factors substantially simultaneously.

**88.** The method according to any one of claims 1-87, wherein the T cell growth factors are one or more factors selected from the group consisting of: IL-2, IL-7, IL-12, IL-15, IL-21, interferon γ, and the functionally active fragments thereof.

**89.** The method according to any one of claims 1-88, wherein the T cell growth factors comprise IL-2 and/or a functionally active fragment thereof.

**90.** The method according to any one of claims 1-89, wherein the step of contacting the TILs with the T cell growth factors comprises adding the T cell growth factors into the cell culture medium of the TILs.

**91.** The method according to any one of claims 1-90, wherein the initial concentration of each of the T cell growth factors in the cell culture medium of the TILs is each independently at least about 300 IU/mL.

**92.** The method according to any one of claims 1-91, wherein the TILs are TILs derived from tumor tissue debris and/or TILs resuscitated after cryopreservation.

**93.** The method according to claim 92, wherein the debris has a volume of about 1 $mm^3$ to about 27 $mm^3$.

**94.** A method for culturing tumor-infiltrating lymphocytes (TILs), comprising:

(A) contacting a first TIL population derived from tumor tissues and not expanded *in vitro* with T cell growth factors, wherein a second TIL population is obtained via the step (A);
(B) improving the expression and/or enhancing the activity of at least one cytokine of the second TIL population, and co-culturing the second TIL population with feeder cells after contacting the second TIL population with T cell activators and/or T cell growth factors for a period of time, wherein a third TIL population is obtained via the step (B).

**95.** A method for culturing tumor-infiltrating lymphocytes (TILs), comprising:

(A) resuscitating and/or continuing culturing an *in vitro* TIL population to obtain a second TIL population, wherein the *in vitro* TIL population comprises a TIL population obtained by *in vitro* expansion of a first TIL population derived from tumor tissues and not expanded *in vitro*;
(B) improving the expression and/or enhancing the activity of at least one cytokine of the second TIL population, and co-culturing the second TIL population with feeder cells after contacting the second TIL population with T cell activators and/or T cell growth factors for a period of time, wherein a third TIL population is obtained via the step (B).

**96.** The method according to claim 95, wherein the *in vitro* TIL population comprises a TIL population obtained by contacting the first TIL population with T cell growth factors.

**97.** The method according to any one of claims 95-96, wherein the *in vitro* TIL population comprises a TIL population obtained by cryopreserving the first TIL population.

**98.** The method according to any one of claims 94-97, wherein the step (A) is carried out for about 7 days to about 14 days.

**99.** The method according to any one of claims 94-98, wherein the step (B) is carried out for about 7 days to about 14 days.

**100.** A method for culturing tumor-infiltrating lymphocytes (TILs), comprising:

(A) contacting a first TIL population derived from tumor tissues and not expanded *in vitro* with T cell growth factors, wherein a second TIL population is obtained via the step (A);
(B) improving the expression and/or enhancing the activity of at least one cytokine of the second TIL population, and contacting the second TIL population with T cell activators and/or T cell growth factors, wherein a third TIL population is obtained via the step (B);
(C) co-culturing the third TIL population with feeder cells, wherein a fourth TIL population is obtained via the step (C).

**101.** A method for culturing tumor-infiltrating lymphocytes (TILs), comprising:

(A) resuscitating and/or continuing culturing an *in vitro* TIL population to obtain a second TIL population, wherein the *in vitro* TIL population comprises a TIL population obtained by *in vitro* expansion of a first TIL population derived from tumor tissues and not expanded *in vitro*;

(B) improving the expression and/or enhancing the activity of at least one cytokine of the second TIL population, and contacting the second TIL population with T cell activators and/or T cell growth factors, wherein a third TIL population is obtained via the step (B);

(C) co-culturing the third TIL population with feeder cells, wherein a fourth TIL population is obtained via the step (C).

102. The method according to claim 101, wherein the *in vitro* TIL population comprises a TIL population obtained by contacting the first TIL population with T cell growth factors.

103. The method according to any one of claims 101-102, wherein the *in vitro* TIL population comprises a TIL population obtained by cryopreserving the first TIL population.

104. The method according to any one of claims 100-103, wherein the step (A) is carried out for about 7 days to about 14 days.

105. The method according to any one of claims 100-104, wherein the step (B) is carried out for about 0 days to about 8 days.

106. The method according to any one of claims 100-105, wherein the step (C) is carried out for about 5 days to about 14 days.

107. A method for culturing tumor-infiltrating lymphocytes (TILs), comprising:

(A) contacting a first TIL population derived from tumor tissues and not expanded *in vitro* with T cell growth factors, wherein a second TIL population is obtained via the step (A);

(B) contacting the second TIL population with T cell activators and/or T cell growth factors, wherein a third TIL population is obtained via the step (B);

(C) improving the expression and/or enhancing the activity of at least one cytokine of the third TIL population, wherein a fourth TIL population is obtained via the step (C);

(D) co-culturing the fourth TIL population with feeder cells, wherein a fifth TIL population is obtained via the step (D).

108. A method for culturing tumor-infiltrating lymphocytes (TILs), comprising:

(A) resuscitating and/or continuing culturing an *in vitro* TIL population to obtain a second TIL population, wherein the *in vitro* TIL population comprises a TIL population obtained by *in vitro* expansion of a first TIL population derived from tumor tissues and not expanded *in vitro*;

(B) contacting the second TIL population with T cell activators and/or T cell growth factors, wherein a third TIL population is obtained via the step (B);

(C) improving the expression and/or enhancing the activity of at least one cytokine of the third TIL population, wherein a fourth TIL population is obtained via the step (C);

(D) co-culturing the fourth TIL population with feeder cells, wherein a fifth TIL population is obtained via the step (D).

109. The method according to claim 108, wherein the *in vitro* TIL population comprises a TIL population obtained by contacting the first TIL population with T cell growth factors.

110. The method according to any one of claims 108-109, wherein the *in vitro* TIL population comprises a TIL population obtained by cryopreserving the first TIL population.

111. The method according to any one of claims 107-110, wherein the step (A) is carried out for about 7 days to about 14 days.

112. The method according to any one of claims 107-111, wherein the step (B) is carried out for about 0 days to about 4 days.

113. The method according to any one of claims 107-112, wherein the step (C) is carried out for about 0 days to about 4 days.

114. The method according to any one of claims 107-113, wherein the step (D) is carried out for about 5 days to about 14 days.

115. The method according to any one of claims 94-114, wherein TILs obtained by improving the expression and/or enhancing the activity of at least one cytokine of the TILs show improved TIL properties, compared to TILs with unchanged expression and/or activity of the cytokine.

116. The method according to claim 115, wherein the improved TIL properties comprise one or more properties selected from the group consisting of: increased number of TIL cells, increased proportion of viable cells, enhanced persistence, improved proportion of T cell subpopulations, enhanced cytokine secretion capacity, enhanced tumor cell killing ability, enhanced T cell receptor (TCR) clonal diversity, and increased number of TIL cells in tissues.

117. The method according to claim 116, wherein the improved proportion of T cell subpopulations comprises one or more subpopulations selected from the group consisting of: increased proportion of central memory T cells, decreased proportion of regulatory T cells, increased proportion of activated T cells, increased proportion of tumor-specific T cells, and increased proportion of stem cell-like T cells.

118. The method according to any one of claims 94-117, wherein the step of improving the expression and/or enhancing the activity of at least one cytokine of the TILs comprises introducing a nucleic acid encoding the cytokine into the TILs.

119. The method according to claim 118, wherein the step of improving the expression and/or enhancing the activity of at least one cytokine of the TILs comprises introducing a vector comprising the nucleic acid into the TILs.

120. The method according to any one of claims 118-119, wherein integrating a nucleic acid encoding the cytokine into the genome of the TILs.

121. The method according to any one of claims 118-120, wherein the vector comprises a viral vector.

122. The method according to claim 121, wherein the viral vector comprises a retroviral vector.

123. The method according to claim 122, wherein the retroviral vector comprises a lentiviral vector.

124. The method according to any one of claims 94-123, wherein the cytokine comprises interleukin (IL).

125. The method according to any one of claims 94-124, wherein the cytokine comprises interleukin-15 (IL-15) and/or a functionally active fragment thereof.

126. The method according to claim 125, wherein the IL-15 comprises membrane-anchored IL-15 and/or secreted IL-15.

127. The method according to any one of claims 125-126, wherein the IL-15 comprises an IL-15 domain.

128. The method according to claim 127, wherein the IL-15 domain comprises an amino acid sequence as shown in SEQ ID NO: 3.

129. The method according to any one of claims 125-128, wherein the IL-15 comprises an IL-15Ra extracellular domain.

130. The method according to claim 129, wherein the IL-15Ra extracellular domain comprises an amino acid sequence as shown in SEQ ID NO: 16.

131. The method according to any one of claims 129-130, wherein the IL-15 domain is directly or indirectly linked to the IL-15Ra extracellular domain.

132. The method according to claim 131, wherein the indirect linkage comprises linking through a linker.

133. The method according to claim 132, wherein the linker comprises an amino acid sequence selected from the group consisting of: SEQ ID NOs: 4-10, (SEQ ID NO: 11)$_1$, (SEQ ID NO: 12)$_m$, (SEQ ID NO: 13)$_n$, (SEQ ID NO: 14)$_p$, and (SEQ ID NO: 15)$_q$, and any combination of the above, wherein l, m, n, p, and q are each independently at least 1.

134. The method according to any one of claims 125-133, wherein the IL-15 comprises a transmembrane domain.

135. The method according to claim 134, wherein the transmembrane domain comprises an amino acid sequence as shown in any one of SEQ ID NOs: 17-22.

136. The method according to any one of claims 134-135, wherein the transmembrane domain is directly or indirectly linked to the IL-15Ra extracellular domain.

137. The method according to any one of claims 125-136, wherein the IL-15 comprises an intracellular domain.

138. The method according to claim 137, wherein the intracellular domain comprises an amino acid sequence as shown in any one of SEQ ID NOs: 23-26.

139. The method according to any one of claims 137-138, wherein the intracellular domain is directly or indirectly linked to the transmembrane domain.

140. The method according to any one of claims 125-139, wherein the IL-15 comprises a signal peptide domain.

141. The method according to claim 140, wherein the signal peptide domain comprises an amino acid sequence as shown in SEQ ID NO: 2.

142. The method according to any one of claims 140-141, wherein the signal peptide domain is directly or indirectly linked to the IL-15 domain.

143. The method according to any one of claims 125-142, wherein the functionally active fragment of the IL-15 comprises an amino acid sequence as shown in SEQ ID NO: 3.

144. The method according to any one of claims 125-143, wherein the IL-15 comprises an amino acid sequence as shown in SEQ ID NO: 1.

145. The method according to any one of claims 94-144, wherein the enhanced expression of the cytokine comprises enhanced synthesis and/or secretion of the cytokine.

146. The method according to any one of claims 94-145, wherein the proportion of cells expressing the cytokine in the TILs obtained by improving the expression and/or enhancing the activity of at least one cytokine of the TILs is increased, compared to TILs with unchanged expression and/or activity of the cytokine.

147. The method according to any one of claims 94-146, wherein the proportion of cells expressing the cytokine in the TILs obtained by improving the expression and/or enhancing the activity of at least one cytokine of the TILs is increased by at least about 5% or above, compared to TILs with unchanged expression and/or activity of the cytokine.

148. The method according to any one of claims 94-147, wherein in the TILs obtained by improving the expression and/or enhancing the activity of at least one cytokine of the TILs, the proportion of cells expressing the cytokine is at least about 5% or above.

149. The method according to any one of claims 94-148, comprising co-culturing the TILs with the feeder cells after contacting the TILs with the T cell activators and/or the T cell growth factors for at least about 2 hours.

150. The method according to any one of claims 94-149, comprising co-culturing the TILs with the feeder cells after contacting the TILs with the T cell activators and/or the T cell growth factors for about 6 hours to about 72 hours.

151. The method according to any one of claims 94-150, comprising co-culturing the TILs with the feeder cells after contacting the TILs with the T cell activators and/or the T cell growth factors for about 12 hours to about 48 hours.

152. The method according to any one of claims 94-150, comprising co-culturing the TILs with the feeder cells after contacting the TILs with the T cell activators and/or the T cell growth factors for about 6 hours, about 12 hours, about 24 hours, about 48 hours, or about 72 hours.

153. The method according to any one of claims 94-152, wherein the feeder cells comprise antigen-presenting cells.

154. The method according to claims 94-153, wherein the feeder cells comprise one or more cells selected from the group consisting of: peripheral mononuclear cells, dendritic cells, and artificial antigen-presenting cells.

155. The method according to claims 94-154, wherein the feeder cells are peripheral mononuclear cells.

156. The method according to claims 94-155, wherein the feeder cells are irradiated feeder cells.

157. The method according to any one of claims 94-156, wherein the step of co-culturing the TILs with the feeder cells comprises contacting the surface of the feeder cells with the surface of the TILs.

158. The method according to any one of claims 94-157, wherein the step of co-culturing the TILs with the feeder cells comprises adding the feeder cells into the cell culture medium of the TILs.

159. The method according to claim 158, wherein the method comprises adding the feeder cells into the cell culture medium of the TILs at a proportion of the feeder cells to the TILs from about 40:1 to about 400:1.

160. The method according to any one of claims 94-159, wherein the T cell activators comprise one or more activators selected from the group consisting of: cluster of differentiation 80 (CD80), CD86, CD276, 4-1BB ligand (4-1BBL), CD27, CD30, CD134, CD275, CD40, CD258, and functionally active fragments thereof.

161. The method according to any one of claims 94-160, wherein the T cell activators comprise agonists of one or more targets selected from the group consisting of: CD3, CD28, herpes virus entry mediator (HVEM), CD40L, OX40, and 4-1BB.

162. The method according to any one of claims 94-161, wherein the T cell activators comprise a CD3 agonist and/or a CD28 agonist.

163. The method according to any one of claims 94-162, wherein the T cell activators comprise a CD3 agonist.

164. The method according to any one of claims 94-163, wherein the T cell activators comprise an anti-CD3 antibody and/or an antigen-binding fragment thereof.

165. The method according to any one of claims 94-164, wherein the T cell activators comprise a CD28 agonist.

166. The method according to any one of claims 94-165, wherein the T cell activators comprise an anti-CD28 antibody and/or an antigen-binding fragment thereof, CD80 and/or a functionally active fragment thereof and/or CD86 and/or a functionally active fragment thereof.

167. The method according to any one of claims 94-166, wherein the step of contacting the TILs with the T cell activators comprises one or more ways selected from the group consisting of: (1) adding the T cell activators into the cell culture medium of the TILs; (2) adding engineered cells expressing the T cell activators into the cell culture medium of the TILs; and (3) adding a solid medium comprising the T cell activators into the cell culture medium of the TILs.

168. The method according to claim 167, wherein the initial concentration of each of the T cell activators in the cell culture medium of the TILs is each independently at least about 30 ng/mL.

169. The method according to any one of claims 167-168, wherein the initial concentration of each of the T cell activators in the cell culture medium of the TILs is each independently about 30 ng/mL to about 300 ng/mL.

170. The method according to any one of claims 167-169, wherein the diameter of the solid medium is about 500 nm to about 10 μm.

171. The method according to any one of claims 167-170, wherein the diameter of the solid medium is about 1 nm to about 500 nm.

172. The method according to any one of claims 170-171, wherein the diameter of the solid medium is measured by transmission electron microscopy.

173. The method according to any one of claims 167-172, wherein the solid medium comprises a polymer.

174. The method according to any one of claims 167-173, wherein the amount of each of the T cell activators comprised in each mg of the solid medium is each independently at least about 25 $\mu$g.

175. The method according to any one of claims 167-174, comprising adding the solid medium comprising the T cell activators into the cell culture medium of the TILs at a proportion of the solid medium to the TILs from about 2:1 to about 1:2.

176. The method according to any one of claims 167-175, comprising adding the solid medium comprising the T cell activators into the cell culture medium of the TILs at a proportion of the solid medium to the TILs from about 1:100 to about 1:2000.

177. The method according to any one of claims 94-176, wherein the method comprises contacting the TILs with the T cell activators and the T cell growth factors substantially simultaneously.

178. The method according to any one of claims 94-177, wherein the T cell growth factors are one or more factors selected from the group consisting of: IL-2, IL-7, IL-12, IL-15, IL-21, interferon $\gamma$, and the functionally active fragments thereof.

179. The method according to any one of claims 94-178, wherein the T cell growth factors comprise IL-2 and/or a functionally active fragment thereof.

180. The method according to any one of claims 94-179, wherein the step of contacting the TILs with the T cell growth factors comprises adding the T cell growth factors into the cell culture medium of the TILs.

181. The method according to any one of claims 94-180, wherein the initial concentration of each of the T cell growth factors in the cell culture medium of the TILs is each independently at least about 300 IU/mL.

182. The method according to any one of claims 94-181, wherein the TILs are TILs derived from tumor tissue debris and/or TILs resuscitated after cryopreservation.

183. The method according to claim 182, wherein the debris has a volume of about 1 mm$^3$ to about 27 mm$^3$.

184. A method for culturing tumor-infiltrating lymphocytes (TILs), wherein the method comprising: improving the expression and/or enhancing the activity of at least one cytokine of the TILs, and contacting the TILs with a CD28 agonist.

185. The method according to claim 184, wherein the method comprising: improving the expression and/or enhancing the activity of at least one cytokine of the TILs after contacting the TILs with the CD28 agonist.

186. The method according to any one of claims 184-185, wherein the method comprising: improving the expression and/or enhancing the activity of at least one cytokine of the TILs before contacting the TILs with the CD28 agonist.

187. A method for culturing tumor-infiltrating lymphocytes (TILs), wherein the method comprising: improving the expression and/or enhancing the activity of at least one cytokine of the TILs, wherein the TILs comprise TILs obtained by contacting the TILs with a CD28 agonist.

188. A method for culturing tumor-infiltrating lymphocytes (TILs), wherein the method comprising: contacting the TILs with a CD28 agonist, wherein the TILs comprise TILs obtained by improving the expression and/or enhancing the activity of at least one cytokine of the TILs.

189. The method according to any one of claims 184-188, wherein TILs obtained by improving the expression and/or enhancing the activity of at least one cytokine of the TILs show improved TIL properties, compared to TILs with unchanged expression and/or activity of the cytokine.

190. The method according to claim 189, wherein the improved TIL properties comprise one or more properties selected from the group consisting of: increased number of TIL cells, increased proportion of viable cells, enhanced persistence, improved proportion of T cell subpopulations, enhanced cytokine secretion capacity, enhanced tumor cell killing ability, enhanced T cell receptor (TCR) clonal diversity, and increased number of TIL cells in tissues.

191. The method according to claim 190, wherein the improved proportion of T cell subpopulations comprises one or more subpopulations selected from the group consisting of: increased proportion of central memory T cells, decreased proportion of regulatory T cells, increased proportion of activated T cells, increased proportion of tumor-specific T cells, and increased proportion of stem cell-like T cells.

192. The method according to any one of claims 184-191, wherein the TILs that have been contacted with the CD28 agonist during at least one stage of *in vitro* expansion show an improved gene editing effect, compared to corresponding TILs that have not been contacted with the CD28 agonist during the stage of *in vitro* expansion.

193. The method according to claim 192, wherein the improved gene editing effect comprises enhanced gene knockout efficiency.

194. The method according to any one of claims 184-193, wherein the step of improving the expression and/or enhancing the activity of at least one cytokine of the TILs comprises introducing a nucleic acid encoding the cytokine into the TILs.

195. The method according to claim 194, wherein the step of improving the expression and/or enhancing the activity of at least one cytokine of the TILs comprises introducing a vector comprising the nucleic acid into the TILs.

196. The method according to any one of claims 194-195, wherein integrating a nucleic acid encoding the cytokine into the genome of the TILs.

197. The method according to any one of claims 195-196, wherein the vector comprises a viral vector.

198. The method according to claim 197, wherein the viral vector comprises a retroviral vector.

199. The method according to claim 198, wherein the retroviral vector comprises a lentiviral vector.

200. The method according to any one of claims 184-199, wherein the cytokine comprises interleukin (IL).

201. The method according to any one of claims 184-200, wherein the cytokine comprises interleukin-15 (IL-15) and/or a functionally active fragment thereof.

202. The method according to claim 201, wherein the IL-15 comprises membrane-anchored IL-15 and/or secreted IL-15.

203. The method according to any one of claims 201-202, wherein the IL-15 comprises an IL-15 domain.

204. The method according to claim 203, wherein the IL-15 domain comprises an amino acid sequence as shown in SEQ ID NO: 3.

205. The method according to any one of claims 201-204, wherein the IL-15 comprises an IL-15Ra extracellular domain.

206. The method according to claim 205, wherein the IL-15Ra extracellular domain comprises an amino acid sequence as shown in SEQ ID NO: 16.

207. The method according to any one of claims 205-206, wherein the IL-15 domain is directly or indirectly linked to the IL-15Ra extracellular domain.

208. The method according to claim 207, wherein the indirect linkage comprises linking through a linker.

209. The method according to claim 208, wherein the linker comprises an amino acid sequence selected from the group consisting of: SEQ ID NOs: 4-10, (SEQ ID NO: 11)$_1$, (SEQ ID NO: 12)$_m$, (SEQ ID NO: 13)$_n$, (SEQ ID NO: 14)$_p$, and

(SEQ ID NO: 15)$_q$, and any combination of the above, wherein l, m, n, p, and q are each independently at least 1.

210. The method according to any one of claims 201-209, wherein the IL-15 comprises a transmembrane domain.

211. The method according to claim 210, wherein the transmembrane domain comprises an amino acid sequence as shown in any one of SEQ ID NOs: 17-22.

212. The method according to any one of claims 210-211, wherein the transmembrane domain is directly or indirectly linked to the IL-15Ra extracellular domain.

213. The method according to any one of claims 201-212, wherein the IL-15 comprises an intracellular domain.

214. The method according to claim 213, wherein the intracellular domain comprises an amino acid sequence as shown in any one of SEQ ID NOs: 23-26.

215. The method according to any one of claims 213-214, wherein the intracellular domain is directly or indirectly linked to the transmembrane domain.

216. The method according to any one of claims 201-215, wherein the IL-15 comprises a signal peptide domain.

217. The method according to claim 216, wherein the signal peptide domain comprises an amino acid sequence as shown in SEQ ID NO: 2.

218. The method according to any one of claims 216-217, wherein the signal peptide domain is directly or indirectly linked to the IL-15 domain.

219. The method according to any one of claims 201-218, wherein the functionally active fragment of the IL-15 comprises an amino acid sequence as shown in SEQ ID NO: 3.

220. The method according to any one of claims 201-219, wherein the IL-15 comprises an amino acid sequence as shown in SEQ ID NO: 1.

221. The method according to any one of claims 184-220, wherein the enhanced expression of the cytokine comprises an enhanced synthesis and/or secretion of the cytokine.

222. The method according to any one of claims 184-221, wherein the proportion of cells expressing the cytokine in the TILs obtained by improving the expression and/or enhancing the activity of at least one cytokine of the TILs is increased, compared to TILs with unchanged expression and/or activity of the cytokine.

223. The method according to any one of claims 184-222, wherein the proportion of cells expressing the cytokine in the TILs obtained by improving the expression and/or enhancing the activity of at least one cytokine of the TILs is increased by at least about 5% or above, compared to TILs with unchanged expression and/or activity of the cytokine.

224. The method according to any one of claims 184-223, wherein in the TILs obtained by improving the expression and/or enhancing the activity of at least one cytokine of the TILs, the proportion of cells expressing the cytokine is at least about 5% or above.

225. The method according to any one of claims 184-224, wherein subjecting the TILs derived from tumor tissues and not expanded *in vitro* to at least one stage *of in vitro* expansion, and wherein contacting the TILs with the CD28 agonist during the at least one stage *of in vitro* expansion.

226. The method according to claim 225, wherein subjecting the TILs derived from tumor tissues and not expanded *in vitro* to a first stage *of in vitro* expansion and a second stage of *in vitro* expansion, and during the second stage of *in vitro* expansion, contacting the TILs from the first stage *of in vitro* expansion with the CD28 agonist.

227. The method according to claim 226, wherein the first stage *of in vitro* expansion is carried out for at least about 7 days.

228. The method according to any one of claims 226-227, wherein the first stage *of in vitro* expansion is carried out for about 7 days to about 14 days.

229. The method according to any one of claims 226-228, wherein the second stage of *in vitro* expansion is carried out for at least about 7 days.

230. The method according to any one of claims 226-229, wherein the second stage of *in vitro* expansion is carried out for about 7 days to about 14 days.

231. The method according to any one of claims 184-230, wherein the CD28 agonist comprises an anti-CD28 antibody and/or an antigen-binding fragment thereof, CD80 and/or a functionally active fragment thereof, and/or CD86 and/or a functionally active fragment thereof.

232. The method according to any one of claims 184-231, wherein the method further comprising: subjecting TILs derived from tumor tissues and not expanded *in vitro* to at least one stage of *in vitro* expansion, wherein contacting the TILs with other T cell activators other than the CD28 agonist during the at least one stage *of in vitro* expansion.

233. The method according to claim 232, wherein during a single stage of *in vitro* expansion, contacting the TILs with the other T cell activators.

234. The method according to any one of claims 232-233, wherein the method comprises increasing the expression of at least one cytokine of the TILs and/or enhancing the activity thereof, and contacting the TILs with the other T cell activators during the single stage *of in vitro* expansion.

235. The method according to any one of claims 232-234, wherein the method comprises subjecting the TILs derived from tumor tissues and not expanded *in vitro* to the first stage of *in vitro* expansion and the second stage of *in vitro* expansion, and during the second stage *of in vitro* expansion, contacting the TILs with the other T cell activators.

236. The method according to any one of claims 232-235, wherein the method comprises contacting the TILs with the CD28 agonist and the other T cell activators substantially simultaneously.

237. The method according to any one of claims 232-236, wherein the other T cell activators comprise agonists of one or more targets selected from the group consisting of: CD3, HVEM, CD40L, OX40, and 4-1BB.

238. The method according to any one of claims 232-237, wherein the other T cell activators comprise a CD3 agonist.

239. The method according to any one of claims 232-238, wherein the other T cell activators comprise an anti-CD3 antibody and/or an antigen-binding fragment thereof.

240. The method according to any one of claims 232-239, wherein the step of contacting the TILs with the CD28 agonist and the other T cell activators comprises one or more ways selected from the group consisting of: (1) adding the CD28 agonist and the other T cell activators into the cell culture medium of the TILs; (2) adding engineered cells expressing the CD28 agonist and the other T cell activators into the cell culture medium of the TILs; (3) adding a solid medium comprising the CD28 agonist and the other T cell activators into the cell culture medium of the TILs.

241. The method according to claim 240, wherein the initial concentration of the other T cell activators in the cell culture medium of the TILs is at least about 30 ng/mL.

242. The method according to any one of claims 240-241, wherein the initial concentration of the other T cell activators in the cell culture medium of the TILs is about 30 ng/mL to about 300 ng/mL.

243. The method according to any one of claims 240-242, wherein the diameter of the solid medium is about 500 nm to about 10 μm.

244. The method according to any one of claims 240-243, wherein the diameter of the solid medium is about 1 nm to about 500 nm.

245. The method according to any one of claims 243-244, wherein the diameter of the solid medium is measured by transmission electron microscopy.

246. The method according to any one of claims 240-245, wherein the solid medium comprises a polymer.

247. The method according to any one of claims 240-246, wherein each mg of the solid medium comprises at least about 25 μg of the CD28 agonist and the other T cell activators.

248. The method according to any one of claims 240-247, comprising adding the solid medium comprising the CD28 agonist and the other T cell activators into the cell culture medium of the TILs at a proportion of the solid medium to the TILs from about 2:1 to about 1:2.

249. The method according to any one of claims 240-248, comprising adding the solid medium comprising the CD28 agonist and the other T cell activators into the cell culture medium of the TILs at a proportion of the solid medium to the TILs from about 1:100 to about 1:2000.

250. The method according to any one of claims 184-249, wherein the method further comprising: subjecting TILs derived from tumor tissues and not expanded *in vitro* to at least one stage of *in vitro* expansion, wherein co-culturing the TILs with feeder cells after contacting the TILs with the CD28 agonist for a period of time during the at least one stage of *in vitro* expansion.

251. The method according to claim 250, wherein co-culturing the TILs with the feeder cells during the single stage of *in vitro* expansion.

252. The method according to any one of claims 250-251, wherein the method comprises contacting the TILs with the CD28 agonist and co-culturing with the feeder cells during the single stage of *in vitro* expansion.

253. The method according to any one of claims 250-252, wherein the method comprises subjecting the TILs derived from tumor tissues and not expanded *in vitro* to the first stage of *in vitro* expansion and the second stage of *in vitro* expansion, and during the second stage of *in vitro* expansion, co-culturing the TILs with the feeder cells.

254. The method according to any one of claims 250-253, comprising co-culturing the TILs with the feeder cells after contacting the TILs with the CD28 agonist for at least about 2 hours.

255. The method according to any one of claims 250-254, comprising co-culturing the TILs with the feeder cells after contacting the TILs with the CD28 agonist for about 6 hours to about 72 hours.

256. The method according to any one of claims 250-255, comprising co-culturing the TILs with the feeder cells after contacting the TILs with the CD28 agonist for about 12 hours to about 48 hours.

257. The method according to any one of claims 250-255, comprising co-culturing the TILs with the feeder cells after contacting the TILs with the CD28 agonist for about 6 hours, about 12 hours, about 24 hours, about 48 hours, or about 72 hours.

258. The method according to any one of claims 250-257, wherein the feeder cells comprise antigen-presenting cells.

259. The method according to any one of claims 250-258, wherein the feeder cells comprise one or more cells selected from the group consisting of: peripheral mononuclear cells, dendritic cells, and artificial antigen-presenting cells.

260. The method according to any one of claims 250-259, wherein the feeder cells are peripheral mononuclear cells.

261. The method according to any one of claims 250-260, wherein the feeder cells are irradiated feeder cells.

262. The method according to any one of claims 250-261, wherein the step of co-culturing the TILs with the feeder cells comprises contacting the surface of the feeder cells with the surface of the TILs.

263. The method according to any one of claims 250-262, wherein the step of co-culturing the TILs with the feeder cells comprises adding the feeder cells into the cell culture medium of the TILs.

264. The method according to any one of claims 250-263, comprising adding the feeder cells into the cell culture medium of the TILs at a proportion of the feeder cells to the TILs from about 40:1 to about 400:1.

265. The method according to any one of claims 184-264, wherein the method further comprising: subjecting TILs derived from tumor tissues and not expanded *in vitro* to at least one stage of *in vitro* expansion, wherein contacting the TILs with T cell growth factors during the at least one stage of *in vitro* expansion.

266. The method according to claim 265, comprising contacting the TILs with the T cell growth factors during the single stage of *in vitro* expansion.

267. The method according to any one of claims 265-266, comprising contacting the TILs with the CD28 agonist and the T cell growth factors during the single stage of *in vitro* expansion.

268. The method according to any one of claims 265-267, wherein the method comprises subjecting the TILs derived from tumor tissues and not expanded *in vitro* to the first stage of *in vitro* expansion and the second stage of *in vitro* expansion, and during the second stage of *in vitro* expansion, contacting the TILs with the T cell growth factors.

269. The method according to any one of claims 265-268, comprising contacting the TILs with the CD28 agonist and the T cell growth factors substantially simultaneously.

270. The method according to any one of claims 265-269, wherein the T cell growth factors are one or more factors selected from the group consisting of: IL-2, IL-7, IL-12, IL-15, IL-21, interferon $\gamma$, and functionally active fragments thereof.

271. The method according to any one of claims 265-270, wherein the T cell growth factors comprise IL-2 and/or a functionally active fragment thereof.

272. The method according to any one of claims 265-271, wherein the step of contacting the TILs with the T cell growth factors comprises adding the T cell growth factors into the cell culture medium of the TILs.

273. The method according to any one of claims 265-272, wherein the initial concentration of each of the T cell growth factors in the cell culture medium of the TILs is each independently at least about 300 IU/mL.

274. The method according to any one of claims 184-273, wherein the TILs are TILs derived from tumor tissue debris and/or TILs resuscitated after cryopreservation.

275. The method according to claim 274, wherein the debris has a volume of about 1 mm$^3$ to about 27 mm$^3$.

276. A method for culturing tumor-infiltrating lymphocytes (TILs), comprising:

(A) contacting a first TIL population derived from tumor tissues and not expanded *in vitro* with T cell growth factors, wherein a second TIL population is obtained via the step (A);
(B) improving the expression and/or enhancing the activity of at least one cytokine of the second TIL population, and contacting the TILs with a CD28 agonist, wherein a third TIL population is obtained via the step (B).

277. A method for culturing tumor-infiltrating lymphocytes (TILs), comprising:

(A) resuscitating and/or continuing culturing an *in vitro* TIL population to obtain a second TIL population, wherein the *in vitro* TIL population comprises a TIL population obtained by *in vitro* expansion of a first TIL population derived from tumor tissues and not expanded *in vitro*;
(B) improving the expression and/or enhancing the activity of at least one cytokine of the second TIL population, and contacting the TILs with a CD28 agonist, wherein a third TIL population is obtained via the step (B).

278. The method according to claim 277, wherein the *in vitro* TIL population comprises a TIL population obtained by contacting the first TIL population with T cell growth factors.

279. The method according to any one of claims 277-278, wherein the *in vitro* TIL population comprises a TIL population obtained by cryopreserving the first TIL population.

280. The method according to any one of claims 276-279, wherein the step (A) is carried out for about 7 days to about 14 days.

281. The method according to any one of claims 276-280, wherein the step (B) is carried out for about 7 days to about 14 days.

282. The method according to any one of claims 276-281, wherein TILs obtained by improving the expression and/or enhancing the activity of at least one cytokine of the TILs show improved TIL properties, compared to TILs with unchanged expression and/or activity of the cytokine.

283. The method according to claim 282, wherein the improved TIL properties comprise one or more properties selected from the group consisting of: increased number of TIL cells, increased proportion of viable cells, enhanced persistence, improved proportion of T cell subpopulations, enhanced cytokine secretion capacity, enhanced tumor cell killing ability, enhanced T cell receptor (TCR) clonal diversity, and increased number of TIL cells in tissues.

284. The method according to claim 283, wherein the improved proportion of T cell subpopulations comprises one or more subpopulations selected from the group consisting of: increased proportion of central memory T cells, decreased proportion of regulatory T cells, increased proportion of activated T cells, increased proportion of tumor-specific T cells, and increased proportion of stem cell-like T cells.

285. The method according to any one of claims 276-284, wherein the TILs that have been contacted with the CD28 agonist during at least one stage of *in vitro* expansion show an improved gene editing effect, compared to corresponding TILs that have not been contacted with the CD28 agonist during the stage of *in vitro* expansion.

286. The method according to claim 285, wherein the improved gene editing effect comprises enhanced gene knockout efficiency.

287. The method according to any one of claims 276-286, wherein the step of improving the expression and/or enhancing the activity of at least one cytokine of the TILs comprises introducing a nucleic acid encoding the cytokine into the TILs.

288. The method according to any one of claims 276-287, wherein the step of improving the expression and/or enhancing the activity of at least one cytokine of the TILs comprises introducing a vector comprising the nucleic acid into the TILs.

289. The method according to any one of claims 287-288, wherein integrating a nucleic acid encoding the cytokine into the genome of the TILs.

290. The method according to any one of claims 288-289, wherein the vector comprises a viral vector.

291. The method according to claim 290, wherein the viral vector comprises a retroviral vector.

292. The method according to claim 291, wherein the retroviral vector comprises a lentiviral vector.

293. The method according to any one of claims 276-292, wherein the cytokine comprises interleukin (IL).

294. The method according to any one of claims 276-293, wherein the cytokine comprises interleukin-15 (IL-15) and/or a functionally active fragment thereof.

295. The method according to claim 294, wherein the IL-15 comprises membrane-anchored IL-15 and/or secreted IL-15.

296. The method according to any one of claims 294-295, wherein the IL-15 comprises an IL-15 domain.

297. The method according to claim 296, wherein the IL-15 domain comprises an amino acid sequence as shown in SEQ ID NO: 3.

298. The method according to any one of claims 294-297, wherein the IL-15 comprises an IL-15Ra extracellular domain.

299. The method according to claim 298, wherein the IL-15Ra extracellular domain comprises an amino acid sequence as shown in SEQ ID NO: 16.

300. The method according to any one of claims 298-299, wherein the IL-15 domain is directly or indirectly linked to the IL-15Ra extracellular domain.

**301.** The method according to claim 300, wherein the indirect linkage comprises linking through a linker.

**302.** The method according to claim 301, wherein the linker comprises an amino acid sequence selected from the group consisting of: SEQ ID NOs: 4-10, (SEQ ID NO: 11)$_l$, (SEQ ID NO: 12)$_m$, (SEQ ID NO: 13)$_n$, (SEQ ID NO: 14)$_p$, and (SEQ ID NO: 15)$_q$, and any combination of the above, wherein l, m, n, p, and q are each independently at least 1.

**303.** The method according to any one of claims 294-302, wherein the IL-15 comprises a transmembrane domain.

**304.** The method according to claim 303, wherein the transmembrane domain comprises an amino acid sequence as shown in any one of SEQ ID NOs: 17-22.

**305.** The method according to any one of claims 303-304, wherein the transmembrane domain is directly or indirectly linked to the IL-15Ra extracellular domain.

**306.** The method according to any one of claims 294-305, wherein the IL-15 comprises an intracellular domain.

**307.** The method according to claim 306, wherein the intracellular domain comprises an amino acid sequence as shown in any one of SEQ ID NOs: 23-26.

**308.** The method according to any one of claims 306-307, wherein the intracellular domain is directly or indirectly linked to the transmembrane domain.

**309.** The method according to any one of claims 294-308, wherein the IL-15 comprises a signal peptide domain.

**310.** The method according to claim 309, wherein the signal peptide domain comprises an amino acid sequence as shown in SEQ ID NO: 2.

**311.** The method according to any one of claims 309-310, wherein the signal peptide domain is directly or indirectly linked to the IL-15 domain.

**312.** The method according to any one of claims 294-311, wherein the functionally active fragment of the IL-15 comprises an amino acid sequence as shown in SEQ ID NO: 3.

**313.** The method according to any one of claims 294-312, wherein the IL-15 comprises an amino acid sequence as shown in SEQ ID NO: 1.

**314.** The method according to any one of claims 276-313, wherein the enhanced expression of the cytokine comprises enhanced synthesis and/or secretion of the cytokine.

**315.** The method according to any one of claims 276-314, wherein the proportion of cells expressing the cytokine in the TILs obtained by improving the expression and/or enhancing the activity of at least one cytokine of the TILs is increased, compared to TILs with unchanged expression and/or activity of the cytokine.

**316.** The method according to any one of claims 276-315, wherein the proportion of cells expressing the cytokine in the TILs obtained by improving the expression and/or enhancing the activity of at least one cytokine of the TILs is increased by at least about 5% or above, compared to TILs with unchanged expression and/or activity of the cytokine.

**317.** The method according to any one of claims 276-316, wherein in the TILs obtained by improving the expression and/or enhancing the activity of at least one cytokine of the TILs, the proportion of cells expressing the cytokine is at least about 5% or above.

**318.** The method according to any one of claims 276-317, wherein the CD28 agonist comprises an anti-CD28 antibody and/or an antigen-binding fragment thereof, CD80 and/or a functionally active fragment thereof, and/or CD86 and/or a functionally active fragment thereof.

**319.** The method according to any one of claims 276-318, comprising contacting the TILs with the CD28 agonist and the other T cell activators substantially simultaneously.

320. The method according to claim 319, wherein the other T cell activators comprise agonists of one or more targets selected from the group consisting of: CD3, HVEM, CD40L, OX40, and 4-1BB.

321. The method according to any one of claims 319-320, wherein the other T cell activators comprise a CD3 agonist.

322. The method according to any one of claims 319-321, wherein the other T cell activators comprise an anti-CD3 antibody and/or an antigen-binding fragment thereof.

323. The method according to any one of claims 319-322, wherein the step of contacting the TILs with the CD28 agonist and the other T cell activators comprises one or more ways selected from the group consisting of: (1) adding the CD28 agonist and the other T cell activators into the cell culture medium of the TILs; (2) adding engineered cells expressing the CD28 agonist and the other T cell activators into the cell culture medium of the TILs; (3) adding a solid medium comprising the CD28 agonist and the other T cell activators into the cell culture medium of the TILs.

324. The method according to claim 323, wherein the initial concentration of the other T cell activators in the cell culture medium of the TILs is at least about 30 ng/mL.

325. The method according to any one of claims 323-324, wherein the initial concentration of the other T cell activators in the cell culture medium of the TILs is about 30 ng/mL to about 300 ng/mL.

326. The method according to any one of claims 323-325, wherein the diameter of the solid medium is about 500 nm to about 10 $\mu$m.

327. The method according to any one of claims 323-326, wherein the diameter of the solid medium is about 1 nm to about 500 nm.

328. The method according to any one of claims 326-327, wherein the diameter of the solid medium is measured by transmission electron microscopy.

329. The method according to any one of claims 323-328, wherein the solid medium comprises a polymer.

330. The method according to any one of claims 323-329, wherein each mg of the solid medium comprises at least about 25 $\mu$g of the CD28 agonist and the other T cell activators.

331. The method according to any one of claims 323-330, comprising adding the solid medium comprising the CD28 agonist and the other T cell activators into the cell culture medium of the TILs at a proportion of the solid medium to the TILs from about 2:1 to about 1:2.

332. The method according to any one of claims 323-331, comprising adding the solid medium comprising the CD28 agonist and the other T cell activators into the cell culture medium of the TILs at a proportion of the solid medium to the TILs from about 1:100 to about 1:2000.

333. The method according to any one of claims 276-332, comprising co-culturing the TILs with the feeder cells after contacting the TILs with the CD28 agonist for at least about 2 hours.

334. The method according to claim 333, comprising co-culturing the TILs with the feeder cells after contacting the TILs with the CD28 agonist for about 6 hours to about 72 hours.

335. The method according to any one of claims 333-334, comprising co-culturing the TILs with the feeder cells after contacting the TILs with the CD28 agonist for about 12 hours to about 48 hours.

336. The method according to any one of claims 333-334, comprising co-culturing the TILs with the feeder cells after contacting the TILs with the CD28 agonist for about 6 hours, about 12 hours, about 24 hours, about 48 hours, or about 72 hours.

337. The method according to any one of claims 333-336, wherein the feeder cells comprise antigen-presenting cells.

338. The method according to any one of claims 333-337, wherein the feeder cells comprise one or more cells selected from the group consisting of: peripheral mononuclear cells, dendritic cells, and artificial antigen-presenting cells.

339. The method according to any one of claims 333-338, wherein the feeder cells are peripheral mononuclear cells.

340. The method according to any one of claims 333-339, wherein the feeder cells are irradiated feeder cells.

341. The method according to any one of claims 333-340, wherein the step of co-culturing the TILs with the feeder cells comprises contacting the surface of the feeder cells with the surface of the TILs.

342. The method according to any one of claims 333-341, wherein the step of co-culturing the TILs with the feeder cells comprises adding the feeder cells into the cell culture medium of the TILs.

343. The method according to any one of claims 333-342, wherein the method comprises adding the feeder cells into the cell culture medium of the TILs at a proportion of the feeder cells to the TILs from about 40:1 to about 400:1.

344. The method according to any one of claims 276-343, comprising contacting the TILs with the CD28 agonist and the T cell growth factors substantially simultaneously.

345. The method according to any one of claims 276-344, wherein the T cell growth factors are one or more factors selected from the group consisting of: IL-2, IL-7, IL-12, IL-15, IL-21, interferon $\gamma$, and functionally active fragments thereof.

346. The method according to any one of claims 276-345, wherein the T cell growth factors comprise IL-2 and/or a functionally active fragment thereof.

347. The method according to any one of claims 276-346, wherein the step of contacting the TILs with the T cell growth factors comprises adding the T cell growth factors into the cell culture medium of the TILs.

348. The method according to any one of claims 276-347, wherein the initial concentration of each of the T cell growth factors in the cell culture medium of the TILs is each independently at least about 300 IU/mL.

349. The method according to any one of claims 276-348, wherein the TILs are TILs derived from tumor tissue debris and/or TILs resuscitated after cryopreservation.

350. The method according to claim 349, wherein the debris has a volume of about 1 mm$^3$ to about 27 mm$^3$.

351. A tumor-infiltrating lymphocyte (TIL) obtainable by the method according to any one of claims 1-350.

352. A composition comprising the TIL as defined in claim 351.

353. A pharmaceutical composition, comprising the TIL as defined in claim 351 and/or the composition as defined in claim 352, and optionally a pharmaceutically acceptable carrier.

354. A method for affecting the tumor cell growth, comprising administering to a subject the TIL as defined in claim 351, the composition as defined in claim 352 and/or the pharmaceutical composition as defined in claim 353.

355. A use of the TIL as defined in claim 351, the composition as defined in claim 352 and/or the pharmaceutical composition as defined in claim 353 for the manufacture of drugs for preventing and/or treating a tumor.

356. The use according to claim 355, wherein the tumor is a solid tumor.

357. The use according to any one of claims 355-356, wherein the tumor is one or more tumors selected from the group consisting of: melanoma, ovarian cancer, cervical cancer, lung cancer, bladder cancer, breast cancer, head and neck cancer, pancreatic cancer, liver cancer, gastric cancer, colorectal cancer, and kidney cancer.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

FIG. 22

FIG. 23

FIG. 24

FIG. 25

FIG. 26

FIG. 27

FIG. 28

FIG. 29

Donor I-B-3

Donor I-B-4

Donor I-B-5

Donor I-B-3

Donor I-B-4

Donor I-B-5

FIG. 30

Donor I-C-1

Donor I-C-2

FIG. 31

EP 4 328 300 A1

FIG. 32

FIG. 33

FIG. 34

FIG. 35

## Donor II-A

FIG. 36

FIG. 37

## Donor II-B-2

☐ Control group

▨ Mixed antibody group

## Donor II-B-2

☐ Control group

▨ Mixed antibody group

## Donor II-B-2

☐ Control group

▨ Mixed antibody group

FIG. 38

FIG. 39

FIG. 40

FIG. 41

FIG. 42

## Donor II-F

FIG. 43

## Donor II-G-1

FIG. 44

## Donor II-G-2

FIG. 45

## Donor II-G-3

FIG. 46

Donor II-G-4

FIG. 47

FIG. 48

FIG. 49

FIG. 50

## Donor II-J-1

FIG. 51

## Donor II-J-2

FIG. 52

## Donor II-K-1

FIG. 53

## Donor II-K-2

FIG. 54

## Donor II-K-3

Group without addition of CD28 agonist
Group with addition of CD28 agonist
Blank group

FIG. 55

## Donor 1

TILs transduced with IL-15
Ctrl

FIG. 56

## Donor 2

FIG. 57

## Donor 3

FIG. 58

Tumor Volume

FIG. 59

IFN-γ in serum

FIG. 60

FIG. 61A

FIG. 61B

**CD8+TCF1+**

FIG. 61C

**Treg**

FIG. 61D

FIG. 61E

CD8+CD103+CD39+

CD4+CD103+CD39+

FIG. 61F

FIG. 61G

CD8+41BB+

CD4+41BB+

FIG. 61H

## CD8+CD25+

## CD4+CD25+

FIG. 61I

FIG. 61J

FIG. 61J (cont.)

FIG. 61K

FIG. 61L

CD8+

FIG. 61M

## Tcm

FIG. 61N

## NKT

FIG. 61O

FIG. 61P

FIG. 61Q

### INTERNATIONAL SEARCH REPORT

| International application No. |
| --- |
| **PCT/CN2022/088338** |

| A. CLASSIFICATION OF SUBJECT MATTER |
| --- |
| C12N 5/0783(2010.01)i; A61K 35/17(2015.01)i; A61K 38/20(2006.01)i |
| According to International Patent Classification (IPC) or to both national classification and IPC |

| B. FIELDS SEARCHED |
| --- |
| Minimum documentation searched (classification system followed by classification symbols) |
| C12N; A61K |
| Documentation searched other than minimum documentation to the extent that such documents are included in the fields searched |
| |
| Electronic data base consulted during the international search (name of data base and, where practicable, search terms used) |
| CNTXT; CNABS; WPABSC; ENTXTC; VCN; DWPI; VEN; Genbank; CNKI; 万方, WANFANG; 中国专利生物序列检索系统, China Patents Biological Sequence Search System: 申请人/发明人, 肿瘤浸润淋巴细胞, 细胞因子, 白细胞介素, 白介素, 转化, 饲养细胞, 抗原递呈细胞, 培养, 颗粒, TIL, tumor infiltrating lymphotcyte, pellet, cytokine, IL-2, IL-15, transfect+, feed, PMBC, APC, CD28, culture, SEQ ID NOs: 1-3 |

| C. DOCUMENTS CONSIDERED TO BE RELEVANT | | |
| --- | --- | --- |
| Category* | Citation of document, with indication, where appropriate, of the relevant passages | Relevant to claim No. |
| PY | WO 2021173964 A1 (KSQ THERAPEUTICS, INC.) 02 September 2021 (2021-09-02) claims 1-11 and 21-28 | 66-67, 71-93, 160-162, 165-357 |
| Y | 王延春 (Wang, Yanchun). "携带IL-15的T细胞对肝癌细胞的体外杀伤研究 (Cytotoxicity Effect of T Cells Harbouring IL-15on Hepatocellular Carcinoma Cell Line In Vitro)" 中国优秀硕士学位论文全文数据库 (China Master's Theses Full-text Database), No. 10, 31 October 2012 (2012-10-31), text, pp. 12-20, 35-41, and 44-56 | 1-357 |
| Y | WO 2020131547 A1 (IOVANCE BIOTHERAPEUTICS, INC.) 25 June 2020 (2020-06-25) claims 2-3, 129, and 149-193, and description, paragraphs 251, 496-510, 537-551, 848-849, and 1775, and embodiments 1-2 | 1-357 |
| Y | WO 2019136459 A1 (IOVANCE BIOTHERAPEUTICS, INC.) 11 July 2019 (2019-07-11) claims 3-42 and 51-52, and description, paragraphs 54, 95, and 117-122, and embodiments 9 and 14-15 | 1-183, 250-357 |
| Y | US 2020164090 A1 (THE BOARD OF TRUSTEES OF THE LELAND STANFORD JUNIOR UNIVERSITY) 28 May 2020 (2020-05-28) claims 1 and 28, and description, paragraphs 7, 50, and 66 | 66-67, 71-93, 160-162, 165-357 |

| ✔ Further documents are listed in the continuation of Box C. | ✔ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **04 July 2022** | **22 July 2022** |

| Name and mailing address of the ISA/CN | Authorized officer |
| --- | --- |
| **China National Intellectual Property Administration (ISA/CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2022/088338** |

### C. DOCUMENTS CONSIDERED TO BE RELEVANT

| Category* | Citation of document, with indication, where appropriate, of the relevant passages | Relevant to claim No. |
| --- | --- | --- |
| Y | CN 106574244 A (POLYBIOCEPT AB) 19 April 2017 (2017-04-19)<br>claims 12-20 | 1-183, 250-357 |
| Y | WO 2020096927 A1 (IOVANCE BIOTHERAPEUTICS, INC.) 14 May 2020 (2020-05-14)<br>claims 21 and 27, and description, paragraphs 4-5 | 1-183, 250-357 |
| A | WO 2020232029 A1 (IOVANCE BIOTHERAPEUTICS, INC.) 19 November 2020<br>(2020-11-19)<br>description, embodiment 5 | 1-357 |
| A | 涂海旋 (TU, Haixuan). "癌性血性胸水TIL细胞体外培养分析 (Analysis of Cancerous<br>Bloody Pleural Effusion TIL Cells In Vitro Culture)"<br>智慧健康 *(Smart Healthcare),* Vol. 6, No. 18, 30 June 2020 (2020-06-30),<br>pp. 38-39 | 1-357 |

Form PCT/ISA/210 (second sheet) (January 2015)

**INTERNATIONAL SEARCH REPORT**

| International application No. |
|---|
| **PCT/CN2022/088338** |

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed:

      ☑ in the form of an Annex C/ST.25 text file.

      ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

      ☐ in the form of an Annex C/ST.25 text file (Rule 13*ter*.1(a)).

      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

## INTERNATIONAL SEARCH REPORT

International application No.

**PCT/CN2022/088338**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **354**
   because they relate to subject matter not required to be searched by this Authority, namely:

   [1]  Claim 354 relates to a method for affecting the growth of a tumor cell in a subject, which belongs to a method for treating a disease as defined in PCT Rule 39.1(iv). The present report was formed on the basis of a use of TILs, a composition, and a pharmaceutical composition thereof in preparation of a drug.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

| International application No. |
|---|
| **PCT/CN2022/088338** |

| Patent document cited in search report | | | Publication date (day/month/year) | Patent family member(s) | | | Publication date (day/month/year) |
|---|---|---|---|---|---|---|---|
| WO | 2021173964 | A1 | 02 September 2021 | None | | | |
| WO | 2020131547 | A1 | 25 June 2020 | EP | 3898949 | A1 | 27 October 2021 |
| | | | | CA | 3123392 | A1 | 25 June 2020 |
| | | | | JP | 2022514023 | A | 09 February 2022 |
| WO | 2019136459 | A1 | 11 July 2019 | None | | | |
| US | 2020164090 | A1 | 28 May 2020 | WO | 2019023622 | A1 | 31 January 2019 |
| CN | 106574244 | A | 19 April 2017 | IL | 249409 | D0 | 28 February 2017 |
| | | | | SG | 11201610350 S | A | 27 January 2017 |
| | | | | CA | 2951749 | A1 | 17 December 2015 |
| | | | | MX | 2016016251 | A | 11 July 2017 |
| | | | | ZA | 201700211 | B | 25 July 2018 |
| | | | | EP | 3838288 | A1 | 23 June 2021 |
| | | | | ES | 2855475 | T3 | 23 September 2021 |
| | | | | US | 2017107490 | A1 | 20 April 2017 |
| | | | | EP | 3154567 | A1 | 19 April 2017 |
| | | | | BR | 112016028996 | A2 | 30 January 2018 |
| | | | | RU | 2016151694 | A | 13 July 2018 |
| | | | | WO | 2015189356 | A1 | 17 December 2015 |
| | | | | WO | 2015189357 | A1 | 17 December 2015 |
| | | | | JP | 2017525754 | A | 07 September 2017 |
| | | | | UA | 123821 | C2 | 09 June 2021 |
| | | | | JP | 2021113200 | A | 05 August 2021 |
| | | | | KR | 20170031139 | A | 20 March 2017 |
| | | | | AU | 2015273501 | A1 | 12 January 2017 |
| | | | | PT | 3154567 | T | 03 March 2021 |
| WO | 2020096927 | A1 | 14 May 2020 | IL | 282774 | D0 | 30 June 2021 |
| | | | | US | 2022033775 | A1 | 03 February 2022 |
| | | | | JP | 2022506508 | A | 17 January 2022 |
| | | | | EP | 3877511 | A1 | 15 September 2021 |
| | | | | CA | 3118493 | A1 | 14 May 2020 |
| WO | 2020232029 | A1 | 19 November 2020 | None | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **TAY, R. E. ; RICHARDSON, E. K.** *Cancer Gene Therapy,* 2020, 1-13

- *Biochem. Pharmacol.,* 1973, vol. 22, 3099